# EUROPEAN PATENT SPECIFICATION

(11) **EP 1 987 360 B1**
(45) Date of publication and mention of the grant of the patent: **07.03.2012**
(21) Application number: 07717460.5
(22) Date of filing: 29.01.2007
(51) Int. Cl.: G01N 33/574

(54) **METHODS FOR IDENTIFYING PATIENTS WITH AN INCREASED LIKELIHOOD OF HAVING OVARIAN CANCER AND COMPOSITIONS THEREFOR**
VERFAHREN ZUR IDENTIFIZIERUNG VON PATIENTEN MIT ERHÖHTER WAHRSCHEINLICHKEIT DES AUFTRETENS EINES OVARIALKARZINOMS UND ZUSAMMENSETZUNGEN DAFÜR
MÉTHODES PERMETTANT D'IDENTIFIER DES PATIENTES PRÉSENTANT UN RISQUE ACCRU D'ÊTRE ATTEINTES D'UN CANCER DE L'OVAIRE ET COMPOSITIONS ASSOCIÉES

(30) Priority: 27.01.2006 US 762760 P
(43) Date of publication of application: 05.11.2008
(60) Divisional application: 11159423.0
(73) Proprietor: Tripath Imaging, Inc., Burlington, NC 27215 (US)
(72) Inventor: FISCHER, Timothy, J., Raleigh, NC 27613 (US); MALINOWSKI, Douglas, P., Hillsborough, NC 27278 (US); HE, Qin, Raleigh, NC 27613 (US); WHITEHEAD, Clark, M., Apex, NC 27502 (US); CHEEK, Robert, L., Mebane, NC 27302 (US); GROELKE, John, W., Raleigh, NC 27613 (US)
(74) Representative: Bentham, Andrew
(86) International application number: PCT/US2007/061205
(87) International publication number: WO 2007/090076

(56) References cited:
- WO-A-2005/016126
- WO-A-2005/083440
- BAST R C ET AL: "Early detection of ovarin cancer: promise and reality" CANCER TREATMENT AND RESEARCH, NIJHOFF, DORDRECHT, NL, vol. 107, 2002, pages 61-97, XP008082826 ISSN: 0927-3042
- ROSEN ET AL: "Potential markers that complement expression of CA125 in epithelial ovarian cancer" GYNECOLOGIC ONCOLOGY, ACADEMIC PRESS, LONDON, GB, vol. 99, no. 2, November 2005 (2005-11), pages 267-277, XP005118976 ISSN: 0090-8258
- LU K H ET AL: "Selection of potential markers for epithelial ovarian cancer with gene expression arrays and recursive descent partition analysis" CLINICAL CANCER RESEARCH, THE AMERICAN ASSOCIATION FOR CANCER RESEARCH, US, vol. 10, 15 May 2004 (2004-05-15), pages 3291-3300, XP003011115 ISSN: 1078-0432
- KOEBL H ET AL: "A COMPARATIVE STUDY OF IMMUNOSUPPRESSIVE ACIDIC PROTEIN (IAP), CA 125 AND ACUTE-PHASE PROTEINS AS PARAMETERS FOR OVARIAN CANCER MONITORING" NEOPLASMA, VEDA, PUBLISHING HOUSE OF SLOVAK ACADEMY OF, CS, vol. 35, no. 2, 1988, pages 215-220, XP009011458 ISSN: 0028-2685
- BAST ROBERT C JR: "Status of tumor markers in ovarian cancer screening." JOURNAL OF CLINICAL ONCOLOGY : OFFICIAL JOURNAL OF THE AMERICAN SOCIETY OF CLINICAL ONCOLOGY 15 MAY 2003, vol. 21, no. 10 Suppl, 15 May 2003 (2003-05-15), pages 200s-205s, XP002448459 ISSN: 1527-7755 cited in the application
- BAST R C JR ET AL: "New tumor markers: CA125 and beyond." INTERNATIONAL JOURNAL OF GYNECOLOGICAL CANCER : OFFICIAL JOURNAL OF THE INTERNATIONAL GYNECOLOGICAL CANCER SOCIETY 2005 NOV-DEC, vol. 15 Suppl 3, November 2005 (2005-11), pages 274-281, XP002448848 ISSN: 1048-891X
- CHECK ROBERT L ET AL: "HE4 and Glycodelin are serum biomarkers for Ovarian Cancer." PROCEEDINGS OF THE AMERICAN ASSOCIATION FOR CANCER RESEARCH ANNUAL MEETING, vol. 47, April 2006 (2006-04), page 1057, XP001248684 & 97TH ANNUAL MEETING OF THE AMERICAN-ASSOCIATION-FOR-CANCER-RESEARCH (AACR); WASHINGTON, DC, USA; APRIL 01 -05, 2006 ISSN: 0197-016X

### SEQUENCE LISTING

<110> Fischer, Timothy J.
   Malinowski, Douglas P.
   He, Qin
   Whitehead, Clark M.
   Cheek, Robert L.
   Groelke, John W.
<120> METHODS FOR IDENTIFYING PATIENTS WITH AN INCREASED LIKELIHOOD OF HAVING OVARIAN CANCER AND COMPOSITIONS THERFOR
<130> 046143/322877
<150> 60/762,760
   <151> 2006-01-27
<160> 64
<170> FastSEQ for Windows Version 4.0
<210> 1
   <211> 124
   <212> PRT
   <213> Homo sapiens
<400> 1
   Met Pro Ala Cys Arg Leu Gly Pro Leu Ala Ala Ala Leu Leu Leu Ser
   1               5                   10                  15
   Leu Leu Leu Phe Gly Phe Thr Leu Val Ser Gly Thr Gly Ala Glu Lys
               20                  25                  30
   Thr Gly Val Cys Pro Glu Leu Gln Ala Asp Gln Asn Cys Thr Gln Glu
           35                  40                  45
   Cys Val Ser Asp Ser Glu Cys Ala Asp Asn Leu Lys Cys Cys Ser Ala
       50                  55                  60
   Gly Cys Ala Thr Phe Cys Ser Leu Pro Asn Asp Lys Glu Gly Ser Cys
   65                  70                  75                  80
   Pro Gln Val Asn Ile Asn Phe Pro Gln Leu Gly Leu Cys Arg Asp Gln
                   85                  90                  95
   Cys Gln Val Asp Ser Gln Cys Pro Gly Gln Met Lys Cys Cys Arg Asn
               100                 105                 110
   Gly Cys Gly Lys Val Ser Cys Val Thr Pro Asn Phe
           115                 120
<210> 2
   <211> 570
   <212> DNA
   <213> Homo sapiens
<400> 2
   cacctgcacc ccgcccgggc atagcaccat gcctgcttgt cgcctaggcc cgctagccgc 60
   cgccctcctc ctcagcctgc tgctgttcgg cttcacccta gtctcaggca caggagcaga 120
   gaagactggc gtgtgccccg agctccaggc tgaccagaac tgcacgcaag agtgcgtctc 180
   ggacagcgaa tgcgccgaca acctcaagtg ctgcagcgcg ggctgtgcca ccttctgctc 240
   tctgcccaat gataaggagg gttcctgccc ccaggtgaac attaactttc cccagctcgg 300
   cctctgtcgg gaccagtgcc aggtggacag ccagtgtcct ggccagatga aatgctgccg 360
   caatggctgt gggaaggtgt cctgtgtcac tcccaatttc tgagctccag ccaccaccag 420
   gctgagcagt gaggagagaa agtttctgcc tggccctgca tctggttcca gcccacctgc 480
   cctccccttt ttcgggactc tgtattccct cttgggctga ccacagcttc tccctttccc 540
   aaccaataaa gtaaccactt tcagcaaaaa                                   570
<210> 3
   <211> 79
   <212> PRT
   <213> Homo sapiens
<400> 3
   Met Pro Ala Cys Arg Leu Gly Pro Leu Ala Ala Ala Leu Leu Leu Ser
   1               5                   10                  15
   Leu Leu Leu Phe Gly Phe Thr Leu Val Ser Gly Thr Gly Ala Glu Lys
               20                  25                  30
   Thr Gly Val Cys Pro Glu Leu Gln Ala Asp Gln Asn Cys Thr Gln Glu
           35                  40                  45
   Cys Val Ser Asp Ser Glu Cys Ala Asp Asn Leu Lys Cys Cys Ser Ala
       50                  55                  60
   Gly Cys Ala Thr Phe Cys Ser Leu Pro Asn Gly Gln Leu Ala Glu
   65                  70                  75
<210> 4
   <211> 694
   <212> DNA
   <213> Homo sapiens
<400> 4
   cacctgcacc ccgcccgggc atagcaccat gcctgcttgt cgcctaggcc cgctagccgc 60
   cgccctcctc ctcagcctgc tgctgttcgg cttcacccta gtctcaggca caggagcaga 120
   gaagactggc gtgtgccccg agctccaggc tgaccagaac tgcacgcaag agtgcgtctc 180
   ggacagcgaa tgcgccgaca acctcaagtg ctgcagcgcg ggctgtgcca ccttctgctc 240
   tctgcccaat ggccaactgg ctgagtgatt cgaagaaagt gaggaatcct ccctggacac 300
   tgtatcgccc ttcgtcgtct ttcagtcaat ctcttccact ctaaggattg agtgagcgcg 360
   agctggggac tctctcaaag ataaggaggg ttcctgcccc caggtgaaca ttaactttcc 420
   ccagctcggc ctctgtcggg accagtgcca ggtggacagc cagtgtcctg gccagatgaa 480
   atgctgccgc aatggctgtg ggaaggtgtc ctgtgtcact cccaatttct gaggtccagc 540
   caccaccagg ctgagcagtg aggagagaaa gtttctgcct ggccctgcat ctggttccag 600
   cccacctgcc ctcccctttt tcgggactct gtattccctc ttgggctgac cacagcttct 660
   ccctttccca accaataaag taaccacttt cagc                            694
<210> 5
   <211> 76
   <212> PRT
   <213> Homo sapiens
<400> 5
   Met Pro Ala Cys Arg Leu Gly Pro Leu Ala Ala Ala Leu Leu Leu Ser
    1               5                  10                  15
   Leu Leu Leu Phe Gly Phe Thr Leu Val Ser Asp Lys Glu Gly Ser Cys
               20                  25                  30
   Pro Gln Val Asn Ile Asn Phe Pro Gln Leu Gly Leu Cys Arg Asp Gln
           35                  40                  45
   Cys Gln Val Asp Ser Gln Cys Pro Gly Gln Met Lys Cys Cys Arg Asn
       50                  55                  60
   Gly Cys Gly Lys Val Ser Cys Val Thr Pro Asn Phe
   65                  70                  75
<210> 6
   <211> 421
   <212> DNA
   <213> Homo sapiens
<400> 6
   cacctgcacc ccgcccgggc atagcaccat gcctgcttgt cgcctaggcc cgctagccgc 60
   cgccctcctc ctcagcctgc tgctgttcgg cttcacccta gtctcagata aggagggttc 120
   ctgcccccag gtgaacatta actttcccca gctcggcctc tgtcgggacc agtgccaggt 180
   ggacagccag tgtcctggcc agatgaaatg ctgccgcaat ggctgtggga aggtgtcctg 240
   tgtcactccc aatttctgag gtccagccac caccaggctg agcagtgagg agagaaagtt 300
   tctgcctggc cctgcatctg gttccagccc acctgccctc ccctttttcg ggactctgta 360
   ttccctcttg ggctgaccac agcttctccc tttcccaacc aataaagtaa ccactttcag 420
   c                                                                 421
<210> 7
   <211> 102
   <212> PRT
   <213> Homo sapiens
<400> 7
   Met Pro Ala Cys Arg Leu Gly Pro Leu Ala Ala Ala Leu Leu Leu Ser
    1              5                   10                  15
   Leu Leu Leu Phe Gly Phe Thr Leu Val Ser Gly Thr Gly Ala Glu Lys
               20                  25                  30
   Thr Gly Val Cys Pro Glu Leu Gln Ala Asp Gln Asn Cys Thr Gln Glu
           35                  40                  45
   Cys Val Ser Asp Ser Glu Cys Ala Asp Asn Leu Lys Cys Cys Ser Ala
       50                  55                  60
   Gly Cys Ala Thr Phe Cys Ser Leu Pro Asn Ala Leu Phe His Trp His
   65                  70                  75                  80
   Leu Lys Thr Arg Arg Leu Trp Glu Ile Ser Gly Pro Arg Pro Arg Arg
                   85                  90                  95
   Pro Thr Trp Asp Ser Ser
               100
<210> 8
   <211> 358
   <212> DNA
   <213> Homo sapiens
<400> 8
   cacctgcacc ccgcccgggc atagcaccat gcctgcttgt cgcctaggcc cgctagccgc 60
   cgccctcctc ctcagcctgc tgctgttcgg cttcacccta gtctcaggca caggagcaga 120
   gaagactggc gtgtgccccg agctccaggc tgaccagaac tgcacgcaag agtgcgtctc 180
   ggacagcgaa tgcgccgaca acctcaagtg ctgcagcgcg ggctgtgcca ccttctgctc 240
   tctgcccaat gcactgttcc actggcacct aaagacacgg aggctctggg agatttctgg 300
   ccctaggcca cgaaggccca cttgggactc aagctgaggt cctgtgattc catttggg   358
<210> 9
   <211> 73
   <212> PRT
   <213> Homo sapiens
<400> 9
   Met Leu Gln Val Gln Val Asn Leu Pro Val Ser Pro Leu Pro Thr Tyr
    1              5                   10                  15
   Pro Tyr Ser Phe Phe Tyr Pro Asp Lys Glu Gly Ser Cys Pro Gln Val
               20                  25                  30
   Asn Ile Asn Phe Pro Gln Leu Gly Leu Cys Arg Asp Gln Cys Gln Val
           35                  40                  45
   Asp Ser Gln Cys Pro Gly Gln Met Lys Cys Cys Arg Asn Gly Cys Gly
       50                  55                  60
   Lys Val Ser Cys Val Thr Pro Asn Phe
   65                  70
<210> 10
   <211> 411
   <212> DNA
   <213> Homo sapiens
<400> 10
   agcccagtga ggggcagtgg gggggccatg ctgcaggtac aagttaatct ccctgtatcg 60
   cctctgccca cttaccctta ctcctttttc tacccagata aggagggttc ctgcccccag 120
   gtgaacatta actttcccca gctcggcctc tgtcgggacc agtgccaggt ggacagccag 180
   tgtcctggcc agatgaaatg ctgccgcaat ggctgtggga aggtgtcctg tgtcactccc 240
   aatttctgag gtccagccac caccaggctg agcagtgagg agagaaagtt tctgcctggc 300
   cctgcatctg gttccagccc acctgccctc ccctttttcg ggactctgta ttccctcttg 360
   ggctgaccac agcttctccc tttcccaacc aataaagtaa ccactttcag c          411
<210> 11
   <211> 244
   <212> PRT
   <213> Homo sapiens
<400> 11
   Met Lys Lys Leu Met Val Val Leu Ser Leu Ile Ala Ala Ala Trp Ala
    1               5                  10                  15
   Glu Glu Gln Asn Lys Leu Val His Gly Gly Pro Cys Asp Lys Thr Ser
               20                  25                  30
   His Pro Tyr Gln Ala Ala Leu Tyr Thr Ser Gly His Leu Leu Cys Gly
           35                  40                  45
   Gly Val Leu Ile His Pro Leu Trp Val Leu Thr Ala Ala His Cys Lys
       50                  55                  60
   Lys Pro Asn Leu Gln Val Phe Leu Gly Lys His Asn Leu Arg Gln Arg
   65                  70                  75                  80
   Glu Ser Ser Gln Glu Gln Ser Ser Val Val Arg Ala Val Ile His Pro
                   85                  90                  95
   Asp Tyr Asp Ala Ala Ser His Asp Gln Asp Ile Met Leu Leu Arg Leu
               100                 105                 110
   Ala Arg Pro Ala Lys Leu Ser Glu Leu Ile Gln Pro Leu Pro Leu Glu
           115                 120                 125
   Arg Asp Cys Ser Ala Asn Thr Thr Ser Cys His Ile Leu Gly Trp Gly
       130                 135                 140
   Lys Thr Ala Asp Gly Asp Phe Pro Asp Thr Ile Gln Cys Ala Tyr Ile
   145                 150                 155                 160
   His Leu Val Ser Arg Glu Glu Cys Glu His Ala Tyr Pro Gly Gln Ile
                   165                 170                 175
   Thr Gln Asn Met Leu Cys Ala Gly Asp Glu Lys Tyr Gly Lys Asp Ser
               180                 185                 190
   Cys Gln Gly Asp Ser Gly Gly Pro Leu Val Cys Gly Asp His Leu Arg
           195                 200                 205
   Gly Leu Val Ser Trp Gly Asn Ile Pro Cys Gly Ser Lys Glu Lys Pro
       210                 215                 220
   Gly Val Tyr Thr Asn Val Cys Arg Tyr Thr Asn Trp Ile Gln Lys Thr
   225                 230                 235                 240
   Ile Gln Ala Lys
<210> 12
   <211> 1527
   <212> DNA
   <213> Homo sapiens
<400> 12
   ggcggacaaa gcccgattgt tcctgggccc tttccccatc gcgcctgggc ctgctcccca 60
   gcccggggca ggggcggggg ccagtgtggt gacacacgct gtagctgtct ccccggctgg 120
   ctggctcgct ctctcctggg gacacagagg tcggcaggca gcacacagag ggacctacgg 180
   gcagctgttc cttcccccga ctcaagaatc cccggaggcc cggaggcctg cagcaggagc 240
   ggccatgaag aagctgatgg tggtgctgag tctgattgct gcagcctggg cagaggagca 300
   gaataagttg gtgcatggcg gaccctgcga caagacatct cacccctacc aagctgccct 360
   ctacacctcg ggccacttgc tctgtggtgg ggtccttatc catccactgt gggtcctcac 420
   agctgcccac tgcaaaaaac cgaatcttca ggtcttcctg gggaagcata accttcggca 480
   aagggagagt tcccaggagc agagttctgt tgtccgggct gtgatccacc ctgactatga 540
   tgccgccagc catgaccagg acatcatgct gttgcgcctg gcacgcccag ccaaactctc 600
   tgaactcatc cagccccttc ccctggagag ggactgctca gccaacacca ccagctgcca 660
   catcctgggc tggggcaaga cagcagatgg tgatttccct gacaccatcc agtgtgcata 720
   catccacctg gtgtcccgtg aggagtgtga gcatgcctac cctggccaga tcacccagaa 780
   catgttgtgt gctggggatg agaagtacgg gaaggattcc tgccagggtg attctggggg 840
   tccgctggta tgtggagacc acctccgagg ccttgtgtca tggggtaaca tcccctgtgg 900
   atcaaaggag aagccaggag tctacaccaa cgtctgcaga tacacgaact ggatccaaaa 960
   aaccattcag gccaagtgac cctgacatgt gacatctacc tcccgaccta ccaccccact 1020
   ggctggttcc agaacgtctc tcacctagac cttgcctccc ctcctctcct gcccagctct 1080
   gaccctgatg cttaataaac gcagcgacgt gagggtcctg attctccctg gttttacccc 1140
   agctccatcc ttgcatcact ggggaggacg tgatgagtga ggacttgggt cctcggtctt 1200
   acccccacca ctaagagaat acaggaaaat cccttctagg catctcctct ccccaaccct 1260
   tccacacgtt tgatttcttc ctgcagaggc ccagccacgt gtctggaatc ccagctccgc 1320
   tgcttactgt cggtgtcccc ttgggatgta cctttcttca ctgcagattt ctcacctgta 1380
   agatgaagat aaggatgata cagtctccat aaggcagtgg ctgttggaaa gatttaaggt 1440
   ttcacaccta tgacatacat ggaatagcac ctgggccacc atgcactcaa taaagaatga 1500
   attttattat gaaaaaaaaa aaaaaaa                                     1527
<210> 13
   <211> 244
   <212> PRT
   <213> Homo sapiens
<400> 13
   Met Lys Lys Leu Met Val Val Leu Ser Leu Ile Ala Ala Ala Trp Ala
    1               5                  10                  15
   Glu Glu Gln Asn Lys Leu Val His Gly Gly Pro Cys Asp Lys Thr Ser
               20                  25                  30
   His Pro Tyr Gln Ala Ala Leu Tyr Thr Ser Gly His Leu Leu Cys Gly
           35                  40                  45
   Gly Val Leu Ile His Pro Leu Trp Val Leu Thr Ala Ala His Cys Lys
       50                  55                  60
   Lys Pro Asn Leu Gln Val Phe Leu Gly Lys His Asn Leu Arg Gln Arg
   65                  70                  75                  80
   Glu Ser Ser Gln Glu Gln Ser Ser Val Val Arg Ala Val Ile His Pro
                   85                  90                  95
   Asp Tyr Asp Ala Ala Ser His Asp Gln Asp Ile Met Leu Leu Arg Leu
               100                 105                 110
   Ala Arg Pro Ala Lys Leu Ser Glu Leu Ile Gln Pro Leu Pro Leu Glu
           115                 120                 125
   Arg Asp Cys Ser Ala Asn Thr Thr Ser Cys His Ile Leu Gly Trp Gly
       130                 135                 140
   Lys Thr Ala Asp Gly Asp Phe Pro Asp Thr Ile Gln Cys Ala Tyr Ile
   145                 150                 155                 160
   His Leu Val Ser Arg Glu Glu Cys Glu His Ala Tyr Pro Gly Gln Ile
                   165                 170                 175
   Thr Gln Asn Met Leu Cys Ala Gly Asp Glu Lys Tyr Gly Lys Asp Ser
               180                 185                 190
   Cys Gln Gly Asp Ser Gly Gly Pro Leu Val Cys Gly Asp His Leu Arg
               195                 200                 205
   Gly Leu Val Ser Trp Gly Asn Ile Pro Cys Gly Ser Lys Glu Lys Pro
       210                 215                 220
   Gly Val Tyr Thr Asn Val Cys Arg Tyr Thr Asn Trp Ile Gln Lys Thr
   225                 230                 235                 240
   Ile Gln Ala Lys
<210> 14
   <211> 1527
   <212> DNA
   <213> Homo sapiens
<400> 14
   aggacgttcc agaagcatct ggggacagaa ccagcctctt ccagggaggc ctgggagctg 60
   ggggtgtgtg tctggcagtc cctgcagccc tgggctctgc ggcccctgcg tcctccgctt 120
   ggctctgcca ctgcatctga gtgtcttctc tcctcacggc tccccgcatt tctaactctt 180
   tctgcctcct cgtctcaaag ctgttccttc ccccgactca agaatccccg gaggcccgga 240
   ggcctgcagc aggagcggcc atgaagaagc tgatggtggt gctgagtctg attgctgcag 300
   cctgggcaga ggagcagaat aagttggtgc atggcggacc ctgcgacaag acatctcacc 360
   cctaccaagc tgccctctac acctcgggcc acttgctctg tggtggggtc cttatccatc 420
   cactgtgggt cctcacagct gcccactgca aaaaaccgaa tcttcaggtc ttcctgggga 480
   agcataacct tcggcaaagg gagagttccc aggagcagag ttctgttgtc cgggctgtga 540
   tccaccctga ctatgatgcc gccagccatg accaggacat catgctgttg cgcctggcac 600
   gcccagccaa actctctgaa ctcatccagc cccttcccct ggagagggac tgctcagcca 660
   acaccaccag ctgccacatc ctgggctggg gcaagacagc agatggtgat ttccctgaca 720
   ccatccagtg tgcatacatc cacctggtgt cccgtgagga gtgtgagcat gcctaccctg 780
   gccagatcac ccagaacatg ttgtgtgctg gggatgagaa gtacgggaag gattcctgcc 840
   agggtgattc tgggggtccg ctggtatgtg gagaccacct ccgaggcctt gtgtcatggg 900
   gtaacatccc ctgtggatca aaggagaagc caggagtcta caccaacgtc tgcagataca 960
   cgaactggat ccaaaaaacc attcaggcca agtgaccctg acatgtgaca tctacctccc 1020
   gacctaccac cccactggct ggttccagaa cgtctctcac ctagaccttg cctcccctcc 1080
   tctcctgccc agctctgacc ctgatgctta ataaacgcag cgacgtgagg gtcctgattc 1140
   tccctggttt taccccagct ccatccttgc atcactgggg aggacgtgat gagtgaggac 1200
   ttgggtcctc ggtcttaccc ccaccactaa gagaatacag gaaaatccct tctaggcatc 1260
   tcctctcccc aacccttcca cacgtttgat ttcttcctgc agaggcccag ccacgtgtct 1320
   ggaatcccag ctccgctgct tactgtcggt gtccccttgg gatgtacctt tcttcactgc 1380
   agatttctca cctgtaagat gaagataagg atgatacagt ctccataagg cagtggctgt 1440
   tggaaagatt taaggtttca cacctatgac atacatggaa tagcacctgg gccaccatgc 1500
   actcaataaa gaatgaattt tattatg                                    1527
<210> 15
   <211> 137
   <212> PRT
   <213> Homo sapiens
<400> 15
   Met Leu Leu Arg Leu Ala Arg Pro Ala Lys Leu Ser Glu Leu Ile Gln
    1               5                  10                  15
   Pro Leu Pro Leu Glu Arg Asp Cys Ser Ala Asn Thr Thr Ser Cys His
               20                  25                  30
   Ile Leu Gly Trp Gly Lys Thr Ala Asp Gly Asp Phe Pro Asp Thr Ile
           35                  40                  45
   Gln Cys Ala Tyr Ile His Leu Val Ser Arg Glu Glu Cys Glu His Ala
       50                  55                  60
   Tyr Pro Gly Gln Ile Thr Gln Asn Met Leu Cys Ala Gly Asp Glu Lys
   65                  70                  75                  80
   Tyr Gly Lys Asp Ser Cys Gln Gly Asp Ser Gly Gly Pro Leu Val Cys
                   85                  90                  95
   Gly Asp His Leu Arg Gly Leu Val Ser Trp Gly Asn Ile Pro Cys Gly
               100                 105                 110
   Ser Lys Glu Lys Pro Gly Val Tyr Thr Asn Val Cys Arg Tyr Thr Asn
           115                 120                 125
   Trp Ile Gln Lys Thr Ile Gln Ala Lys
       130                 135
<210> 16
   <211> 1495
   <212> DNA
   <213> Homo sapiens
<400> 16
   aggacgttcc agaagcatct ggggacagaa ccagcctctt ccagggaggc ctgggagctg 60
   ggggtgtgtg tctggcagtc cctgcagccc tgggctctgc ggcccctgcg tcctccgctt 120
   ggctctgcca ctgcatctga gtgtcttctc tcctcacggc tccccgcatt tctaactctt 180
   tctgcctcct cgtctcaaag ctgttccttc ccccgactca agaatccccg gaggcccgga 240
   ggcctgcagc agcctgggca gaggagcaga ataagttggt gcatggcgga ccctgcgaca 300
   agacatctca cccctaccaa gctgccctct acacctcggg ccacttgctc tgtggtgggg 360
   tccttatcca tccactgtgg gtcctcacag ctgcccactg caaaaaaccg aatcttcagg 420
   tcttcctggg gaagcataac cttcggcaaa gggagagttc ccaggagcag agttctgttg 480
   tccgggctgt gatccaccct gactatgatg ccgccagcca tgaccaggac atcatgctgt 540
   tgcgcctggc acgcccagcc aaactctctg aactcatcca gccccttccc ctggagaggg 600
   actgctcagc caacaccacc agctgccaca tcctgggctg gggcaagaca gcagatggtg 660
   atttccctga caccatccag tgtgcataca tccacctggt gtcccgtgag gagtgtgagc 720
   atgcctaccc tggccagatc acccagaaca tgttgtgtgc tggggatgag aagtacggga 780
   aggattcctg ccagggtgat tctgggggtc cgctggtatg tggagaccac ctccgaggcc 840
   ttgtgtcatg gggtaacatc ccctgtggat caaaggagaa gccaggagtc tacaccaacg 900
   tctgcagata cacgaactgg atccaaaaaa ccattcaggc caagtgaccc tgacatgtga 960
   catctacctc ccgacctacc accccactgg ctggttccag aacgtctctc acctagacct 1020
   tgcctcccct cctctcctgc ccagctctga ccctgatgct taataaacgc agcgacgtga 1080
   gggtcctgat tctccctggt tttaccccag ctccatcctt gcatcactgg ggaggacgtg 1140
   atgagtgagg acttgggtcc tcggtcttac ccccaccact aagagaatac aggaaaatcc 1200
   cttctaggca tctcctctcc ccaacccttc cacacgtttg atttcttcct gcagaggccc 1260
   agccacgtgt ctggaatccc agctccgctg cttactgtcg gtgtcccctt gggatgtacc 1320
   tttcttcact gcagatttct cacctgtaag atgaagataa ggatgataca gtctccataa 1380
   ggcagtggct gttggaaaga tttaaggttt cacacctatg acatacatgg aatagcacct 1440
   gggccaccat gcactcaata aagaatgaat tttattatga aaaaaaaaaa aaaaa      1495
<210> 17
   <211> 40
   <212> PRT
   <213> Homo sapiens
<400> 17
   Met Lys Lys Leu Met Val Val Leu Ser Leu Ile Ala Ala Gly Ile Phe
   1               5                   10                  15
   Arg Ser Ser Trp Gly Ser Ile Thr Phe Gly Lys Gly Arg Val Pro Arg
               20                  25                  30
   Ser Arg Val Leu Leu Ser Gly Leu
           35                  40
<210> 18
   <211> 1386
   <212> DNA
   <213> Homo sapiens
<400> 18
   aggacgttcc agaagcatct ggggacagaa ccagcctctt ccagggaggc ctgggagctg 60
   ggggtgtgtg tctggcagtc cctgcagccc tgggctctgc ggcccctgcg tcctccgctt 120
   ggctctgcca ctgcatctga gtgtcttctc tcctcacggc tccccgcatt tctaactctt 180
   tctgcctcct cgtctcaaag ctgttccttc ccccgactca agaatccccg gaggcccgga 240
   ggcctgcagc aggagcggcc atgaagaagc tgatggtggt gctgagtctg attgctgcag 300
   gaatcttcag gtcttcctgg ggaagcataa ccttcggcaa agggagagtt cccaggagca 360
   gagttctgtt gtccgggctg tgatccaccc tgactatgat gccgccagcc atgaccagga 420
   catcatgctg ttgcgcctgg cacgcccagc caaactctct gaactcatcc agccccttcc 480
   cctggagagg gactgctcag ccaacaccac cagctgccac atcctgggct ggggcaagac 540
   agcagatggt gatttccctg acaccatcca gtgtgcatac atccacctgg tgtcccgtga 600
   ggagtgtgag catgcctacc ctggccagat cacccagaac atgttgtgtg ctggggatga 660
   gaagtacggg aaggattcct gccagggtga ttctgggggt ccgctggtat gtggagacca 720
   cctccgaggc cttgtgtcat ggggtaacat cccctgtgga tcaaaggaga agccaggagt 780
   ctacaccaac gtctgcagat acacgaactg gatccaaaaa accattcagg ccaagtgacc 840
   ctgacatgtg acatctacct cccgacctac caccccactg gctggttcca gaacgtctct 900
   cacctagacc ttgcctcccc tcctctcctg cccagctctg accctgatgc ttaataaacg 960
   cagcgacgtg agggtcctga ttctccctgg ttttacccca gctccatcct tgcatcactg 1020
   gggaggacgt gatgagtgag gacttgggtc ctcggtctta cccccaccac taagagaata 1080
   caggaaaatc ccttctaggc atctcctctc cccaaccctt ccacacgttt gatttcttcc 1140
   tgcagaggcc cagccacgtg tctggaatcc cagctccgct gcttactgtc ggtgtcccct 1200
   tgggatgtac ctttcttcac tgcagatttc tcacctgtaa gatgaagata aggatgatac 1260
   agtctccata aggcagtggc tgttggaaag atttaaggtt tcacacctat gacatacatg 1320
   gaatagcacc tgggccacca tgcactcaat aaagaatgaa ttttattatg aaaaaaaaaa 1380
   aaaaaa                                                            1386
<210> 19
   <211> 276
   <212> PRT
   <213> Homo sapiens
<400> 19
   Met Arg Ala Pro His Leu His Leu Ser Ala Ala Ser Gly Ala Arg Ala
    1               5                  10                  15
   Leu Ala Lys Leu Leu Pro Leu Leu Met Ala Gln Leu Trp Ala Ala Glu
               20                  25                  30
   Ala Ala Leu Leu Pro Gln Asn Asp Thr Arg Leu Asp Pro Glu Ala Tyr
           35                  40                  45
   Gly Ser Pro Cys Ala Arg Gly Ser Gln Pro Trp Gln Val Ser Leu Phe
       50                  55                  60
   Asn Gly Leu Ser Phe His Cys Ala Gly Val Leu Val Asp Gln Ser Trp
   65                  70                  75                  80
   Val Leu Thr Ala Ala His Cys Gly Asn Lys Pro Leu Trp Ala Arg Val
                   85                  90                  95
   Gly Asp Asp His Leu Leu Leu Leu Gln Gly Glu Gln Leu Arg Arg Thr
               100                 105                 110
   Thr Arg Ser Val Val His Pro Lys Tyr His Gln Gly Ser Gly Pro Ile
           115                 120                 125
   Leu Pro Arg Arg Thr Asp Glu His Asp Leu Met Leu Leu Lys Leu Ala
       130                 135                 140
   Arg Pro Val Val Leu Gly Pro Arg Val Arg Ala Leu Gln Leu Pro Tyr
   145                 150                 155                 160
   Arg Cys Ala Gln Pro Gly Asp Gln Cys Gln Val Ala Gly Trp Gly Thr
                   165                 170                 175
   Thr Ala Ala Arg Arg Val Lys Tyr Asn Lys Gly Leu Thr Cys Ser Ser
               180                 185                 190
   Ile Thr Ile Leu Ser Pro Lys Glu Cys Glu Val Phe Tyr Pro Gly Val
           195                 200                 205
   Val Thr Asn Asn Met Ile Cys Ala Gly Leu Asp Arg Gly Gln Asp Pro
       210                 215                 220
   Cys Gln Ser Asp Ser Gly Gly Pro Leu Val Cys Asp Glu Thr Leu Gln
   225                 230                 235                 240
   Gly Ile Leu Ser Trp Gly Val Tyr Pro Cys Gly Ser Ala Gln His Pro
                   245                 250                 255
   Ala Val Tyr Thr Gln Ile Cys Lys Tyr Met Ser Trp Ile Asn Lys Val
               260                 265                 270
   Ile Arg Ser Asn
           275
<210> 20
   <211> 1580
   <212> DNA
   <213> Homo sapiens
<400> 20
   catcctgcca cccctagcct tgctggggac gtgaaccctc tccccgcgcc tgggaagcct 60
   tcttggcacc gggacccgga gaatccccac ggaagccagt tccaaaaggg atgaaaaggg 120
   ggcgtttcgg gcactgggag aagcctgtat tccagggccc ctcccagagc aggaatctgg 180
   gacccaggag tgccagcctc acccacgcag atcctggcca tgagagctcc gcacctccac 240
   ctctccgccg cctctggcgc ccgggctctg gcgaagctgc tgccgctgct gatggcgcaa 300
   ctctgggccg cagaggcggc gctgctcccc caaaacgaca cgcgcttgga ccccgaagcc 360
   tatggctccc cgtgcgcgcg cggctcgcag ccctggcagg tctcgctctt caacggcctc 420
   tcgttccact gcgcgggtgt cctggtggac cagagttggg tgctgacggc cgcgcactgc 480
   ggaaacaagc cactgtgggc tcgagtaggg gatgaccacc tgctgcttct tcagggagag 540
   cagctccgcc ggaccactcg ctctgttgtc catcccaagt accaccaggg ctcaggcccc 600
   atcctgccaa ggcgaacgga tgagcacgat ctcatgttgc tgaagctggc caggcccgta 660
   gtgctggggc cccgcgtccg ggccctgcag cttccctacc gctgtgctca gcccggagac 720
   cagtgccagg ttgctggctg gggcaccacg gccgcccgga gagtgaagta caacaagggc 780
   ctgacctgct ccagcatcac tatcctgagc cctaaagagt gtgaggtctt ctaccctggc 840
   gtggtcacca acaacatgat atgtgctgga ctggaccggg gccaggaccc ttgccagagt 900
   gactctggag gccccctggt ctgtgacgag accctccaag gcatcctctc gtggggtgtt 960
   tacccctgtg gctctgccca gcatccagct gtctacaccc agatctgcaa atacatgtcc 1020
   tggatcaata aagtcatacg ctccaactga tccagatgct acgctccagc tgatccagat 1080
   gttatgctcc tgctgatcca gatgcccaga ggctccatcg tccatcctct tcctccccag 1140
   tcggctgaac tctccccttg tctgcactgt tcaaacctct gccgccctcc acacctctaa 1200
   acatctcccc tctcacctca ttcccccacc tatccccatt ctctgcctgt actgaagctg 1260
   aaatgcagga agtggtggca aaggtttatt ccagagaagc caggaagccg gtcatcaccc 1320
   agcctctgag agcagttact ggggtcaccc aacctgactt cctctgccac tccctgctgt 1380
   gtgactttgg gcaagccaag tgccctctct gaacctcagt ttcctcatct gcaaaatggg 1440
   aacaatgacg tgcctacctc ttagacatgt tgtgaggaga ctatgatata acatgtgtat 1500
   gtaaatcttc atggtgattg tcatgtaagg cttaacacag tgggtggtga gttctgacta 1560
   aaggttacct gttgtcgtga                                            1580
<210> 21
   <211> 276
   <212> PRT
   <213> Homo sapiens
<400> 21
   Met Arg Ala Pro His Leu His Leu Ser Ala Ala Ser Gly Ala Arg Ala
    1               5                  10                  15
   Leu Ala Lys Leu Leu Pro Leu Leu Met Ala Gln Leu Trp Ala Ala Glu
               20                  25                  30
   Ala Ala Leu Leu Pro Gln Asn Asp Thr Arg Leu Asp Pro Glu Ala Tyr
           35                  40                  45
   Gly Ser Pro Cys Ala Arg Gly Ser Gln Pro Trp Gln Val Ser Leu Phe
       50                  55                  60
   Asn Gly Leu Ser Phe His Cys Ala Gly Val Leu Val Asp Gln Ser Trp
   65                  70                  75                  80
   Val Leu Thr Ala Ala His Cys Gly Asn Lys Pro Leu Trp Ala Arg Val
                   85                  90                  95
   Gly Asp Asp His Leu Leu Leu Leu Gln Gly Glu Gln Leu Arg Arg Thr
               100                 105                 110
   Thr Arg Ser Val Val His Pro Lys Tyr His Gln Gly Ser Gly Pro Ile
           115                     120                     125
   Leu Pro Arg Arg Thr Asp Glu His Asp Leu Met Leu Leu Lys Leu Ala
       130                     135                     140
   Arg Pro Val Val Leu Gly Pro Arg Val Arg Ala Leu Gln Leu Pro Tyr
   145                     150                     155                     160
   Arg Cys Ala Gln Pro Gly Asp Gln Cys Gln Val Ala Gly Trp Gly Thr
                   165                     170                     175
   Thr Ala Ala Arg Arg Val Lys Tyr Asn Lys Gly Leu Thr Cys Ser Ser
               180                     185                     190
   Ile Thr Ile Leu Ser Pro Lys Glu Cys Glu Val Phe Tyr Pro Gly Val
           195                     200                     205
   Val Thr Asn Asn Met Ile Cys Ala Gly Leu Asp Arg Gly Gln Asp Pro
       210                     215                     220
   Cys Gln Ser Asp Ser Gly Gly Pro Leu Val Cys Asp Glu Thr Leu Gln
   225                     230                     235                     240
   Gly Ile Leu Ser Trp Gly Val Tyr Pro Cys Gly Ser Ala Gln His Pro
                   245                     250                     255
   Ala Val Tyr Thr Gln Ile Cys Lys Tyr Met Ser Trp Ile Asn Lys Val
               260                     265                     270
   Ile Arg Ser Asn
           275
<210> 22
   <211> 1443
   <212> DNA
   <213> Homo sapiens
<400> 22
   accagcggca gaccacaggc agggcagagg cacgtctggg tcccctccct ccttcctatc 60
   ggcgactccc aggatcctgg ccatgagagc tccgcacctc cacctctccg ccgcctctgg 120
   cgcccgggct ctggcgaagc tgctgccgct gctgatggcg caactctggg ccgcagaggc 180
   ggcgctgctc ccccaaaacg acacgcgctt ggaccccgaa gcctatggct ccccgtgcgc 240
   gcgcggctcg cagccctggc aggtctcgct cttcaacggc ctctcgttcc actgcgcggg 300
   tgtcctggtg gaccagagtt gggtgctgac ggccgcgcac tgcggaaaca agccactgtg 360
   ggctcgagta ggggatgacc acctgctgct tcttcaggga gagcagctcc gccggaccac 420
   tcgctctgtt gtccatccca agtaccacca gggctcaggc cccatcctgc caaggcgaac 480
   ggatgagcac gatctcatgt tgctgaagct ggccaggccc gtagtgctgg ggccccgcgt 540
   ccgggccctg cagcttccct accgctgtgc tcagcccgga gaccagtgcc aggttgctgg 600
   ctggggcacc acggccgccc ggagagtgaa gtacaacaag ggcctgacct gctccagcat 660
   cactatcctg agccctaaag agtgtgaggt cttctaccct ggcgtggtca ccaacaacat 720
   gatatgtgct ggactggacc ggggccagga cccttgccag agtgactctg gaggccccct 780
   ggtctgtgac gagaccctcc aaggcatcct ctcgtggggt gtttacccct gtggctctgc 840
   ccagcatcca gctgtctaca cccagatctg caaatacatg tcctggatca ataaagtcat 900
   acgctccaac tgatccagat gctacgctcc agctgatcca gatgttatgc tcctgctgat 960
   ccagatgccc agaggctcca tcgtccatcc tcttcctccc cagtcggctg aactctcccc 1020
   ttgtctgcac tgttcaaacc tctgccgccc tccacacctc taaacatctc ccctctcacc 1080
   tcattccccc acctatcccc attctctgcc tgtactgaag ctgaaatgca ggaagtggtg 1140
   gcaaaggttt attccagaga agccaggaag ccggtcatca cccagcctct gagagcagtt 1200
   actggggtca cccaacctga cttcctctgc cactccctgc tgtgtgactt tgggcaagcc 1260
   aagtgccctc tctgaacctc agtttcctca tctgcaaaat gggaacaatg acgtgcctac 1320
   ctcttagaca tgttgtgagg agactatgat ataacatgtg tatgtaaatc ttcatggtga 1380
   ttgtcatgta aggcttaaca cagtgggtgg tgagttctga ctaaaggtta cctgttgtcg 1440
   tga                                                               1443
<210> 23
   <211> 180
   <212> PRT
   <213> Homo sapiens
<400> 23
   Met Leu Cys Leu Leu Leu Thr Leu Gly Val Ala Leu Val Cys Gly Val
    1               5                  10                  15
   Pro Ala Met Asp Ile Pro Gln Thr Lys Gln Asp Leu Glu Leu Pro Lys
               20                  25                  30
   Leu Ala Gly Thr Trp His Ser Met Ala Met Ala Thr Asn Asn Ile Ser
           35                  40                  45
   Leu Met Ala Thr Leu Lys Ala Pro Leu Arg Val His Ile Thr Ser Leu
       50                  55                  60
   Leu Pro Thr Pro Glu Asp Asn Leu Glu Ile Val Leu His Arg Trp Glu
   65                  70                  75                  80
   Asn Asn Ser Cys Val Glu Lys Lys Val Leu Gly Glu Lys Thr Glu Asn
                   85                  90                  95
   Pro Lys Lys Phe Lys Ile Asn Tyr Thr Val Ala Asn Glu Ala Thr Leu
               100                 105                 110
   Leu Asp Thr Asp Tyr Asp Asn Phe Leu Phe Leu Cys Leu Gln Asp Thr
           115                 120                 125
   Thr Thr Pro Ile Gln Ser Met Met Cys Gln Tyr Leu Ala Arg Val Leu
       130                 135                 140
   Val Glu Asp Asp Glu Ile Met Gln Gly Phe Ile Arg Ala Phe Arg Pro
   145                 150                 155                 160
   Leu Pro Arg His Leu Trp Tyr Leu Leu Asp Leu Lys Gln Met Glu Glu
                   165                 170                 175
   Pro Cys Arg Phe
               180
<210> 24
   <211> 857
   <212> DNA
   <213> Homo sapiens
<400> 24
   catccctctg gctccagagc tcagagccac ccacagccgc agccatgctg tgcctcctgc 60
   tcaccctggg cgtggccctg gtctgtggtg tcccggccat ggacatcccc cagaccaagc 120
   aggacctgga gctcccaaag ttggcaggga cctggcactc catggccatg gcgaccaaca 180
   acatctccct catggcgaca ctgaaggccc ctctgagggt ccacatcacc tcactgttgc 240
   ccacccccga ggacaacctg gagatcgttc tgcacagatg ggagaacaac agctgtgttg 300
   agaagaaggt ccttggagag aagactgaga atccaaagaa gttcaagatc aactatacgg 360
   tggcgaacga ggccacgctg ctcgatactg actacgacaa tttcctgttt ctctgcctac 420
   aggacaccac cacccccatc cagagcatga tgtgccagta cctggccaga gtcctggtgg 480
   aggacgatga gatcatgcag ggattcatca gggctttcag gcccctgccc aggcacctat 540
   ggtacttgct ggacttgaaa cagatggaag agccgtgccg tttctaggtg agctcctgcc 600
   tggtcctgcc tcctggctca cctccgcctc caggaagacc agactcccac ccttccacac 660
   ctccagagca gtgggacttc ctcctgccct ttcaaagaat aaccacagct cagaagacga 720
   tgacgtggtc atctgtgtcg ccatcccctt cctgctgcac acctgcacca cggccatggg 780
   gaggctgctc cctgggggca gagtctctgg cagaggttat taataaaccc ttggagcatg 840
   aaaaaaaaaa aaaaaaa                                                857
<210> 25
   <211> 180
   <212> PRT
   <213> Homo sapiens
<400> 25
   Met Leu Cys Leu Leu Leu Thr Leu Gly Val Ala Leu Val Cys Gly Val
   1 5 10 15
   Pro Ala Met Asp Ile Pro Gln Thr Lys Gln Asp Leu Glu Leu Pro Lys
   20 25 30
   Leu Ala Gly Thr Trp His Ser Met Ala Met Ala Thr Asn Asn Ile Ser
   35 40 45
   Leu Met Ala Thr Leu Lys Ala Pro Leu Arg Val His Ile Thr Ser Leu
   50 55 60
   Leu Pro Thr Pro Glu Asp Asn Leu Glu Ile Val Leu His Arg Trp Glu
   65 70 75 80
   Asn Asn Ser Cys Val Glu Lys Lys Val Leu Gly Glu Lys Thr Glu Asn
   85 90 95
   Pro Lys Lys Phe Lys Ile Asn Tyr Thr Val Ala Asn Glu Ala Thr Leu
   100 105 110
   Leu Asp Thr Asp Tyr Asp Asn Phe Leu Phe Leu Cys Leu Gln Asp Thr
   115 120 125
   Thr Thr Pro Ile Gln Ser Met Met Cys Gln Tyr Leu Ala Arg Val Leu
   130 135 140
   Val Glu Asp Asp Glu Ile Met Gln Gly Phe Ile Arg Ala Phe Arg Pro
   145 150 155 160
   Leu Pro Arg His Leu Trp Tyr Leu Leu Asp Leu Lys Gln Met Glu Glu
   165 170 175
   Pro Cys Arg Phe
   180
<210> 26
   <211> 828
   <212> DNA
   <213> Homo sapiens
<400> 26
   catccctctg gctccagagc tcagagccac ccacagccgc agccatgctg tgcctcctgc 60
   tcaccctggg cgtggccctg gtctgtggtg tcccggccat ggacatcccc cagaccaagc 120
   aggacctgga gctcccaaag ttggcaggga cctggcactc catggccatg gcgaccaaca 180
   acatctccct catggcgaca ctgaaggccc ctctgagggt ccacatcacc tcactgttgc 240
   ccacccccga ggacaacctg gagatcgttc tgcacagatg ggagaacaac agctgtgttg 300
   agaagaaggt ccttggagag aagactgaga atccaaagaa gttcaagatc aactatacgg 360
   tggcgaacga ggccacgctg ctcgatactg actacgacaa tttcctgttt ctctgcctac 420
   aggacaccac cacccccatc cagagcatga tgtgccagta cctggccaga gtcctggtgg 480
   aggacgatga gatcatgcag ggattcatca gggctttcag gcccctgccc aggcacctat 540
   ggtacttgct ggacttgaaa cagatggaag agccgtgccg tttctagctc acctccgcct 600
   ccaggaagac cagactccca cccttccaca cctccagagc agtgggactt cctcctgccc 660
   tttcaaagaa taaccacagc tcagaagacg atgacgtggt catctgtgtc gccatcccct 720
   tcctgctgca cacctgcacc acggccatgg ggaggctgct ccctgggggc agagtctctg 780
   gcagaggtta ttaataaacc cttggagcat gaaaaaaaaa aaaaaaaa 828
<210> 27
   <211> 402
   <212> PRT
   <213> Homo sapiens
<400> 27
<210> 28
   <211> 2876
   <212> DNA
   <213> Homo sapiens
<400> 28
   gaattcctgc agctcagcag ccgccgccag agcaggacga accgccaatc gcaaggcacc 60
   tctgagaact tcaggatgca gatgtctcca gccctcacct gcctagtcct gggcctggcc 120
   cttgtctttg gtgaagggtc tgctgtgcac catcccccat cctacgtggc ccacctggcc 180
   tcagacttcg gggtgagggt gtttcagcag gtggcgcagg cctccaagga ccgcaacgtg 240
   gttttctcac cctatggggt ggcctcggtg ttggccatgc tccagctgac aacaggagga 300
   gaaacccagc agcagattca agcagctatg ggattcaaga ttgatgacaa gggcatggcc 360
   cccgccctcc ggcatctgta caaggagctc atggggccat ggaacaagga tgagatcagc 420
   accacagacg cgatcttcgt ccagcgggat ctgaagctgg tccagggctt catgccccac 480
   ttcttcaggc tgttccggag cacggtcaag caagtggact tttcagaggt ggagagagcc 540
   agattcatca tcaatgactg ggtgaagaca cacacaaaag gtatgatcag caacttgctt 600
   gggaaaggag ccgtggacca gctgacacgg ctggtgctgg tgaatgccct ctacttcaac 660
   ggccagtgga agactccctt ccccgactcc agcacccacc gccgcctctt ccacaaatca 720
   gacggcagca ctgtctctgt gcccatgatg gctcagacca acaagttcaa ctatactgag 780
   ttcaccacgc ccgatggcca ttactacgac atcctggaac tgccctacca cggggacacc 840
   ctcagcatgt tcattgctgc cccttatgaa aaagaggtgc ctctctctgc cctcaccaac 900
   attctgagtg cccagctcat cagccactgg aaaggcaaca tgaccaggct gccccgcctc 960
   ctggttctgc ccaagttctc cctggagact gaagtcgacc tcaggaagcc cctagagaac 1020
   ctgggaatga ccgacatgtt cagacagttt caggctgact tcacgagtct ttcagaccaa 1080
   gagcctctcc acgtcgcgca ggcgctgcag aaagtgaaga tcgaggtgaa cgagagtggc 1140
   acggtggcct cctcatccac agctgtcata gtctcagccc gcatggcccc cgaggagatc 1200
   atcatggaca gacccttcct ctttgtggtc cggcacaacc ccacaggaac agtccttttc 1260
   atgggccaag tgatggaacc ctgaccctgg ggaaagacgc cttcatctgg gacaaaactg 1320
   gagatgcatc gggaaagaag aaactccgaa gaaaagaatt ttagtgttaa tgactctttc 1380
   tgaaggaaga gaagacattt gccttttgtt aaaagatggt aaaccagatc tgtctccaag 1440
   accttggcct ctccttggag gacctttagg tcaaactccc tagtctccac ctgagaccct 1500
   gggagagaag tttgaagcac aactccctta aggtctccaa accagacggt gacgcctgcg 1560
   ggaccatctg gggcacctgc ttccacccgt ctctctgccc actcgggtct gcagacctgg 1620
   ttcccactga ggccctttgc aggatggaac tacggggctt acaggagctt ttgtgtgcct 1680
   ggtagaaact atttctgttc cagtcacatt gccatcactc ttgtactgcc tgccaccgcg 1740
   gaggaggctg gtgacaggcc aaaggccagt ggaagaaaca ccctttcatc tcagagtcca 1800
   ctgtggcact ggccacccct ccccagtaca ggggtgctgc aggtggcaga gtgaatgtcc 1860
   cccatcatgt ggcccaactc tcctggcctg gccatctccc tccccagaaa cagtgtgcat 1920
   gggttatttt ggagtgtagg tgacttgttt actcattgaa gcagatttct gcttcctttt 1980
   atttttatag gaatagagga agaaatgtca gatgcgtgcc cagctcttca ccccccaatc 2040
   tcttggtggg gaggggtgta cctaaatatt tatcatatcc ttgcccttga gtgcttgtta 2100
   gagagaaaga gaactactaa ggaaaataat attatttaaa ctcgctccta gtgtttcttt 2160
   gtggtctgtg tcaccgtatc tcaggaagtc cagccacttg actggcacac acccctccgg 2220
   acatccagcg tgacggagcc cacactgcca ccttgtggcc gcctgagacc ctcgcgcccc 2280
   ccgcgccccc cgcgcccctc tttttcccct tgatggaaat tgaccataca atttcatcct 2340
   ccttcagggg atcaaaagga cggagtgggg ggacagagac tcagatgagg acagagtggt 2400
   ttccaatgtg ttcaatagat ttaggagcag aaatgcaagg ggctgcatga cctaccagga 2460
   cagaactttc cccaattaca gggtgactca cagccgcatt ggtgactcac ttcaatgtgt 2520
   catttccggc tgctgtgtgt gagcagtgga cacgtgaggg gggggtgggt gagagagaca 2580
   ggcagctcgg attcaactac cttagataat atttctgaaa acctaccagc cagagggtag 2640
   ggcacaaaga tggatgtaat gcactttggg aggccaaggc gggaggattg cttgagccca 2700
   ggagttcaag accagcctgg gcaacatacc aagacccccg tctctttaaa aatatatata 2760
   ttttaaatat acttaaatat atatttctaa tatctttaaa tatatatata tattttaaag 2820
   accaatttat gggagaattg cacacagatg tgaaatgaat gtaatctaat agaagc 2876
<210> 29
   <211> 273
   <212> PRT
   <213> Homo sapiens
<400> 29
   Met Thr Pro Gly Thr Gln Ser Pro Phe Phe Leu Leu Leu Leu Leu Thr
    1               5                  10                  15
   Val Leu Thr Val Val Thr Gly Ser Gly His Ala Ser Ser Thr Pro Gly
               20                  25                  30
   Gly Glu Lys Glu Thr Ser Ala Thr Gln Arg Ser Ser Val Pro Ser Ser
           35                  40                  45
   Thr Glu Lys Asn Ala Leu Ser Thr Gly Val Ser Phe Phe Phe Leu Ser
       50                  55                  60
   Phe His Ile Ser Asn Leu Gln Phe Asn Ser Ser Leu Glu Asp Pro Ser
   65                  70                  75                  80
   Thr Asp Tyr Tyr Gln Glu Leu Gln Arg Asp Ile Ser Glu Met Phe Leu
                   85                  90                  95
   Gln Ile Tyr Lys Gln Gly Gly Phe Leu Gly Leu Ser Asn Ile Lys Phe
               100                 105                 110
   Arg Pro Gly Ser Val Val Val Gln Leu Thr Leu Ala Phe Arg Glu Gly
           115                 120                 125
   Thr Ile Asn Val His Asp Val Glu Thr Gln Phe Asn Gln Tyr Lys Thr
       130                 135                 140
   Glu Ala Ala Ser Arg Tyr Asn Leu Thr Ile Ser Asp Val Ser Val Ser
   145                 150                 155                 160
   Asp Val Pro Phe Pro Phe Ser Ala Gln Ser Gly Ala Gly Val Pro Gly
                   165                 170                 175
   Trp Gly Ile Ala Leu Leu Val Leu Val Cys Val Leu Val Ala Leu Ala
               180                 185                 190
   Ile Val Tyr Leu Ile Ala Leu Ala Val Cys Gln Cys Arg Arg Lys Asn
           195                 200                 205
   Tyr Gly Gln Leu Asp Ile Phe Pro Ala Arg Asp Thr Tyr His Pro Met
       210                 215                 220
   Ser Glu Tyr Pro Thr Tyr His Thr His Gly Arg Tyr Val Pro Pro Ser
   225                 230                 235                 240
   Ser Thr Asp Arg Ser Pro Tyr Glu Lys Val Ser Ala Gly Asn Gly Gly
                   245                 250                 255
   Ser Ser Leu Ser Tyr Thr Asn Pro Ala Val Ala Ala Thr Ser Ala Asn
               260                 265                 270
   Leu
<210> 30
   <211> 1209
   <212> DNA
   <213> Homo sapiens
<400> 30
   acctctcaag cagccagcgc ctgcctgaat ctgttctgcc ccctccccac ccatttcacc 60
   accaccatga caccgggcac ccagtctcct ttcttcctgc tgctgctcct cacagtgctt 120
   acagttgtta cgggttctgg tcatgcaagc tctaccccag gtggagaaaa ggagacttcg 180
   gctacccaga gaagttcagt gcccagctct actgagaaga atgctttgtc tactggggtc 240
   tctttctttt tcctgtcttt tcacatttca aacctccagt ttaattcctc tctggaagat 300
   cccagcaccg actactacca agagctgcag agagacattt ctgaaatgtt tttgcagatt 360
   tataaacaag ggggttttct gggcctctcc aatattaagt tcaggccagg atctgtggtg 420
   gtacaattga ctctggcctt ccgagaaggt accatcaatg tccacgacgt ggagacacag 480
   ttcaatcagt ataaaacgga agcagcctct cgatataacc tgacgatctc agacgtcagc 540
   gtgagtgatg tgccatttcc tttctctgcc cagtctgggg ctggggtgcc aggctggggc 600
   atcgcgctgc tggtgctggt ctgtgttctg gttgcgctgg ccattgtcta tctcattgcc 660
   ttggctgtct gtcagtgccg ccgaaagaac tacgggcagc tggacatctt tccagcccgg 720
   gatacctacc atcctatgag cgagtacccc acctaccaca cccatgggcg ctatgtgccc 780
   cctagcagta ccgatcgtag cccctatgag aaggtttctg caggtaatgg tggcagcagc 840
   ctctcttaca caaacccagc agtggcagcc acttctgcca acttgtaggg gcacgtcgcc 900
   cgctgagctg agtggccagc cagtgccatt ccactccact caggttcttc agggccagag 960
   cccctgcacc ctgtttgggc tggtgagctg ggagttcagg tgggctgctc acagcctcct 1020
   tcagaggccc caccaatttc tcggacactt ctcagtgtgt ggaagctcat gtgggcccct 1080
   gagggctcat gcctgggaag tgttgtggtg ggggctccca ggaggactgg cccagagagc 1140
   cctgagatag cggggatcct gaactggact gaataaaacg tggtctccca ctgcgccaaa 1200
   aaaaaaaaa                                                         1209
<210> 31
   <211> 264
   <212> PRT
   <213> Homo sapiens
<400> 31
   Met Thr Pro Gly Thr Gln Ser Pro Phe Phe Leu Leu Leu Leu Leu Thr
   1               5                   10                  15
   Val Leu Thr Ala Thr Thr Ala Pro Lys Pro Ala Thr Val Val Thr Gly
               20                  25                  30
   Ser Gly His Ala Ser Ser Thr Pro Gly Gly Glu Lys Glu Thr Ser Ala
           35                  40                  45
   Thr Gln Arg Ser Ser Val Pro Ser Ser Thr Glu Lys Asn Ala Phe Asn
       50                  55                  60
   Ser Ser Leu Glu Asp Pro Ser Thr Asp Tyr Tyr Gln Glu Leu Gln Arg
   65                  70                  75                  80
   Asp Ile Ser Glu Met Phe Leu Gln Ile Tyr Lys Gln Gly Gly Phe Leu
                   85                  90                  95
   Gly Leu Ser Asn Ile Lys Phe Arg Pro Gly Ser Val Val Val Gln Leu
               100                 105                 110
   Thr Leu Ala Phe Arg Glu Gly Thr Ile Asn Val His Asp Val Glu Thr
           115                 120                 125
   Gln Phe Asn Gln Tyr Lys Thr Glu Ala Ala Ser Arg Tyr Asn Leu Thr
       130                 135                 140
   Ile Ser Asp Val Ser Val Ser Asp Val Pro Phe Pro Phe Ser Ala Gln
   145                 150                 155                 160
   Ser Gly Ala Gly Val Pro Gly Trp Gly Ile Ala Leu Leu Val Leu Val
                   165                 170                 175
   Cys Val Leu Val Ala Leu Ala Ile Val Tyr Leu Ile Ala Leu Ala Val
               180                 185                 190
   Cys Gln Cys Arg Arg Lys Asn Tyr Gly Gln Leu Asp Ile Phe Pro Ala
           195                 200                 205
   Arg Asp Thr Tyr His Pro Met Ser Glu Tyr Pro Thr Tyr His Thr His
       210                 215                 220
   Gly Arg Tyr Val Pro Pro Ser Ser Thr Asp Arg Ser Pro Tyr Glu Lys
   225                 230                 235                 240
   Val Ser Ala Gly Asn Gly Gly Ser Ser Leu Ser Tyr Thr Asn Pro Ala
                   245                 250                 255
   Val Ala Ala Thr Ser Ala Asn Leu
               260
<210> 32
   <211> 1182
   <212> DNA
   <213> Homo sapiens
<400> 32
   acctctcaag cagccagcgc ctgcctgaat ctgttctgcc ccctccccac ccatttcacc 60
   accaccatga caccgggcac ccagtctcct ttcttcctgc tgctgctcct cacagtgctt 120
   acagctacca cagcccctaa acccgcaaca gttgttacgg gttctggtca tgcaagctct 180
   accccaggtg gagaaaagga gacttcggct acccagagaa gttcagtgcc cagctctact 240
   gagaagaatg cttttaattc ctctctggaa gatcccagca ccgactacta ccaagagctg 300
   cagagagaca tttctgaaat gtttttgcag atttataaac aagggggttt tctgggcctc 360
   tccaatatta agttcaggcc aggatctgtg gtggtacaat tgactctggc cttccgagaa 420
   ggtaccatca atgtccacga cgtggagaca cagttcaatc agtataaaac ggaagcagcc 480
   tctcgatata acctgacgat ctcagacgtc agcgtgagtg atgtgccatt tcctttctct 540
   gcccagtctg gggctggggt gccaggctgg ggcatcgcgc tgctggtgct ggtctgtgtt 600
   ctggttgcgc tggccattgt ctatctcatt gccttggctg tctgtcagtg ccgccgaaag 660
   aactacgggc agctggacat ctttccagcc cgggatacct accatcctat gagcgagtac 720
   cccacctacc acacccatgg gcgctatgtg ccccctagca gtaccgatcg tagcccctat 780
   gagaaggttt ctgcaggtaa tggtggcagc agcctctctt acacaaaccc agcagtggca 840
   gccacttctg ccaacttgta ggggcacgtc gcccgctgag ctgagtggcc agccagtgcc 900
   attccactcc actcaggttc ttcagggcca gagcccctgc accctgtttg ggctggtgag 960
   ctgggagttc aggtgggctg ctcacagcct ccttcagagg ccccaccaat ttctcggaca 1020
   cttctcagtg tgtggaagct catgtgggcc cctgagggct catgcctggg aagtgttgtg 1080
   gtgggggctc ccaggaggac tggcccagag agccctgaga tagcggggat cctgaactgg 1140
   actgaataaa acgtggtctc ccactgcgcc aaaaaaaaaa aa                   1182
<210> 33
   <211> 255
   <212> PRT
   <213> Homo sapiens
<400> 33
   Met Thr Pro Gly Thr Gln Ser Pro Phe Phe Leu Leu Leu Leu Leu Thr
    1               5                  10                  15
   Val Leu Thr Val Val Thr Gly Ser Gly His Ala Ser Ser Thr Pro Gly
               20                  25                  30
   Gly Glu Lys Glu Thr Ser Ala Thr Gln Arg Ser Ser Val Pro Ser Ser
           35                  40                  45
   Thr Glu Lys Asn Ala Phe Asn Ser Ser Leu Glu Asp Pro Ser Thr Asp
       50                  55                  60
   Tyr Tyr Gln Glu Leu Gln Arg Asp Ile Ser Glu Met Phe Leu Gln Ile
   65                  70                  75                  80
   Tyr Lys Gln Gly Gly Phe Leu Gly Leu Ser Asn Ile Lys Phe Arg Pro
                   85                  90                  95
   Gly Ser Val Val Val Gln Leu Thr Leu Ala Phe Arg Glu Gly Thr Ile
               100                 105                 110
   Asn Val His Asp Val Glu Thr Gln Phe Asn Gln Tyr Lys Thr Glu Ala
           115                 120                 125
   Ala Ser Arg Tyr Asn Leu Thr Ile Ser Asp Val Ser Val Ser Asp Val
       130                 135                 140
   Pro Phe Pro Phe Ser Ala Gln Ser Gly Ala Gly Val Pro Gly Trp Gly
   145                 150                 155                 160
   Ile Ala Leu Leu Val Leu Val Cys Val Leu Val Ala Leu Ala Ile Val
                   165                 170                 175
   Tyr Leu Ile Ala Leu Ala Val Cys Gln Cys Arg Arg Lys Asn Tyr Gly
               180                 185                 190
   Gln Leu Asp Ile Phe Pro Ala Arg Asp Thr Tyr His Pro Met Ser Glu
           195                 200                 205
   Tyr Pro Thr Tyr His Thr His Gly Arg Tyr Val Pro Pro Ser Ser Thr
       210                 215                 220
   Asp Arg Ser Pro Tyr Glu Lys Val Ser Ala Gly Asn Gly Gly Ser Ser
   225                 230                 235                 240
   Leu Ser Tyr Thr Asn Pro Ala Val Ala Ala Thr Ser Ala Asn Leu
                   245                 250                 255
<210> 34
   <211> 1155
   <212> DNA
   <213> Homo sapiens
<400> 34
   acctctcaag cagccagcgc ctgcctgaat ctgttctgcc ccctccccac ccatttcacc 60
   accaccatga caccgggcac ccagtctcct ttcttcctgc tgctgctcct cacagtgctt 120
   acagttgtta cgggttctgg tcatgcaagc tctaccccag gtggagaaaa ggagacttcg 180
   gctacccaga gaagttcagt gcccagctct actgagaaga atgcttttaa ttcctctctg 240
   gaagatccca gcaccgacta ctaccaagag ctgcagagag acatttctga aatgtttttg 300
   cagatttata aacaaggggg ttttctgggc ctctccaata ttaagttcag gccaggatct 360
   gtggtggtac aattgactct ggccttccga gaaggtacca tcaatgtcca cgacgtggag 420
   acacagttca atcagtataa aacggaagca gcctctcgat ataacctgac gatctcagac 480
   gtcagcgtga gtgatgtgcc atttcctttc tctgcccagt ctggggctgg ggtgccaggc 540
   tggggcatcg cgctgctggt gctggtctgt gttctggttg cgctggccat tgtctatctc 600
   attgccttgg ctgtctgtca gtgccgccga aagaactacg ggcagctgga catctttcca 660
   gcccgggata cctaccatcc tatgagcgag taccccacct accacaccca tgggcgctat 720
   gtgcccccta gcagtaccga tcgtagcccc tatgagaagg tttctgcagg taatggtggc 780
   agcagcctct cttacacaaa cccagcagtg gcagccactt ctgccaactt gtaggggcac 840
   gtcgcccgct gagctgagtg gccagccagt gccattccac tccactcagg ttcttcaggg 900
   ccagagcccc tgcaccctgt ttgggctggt gagctgggag ttcaggtggg ctgctcacag 960
   cctccttcag aggccccacc aatttctcgg acacttctca gtgtgtggaa gctcatgtgg 1020
   gcccctgagg gctcatgcct gggaagtgtt gtggtggggg ctcccaggag gactggccca 1080
   gagagccctg agatagcggg gatcctgaac tggactgaat aaaacgtggt ctcccactgc 1140
   gccaaaaaaa aaaaa                                                  1155
<210> 35
   <211> 96
   <212> PRT
   <213> Homo sapiens
<400> 35
   Met Thr Pro Gly Thr Gln Ser Pro Phe Phe Leu Leu Leu Leu Leu Thr
    1               5                  10                  15
   Val Leu Thr Val Val Thr Gly Ser Gly His Ala Ser Ser Thr Pro Gly
               20                  25                  30
   Gly Glu Lys Glu Thr Ser Ala Thr Gln Arg Ser Ser Val Pro Ser Ser
           35                  40                  45
   Thr Glu Lys Asn Ala Ile Pro Ala Pro Thr Thr Thr Lys Ser Cys Arg
       50                  55                  60
   Glu Thr Phe Leu Lys Cys Phe Cys Arg Phe Ile Asn Lys Gly Val Phe
   65                  70                  75                  80
   Trp Ala Ser Pro Ile Leu Ser Ser Gly Gln Asp Leu Trp Trp Tyr Asn
                   85                  90                  95
<210> 36
   <211> 1136
   <212> DNA
   <213> Homo sapiens
<400> 36
   acctctcaag cagccagcgc ctgcctgaat ctgttctgcc ccctccccac ccatttcacc 60
   accaccatga caccgggcac ccagtctcct ttcttcctgc tgctgctcct cacagtgctt 120
   acagttgtta cgggttctgg tcatgcaagc tctaccccag gtggagaaaa ggagacttcg 180
   gctacccaga gaagttcagt gcccagctct actgagaaga atgctatccc agcaccgact 240
   actaccaaga gctgcagaga gacatttctg aaatgttttt gcagatttat aaacaagggg 300
   gttttctggg cctctccaat attaagttca ggccaggatc tgtggtggta caattgactc 360
   tggccttccg agaaggtacc atcaatgtcc acgacgtgga gacacagttc aatcagtata 420
   aaacggaagc agcctctcga tataacctga cgatctcaga cgtcagcgtg agtgatgtgc 480
   catttccttt ctctgcccag tctggggctg gggtgccagg ctggggcatc gcgctgctgg 540
   tgctggtctg tgttctggtt gcgctggcca ttgtctatct cattgccttg gctgtctgtc 600
   agtgccgccg aaagaactac gggcagctgg acatctttcc agcccgggat acctaccatc 660
   ctatgagcga gtaccccacc taccacaccc atgggcgcta tgtgccccct agcagtaccg 720
   atcgtagccc ctatgagaag gtttctgcag gtaatggtgg cagcagcctc tcttacacaa 780
   acccagcagt ggcagccact tctgccaact tgtaggggca cgtcgcccgc tgagctgagt 840
   ggccagccag tgccattcca ctccactcag gttcttcagg gccagagccc ctgcaccctg 900
   tttgggctgg tgagctggga gttcaggtgg gctgctcaca gcctccttca gaggccccac 960
   caatttctcg gacacttctc agtgtgtgga agctcatgtg ggcccctgag ggctcatgcc 1020
   tgggaagtgt tgtggtgggg gctcccagga ggactggccc agagagccct gagatagcgg 1080
   ggatcctgaa ctggactgaa taaaacgtgg tctcccactg cgccaaaaaa aaaaaa     1136
<210> 37
   <211> 418
   <212> PRT
   <213> Homo sapiens
<400> 37
<210> 38
   <211> 1607
   <212> DNA
   <213> Homo sapiens
<400> 38
   aatgactcct ttcggtaagt gcagtggaag ctgtacactg cccaggcaaa gcgtccgggc 60
   agcgtaggcg ggcgactcag atcccagcca gtggacttag cccctgtttg ctcctccgat 120
   aactggggtg accttggtta atattcacca gcagcctccc ccgttgcccc tctggatcca 180
   ctgcttaaat acggacgagg acagggccct gtctcctcag cttcaggcac caccactgac 240
   ctgggacagt gaatcgacaa tgccgtcttc tgtctcgtgg ggcatcctcc tgctggcagg 300
   cctgtgctgc ctggtccctg tctccctggc tgaggatccc cagggagatg ctgcccagaa 360
   gacagataca tcccaccatg atcaggatca cccaaccttc aacaagatca cccccaacct 420
   ggctgagttc gccttcagcc tataccgcca gctggcacac cagtccaaca gcaccaatat 480
   cttcttctcc ccagtgagca tcgctacagc ctttgcaatg ctctccctgg ggaccaaggc 540
   tgacactcac gatgaaatcc tggagggcct gaatttcaac ctcacggaga ttccggaggc 600
   tcagatccat gaaggcttcc aggaactcct ccgtaccctc aaccagccag acagccagct 660
   ccagctgacc accggcaatg gcctgttcct cagcgagggc ctgaagctag tggataagtt 720
   tttggaggat gttaaaaagt tgtaccactc agaagccttc actgtcaact tcggggacac 780
   cgaagaggcc aagaaacaga tcaacgatta cgtggagaag ggtactcaag ggaaaattgt 840
   ggatttggtc aaggagcttg acagagacac agtttttgct ctggtgaatt acatcttctt 900
   taaaggcaaa tgggagagac cctttgaagt caaggacacc gaggaagagg acttccacgt 960
   ggaccaggtg accaccgtga aggtgcctat gatgaagcgt ttaggcatgt ttaacatcca 1020
   gcactgtaag aagctgtcca gctgggtgct gctgatgaaa tacctgggca atgccaccgc 1080
   catcttcttc ctgcctgatg aggggaaact acagcacctg gaaaatgaac tcacccacga 1140
   tatcatcacc aagttcctgg aaaatgaaga cagaaggtct gccagcttac atttacccaa 1200
   actgtccatt actggaacct atgatctgaa gagcgtcctg ggtcaactgg gcatcactaa 1260
   ggtcttcagc aatggggctg acctctccgg ggtcacagag gaggcacccc tgaagctctc 1320
   caaggccgtg cataaggctg tgctgaccat cgacgagaaa gggactgaag ctgctggggc 1380
   catgttttta gaggccatac ccatgtctat cccccccgag gtcaagttca acaaaccctt 1440
   tgtcttctta atgattgaac aaaataccaa gtctcccctc ttcatgggaa aagtggtgaa 1500
   tcccacccaa aaataactgc ctctcgctcc tcaacccctc ccctccatcc ctggccccct 1560
   ccctggatga cattaaagaa gggttgagct ggtccctgcc tgcaaaa               1607
<210> 39
   <211> 418
   <212> PRT
   <213> Homo sapiens
<400> 39
<210> 40
   <211> 1588
   <212> DNA
   <213> Homo sapiens
<400> 40
   tgggcaggaa ctgggcactg tgcccagggc atgcactgcc tccacgcagc aaccctcaga 60
   gtcctgagct gaaccaagaa ggaggagggg gtcgggcctc cgaggaaggc ctagccgctg 120
   ctgctgccag gaattccagg ttggaggggc ggcaacctcc tgccagcctt caggccactc 180
   tcctgtgcct gccagaagag acagagcttg aggagagctt gaggagagca ggaaaggaca 240
   atgccgtctt ctgtctcgtg gggcatcctc ctgctggcag gcctgtgctg cctggtccct 300
   gtctccctgg ctgaggatcc ccagggagat gctgcccaga agacagatac atcccaccat 360
   gatcaggatc acccaacctt caacaagatc acccccaacc tggctgagtt cgccttcagc 420
   ctataccgcc agctggcaca ccagtccaac agcaccaata tcttcttctc cccagtgagc 480
   atcgctacag cctttgcaat gctctccctg gggaccaagg ctgacactca cgatgaaatc 540
   ctggagggcc tgaatttcaa cctcacggag attccggagg ctcagatcca tgaaggcttc 600
   caggaactcc tccgtaccct caaccagcca gacagccagc tccagctgac caccggcaat 660
   ggcctgttcc tcagcgaggg cctgaagcta gtggataagt ttttggagga tgttaaaaag 720
   ttgtaccact cagaagcctt cactgtcaac ttcggggaca ccgaagaggc caagaaacag 780
   atcaacgatt acgtggagaa gggtactcaa gggaaaattg tggatttggt caaggagctt 840
   gacagagaca cagtttttgc tctggtgaat tacatcttct ttaaaggcaa atgggagaga 900
   ccctttgaag tcaaggacac cgaggaagag gacttccacg tggaccaggt gaccaccgtg 960
   aaggtgccta tgatgaagcg tttaggcatg tttaacatcc agcactgtaa gaagctgtcc 1020
   agctgggtgc tgctgatgaa atacctgggc aatgccaccg ccatcttctt cctgcctgat 1080
   gaggggaaac tacagcacct ggaaaatgaa ctcacccacg atatcatcac caagttcctg 1140
   gaaaatgaag acagaaggtc tgccagctta catttaccca aactgtccat tactggaacc 1200
   tatgatctga agagcgtcct gggtcaactg ggcatcacta aggtcttcag caatggggct 1260
   gacctctccg gggtcacaga ggaggcaccc ctgaagctct ccaaggccgt gcataaggct 1320
   gtgctgacca tcgacgagaa agggactgaa gctgctgggg ccatgttttt agaggccata 1380
   cccatgtcta tcccccccga ggtcaagttc aacaaaccct ttgtcttctt aatgattgaa 1440
   caaaatacca agtctcccct cttcatggga aaagtggtga atcccaccca aaaataactg 1500
   cctctcgctc ctcaacccct cccctccatc cctggccccc tccctggatg acattaaaga 1560
   agggttgagc tggtccctgc ctgcaaaa                                    1588
<210> 41
   <211> 418
   <212> PRT
   <213> Homo sapiens
<400> 41
<210> 42
   <211> 1902
   <212> DNA
   <213> Homo sapiens
<400> 42
   tgggcaggaa ctgggcactg tgcccagggc atgcactgcc tccacgcagc aaccctcaga 60
   gtcctgagct gaaccaagaa ggaggagggg gtcgggcctc cgaggaaggc ctagccgctg 120
   ctgctgccag gaattccagg ttggaggggc ggcaacctcc tgccagcctt caggccactc 180
   tcctgtgcct gccagaagag acagagcttg aggagagctt gaggagagca ggaaagggcg 240
   gcagtaagtc ttcagcatca ggcattttgg ggtgactcag taaatggtag atcttgctac 300
   cagtggaaca gccactaagg attctgcagt gagagcagag ggccagctaa gtggtactct 360
   cccagagact gtctgactca cgccaccccc tccaccttgg acacaggacg ctgtggtttc 420
   tgagccaggt acaatgactc ctttcgcagc ctcccccgtt gcccctctgg atccactgct 480
   taaatacgga cgaggacagg gccctgtctc ctcagcttca ggcaccacca ctgacctggg 540
   acagtgaatc gacaatgccg tcttctgtct cgtggggcat cctcctgctg gcaggcctgt 600
   gctgcctggt ccctgtctcc ctggctgagg atccccaggg agatgctgcc cagaagacag 660
   atacatccca ccatgatcag gatcacccaa ccttcaacaa gatcaccccc aacctggctg 720
   agttcgcctt cagcctatac cgccagctgg cacaccagtc caacagcacc aatatcttct 780
   tctccccagt gagcatcgct acagcctttg caatgctctc cctggggacc aaggctgaca 840
   ctcacgatga aatcctggag ggcctgaatt tcaacctcac ggagattccg gaggctcaga 900
   tccatgaagg cttccaggaa ctcctccgta ccctcaacca gccagacagc cagctccagc 960
   tgaccaccgg caatggcctg ttcctcagcg agggcctgaa gctagtggat aagtttttgg 1020
   aggatgttaa aaagttgtac cactcagaag ccttcactgt caacttcggg gacaccgaag 1080
   aggccaagaa acagatcaac gattacgtgg agaagggtac tcaagggaaa attgtggatt 1140
   tggtcaagga gcttgacaga gacacagttt ttgctctggt gaattacatc ttctttaaag 1200
   gcaaatggga gagacccttt gaagtcaagg acaccgagga agaggacttc cacgtggacc 1260
   aggtgaccac cgtgaaggtg cctatgatga agcgtttagg catgtttaac atccagcact 1320
   gtaagaagct gtccagctgg gtgctgctga tgaaatacct gggcaatgcc accgccatct 1380
   tcttcctgcc tgatgagggg aaactacagc acctggaaaa tgaactcacc cacgatatca 1440
   tcaccaagtt cctggaaaat gaagacagaa ggtctgccag cttacattta cccaaactgt 1500
   ccattactgg aacctatgat ctgaagagcg tcctgggtca actgggcatc actaaggtct 1560
   tcagcaatgg ggctgacctc tccggggtca cagaggaggc acccctgaag ctctccaagg 1620
   ccgtgcataa ggctgtgctg accatcgacg agaaagggac tgaagctgct ggggccatgt 1680
   ttttagaggc catacccatg tctatccccc ccgaggtcaa gttcaacaaa ccctttgtct 1740
   tcttaatgat tgaacaaaat accaagtctc ccctcttcat gggaaaagtg gtgaatccca 1800
   cccaaaaata actgcctctc gctcctcaac ccctcccctc catccctggc cccctccctg 1860
   gatgacatta aagaagggtt gagctggtcc ctgcctgcaa aa                    1902
<210> 43
   <211> 335
   <212> PRT
   <213> Homo sapiens
<400> 43
   Met Gly His Pro Pro Leu Leu Pro Leu Leu Leu Leu Leu His Thr Cys
    1               5                  10                  15
   Val Pro Ala Ser Trp Gly Leu Arg Cys Met Gln Cys Lys Thr Asn Gly
               20                  25                  30
   Asp Cys Arg Val Glu Glu Cys Ala Leu Gly Gln Asp Leu Cys Arg Thr
           35                  40                  45
   Thr Ile Val Arg Leu Trp Glu Glu Gly Glu Glu Leu Glu Leu Val Glu
       50                  55                  60
   Lys Ser Cys Thr His Ser Glu Lys Thr Asn Arg Thr Leu Ser Tyr Arg
   65                  70                  75                  80
   Thr Gly Leu Lys Ile Thr Ser Leu Thr Glu Val Val Cys Gly Leu Asp
                   85                  90                  95
   Leu Cys Asn Gln Gly Asn Ser Gly Arg Ala Val Thr Tyr Ser Arg Ser
               100                 105                 110
   Arg Tyr Leu Glu Cys Ile Ser Cys Gly Ser Ser Asp Met Ser Cys Glu
           115                 120                 125
   Arg Gly Arg His Gln Ser Leu Gln Cys Arg Ser Pro Glu Glu Gln Cys
       130                 135                 140
   Leu Asp Val Val Thr His Trp Ile Gln Glu Gly Glu Glu Gly Arg Pro
   145                 150                 155                 160
   Lys Asp Asp Arg His Leu Arg Gly Cys Gly Tyr Leu Pro Gly Cys Pro
                   165                 170                 175
   Gly Ser Asn Gly Phe His Asn Asn Asp Thr Phe His Phe Leu Lys Cys
               180                 185                 190
   Cys Asn Thr Thr Lys Cys Asn Glu Gly Pro Ile Leu Glu Leu Glu Asn
           195                 200                 205
   Leu Pro Gln Asn Gly Arg Gln Cys Tyr Ser Cys Lys Gly Asn Ser Thr
       210                 215                 220
   His Gly Cys Ser Ser Glu Glu Thr Phe Leu Ile Asp Cys Arg Gly Pro
   225                 230                 235                 240
   Met Asn Gln Cys Leu Val Ala Thr Gly Thr His Glu Pro Lys Asn Gln
                   245                 250                 255
   Ser Tyr Met Val Arg Gly Cys Ala Thr Ala Ser Met Cys Gln His Ala
               260                 265                 270
   His Leu Gly Asp Ala Phe Ser Met Asn His Ile Asp Val Ser Cys Cys
           275                 280                 285
   Thr Lys Ser Gly Cys Asn His Pro Asp Leu Asp Val Gln Tyr Arg Ser
       290                 295                 300
   Gly Ala Ala Pro Gln Pro Gly Pro Ala His Leu Ser Leu Thr Ile Thr
   305                 310                 315                 320
   Leu Leu Met Thr Ala Arg Leu Trp Gly Gly Thr Leu Leu Trp Thr
                   325                 330                 335
<210> 44
   <211> 1548
   <212> DNA
   <213> Homo sapiens
<400> 44
   cagggccgag ccagcccctt caccaccagc cggccgcgcc ccgggaaggg aagtttgtgg 60
   cggaggaggt tcgtacggga ggagggggag gcgcccacgc atctggggct gactcgctct 120
   ttcgcaaaac gtctgggagg agtccctggg gccacaaaac tgcctccttc ctgaggccag 180
   aaggagagaa gacgtgcagg gaccccgcgc acaggagctg ccctcgcgac atgggtcacc 240
   cgccgctgct gccgctgctg ctgctgctcc acacctgcgt cccagcctct tggggcctgc 300
   ggtgcatgca gtgtaagacc aacggggatt gccgtgtgga agagtgcgcc ctgggacagg 360
   acctctgcag gaccacgatc gtgcgcttgt gggaagaagg agaagagctg gagctggtgg 420
   agaaaagctg tacccactca gagaagacca acaggaccct gagctatcgg actggcttga 480
   agatcaccag ccttaccgag gttgtgtgtg ggttagactt gtgcaaccag ggcaactctg 540
   gccgggctgt cacctattcc cgaagccgtt acctcgaatg catttcctgt ggctcatcag 600
   acatgagctg tgagaggggc cggcaccaga gcctgcagtg ccgcagccct gaagaacagt 660
   gcctggatgt ggtgacccac tggatccagg aaggtgaaga agggcgtcca aaggatgacc 720
   gccacctccg tggctgtggc taccttcccg gctgcccggg ctccaatggt ttccacaaca 780
   acgacacctt ccacttcctg aaatgctgca acaccaccaa atgcaacgag ggcccaatcc 840
   tggagcttga aaatctgccg cagaatggcc gccagtgtta cagctgcaag gggaacagca 900
   cccatggatg ctcctctgaa gagactttcc tcattgactg ccgaggcccc atgaatcaat 960
   gtctggtagc caccggcact cacgaaccga aaaaccaaag ctatatggta agaggctgtg 1020
   caaccgcctc aatgtgccaa catgcccacc tgggtgacgc cttcagcatg aaccacattg 1080
   atgtctcctg ctgtactaaa agtggctgta accacccaga cctggatgtc cagtaccgca 1140
   gtggggctgc tcctcagcct ggccctgccc atctcagcct caccatcacc ctgctaatga 1200
   ctgccagact gtggggaggc actctcctct ggacctaaac ctgaaatccc cctctctgcc 1260
   ctggctggat ccgggggacc cctttgccct tccctcggct cccagcccta cagacttgct 1320
   gtgtgacctc aggccagtgt gccgacctct ctgggcctca gttttcccag ctatgaaaac 1380
   agctatctca caaagttgtg tgaagcagaa gagaaaagct ggaggaaggc cgtgggccaa 1440
   tgggagagct cttgttatta ttaatattgt tgccgctgtt gtgttgttgt tattaattaa 1500
   tattcatatt atttatttta tacttacata aagattttgt accagtgg             1548
<210> 45
   <211> 281
   <212> PRT
   <213> Homo sapiens
<400> 45
   Met Gly His Pro Pro Leu Leu Pro Leu Leu Leu Leu Leu His Thr Cys
    1               5                  10                  15
   Val Pro Ala Ser Trp Gly Leu Arg Cys Met Gln Cys Lys Thr Asn Gly
               20                  25                  30
   Asp Cys Arg Val Glu Glu Cys Ala Leu Gly Gln Asp Leu Cys Arg Thr
           35                  40                  45
   Thr Ile Val Arg Leu Trp Glu Glu Gly Glu Glu Leu Glu Leu Val Glu
       50                  55                  60
   Lys Ser Cys Thr His Ser Glu Lys Thr Asn Arg Thr Leu Ser Tyr Arg
   65                  70                  75                  80
   Thr Gly Leu Lys Ile Thr Ser Leu Thr Glu Val Val Cys Gly Leu Asp
                   85                  90                  95
   Leu Cys Asn Gln Gly Asn Ser Gly Arg Ala Val Thr Tyr Ser Arg Ser
               100                 105                 110
   Arg Tyr Leu Glu Cys Ile Ser Cys Gly Ser Ser Asp Met Ser Cys Glu
           115                 120                 125
   Arg Gly Arg His Gln Ser Leu Gln Cys Arg Ser Pro Glu Glu Gln Cys
       130                 135                 140
   Leu Asp Val Val Thr His Trp Ile Gln Glu Gly Glu Glu Gly Arg Pro
   145                 150                 155                 160
   Lys Asp Asp Arg His Leu Arg Gly Cys Gly Tyr Leu Pro Gly Cys Pro
                   165                 170                 175
   Gly Ser Asn Gly Phe His Asn Asn Asp Thr Phe His Phe Leu Lys Cys
               180                 185                 190
   Cys Asn Thr Thr Lys Cys Asn Glu Gly Pro Ile Leu Glu Leu Glu Asn
           195                 200                 205
   Leu Pro Gln Asn Gly Arg Gln Cys Tyr Ser Cys Lys Gly Asn Ser Thr
       210                 215                 220
   His Gly Cys Ser Ser Glu Glu Thr Phe Leu Ile Asp Cys Arg Gly Pro
   225                 230                 235                 240
   Met Asn Gln Cys Leu Val Ala Thr Gly Thr His Glu Arg Ser Leu Trp
                   245                 250                 255
   Gly Ser Trp Leu Pro Cys Lys Ser Thr Thr Ala Leu Arg Pro Pro Cys
               260                 265                 270
   Cys Glu Glu Ala Gln Ala Thr His Val
           275                 280
<210> 46
   <211> 1437
   <212> DNA
   <213> Homo sapiens
<400> 46
   cagggccgag ccagcccctt caccaccagc cggccgcgcc ccgggaaggg aagtttgtgg 60
   cggaggaggt tcgtacggga ggagggggag gcgcccacgc atctggggct gactcgctct 120
   ttcgcaaaac gtctgggagg agtccctggg gccacaaaac tgcctccttc ctgaggccag 180
   aaggagagaa gacgtgcagg gaccccgcgc acaggagctg ccctcgcgac atgggtcacc 240
   cgccgctgct gccgctgctg ctgctgctcc acacctgcgt cccagcctct tggggcctgc 300
   ggtgcatgca gtgtaagacc aacggggatt gccgtgtgga agagtgcgcc ctgggacagg 360
   acctctgcag gaccacgatc gtgcgcttgt gggaagaagg agaagagctg gagctggtgg 420
   agaaaagctg tacccactca gagaagacca acaggaccct gagctatcgg actggcttga 480
   agatcaccag ccttaccgag gttgtgtgtg ggttagactt gtgcaaccag ggcaactctg 540
   gccgggctgt cacctattcc cgaagccgtt acctcgaatg catttcctgt ggctcatcag 600
   acatgagctg tgagaggggc cggcaccaga gcctgcagtg ccgcagccct gaagaacagt 660
   gcctggatgt ggtgacccac tggatccagg aaggtgaaga agggcgtcca aaggatgacc 720
   gccacctccg tggctgtggc taccttcccg gctgcccggg ctccaatggt ttccacaaca 780
   acgacacctt ccacttcctg aaatgctgca acaccaccaa atgcaacgag ggcccaatcc 840
   tggagcttga aaatctgccg cagaatggcc gccagtgtta cagctgcaag gggaacagca 900
   cccatggatg ctcctctgaa gagactttcc tcattgactg ccgaggcccc atgaatcaat 960
   gtctggtagc caccggcact cacgaacgct cactctgggg aagctggttg ccatgtaaaa 1020
   gtactactgc cctgagacca ccatgctgtg aggaagccca agctactcat gtataaatgc 1080
   catgtggaga tagagcccca gatgtttcag ccatctcagc ccaggcacca gacaagtggg 1140
   tgaagaagcc accttggaca tgtagcccca gcagatgtga tatagagaag aaacaggaaa 1200
   cttggctata ttagtttcct agggctgcct gtgataaatt attacaaact ttataaacta 1260
   acacattgtg tgcctatatc aaaacatcat ggaaggacag gcacagtggc tcatgcctgt 1320
   agtcctagca ctttgggagg gtgagaaagg aagatctctt gagctcagga gttcaagatc 1380
   agcctgggca acacagtgag acctcatctc cactaaaaat aaaaaaaaat tggctgg    1437
<210> 47
   <211> 290
   <212> PRT
   <213> Homo sapiens
<400> 47
   Met Gly His Pro Pro Leu Leu Pro Leu Leu Leu Leu Leu His Thr Cys
    1               5                  10                  15
   Val Pro Ala Ser Trp Gly Leu Arg Cys Met Gln Cys Lys Thr Asn Gly
               20                  25                  30
   Asp Cys Arg Val Glu Glu Cys Ala Leu Gly Gln Asp Leu Cys Arg Thr
           35                  40                  45
   Thr Ile Val Arg Leu Trp Glu Glu Gly Glu Glu Leu Glu Leu Val Glu
       50                  55                  60
   Lys Ser Cys Thr His Ser Glu Lys Thr Asn Arg Thr Leu Ser Tyr Arg
   65                  70                  75                  80
   Thr Gly Leu Lys Ile Thr Ser Leu Thr Glu Val Val Cys Gly Leu Asp
                   85                  90                  95
   Leu Cys Asn Gln Gly Asn Ser Gly Arg Ala Val Thr Tyr Ser Arg Ser
               100                 105                 110
   Arg Tyr Leu Glu Cys Ile Ser Cys Gly Ser Ser Asp Met Ser Cys Glu
           115                 120                 125
   Arg Gly Arg His Gln Ser Leu Gln Cys Arg Ser Pro Glu Glu Gln Cys
       130                 135                 140
   Leu Asp Val Val Thr His Trp Ile Gln Glu Gly Glu Glu Val Leu Glu
   145                 150                 155                 160
   Leu Glu Asn Leu Pro Gln Asn Gly Arg Gln Cys Tyr Ser Cys Lys Gly
                   165                 170                 175
   Asn Ser Thr His Gly Cys Ser Ser Glu Glu Thr Phe Leu Ile Asp Cys
               180                 185                 190
   Arg Gly Pro Met Asn Gln Cys Leu Val Ala Thr Gly Thr His Glu Pro
           195                 200                 205
   Lys Asn Gln Ser Tyr Met Val Arg Gly Cys Ala Thr Ala Ser Met Cys
       210                 215                 220
   Gln His Ala His Leu Gly Asp Ala Phe Ser Met Asn His Ile Asp Val
   225                 230                 235                 240
   Ser Cys Cys Thr Lys Ser Gly Cys Asn His Pro Asp Leu Asp Val Gln
                   245                 250                 255
   Tyr Arg Ser Gly Ala Ala Pro Gln Pro Gly Pro Ala His Leu Ser Leu
               260                 265                 270
   Thr Ile Thr Leu Leu Met Thr Ala Arg Leu Trp Gly Gly Thr Leu Leu
           275                 280                 285
   Trp Thr
       290
<210> 48
   <211> 1360
   <212> DNA
   <213> Homo sapiens
<400> 48
   cagggccgag ccagcccctt caccaccagc cggccgcgcc ccgggaaggg aagtttgtgg 60
   cggaggaggt tcgtacggga ggagggggag gcgcccacgc atctggggct gactcgctct 120
   ttcgcaaaac gtctgggagg agtccctggg gccacaaaac tgcctccttc ctgaggccag 180
   aaggagagaa gacgtgcagg gaccccgcgc acaggagctg ccctcgcgac atgggtcacc 240
   cgccgctgct gccgctgctg ctgctgctcc acacctgcgt cccagcctct tggggcctgc 300
   ggtgcatgca gtgtaagacc aacggggatt gccgtgtgga agagtgcgcc ctgggacagg 360
   acctctgcag gaccacgatc gtgcgcttgt gggaagaagg agaagagctg gagctggtgg 420
   agaaaagctg tacccactca gagaagacca acaggaccct gagctatcgg actggcttga 480
   agatcaccag ccttaccgag gttgtgtgtg ggttagactt gtgcaaccag ggcaactctg 540
   gccgggctgt cacctattcc cgaagccgtt acctcgaatg catttcctgt ggctcatcag 600
   acatgagctg tgagaggggc cggcaccaga gcctgcagtg ccgcagccct gaagaacagt 660
   gcctggatgt ggtgacccac tggatccagg aaggtgaaga agtcctggag cttgaaaatc 720
   tgccgcagaa tggccgccag tgttacagct gcaaggggaa cagcacccat ggatgctcct 780
   ctgaagagac tttcctcatt gactgccgag gccccatgaa tcaatgtctg gtagccaccg 840
   gcactcacga accgaaaaac caaagctata tggtaagagg ctgtgcaacc gcctcaatgt 900
   gccaacatgc ccacctgggt gacgccttca gcatgaacca cattgatgtc tcctgctgta 960
   ctaaaagtgg ctgtaaccac ccagacctgg atgtccagta ccgcagtggg gctgctcctc 1020
   agcctggccc tgcccatctc agcctcacca tcaccctgct aatgactgcc agactgtggg 1080
   gaggcactct cctctggacc taaacctgaa atccccctct ctgccctggc tggatccggg 1140
   ggaccccttt gcccttccct cggctcccag ccctacagac ttgctgtgtg acctcaggcc 1200
   agtgtgccga cctctctggg cctcagtttt cccagctatg aaaacagcta tctcacaaag 1260
   ttgtgtgaag cagaagagaa aagctggagg aaggccgtgg gccaatggga gagctcttgt 1320
   tattattaat attgttgccg ctgttgtgtt gttgttatta                     1360
<210> 49
   <211> 243
   <212> PRT
   <213> Homo sapiens
<400> 49
   Met Arg Pro Gln Gly Pro Ala Ala Ser Pro Gln Arg Leu Arg Gly Leu
    1               5                  10                  15
   Leu Leu Leu Leu Leu Leu Gln Leu Pro Ala Pro Ser Ser Ala Ser Glu
               20                  25                  30
   Ile Pro Lys Gly Lys Gln Lys Ala Gln Leu Arg Gln Arg Glu Val Val
           35                  40                  45
   Asp Leu Tyr Asn Gly Met Cys Leu Gln Gly Pro Ala Gly Val Pro Gly
       50                  55                  60
   Arg Asp Gly Ser Pro Gly Ala Asn Gly Ile Pro Gly Thr Pro Gly Ile
   65                  70                  75                  80
   Pro Gly Arg Asp Gly Phe Lys Gly Glu Lys Gly Glu Cys Leu Arg Glu
                   85                  90                  95
   Ser Phe Glu Glu Ser Trp Thr Pro Asn Tyr Lys Gln Cys Ser Trp Ser
               100                 105                 110
   Ser Leu Asn Tyr Gly Ile Asp Leu Gly Lys Ile Ala Glu Cys Thr Phe
           115                 120                 125
   Thr Lys Met Arg Ser Asn Ser Ala Leu Arg Val Leu Phe Ser Gly Ser
       130                 135                 140
   Leu Arg Leu Lys Cys Arg Asn Ala Cys Cys Gln Arg Trp Tyr Phe Thr
   145                 150                 155                 160
   Phe Asn Gly Ala Glu Cys Ser Gly Pro Leu Pro Ile Glu Ala Ile Ile
                   165                 170                 175
   Tyr Leu Asp Gln Gly Ser Pro Glu Met Asn Ser Thr Ile Asn Ile His
               180                 185                 190
   Arg Thr Ser Ser Val Glu Gly Leu Cys Glu Gly Ile Gly Ala Gly Leu
           195                 200                 205
   Val Asp Val Ala Ile Trp Val Gly Thr Cys Ser Asp Tyr Pro Lys Gly
       210                 215                 220
   Asp Ala Ser Thr Gly Trp Asn Ser Val Ser Arg Ile Ile Ile Glu Glu
   225                 230                 235                 240
   Leu Pro Lys
<210> 50
   <211> 1236
   <212> DNA
   <213> Homo sapiens
<400> 50
   ctgcggcggc ctcggagcgc ggcggagcca gacgctgacc acgttcctct cctcggtctc 60
   ctccgcctcc agctccgcgc tgcccggcag ccgggagcca tgcgacccca gggccccgcc 120
   gcctccccgc agcggctccg cggcctcctg ctgctcctgc tgctgcagct gcccgcgccg 180
   tcgagcgcct ctgagatccc caaggggaag caaaaggcgc agctccggca gagggaggtg 240
   gtggacctgt ataatggaat gtgcttacaa gggccagcag gagtgcctgg tcgagacggg 300
   agccctgggg ccaatggcat tccgggtaca cctgggatcc caggtcggga tggattcaaa 360
   ggagaaaagg gggaatgtct gagggaaagc tttgaggagt cctggacacc caactacaag 420
   cagtgttcat ggagttcatt gaattatggc atagatcttg ggaaaattgc ggagtgtaca 480
   tttacaaaga tgcgttcaaa tagtgctcta agagttttgt tcagtggctc acttcggcta 540
   aaatgcagaa atgcatgctg tcagcgttgg tatttcacat tcaatggagc tgaatgttca 600
   ggacctcttc ccattgaagc tataatttat ttggaccaag gaagccctga aatgaattca 660
   acaattaata ttcatcgcac ttcttctgtg gaaggacttt gtgaaggaat tggtgctgga 720
   ttagtggatg ttgctatctg ggttggcact tgttcagatt acccaaaagg agatgcttct 780
   actggatgga attcagtttc tcgcatcatt attgaagaac taccaaaata aatgctttaa 840
   ttttcatttg ctacctcttt ttttattatg ccttggaatg gttcacttaa atgacatttt 900
   aaataagttt atgtatacat ctgaatgaaa agcaaagcta aatatgttta cagaccaaag 960
   tgtgatttca cactgttttt aaatctagca ttattcattt tgcttcaatc aaaagtggtt 1020
   tcaatatttt ttttagttgg ttagaatact ttcttcatag tcacattctc tcaacctata 1080
   atttggaata ttgttgtggt cttttgtttt ttctcttagt atagcatttt taaaaaaata 1140
   taaaagctac caatctttgt acaatttgta aatgttaaga atttttttta tatctgttaa 1200
   ataaaaatta tttccaacaa aaaaaaaaaa aaaaaa                           1236
<210> 51
   <211> 366
   <212> PRT
   <213> Homo sapiens
<400> 51
   Met Val Leu His Leu Leu Leu Phe Leu Leu Leu Thr Pro Gln Gly Gly
   1               5                   10                  15
   His Ser Cys Gln Gly Leu Glu Leu Ala Arg Glu Leu Val Leu Ala Lys
               20                  25                  30
   Val Arg Ala Leu Phe Leu Asp Ala Leu Gly Pro Pro Ala Val Thr Arg
           35                  40                  45
   Glu Gly Gly Asp Pro Gly Val Arg Arg Leu Pro Arg Arg His Ala Leu
       50                  55                  60
   Gly Gly Phe Thr His Arg Gly Ser Glu Pro Glu Glu Glu Glu Asp Val
   65                  70                  75                  80
   Ser Gln Ala Ile Leu Phe Pro Ala Thr Asp Ala Ser Cys Glu Asp Lys
                   85                  90                  95
   Ser Ala Ala Arg Gly Leu Ala Gln Glu Ala Glu Glu Gly Leu Phe Arg
               100                 105                 110
   Tyr Met Phe Arg Pro Ser Gln His Thr Arg Ser Arg Gln Val Thr Ser
           115                 120                 125
   Ala Gln Leu Trp Phe His Thr Gly Leu Asp Arg Gln Gly Thr Ala Ala
       130                 135                 140
   Ser Asn Ser Ser Glu Pro Leu Leu Gly Leu Leu Ala Leu Ser Pro Gly
   145                 150                 155                 160
   Gly Pro Val Ala Val Pro Met Ser Leu Gly His Ala Pro Pro His Trp
                   165                 170                 175
   Ala Val Leu His Leu Ala Thr Ser Ala Leu Ser Leu Leu Thr His Pro
               180                 185                 190
   Val Leu Val Leu Leu Leu Arg Cys Pro Leu Cys Thr Cys Ser Ala Arg
           195                 200                 205
   Pro Glu Ala Thr Pro Phe Leu Val Ala His Thr Arg Thr Arg Pro Pro
       210                 215                 220
   Ser Gly Gly Glu Arg Ala Arg Arg Ser Thr Pro Leu Met Ser Trp Pro
   225                 230                 235                 240
   Trp Ser Pro Ser Ala Leu Arg Leu Leu Gln Arg Pro Pro Glu Glu Pro
                   245                 250                 255
   Ala Ala His Ala Asn Cys His Arg Val Ala Leu Asn Ile Ser Phe Gln
               260                 265                 270
   Glu Leu Gly Trp Glu Arg Trp Ile Val Tyr Pro Pro Ser Phe Ile Phe
           275                 280                 285
   His Tyr Cys His Gly Gly Cys Gly Leu His Ile Pro Pro Asn Leu Ser
       290                 295                 300
   Leu Pro Val Pro Gly Ala Pro Pro Thr Pro Ala Gln Pro Tyr Ser Leu
   305                 310                 315                 320
   Leu Pro Gly Ala Gln Pro Cys Cys Ala Ala Leu Pro Gly Thr Met Arg
                   325                 330                 335
   Pro Leu His Val Arg Thr Thr Ser Asp Gly Gly Tyr Ser Phe Lys Tyr
               340                 345                 350
   Glu Thr Val Pro Asn Leu Leu Thr Gln His Cys Ala Cys Ile
           355                 360                 365
<210> 52
   <211> 1424
   <212> DNA
   <213> Homo sapiens
<400> 52
   gaaggactgg ggaagactgg atgagaaggg tagaagaggg tgggtgtggg atggggaggg 60
   gagagtggaa aggccctggg cagaccctgg cagaaggggc acggggcagg gtgtgagttc 120
   cccactagca gggccaggtg agctatggtg ctgcacctac tgctcttctt gctgctgacc 180
   ccacagggtg ggcacagctg ccaggggctg gagctggccc gggaacttgt tctggccaag 240
   gtgagggccc tgttcttgga tgccttgggg ccccccgcgg tgaccaggga aggtggggac 300
   cctggagtca ggcggctgcc ccgaagacat gccctggggg gcttcacaca caggggctct 360
   gagcccgagg aagaggagga tgtctcccaa gccatccttt tcccagccac agatgccagc 420
   tgtgaggaca agtcagctgc cagagggctg gcccaggagg ctgaggaggg cctcttcaga 480
   tacatgttcc ggccatccca gcatacacgc agccgccagg tgacttcagc ccagctgtgg 540
   ttccacaccg ggctggacag gcagggcaca gcagcctcca atagctctga gcccctgcta 600
   ggcctgctgg cactgtcacc gggaggaccc gtggctgtgc ccatgtcttt gggccatgct 660
   ccccctcact gggccgtgct gcacctggcc acctctgctc tctctctgct gacccacccc 720
   gtcctggtgc tgctgctgcg ctgtcccctc tgtacctgct cagcccggcc tgaggccacg 780
   cccttcctgg tggcccacac tcggaccaga ccacccagtg gaggggagag agcccgacgc 840
   tcaactcccc tgatgtcctg gccttggtct ccctctgctc tgcgcctgct gcagaggcct 900
   ccggaggaac cggctgccca tgccaactgc cacagagtag cactgaacat ctccttccag 960
   gagctgggct gggaacggtg gatcgtgtac cctcccagtt tcatcttcca ctactgtcat 1020
   ggtggttgtg ggctgcacat cccaccaaac ctgtcccttc cagtccctgg ggctccccct 1080
   accccagccc agccctactc cttgctgcca ggggcccagc cctgctgtgc tgctctccca 1140
   gggaccatga ggcccctaca tgtccgcacc acctcggatg gaggttactc tttcaagtat 1200
   gagacagtgc ccaaccttct cacgcagcac tgtgcttgta tctaagggtg gggggtcttc 1260
   cttcttaatc ccatggctgg tggccacgcc cccaccatca tcagctggga ggaaaggcag 1320
   agttgggaaa tagatggctc ccactcctcc ctcctttcac ttctctgcct atgggctacc 1380
   ctccccaccc cacttctatc tcaataaaga acacagtgca tatg                  1424
<210> 53
   <211> 407
   <212> PRT
   <213> Homo sapiens
<400> 53
   Met Asp Gly Leu Pro Gly Arg Ala Leu Gly Ala Ala Cys Leu Leu Leu
    1               5                  10                  15
   Leu Ala Ala Gly Trp Leu Gly Pro Glu Ala Trp Gly Ser Pro Thr Pro
               20                  25                  30
   Pro Pro Thr Pro Ala Ala Pro Pro Pro Pro Pro Pro Pro Gly Ala Pro
           35                  40                  45
   Gly Gly Ser Gln Asp Thr Cys Thr Ser Cys Gly Gly Phe Arg Arg Pro
       50                  55                  60
   Glu Glu Leu Gly Arg Val Asp Gly Asp Phe Leu Glu Ala Val Lys Arg
   65                  70                  75                  80
   His Ile Leu Ser Arg Leu Gln Met Arg Gly Arg Pro Asn Ile Thr His
                   85                  90                  95
   Ala Val Pro Lys Ala Ala Met Val Thr Ala Leu Arg Lys Leu His Ala
               100                 105                 110
   Gly Lys Val Arg Glu Asp Gly Arg Val Glu Ile Pro His Leu Asp Gly
           115                 120                 125
   His Ala Ser Pro Gly Ala Asp Gly Gln Glu Arg Val Ser Glu Ile Ile
       130                 135                 140
   Ser Phe Ala Glu Thr Asp Gly Leu Ala Ser Ser Arg Val Arg Leu Tyr
   145                 150                 155                 160
   Phe Phe Ile Ser Asn Glu Gly Asn Gln Asn Leu Phe Val Val Gln Ala
                   165                 170                 175
   Ser Leu Trp Leu Tyr Leu Lys Leu Leu Pro Tyr Val Leu Glu Lys Gly
               180                 185                 190
   Ser Arg Arg Lys Val Arg Val Lys Val Tyr Phe Gln Glu Gln Gly His
           195                 200                 205
   Gly Asp Arg Trp Asn Met Val Glu Lys Arg Val Asp Leu Lys Arg Ser
       210                 215                 220
   Gly Trp His Thr Phe Pro Leu Thr Glu Ala Ile Gln Ala Leu Phe Glu
   225                 230                 235                 240
   Arg Gly Glu Arg Arg Leu Asn Leu Asp Val Gln Cys Asp Ser Cys Gln
                   245                 250                 255
   Glu Leu Ala Val Val Pro Val Phe Val Asp Pro Gly Glu Glu Ser His
               260                 265                 270
   Arg Pro Phe Val Val Val Gln Ala Arg Leu Gly Asp Ser Arg His Arg
           275                 280                 285
   Ile Arg Lys Arg Gly Leu Glu Cys Asp Gly Arg Thr Asn Leu Cys Cys
       290                 295                 300
   Arg Gln Gln Phe Phe Ile Asp Phe Arg Leu Ile Gly Trp Asn Asp Trp
   305                 310                 315                 320
   Ile Ile Ala Pro Thr Gly Tyr Tyr Gly Asn Tyr Cys Glu Gly Ser Cys
                   325                 330                 335
   Pro Ala Tyr Leu Ala Gly Val Pro Gly Ser Ala Ser Ser Phe His Thr
               340                 345                 350
   Ala Val Val Asn Gln Tyr Arg Met Arg Gly Leu Asn Pro Gly Thr Val
           355                 360                 365
   Asn Ser Cys Cys Ile Pro Thr Lys Leu Ser Thr Met Ser Met Leu Tyr
       370                 375                 380
   Phe Asp Asp Glu Tyr Asn Ile Val Lys Arg Asp Val Pro Asn Met Ile
   385                 390                 395                 400
   Val Glu Glu Cys Gly Cys Ala
                   405
<210> 54
   <211> 3516
   <212> DNA
   <213> Homo sapiens
<400> 54
   ggatcctgga gacaactttg ccgtgtgacg cgccgggagg actgcagggc ccgcggccga 60
   gggctcggcg ccgcctgtga gcgggcccgc gcggccggct ctcccgggca ccaagcttgc 120
   tccgcgccac tgcccgccgg cccgcggcga ggacgacctg cccgtctccg ccgccggcgg 180
   cccttcctgg cgcgaggcag tgagggcgag gcgctcaggt gcgagcgcgg ggccccgccg 240
   cagcgcccgc cgcagcgccg cgccaagccg cgcccggctc cgctccgggg ggctccagcg 300
   ccttcgcttc cgtctcagcc aagttgcgtg gacccgctct ttcgccacct tccccagccg 360
   ccggccgaac cgccgctccc actgacgctg ctttcgcttc acccgaaccg gggctgcggg 420
   gcccccgacg cggaaaggat ggggagaagg ctgcagatgc cgaggcgccc cgagacgccc 480
   gtgcggcagt gacccgcgac ctccgccccg cccggcgcgc ccctcgggcc cccggggccc 540
   tcggcgcccc ttccctgccg cgcgggaacc cccgaggccc ggccggcccc ctccccctgc 600
   gagccggcgg cagccctccc ggcgggcggg cgggcggagg cccgggcggg cgcgggcgcg 660
   ggcgggggcg gggcggggcg gcgcgcccgg agcccggagc ccggccctgc gctcggctcg 720
   actcggctcg cctcgcggcg ggcgccctcg tcgccagcgg cgcaccatgg acgggctgcc 780
   cggtcgggcg ctgggggccg cctgccttct gctgctggcg gccggctggc tggggcctga 840
   ggcctggggc tcacccacgc ccccgccgac gcctgccgcg ccgccgccac ccccgccacc 900
   cggagccccg ggtggctcgc aggacacctg tacgtcgtgc ggcggcttcc ggcggccaga 960
   ggagctcggc cgagtggacg gcgacttcct ggaggcggtg aagcggcaca tcttgagccg 1020
   cctgcagatg cggggccggc ccaacatcac gcacgccgtg cctaaggccg ccatggtcac 1080
   ggccctgcgc aagctgcacg cgggcaaggt gcgcgaggac ggccgcgtgg agatcccgca 1140
   cctcgacggc cacgccagcc cgggcgccga cggccaggag cgcgtttccg aaatcatcag 1200
   cttcgccgag acagatggcc tcgcctcctc ccgggtccgc ctatacttct tcatctccaa 1260
   cgaaggcaac cagaacctgt ttgtggtcca ggccagcctg tggctttacc tgaaactcct 1320
   gccctacgtc ctggagaagg gcagccggcg gaaggtgcgg gtcaaagtgt acttccagga 1380
   gcagggccac ggtgacaggt ggaacatggt ggagaagagg gtggacctca agcgcagcgg 1440
   ctggcatacc ttcccactca cggaggccat ccaggccttg tttgagcggg gcgagcggcg 1500
   actcaaccta gacgtgcagt gtgacagctg ccaggagctg gccgtggtgc cggtgttcgt 1560
   ggacccaggc gaagagtcgc accgaccctt tgtggtggtg caggctcggc tgggcgacag 1620
   caggcaccgc attcgcaagc gaggcctgga gtgcgatggc cggaccaacc tctgttgcag 1680
   gcaacagttc ttcattgact tccgcctcat cggctggaac gactggatca tagcacccac 1740
   cggctactac ggcaactact gtgagggcag ctgcccagcc tacctggcag gggtccccgg 1800
   ctctgcctcc tccttccaca cggctgtggt gaaccagtac cgcatgcggg gtctgaaccc 1860
   cggcacggtg aactcctgct gcattcccac caagctgagc accatgtcca tgctgtactt 1920
   cgatgatgag tacaacatcg tcaagcggga cgtgcccaac atgattgtgg aggagtgcgg 1980
   ctgcgcctga cagtgcaagg caggggcacg gtggtggggc acggagggca gtcccgggtg 2040
   ggcttcttcc agccccccgc gggaacgggg tacacggtgg gctgagtaca gtcattctgt 2100
   tgggctgtgg agatagtgcc agggtgcggc ctgagatatt tttctacagc ttcatagagc 2160
   aaccagtcaa aaccagagcg agaaccctca actgacatga aatactttaa aatgcacacg 2220
   tagccacgca cagccagacg catcctgcca cccacacagc agcctccagg ataccagcaa 2280
   atggatgcgg tgacaaatgg cagcttagct acaaatgcct gtcagtcgga gagaatgggg 2340
   tgagcagcca ccattccacc agctggcccg gccacgtctc gaagttgcgc cttcccgagc 2400
   acacataaaa gcacaaagac agagacgcag agagagagag agagccacgg agaggaaaag 2460
   cagatgcagg ggtggggagc gcagctcggc ggaggctgcg tgtgccccgt ggcttttacc 2520
   aggcctgctc tgcctggctc gatgtctgct tcttcccagc ctgggatcct tcgtgcttca 2580
   aggcctgggg agcctgtcct tccatgccct tgtcgaggga aagagaccca gaaaggacac 2640
   aacccgtcag agacctggga gcaggggcaa tgaccgtttg actgtttgtg gcttgggcct 2700
   ctgacatgac ttatgtgtgt gtgtgttttt ggggtgggga gggagggaga gaagaggggg 2760
   ctaaatttga tgctttaact gatctccaac agttgacagg tcatccttgc cagttgtata 2820
   actgaaaaag gacttttcta ccaggtatga ccttttaagt gaaaatctga attgttctaa 2880
   atggaaagaa aaaaagttgc aatctgtgcc cttcattggg gacattcctc taggactggt 2940
   ttggggacgg gtgggaatga cccctaggca aggggatgag accgcaggag gaaatggcgg 3000
   ggaggtggca ttcttgaact gctgaggatg gggggtgtcc cctcagcgga ggccaaggga 3060
   ggggagcagc ctagttggtc ttggagagat ggggaaggct ttcagctgat ttgcagaagt 3120
   tgcccatgtg ggcccaacca tcagggctgg ccgtggacgt ggcccctgcc cactcacctg 3180
   cccgcctgcc cgcccgcccg catagcactt gcagacctgc ctgaacgcac atgacatagc 3240
   acttgccgat ctgcgtgtgc ccagaagtgg cccttggccg agcgccgaac tcgctcgccc 3300
   tctagatgtc caagtgccac gtgaactatg caatttaaag ggttgaccca cactagacga 3360
   aactggactc gtacgactct ttttatattt tttatacttg aaatgaaatc ctttgcttct 3420
   tttttaagcg aatgattgct tttaatgttt gcactgattt agttgcatga ttagtcagaa 3480
   actgccattt gaaaaaaaag ttatttttat agcagc                           3516
<210> 55
   <211> 426
   <212> PRT
   <213> Homo sapiens
<400> 55
   Met Pro Leu Leu Trp Leu Arg Gly Phe Leu Leu Ala Ser Cys Trp Ile
   1               5                   10                  15
   Ile Val Arg Ser Ser Pro Thr Pro Gly Ser Glu Gly His Ser Ala Ala
               20                  25                  30
   Pro Asp Cys Pro Ser Cys Ala Leu Ala Ala Leu Pro Lys Asp Val Pro
           35                  40                  45
   Asn Ser Gln Pro Glu Met Val Glu Ala Val Lys Lys His Ile Leu Asn
       50                  55                  60
   Met Leu His Leu Lys Lys Arg Pro Asp Val Thr Gln Pro Val Pro Lys
   65                  70                  75                  80
   Ala Ala Leu Leu Asn Ala Ile Arg Lys Leu His Val Gly Lys Val Gly
                   85                  90                  95
   Glu Asn Gly Tyr Val Glu Ile Glu Asp Asp Ile Gly Arg Arg Ala Glu
               100                 105                 110
   Met Asn Glu Leu Met Glu Gln Thr Ser Glu Ile Ile Thr Phe Ala Glu
           115                 120                 125
   Ser Gly Thr Ala Arg Lys Thr Leu His Phe Glu Ile Ser Lys Glu Gly
       130                 135                 140
   Ser Asp Leu Ser Val Val Glu Arg Ala Glu Val Trp Leu Phe Leu Lys
   145                 150                 155                 160
   Val Pro Lys Ala Asn Arg Thr Arg Thr Lys Val Thr Ile Arg Leu Phe
                   165                 170                 175
   Gln Gln Gln Lys His Pro Gln Gly Ser Leu Asp Thr Gly Glu Glu Ala
               180                 185                 190
   Glu Glu Val Gly Leu Lys Gly Glu Arg Ser Glu Leu Leu Leu Ser Glu
           195                 200                 205
   Lys Val Val Asp Ala Arg Lys Ser Thr Trp His Val Phe Pro Val Ser
       210                 215                 220
   Ser Ser Ile Gln Arg Leu Leu Asp Gln Gly Lys Ser Ser Leu Asp Val
   225                 230                 235                 240
   Arg Ile Ala Cys Glu Gln Cys Gln Glu Ser Gly Ala Ser Leu Val Leu
                   245                 250                 255
   Leu Gly Lys Lys Lys Lys Lys Glu Glu Glu Gly Glu Gly Lys Lys Lys
               260                 265                 270
   Gly Gly Gly Glu Gly Gly Ala Gly Ala Asp Glu Glu Lys Glu Gln Ser
           275                 280                 285
   His Arg Pro Phe Leu Met Leu Gln Ala Arg Gln Ser Glu Asp His Pro
       290                 295                 300
   His Arg Arg Arg Arg Arg Gly Leu Glu Cys Asp Gly Lys Val Asn Ile
   305                 310                 315                 320
   Cys Cys Lys Lys Gln Phe Phe Val Ser Phe Lys Asp Ile Gly Trp Asn
                   325                 330                 335
   Asp Trp Ile Ile Ala Pro Ser Gly Tyr His Ala Asn Tyr Cys Glu Gly
               340                 345                 350
   Glu Cys Pro Ser His Ile Ala Gly Thr Ser Gly Ser Ser Leu Ser Phe
           355                 360                 365
   His Ser Thr Val Ile Asn His Tyr Arg Met Arg Gly His Ser Pro Phe
       370                 375                 380
   Ala Asn Leu Lys Ser Cys Cys Val Pro Thr Lys Leu Arg Pro Met Ser
   385                 390                 395                 400
   Met Leu Tyr Tyr Asp Asp Gly Gln Asn Ile Ile Lys Lys Asp Ile Gln
                   405                 410                 415
   Asn Met Ile Val Glu Glu Cys Gly Cys Ser
               420                 425
<210> 56
   <211> 2175
   <212> DNA
   <213> Homo sapiens
<400> 56
   agtacagtat aaaacttcac agtgccaata ccatgaagag gagctcagac agctcttacc 60
   acatgataca agagccggct ggtggaagag tggggaccag aaagagaatt tgctgaagag 120
   gagaaggaaa aaaaaaacac caaaaaaaaa aataaaaaaa tccacacaca caaaaaaacc 180
   tgcgcgtgag gggggaggaa aagcagggcc ttttaaaaag gcaatcacaa caacttttgc 240
   tgccaggatg cccttgcttt ggctgagagg atttctgttg gcaagttgct ggattatagt 300
   gaggagttcc cccaccccag gatccgaggg gcacagcgcg gcccccgact gtccgtcctg 360
   tgcgctggcc gccctcccaa aggatgtacc caactctcag ccagagatgg tggaggccgt 420
   caagaagcac attttaaaca tgctgcactt gaagaagaga cccgatgtca cccagccggt 480
   acccaaggcg gcgcttctga acgcgatcag aaagcttcat gtgggcaaag tcggggagaa 540
   cgggtatgtg gagatagagg atgacattgg aaggagggca gaaatgaatg aacttatgga 600
   gcagacctcg gagatcatca cgtttgccga gtcaggaaca gccaggaaga cgctgcactt 660
   cgagatttcc aaggaaggca gtgacctgtc agtggtggag cgtgcagaag tctggctctt 720
   cctaaaagtc cccaaggcca acaggaccag gaccaaagtc accatccgcc tcttccagca 780
   gcagaagcac ccgcagggca gcttggacac aggggaagag gccgaggaag tgggcttaaa 840
   gggggagagg agtgaactgt tgctctctga aaaagtagta gacgctcgga agagcacctg 900
   gcatgtcttc cctgtctcca gcagcatcca gcggttgctg gaccagggca agagctccct 960
   ggacgttcgg attgcctgtg agcagtgcca ggagagtggc gccagcttgg ttctcctggg 1020
   caagaagaag aagaaagaag aggaggggga agggaaaaag aagggcggag gtgaaggtgg 1080
   ggcaggagca gatgaggaaa aggagcagtc gcacagacct ttcctcatgc tgcaggcccg 1140
   gcagtctgaa gaccaccctc atcgccggcg tcggcggggc ttggagtgtg atggcaaggt 1200
   caacatctgc tgtaagaaac agttctttgt cagtttcaag gacatcggct ggaatgactg 1260
   gatcattgct ccctctggct atcatgccaa ctactgcgag ggtgagtgcc cgagccatat 1320
   agcaggcacg tccgggtcct cactgtcctt ccactcaaca gtcatcaacc actaccgcat 1380
   gcggggccat agcccctttg ccaacctcaa atcgtgctgt gtgcccacca agctgagacc 1440
   catgtccatg ttgtactatg atgatggtca aaacatcatc aaaaaggaca ttcagaacat 1500
   gatcgtggag gagtgtgggt gctcatagag ttgcccagcc cagggggaaa gggagcaaga 1560
   gttgtccaga gaagacagtg gcaaaatgaa gaaattttta aggtttctga gttaaccaga 1620
   aaaatagaaa ttaaaaacaa aacaaaaaaa aaaacaaaaa aaaacaaaag taaattaaaa 1680
   acaaaacctg atgaaacaga tgaaggaaga tgtggaaaaa atccttagcc aggcctcaga 1740
   gatgaagcag tgaaagagac aggaattggg agggaaaggg agaatggtgt accctttatt 1800
   tcttctgaaa tcacactgat gacatcagtt gtttaaacgg ggtattgtcc tttcccccct 1860
   tgaggttccc ttgtgagcct tgaatcaacc aatctagtct gcagtagtgt ggactagaac 1920
   aacccaaata gcatctagaa agccatgagt ttgaaagggc ccatcacagg cactttccta 1980
   cccaattacc caggtcataa ggtatgtctg tgtgacactt atctctgtgt atatcagcat 2040
   acacacacac acacacacac acacacacac acacaggcat ttccacacat tacatatata 2100
   cacatactgg taaaagaaca atcgtgtgca ggtggtcaca cttccttttt ctgtaccact 2160
   tttgcaacaa aacaa                                                  2175
<210> 57
   <211> 14507
   <212> PRT
   <213> Homo sapiens
<400> 57
   Met Leu Lys Pro Ser Gly Leu Pro Gly Ser Ser Ser Pro Thr Arg Ser
    1              5                   10                  15
   Leu Met Thr Gly Ser Arg Ser Thr Lys Ala Thr Pro Glu Met Asp Ser
               20                  25                  30
   Gly Leu Thr Gly Ala Thr Leu Ser Pro Lys Thr Ser Thr Gly Ala Ile
           35                  40                  45
   Val Val Thr Glu His Thr Leu Pro Phe Thr Ser Pro Asp Lys Thr Leu
       50                  55                  60
   Ala Ser Pro Thr Ser Ser Val Val Gly Arg Thr Thr Gln Ser Leu Gly
   65                  70                  75                  80
   Val Met Ser Ser Ala Leu Pro Glu Ser Thr Ser Arg Gly Met Thr His
                   85                  90                  95
   Ser Glu Gln Arg Thr Ser Pro Ser Leu Ser Pro Gln Val Asn Gly Thr
               100                 105                 110
   Pro Ser Arg Asn Tyr Pro Ala Thr Ser Met Val Ser Gly Leu Ser Ser
           115                 120                 125
   Pro Arg Thr Arg Thr Ser Ser Thr Glu Gly Asn Phe Thr Lys Glu Ala
       130                 135                 140
   Ser Thr Tyr Thr Leu Thr Val Glu Thr Thr Ser Gly Pro Val Thr Glu
   145                 150                 155                 160
   Lys Tyr Thr Val Pro Thr Glu Thr Ser Thr Thr Glu Gly Asp Ser Thr
                   165                 170                 175
   Glu Thr Pro Trp Asp Thr Arg Tyr Ile Pro Val Lys Ile Thr Ser Pro
               180                 185                 190
   Met Lys Thr Phe Ala Asp Ser Thr Ala Ser Lys Glu Asn Ala Pro Val
           195                 200                 205
   Ser Met Thr Pro Ala Glu Thr Thr Val Thr Asp Ser His Thr Pro Gly
       210                 215                 220
   Arg Thr Asn Pro Ser Phe Gly Thr Leu Tyr Ser Ser Phe Leu Asp Leu
   225                 230                 235                 240
   Ser Pro Lys Gly Thr Pro Asn Ser Arg Gly Glu Thr Ser Leu Glu Leu
                   245                 250                 255
   Ile Leu Ser Thr Thr Gly Tyr Pro Phe Ser Ser Pro Glu Pro Gly Ser
               260                 265                 270
   Ala Gly His Ser Arg Ile Ser Thr Ser Ala Pro Leu Ser Ser Ser Ala
           275                 280                 285
   Ser Val Leu Asp Asn Lys Ile Ser Glu Thr Ser Ile Phe Ser Gly Gln
       290                 295                 300
   Ser Leu Thr Ser Pro Leu Ser Pro Gly Val Pro Glu Ala Arg Ala Ser
   305                 310                 315                 320
   Thr Met Pro Asn Ser Ala Ile Pro Phe Ser Met Thr Leu Ser Asn Ala
                   325                 330                 335
   Glu Thr Ser Ala Glu Arg Val Arg Ser Thr Ile Ser Ser Leu Gly Thr
               340                 345                 350
   Pro Ser Ile Ser Thr Lys Gln Thr Ala Glu Thr Ile Leu Thr Phe His
           355                 360                 365
   Ala Phe Ala Glu Thr Met Asp Ile Pro Ser Thr His Ile Ala Lys Thr
       370                 375                 380
   Leu Ala Ser Glu Trp Leu Gly Ser Pro Gly Thr Leu Gly Gly Thr Ser
   385                 390                 395                 400
   Thr Ser Ala Leu Thr Thr Thr Ser Pro Ser Thr Thr Leu Val Ser Glu
                   405                 410                 415
   Glu Thr Asn Thr His His Ser Thr Ser Gly Lys Glu Thr Glu Gly Thr
               420                 425                 430
   Leu Asn Thr Ser Met Thr Pro Leu Glu Thr Ser Ala Pro Gly Glu Glu
           435                 440                 445
   Ser Glu Met Thr Ala Thr Leu Val Pro Thr Leu Gly Phe Thr Thr Leu
       450                 455                 460
   Asp Ser Lys Ile Arg Ser Pro Ser Gln Val Ser Ser Ser His Pro Thr
   465                 470                 475                 480
   Arg Glu Leu Arg Thr Thr Gly Ser Thr Ser Gly Arg Gln Ser Ser Ser
                   485                 490                 495
   Thr Ala Ala His Gly Ser Ser Asp Ile Leu Arg Ala Thr Thr Ser Ser
               500                 505                 510
   Thr Ser Lys Ala Ser Ser Trp Thr Ser Glu Ser Thr Ala Gln Gln Phe
           515                 520                 525
   Ser Glu Pro Gln His Thr Gln Trp Val Glu Thr Ser Pro Ser Met Lys
       530                 535                 540
   Thr Glu Arg Pro Pro Ala Ser Thr Ser Val Ala Ala Pro Ile Thr Thr
   545                 550                 555                 560
   Ser Val Pro Ser Val Val Ser Gly Phe Thr Thr Leu Lys Thr Ser Ser
                   565                 570                 575
   Thr Lys Gly Ile Trp Leu Glu Glu Thr Ser Ala Asp Thr Leu Ile Gly
               580                 585                 590
   Glu Ser Thr Ala Gly Pro Thr Thr His Gln Phe Ala Val Pro Thr Gly
           595                 600                 605
   Ile Ser Met Thr Gly Gly Ser Ser Thr Arg Gly Ser Gln Gly Thr Thr
       610                 615                 620
   His Leu Leu Thr Arg Ala Thr Ala Ser Ser Glu Thr Ser Ala Asp Leu
   625                 630                 635                 640
   Thr Leu Ala Thr Asn Gly Val Pro Val Ser Val Ser Pro Ala Val Ser
                   645                 650                 655
   Lys Thr Ala Ala Gly Ser Ser Pro Pro Gly Gly Thr Lys Pro Ser Tyr
               660                 665                 670
   Thr Met Val Ser Ser Val Ile Pro Glu Thr Ser Ser Leu Gln Ser Ser
           675                 680                 685
   Ala Phe Arg Glu Gly Thr Ser Leu Gly Leu Thr Pro Leu Asn Thr Arg
       690                 695                 700
   His Pro Phe Ser Ser Pro Glu Pro Asp Ser Ala Gly His Thr Lys Ile
   705                 710                 715                 720
   Ser Thr Ser Ile Pro Leu Leu Ser Ser Ala Ser Val Leu Glu Asp Lys
                   725                 730                 735
   Val Ser Ala Thr Ser Thr Phe Ser His His Lys Ala Thr Ser Ser Ile
               740                 745                 750
   Thr Thr Gly Thr Pro Glu Ile Ser Thr Lys Thr Lys Pro Ser Ser Ala
           755                 760                 765
   Val Leu Ser Ser Met Thr Leu Ser Asn Ala Ala Thr Ser Pro Glu Arg
       770                 775                 780
   Val Arg Asn Ala Thr Ser Pro Leu Thr His Pro Ser Pro Ser Gly Glu
   785                 790                 795                 800
   Glu Thr Ala Gly Ser Val Leu Thr Leu Ser Thr Ser Ala Glu Thr Thr
                   805                 810                 815
   Asp Ser Pro Asn Ile His Pro Thr Gly Thr Leu Thr Ser Glu Ser Ser
               820                 825                 830
   Glu Ser Pro Ser Thr Leu Ser Leu Pro Ser Val Ser Gly Val Lys Thr
           835                 840                 845
   Thr Phe Ser Ser Ser Thr Pro Ser Thr His Leu Phe Thr Ser Gly Glu
       850                 855                 860
   Glu Thr Glu Glu Thr Ser Asn Pro Ser Val Ser Gln Pro Glu Thr Ser
   865                 870                 875                 880
   Val Ser Arg Val Arg Thr Thr Leu Ala Ser Thr Ser Val Pro Thr Pro
                   885                 890                 895
   Val Phe Pro Thr Met Asp Thr Trp Pro Thr Arg Ser Ala Gln Phe Ser
               900                 905                 910
   Ser Ser His Leu Val Ser Glu Leu Arg Ala Thr Ser Ser Thr Ser Val
           915                 920                 925
   Thr Asn Ser Thr Gly Ser Ala Leu Pro Lys Ile Ser His Leu Thr Gly
       930                 935                 940
   Thr Ala Thr Met Ser Gln Thr Asn Arg Asp Thr Phe Asn Asp Ser Ala
   945                 950                 955                 960
   Ala Pro Gln Ser Thr Thr Trp Pro Glu Thr Ser Pro Arg Phe Lys Thr
                   965                 970                 975
   Gly Leu Pro Ser Ala Thr Thr Thr Val Ser Thr Ser Ala Thr Ser Leu
               980                 985                 990
   Ser Ala Thr Val Met Val Ser Lys Phe Thr Ser Pro Ala Thr Ser Ser
           995                 1000                1005
   Met Glu Ala Thr Ser Ile Arg Glu Pro Ser Thr Thr Ile Leu Thr Thr
       1010                1015                1020
   Glu Thr Thr Asn Gly Pro Gly Ser Met Ala Val Ala Ser Thr Asn Ile
   1025                1030                1035                1040
   Pro Ile Gly Lys Gly Tyr Ile Thr Glu Gly Arg Leu Asp Thr Ser His
                   1045                1050                1055
   Leu Pro Ile Gly Thr Thr Ala Ser Ser Glu Thr Ser Met Asp Phe Thr
               1060                1065                1070
   Met Ala Lys Glu Ser Val Ser Met Ser Val Ser Pro Ser Gln Ser Met
           1075                1080                1085
   Asp Ala Ala Gly Ser Ser Thr Pro Gly Arg Thr Ser Gln Phe Val Asp
       1090                1095                1100
   Thr Phe Ser Asp Asp Val Tyr His Leu Thr Ser Arg Glu Ile Thr Ile
   1105                1110                1115                1120
   Pro Arg Asp Gly Thr Ser Ser Ala Leu Thr Pro Gln Met Thr Ala Thr
                   1125                1130                1135
   His Pro Pro Ser Pro Asp Pro Gly Ser Ala Arg Ser Thr Trp Leu Gly
               1140                1145                1150
   Ile Leu Ser Ser Ser Pro Ser Ser Pro Thr Pro Lys Val Thr Met Ser
           1155                1160                1165
   Ser Thr Phe Ser Thr Gln Arg Val Thr Thr Ser Met Ile Met Asp Thr
       1170                1175                1180
   Val Glu Thr Ser Arg Trp Asn Met Pro Asn Leu Pro Ser Thr Thr Ser
   1185                1190                1195                1200
   Leu Thr Pro Ser Asn Ile Pro Thr Ser Gly Ala Ile Gly Lys Ser Thr
                   1205                1210                1215
   Leu Val Pro Leu Asp Thr Pro Ser Pro Ala Thr Ser Leu Glu Ala Ser
               1220                1225                1230
   Glu Gly Gly Leu Pro Thr Leu Ser Thr Tyr Pro Glu Ser Thr Asn Thr
           1235                1240                1245
   Pro Ser Ile His Leu Gly Ala His Ala Ser Ser Glu Ser Pro Ser Thr
       1250                1255                1260
   Ile Lys Leu Thr Met Ala Ser Val Val Lys Pro Gly Ser Tyr Thr Pro
   1265                1270                1275                1280
   Leu Thr Phe Pro Ser Ile Glu Thr His Ile His Val Ser Thr Ala Arg
                   1285                1290                1295
   Met Ala Tyr Ser Ser Gly Ser Ser Pro Glu Met Thr Ala Pro Gly Glu
               1300                1305                1310
   Thr Asn Thr Gly Ser Thr Trp Asp Pro Thr Thr Tyr Ile Thr Thr Thr
           1315                1320                1325
   Asp Pro Lys Asp Thr Ser Ser Ala Gln Val Ser Thr Pro His Ser Val
       1330                1335                1340
   Arg Thr Leu Arg Thr Thr Glu Asn His Pro Lys Thr Glu Ser Ala Thr
   1345                1350                1355                1360
   Pro Ala Ala Tyr Ser Gly Ser Pro Lys Ile Ser Ser Ser Pro Asn Leu
                   1365                1370                1375
   Thr Ser Pro Ala Thr Lys Ala Trp Thr Ile Thr Asp Thr Thr Glu His
               1380                1385                1390
   Ser Thr Gln Leu His Tyr Thr Lys Leu Ala Glu Lys Ser Ser Gly Phe
           1395                1400                1405
   Glu Thr Gln Ser Ala Pro Gly Pro Val Ser Val Val Ile Pro Thr Ser
       1410                1415                1420
   Pro Thr Ile Gly Ser Ser Thr Leu Glu Leu Thr Ser Asp Val Pro Gly
   1425                1430                1435                1440
   Glu Pro Leu Val Leu Ala Pro Ser Glu Gln Thr Thr Ile Thr Leu Pro
                   1445                1450                1455
   Met Ala Thr Trp Leu Ser Thr Ser Leu Thr Glu Glu Met Ala Ser Thr
               1460                1465                1470
   Asp Leu Asp Ile Ser Ser Pro Ser Ser Pro Met Ser Thr Phe Ala Ile
           1475                1480                1485
   Phe Pro Pro Met Ser Thr Pro Ser His Glu Leu Ser Lys Ser Glu Ala
       1490                1495                1500
   Asp Thr Ser Ala Ile Arg Asn Thr Asp Ser Thr Thr Leu Asp Gln His
   1505                1510                1515                1520
   Leu Gly Ile Arg Ser Leu Gly Arg Thr Gly Asp Leu Thr Thr Val Pro
                   1525                1530                1535
   Ile Thr Pro Leu Thr Thr Thr Trp Thr Ser Val Ile Glu His Ser Thr
               1540                1545                1550
   Gln Ala Gln Asp Thr Leu Ser Ala Thr Met Ser Pro Thr His Val Thr
           1555                1560                1565
   Gln Ser Leu Lys Asp Gln Thr Ser Ile Pro Ala Ser Ala Ser Pro Ser
       1570                1575                1580
   His Leu Thr Glu Val Tyr Pro Glu Leu Gly Thr Gln Gly Arg Ser Ser
   1585 1590 1595 1600
   Ser Glu Ala Thr Thr Phe Trp Lys Pro Ser Thr Asp Thr Leu Ser Arg
   1605 1610 1615
   Glu Ile Glu Thr Gly Pro Thr Asn Ile Gln Ser Thr Pro Pro Met Asp
   1620 1625 1630
   Asn Thr Thr Thr Gly Ser Ser Ser Ser Gly Val Thr Leu Gly Ile Ala
   1635 1640 1645
   His Leu Pro Ile Gly Thr Ser Ser Pro Ala Glu Thr Ser Thr Asn Met
   1650 1655 1660
   Ala Leu Glu Arg Arg Ser Ser Thr Ala Thr Val Ser Met Ala Gly Thr
   1665 1670 1675 1680
   Met Gly Leu Leu Val Thr Ser Ala Pro Gly Arg Ser Ile Ser Gln Ser
   1685 1690 1695
   Leu Gly Arg Val Ser Ser Val Leu Ser Glu Ser Thr Thr Glu Gly Val
   1700 1705 1710
   Thr Asp Ser Ser Lys Gly Ser Ser Pro Arg Leu Asn Thr Gln Gly Asn
   1715 1720 1725
   Thr Ala Leu Ser Ser Ser Leu Glu Pro Ser Tyr Ala Glu Gly Ser Gln
   1730 1735 1740
   Met Ser Thr Ser Ile Pro Leu Thr Ser Ser Pro Thr Thr Pro Asp Val
   1745 1750 1755 1760
   Glu Phe Ile Gly Gly Ser Thr Phe Trp Thr Lys Glu Val Thr Thr Val
   1765 1770 1775
   Met Thr Ser Asp Ile Ser Lys Ser Ser Ala Arg Thr Glu Ser Ser Ser
   1780 1785 1790
   Ala Thr Leu Met Ser Thr Ala Leu Gly Ser Thr Glu Asn Thr Gly Lys
   1795 1800 1805
   Glu Lys Leu Arg Thr Ala Ser Met Asp Leu Pro Ser Pro Thr Pro Ser
   1810 1815 1820
   Met Glu Val Thr Pro Trp Ile Ser Leu Thr Leu Ser Asn Ala Pro Asn
   1825 1830 1835 1840
   Thr Thr Asp Ser Leu Asp Leu Ser His Gly Val His Thr Ser Ser Ala
   1845 1850 1855
   Gly Thr Leu Ala Thr Asp Arg Ser Leu Asn Thr Gly Val Thr Arg Ala
   1860 1865 1870
   Ser Arg Leu Glu Asn Gly Ser Asp Thr Ser Ser Lys Ser Leu Ser Met
   1875 1880 1885
   Gly Asn Ser Thr His Thr Ser Met Thr Tyr Thr Glu Lys Ser Glu Val
   1890 1895 1900
   Ser Ser Ser Ile His Pro Arg Pro Glu Thr Ser Ala Pro Gly Ala Glu
   1905 1910 1915 1920
   Thr Thr Leu Thr Ser Thr Pro Gly Asn Arg Ala Ile Ser Leu Thr Leu
   1925 1930 1935
   Pro Phe Ser Ser Ile Pro Val Glu Glu Val Ile Ser Thr Gly Ile Thr
   1940 1945 1950
   Ser Gly Pro Asp Ile Asn Ser Ala Pro Met Thr His Ser Pro Ile Thr
   1955 1960 1965
   Pro Pro Thr Ile Val Trp Thr Ser Thr Gly Thr Ile Glu Gln Ser Thr
   1970 1975 1980
   Gln Pro Leu His Ala Val Ser Ser Glu Lys Val Ser Val Gln Thr Gln
   1985 1990 1995 2000
   Ser Thr Pro Tyr Val Asn Ser Val Ala Val Ser Ala Ser Pro Thr His
   2005 2010 2015
   Glu Asn Ser Val Ser Ser Gly Ser Ser Thr Ser Ser Pro Tyr Ser Ser
   2020 2025 2030
   Ala Ser Leu Glu Ser Leu Asp Ser Thr Ile Ser Arg Arg Asn Ala Ile
   2035 2040 2045
   Thr Ser Trp Leu Trp Asp Leu Thr Thr Ser Leu Pro Thr Thr Thr Trp
   2050 2055 2060
   Pro Ser Thr Ser Leu Ser Glu Ala Leu Ser Ser Gly His Ser Gly Val
   2065 2070 2075 2080
   Ser Asn Pro Ser Ser Thr Thr Thr Glu Phe Pro Leu Phe Ser Ala Ala
   2085 2090 2095
   Ser Thr Ser Ala Ala Lys Gln Arg Asn Pro Glu Thr Glu Thr His Gly
   2100 2105 2110
   Pro Gln Asn Thr Ala Ala Ser Thr Leu Asn Thr Asp Ala Ser Ser Val
   2115 2120 2125
   Thr Gly Leu Ser Glu Thr Pro Val Gly Ala Ser Ile Ser Ser Glu Val
   2130 2135 2140
   Pro Leu Pro Met Ala Ile Thr Ser Arg Ser Asp Val Ser Gly Leu Thr
   2145 2150 2155 2160
   Ser Glu Ser Thr Ala Asn Pro Ser Leu Gly Thr Ala Ser Ser Ala Gly
   2165 2170 2175
   Thr Lys Leu Thr Arg Thr Ile Ser Leu Pro Thr Ser Glu Ser Leu Val
   2180 2185 2190
   Ser Phe Arg Met Asn Lys Asp Pro Trp Thr Val Ser Ile Pro Leu Gly
   2195 2200 2205
   Ser His Pro Thr Thr Asn Thr Glu Thr Ser Ile Pro Val Asn Ser Ala
   2210 2215 2220
   Gly Pro Pro Gly Leu Ser Thr Val Ala Ser Asp Val Ile Asp Thr Pro
   2225 2230 2235 2240
   Ser Asp Gly Ala Glu Ser Ile Pro Thr Val Ser Phe Ser Pro Ser Pro
   2245 2250 2255
   Asp Thr Glu Val Thr Thr Ile Ser His Phe Pro Glu Lys Thr Thr His
   2260 2265 2270
   Ser Phe Arg Thr Ile Ser Ser Leu Thr His Glu Leu Thr Ser Arg Val
   2275 2280 2285
   Thr Pro Ile Pro Gly Asp Trp Met Ser Ser Ala Met Ser Thr Lys Pro
   2290 2295 2300
   Thr Gly Ala Ser Pro Ser Ile Thr Leu Gly Glu Arg Arg Thr Ile Thr
   2305 2310 2315 2320
   Ser Ala Ala Pro Thr Thr Ser Pro Ile Val Leu Thr Ala Ser Phe Thr
   2325 2330 2335
   Glu Thr Ser Thr Val Ser Leu Asp Asn Glu Thr Thr Val Lys Thr Ser
   2340 2345 2350
   Asp Ile Leu Asp Ala Arg Lys Thr Asn Glu Leu Pro Ser Asp Ser Ser
   2355 2360 2365
   Ser Ser Ser Asp Leu Ile Asn Thr Ser Ile Ala Ser Ser Thr Met Asp
   2370 2375 2380
   Val Thr Lys Thr Ala Ser Ile Ser Pro Thr Ser Ile Ser Gly Met Thr
   2385 2390 2395 2400
   Ala Ser Ser Ser Pro Ser Leu Phe Ser Ser Asp Arg Pro Gln Val Pro
   2405 2410 2415
   Thr Ser Thr Thr Glu Thr Asn Thr Ala Thr Ser Pro Ser Val Ser Ser
   2420 2425 2430
   Asn Thr Tyr Ser Leu Asp Gly Gly Ser Asn Val Gly Gly Thr Pro Ser
   2435 2440 2445
   Thr Leu Pro Pro Phe Thr Ile Thr His Pro Val Glu Thr Ser Ser Ala
   2450 2455 2460
   Leu Leu Ala Trp Ser Arg Pro Val Arg Thr Phe Ser Thr Met Val Ser
   2465 2470 2475 2480
   Thr Asp Thr Ala Ser Gly Glu Asn Pro Thr Ser Ser Asn Ser Val Val
   2485 2490 2495
   Thr Ser Val Pro Ala Pro Gly Thr Trp Thr Ser Val Gly Ser Thr Thr
   2500 2505 2510
   Asp Leu Pro Ala Met Gly Phe Leu Lys Thr Ser Pro Ala Gly Glu Ala
   2515 2520 2525
   His Ser Leu Leu Ala Ser Thr Ile Glu Pro Ala Thr Ala Phe Thr Pro
   2530 2535 2540
   His Leu Ser Ala Ala Val Val Thr Gly Ser Ser Ala Thr Ser Glu Ala
   2545 2550 2555 2560
   Ser Leu Leu Thr Thr Ser Glu Ser Lys Ala Ile His Ser Ser Pro Gln
   2565 2570 2575
   Thr Pro Thr Thr Pro Thr Ser Gly Ala Asn Trp Glu Thr Ser Ala Thr
   2580 2585 2590
   Pro Glu Ser Leu Leu Val Val Thr Glu Thr Ser Asp Thr Thr Leu Thr
   2595 2600 2605
   Ser Lys Ile Leu Val Thr Asp Thr Ile Leu Phe Ser Thr Val Ser Thr
   2610 2615 2620
   Pro Pro Ser Lys Phe Pro Ser Thr Gly Thr Leu Ser Gly Ala Ser Phe
   2625 2630 2635 2640
   Pro Thr Leu Leu Pro Asp Thr Pro Ala Ile Pro Leu Thr Ala Thr Glu
   2645 2650 2655
   Pro Thr Ser Ser Leu Ala Thr Ser Phe Asp Ser Thr Pro Leu Val Thr
   2660 2665 2670
   Ile Ala Ser Asp Ser Leu Gly Thr Val Pro Glu Thr Thr Leu Thr Met
   2675 2680 2685
   Ser Glu Thr Ser Asn Gly Asp Ala Leu Val Leu Lys Thr Val Ser Asn
   2690 2695 2700
   Pro Asp Arg Ser Ile Pro Gly Ile Thr Ile Gln Gly Val Thr Glu Ser
   2705 2710 2715 2720
   Pro Leu His Pro Ser Ser Thr Ser Pro Ser Lys Ile Val Ala Pro Arg
   2725 2730 2735
   Asn Thr Thr Tyr Glu Gly Ser Ile Thr Val Ala Leu Ser Thr Leu Pro
   2740 2745 2750
   Ala Gly Thr Thr Gly Ser Leu Val Phe Ser Gln Ser Ser Glu Asn Ser
   2755 2760 2765
   Glu Thr Thr Ala Leu Val Asp Ser Ser Ala Gly Leu Glu Arg Ala Ser
   2770 2775 2780
   Val Met Pro Leu Thr Thr Gly Ser Gln Gly Met Ala Ser Ser Gly Gly
   2785 2790 2795 2800
   Ile Arg Ser Gly Ser Thr His Ser Thr Gly Thr Lys Thr Phe Ser Ser
   2805 2810 2815
   Leu Pro Leu Thr Met Asn Pro Gly Glu Val Thr Ala Met Ser Glu Ile
   2820 2825 2830
   Thr Thr Asn Arg Leu Thr Ala Thr Gln Ser Thr Ala Pro Lys Gly Ile
   2835 2840 2845
   Pro Val Lys Pro Thr Ser Ala Glu Ser Gly Leu Leu Thr Pro Val Ser
   2850 2855 2860
   Ala Ser Ser Ser Pro Ser Lys Ala Phe Ala Ser Leu Thr Thr Ala Pro
   2865 2870 2875 2880
   Pro Thr Trp Gly Ile Pro Gln Ser Thr Leu Thr Phe Glu Phe Ser Glu
   2885 2890 2895
   Val Pro Ser Leu Asp Thr Lys Ser Ala Ser Leu Pro Thr Pro Gly Gln
   2900 2905 2910
   Ser Leu Asn Thr Ile Pro Asp Ser Asp Ala Ser Thr Ala Ser Ser Ser
   2915 2920 2925
   Leu Ser Lys Ser Pro Glu Lys Asn Pro Arg Ala Arg Met Met Thr Ser
   2930 2935 2940
   Thr Lys Ala Ile Ser Ala Ser Ser Phe Gln Ser Thr Gly Phe Thr Glu
   2945 2950 2955 2960
   Thr Pro Glu Gly Ser Ala Ser Pro Ser Met Ala Gly His Glu Pro Arg
   2965 2970 2975
   Val Pro Thr Ser Gly Thr Gly Asp Pro Arg Tyr Ala Ser Glu Ser Met
   2980 2985 2990
   Ser Tyr Pro Asp Pro Ser Lys Ala Ser Ser Ala Met Thr Ser Thr Ser
   2995 3000 3005
   Leu Ala Ser Lys Leu Thr Thr Leu Phe Ser Thr Gly Gln Ala Ala Arg
   3010 3015 3020
   Ser Gly Ser Ser Ser Ser Pro Ile Ser Leu Ser Thr Glu Lys Glu Thr
   3025 3030 3035 3040
   Ser Phe Leu Ser Pro Thr Ala Ser Thr Ser Arg Lys Thr Ser Leu Phe
                   3045                3050                3055
   Leu Gly Pro Ser Met Ala Arg Gln Pro Asn Ile Leu Val His Leu Gln
               3060                3065                3070
   Thr Ser Ala Leu Thr Leu Ser Pro Thr Ser Thr Leu Asn Met Ser Gln
           3075                3080                3085
   Glu Glu Pro Pro Glu Leu Thr Ser Ser Gln Thr Ile Ala Glu Glu Glu
       3090                3095                3100
   Gly Thr Thr Ala Glu Thr Gln Thr Leu Thr Phe Thr Pro Ser Glu Thr
   3105                3110                3115                3120
   Pro Thr Ser Leu Leu Pro Val Ser Ser Pro Thr Glu Pro Thr Ala Arg
                   3125                3130                3135
   Arg Lys Ser Ser Pro Glu Thr Trp Ala Ser Ser Ile Ser Val Pro Ala
               3140                3145                3150
   Lys Thr Ser Leu Val Glu Thr Thr Asp Gly Thr Leu Val Thr Thr Ile
           3155                3160                3165
   Lys Met Ser Ser Gln Ala Ala Gln Gly Asn Ser Thr Trp Pro Ala Pro
       3170                3175                3180
   Ala Glu Glu Thr Gly Ser Ser Pro Ala Gly Thr Ser Pro Gly Ser Pro
   3185                3190                3195                3200
   Glu Met Ser Thr Thr Leu Lys Ile Met Ser Ser Lys Glu Pro Ser Ile
                   3205                3210                3215
   Ser Pro Glu Ile Arg Ser Thr Val Arg Asn Ser Pro Trp Lys Thr Pro
               3220                3225                3230
   Glu Thr Thr Val Pro Met Glu Thr Thr Val Glu Pro Val Thr Leu Gln
           3235                3240                3245
   Ser Thr Ala Leu Gly Ser Gly Ser Thr Ser Ile Ser His Leu Pro Thr
       3250                3255                3260
   Gly Thr Thr Ser Pro Thr Lys Ser Pro Thr Glu Asn Met Leu Ala Thr
   3265                3270                3275                3280
   Glu Arg Val Ser Leu Ser Pro Ser Pro Pro Glu Ala Trp Thr Asn Leu
                   3285                3290                3295
   Tyr Ser Gly Thr Pro Gly Gly Thr Arg Gln Ser Leu Ala Thr Met Ser
               3300                3305                3310
   Ser Val Ser Leu Glu Ser Pro Thr Ala Arg Ser Ile Thr Gly Thr Gly
           3315                3320                3325
   Gln Gln Ser Ser Pro Glu Leu Val Ser Lys Thr Thr Gly Met Glu Phe
       3330                3335                3340
   Ser Met Trp His Gly Ser Thr Gly Gly Thr Thr Gly Asp Thr His Val
   3345                3350                3355                3360
   Ser Leu Ser Thr Ser Ser Asn Ile Leu Glu Asp Pro Val Thr Ser Pro
                   3365                3370                3375
   Asn Ser Val Ser Ser Leu Thr Asp Lys Ser Lys His Lys Thr Glu Thr
               3380                3385                3390
   Trp Val Ser Thr Thr Ala Ile Pro Ser Thr Val Leu Asn Asn Lys Ile
           3395                3400                3405
   Met Ala Ala Glu Gln Gln Thr Ser Arg Ser Val Asp Glu Ala Tyr Ser
       3410                3415                3420
   Ser Thr Ser Ser Trp Ser Asp Gln Thr Ser Gly Ser Asp Ile Thr Leu
   3425                3430                3435                3440
   Gly Ala Ser Pro Asp Val Thr Asn Thr Leu Tyr Ile Thr Ser Thr Ala
                   3445                3450                3455
   Gln Thr Thr Ser Leu Val Ser Leu Pro Ser Gly Asp Gln Gly Ile Thr
               3460                3465                3470
   Ser Leu Thr Asn Pro Ser Gly Gly Lys Thr Ser Ser Ala Ser Ser Val
           3475                3480                3485
   Thr Ser Pro Ser Ile Gly Leu Glu Thr Leu Arg Ala Asn Val Ser Ala
       3490                3495                3500
   Val Lys Ser Asp Ile Ala Pro Thr Ala Gly His Leu Ser Gln Thr Ser
   3505                3510                3515                3520
   Ser Pro Ala Glu Val Ser Ile Leu Asp Val Thr Thr Ala Pro Thr Pro
                   3525                3530                3535
   Gly Ile Ser Thr Thr Ile Thr Thr Met Gly Thr Asn Ser Ile Ser Thr
               3540                3545                3550
   Thr Thr Pro Asn Pro Glu Val Gly Met Ser Thr Met Asp Ser Thr Pro
           3555                3560                3565
   Ala Thr Glu Arg Arg Thr Thr Ser Thr Glu His Pro Ser Thr Trp Ser
       3570                3575                3580
   Ser Thr Ala Ala Ser Asp Ser Trp Thr Val Thr Asp Met Thr Ser Asn
   3585                3590                3595                3600
   Leu Lys Val Ala Arg Ser Pro Gly Thr Ile Ser Thr Met His Thr Thr
                   3605                3610                3615
   Ser Phe Leu Ala Ser Ser Thr Glu Leu Asp Ser Met Ser Thr Pro His
               3620                3625                3630
   Gly Arg Ile Thr Val Ile Gly Thr Ser Leu Val Thr Pro Ser Ser Asp
           3635                3640                3645
   Ala Ser Ala Val Lys Thr Glu Thr Ser Thr Ser Glu Arg Thr Leu Ser
       3650                3655                3660
   Pro Ser Asp Thr Thr Ala Ser Thr Pro Ile Ser Thr Phe Ser Arg Val
   3665                3670                3675                3680
   Gln Arg Met Ser Ile Ser Val Pro Asp Ile Leu Ser Thr Ser Trp Thr
                   3685                3690                3695
   Pro Ser Ser Thr Glu Ala Glu Asp Val Pro Val Ser Met Val Ser Thr
               3700                3705                3710
   Asp His Ala Ser Thr Lys Thr Asp Pro Asn Thr Pro Leu Ser Thr Phe
           3715                3720                3725
   Leu Phe Asp Ser Leu Ser Thr Leu Asp Trp Asp Thr Gly Arg Ser Leu
       3730                3735                3740
   Ser Ser Ala Thr Ala Thr Thr Ser Ala Pro Gln Gly Ala Thr Thr Pro
   3745                3750                3755                3760
   Gln Glu Leu Thr Leu Glu Thr Met Ile Ser Pro Ala Thr Ser Gln Leu
                   3765                3770                3775
   Pro Phe Ser Ile Gly His Ile Thr Ser Ala Val Thr Pro Ala Ala Met
               3780                3785                3790
   Ala Arg Ser Ser Gly Val Thr Phe Ser Arg Pro Asp Pro Thr Ser Lys
           3795                3800                3805
   Lys Ala Glu Gln Thr Ser Thr Gln Leu Pro Thr Thr Thr Ser Ala His
       3810                3815                3820
   Pro Gly Gln Val Pro Arg Ser Ala Ala Thr Thr Leu Asp Val Ile Pro
   3825                3830                3835                3840
   His Thr Ala Lys Thr Pro Asp Ala Thr Phe Gln Arg Gln Gly Gln Thr
                   3845                3850                3855
   Ala Leu Thr Thr Glu Ala Arg Ala Thr Ser Asp Ser Trp Asn Glu Lys
               3860                3865                3870
   Glu Lys Ser Thr Pro Ser Ala Pro Trp Ile Thr Glu Met Met Asn Ser
           3875                3880                3885
   Val Ser Glu Asp Thr Ile Lys Glu Val Thr Ser Ser Ser Ser Val Leu
       3890                3895                3900
   Arg Thr Leu Asn Thr Leu Asp Ile Asn Leu Glu Ser Gly Thr Thr Ser
   3905                3910                3915                3920
   Ser Pro Ser Trp Lys Ser Ser Pro Tyr Glu Arg Ile Ala Pro Ser Glu
                   3925                3930                3935
   Ser Thr Thr Asp Lys Glu Ala Ile His Pro Ser Thr Asn Thr Val Glu
               3940                3945                3950
   Thr Thr Gly Trp Val Thr Ser Ser Glu His Ala Ser His Ser Thr Ile
           3955                3960                3965
   Pro Ala His Ser Ala Ser Ser Lys Leu Thr Ser Pro Val Val Thr Thr
       3970                3975                3980
   Ser Thr Arg Glu Gln Ala Ile Val Ser Met Ser Thr Thr Thr Trp Pro
   3985                3990                3995                4000
   Glu Ser Thr Arg Ala Arg Thr Glu Pro Asn Ser Phe Leu Thr Ile Glu
                   4005                4010                4015
   Leu Arg Asp Val Ser Pro Tyr Met Asp Thr Ser Ser Thr Thr Gln Thr
               4020                4025                4030
   Ser Ile Ile Ser Ser Pro Gly Ser Thr Ala Ile Thr Lys Gly Pro Arg
           4035                4040                4045
   Thr Glu Ile Thr Ser Ser Lys Arg Ile Ser Ser Ser Phe Leu Ala Gln
       4050                4055                4060
   Ser Met Arg Ser Ser Asp Ser Pro Ser Glu Ala Ile Thr Arg Leu Ser
   4065                4070                4075                4080
   Asn Phe Pro Ala Met Thr Glu Ser Gly Gly Met Ile Leu Ala Met Gln
                   4085                4090                4095
   Thr Ser Pro Pro Gly Ala Thr Ser Leu Ser Ala Pro Thr Leu Asp Thr
               4100                4105                4110
   Ser Ala Thr Ala Ser Trp Thr Gly Thr Pro Leu Ala Thr Thr Gln Arg
           4115                4120                4125
   Phe Thr Tyr Ser Glu Lys Thr Thr Leu Phe Ser Lys Gly Pro Glu Asp
       4130                4135                4140
   Thr Ser Gln Pro Ser Pro Pro Ser Val Glu Glu Thr Ser Ser Ser Ser
   4145                4150                4155                4160
   Ser Leu Val Pro Ile His Ala Thr Thr Ser Pro Ser Asn Ile Leu Leu
                   4165                4170                4175
   Thr Ser Gln Gly His Ser Pro Ser Ser Thr Pro Pro Val Thr Ser Val
               4180                4185                4190
   Phe Leu Ser Glu Thr Ser Gly Leu Gly Lys Thr Thr Asp Met Ser Arg
           4195                4200                4205
   Ile Ser Leu Glu Pro Gly Thr Ser Leu Pro Pro Asn Leu Ser Ser Thr
       4210                4215                4220
   Ala Gly Glu Ala Leu Ser Thr Tyr Glu Ala Ser Arg Asp Thr Lys Ala
   4225                4230                4235                4240
   Ile His His Ser Ala Asp Thr Ala Val Thr Asn Met Glu Ala Thr Ser
                   4245                4250                4255
   Ser Glu Tyr Ser Pro Ile Pro Gly His Thr Lys Pro Ser Lys Ala Thr
               4260                4265                4270
   Ser Pro Leu Val Thr Ser His Ile Met Gly Asp Ile Thr Ser Ser Thr
           4275                4280                4285
   Ser Val Phe Gly Ser Ser Glu Thr Thr Glu Ile Glu Thr Val Ser Ser
       4290                4295                4300
   Val Asn Gln Gly Leu Gln Glu Arg Ser Thr Ser Gln Val Ala Ser Ser
   4305                4310                4315                4320
   Ala Thr Glu Thr Ser Thr Val Ile Thr His Val Ser Ser Gly Asp Ala
                   4325                4330                4335
   Thr Thr His Val Thr Lys Thr Gln Ala Thr Phe Ser Ser Gly Thr Ser
               4340                4345                4350
   Ile Ser Ser Pro His Gln Phe Ile Thr Ser Thr Asn Thr Phe Thr Asp
           4355                4360                4365
   Val Ser Thr Asn Pro Ser Thr Ser Leu Ile Met Thr Glu Ser Ser Gly
       4370                4375                4380
   Val Thr Ile Thr Thr Gln Thr Gly Pro Thr Gly Ala Ala Thr Gln Gly
   4385                4390                4395                4400
   Pro Tyr Leu Leu Asp Thr Ser Thr Met Pro Tyr Leu Thr Glu Thr Pro
                   4405                4410                4415
   Leu Ala Val Thr Pro Asp Phe Met Gln Ser Glu Lys Thr Thr Leu Ile
               4420                4425                4430
   Ser Lys Gly Pro Lys Asp Val Ser Trp Thr Ser Pro Pro Ser Val Ala
           4435                4440                4445
   Glu Thr Ser Tyr Pro Ser Ser Leu Thr Pro Phe Leu Val Thr Thr Ile
       4450                4455                4460
   Pro Pro Ala Thr Ser Thr Leu Gln Gly Gln His Thr Ser Ser Pro Val
   4465                4470                4475                4480
   Ser Ala Thr Ser Val Leu Thr Ser Gly Leu Val Lys Thr Thr Asp Met
                   4485                4490                4495
   Leu Asn Thr Ser Met Glu Pro Val Thr Asn Ser Pro Gln Asn Leu Asn
               4500                4505                4510
   Asn Pro Ser Asn Glu Ile Leu Ala Thr Leu Ala Ala Thr Thr Asp Ile
   4515 4520 4525
   Glu Thr Ile His Pro Ser Ile Asn Lys Ala Val Thr Asn Met Gly Thr
   4530 4535 4540
   Ala Ser Ser Ala His Val Leu His Ser Thr Leu Pro Val Ser Ser Glu
   4545 4550 4555 4560
   Pro Ser Thr Ala Thr Ser Pro Met Val Pro Ala Ser Ser Met Gly Asp
   4565 4570 4575
   Ala Leu Ala Ser Ile Ser Ile Pro Gly Ser Glu Thr Thr Asp Ile Glu
   4580 4585 4590
   Gly Glu Pro Thr Ser Ser Leu Thr Ala Gly Arg Lys Glu Asn Ser Thr
   4595 4600 4605
   Leu Gln Glu Met Asn Ser Thr Thr Glu Ser Asn Ile Ile Leu Ser Asn
   4610 4615 4620
   Val Ser Val Gly Ala Ile Thr Glu Ala Thr Lys Met Glu Val Pro Ser
   4625 4630 4635 4640
   Phe Asp Ala Thr Phe Ile Pro Thr Pro Ala Gln Ser Thr Lys Phe Pro
   4645 4650 4655
   Asp Ile Phe Ser Val Ala Ser Ser Arg Leu Ser Asn Ser Pro Pro Met
   4660 4665 4670
   Thr Ile Ser Thr His Met Thr Thr Thr Gln Thr Gly Ser Ser Gly Ala
   4675 4680 4685
   Thr Ser Lys Ile Pro Leu Ala Leu Asp Thr Ser Thr Leu Glu Thr Ser
   4690 4695 4700
   Ala Gly Thr Pro Ser Val Val Thr Glu Gly Phe Ala His Ser Lys Ile
   4705 4710 4715 4720
   Thr Thr Ala Met Asn Asn Asp Val Lys Asp Val Ser Gln Thr Asn Pro
   4725 4730 4735
   Pro Phe Gln Asp Glu Ala Ser Ser Pro Ser Ser Gln Ala Pro Val Leu
   4740 4745 4750
   Val Thr Thr Leu Pro Ser Ser Val Ala Phe Thr Pro Gln Trp His Ser
   4755 4760 4765
   Thr Ser Ser Pro Val Ser Met Ser Ser Val Leu Thr Ser Ser Leu Val
   4770 4775 4780
   Lys Thr Ala Gly Lys Val Asp Thr Ser Leu Glu Thr Val Thr Ser Ser
   4785 4790 4795 4800
   Pro Gln Ser Met Ser Asn Thr Leu Asp Asp Ile Ser Val Thr Ser Ala
   4805 4810 4815
   Ala Thr Thr Asp Ile Glu Thr Thr His Pro Ser Ile Asn Thr Val Val
   4820 4825 4830
   Thr Asn Val Gly Thr Thr Gly Ser Ala Phe Glu Ser His Ser Thr Val
   4835 4840 4845
   Ser Ala Tyr Pro Glu Pro Ser Lys Val Thr Ser Pro Asn Val Thr Thr
   4850 4855 4860
   Ser Thr Met Glu Asp Thr Thr Ile Ser Arg Ser Ile Pro Lys Ser Ser
   4865 4870 4875 4880
   Lys Thr Thr Arg Thr Glu Thr Glu Thr Thr Ser Ser Leu Thr Pro Lys
   4885 4890 4895
   Leu Arg Glu Thr Ser Ile Ser Gln Glu Ile Thr Ser Ser Thr Glu Thr
   4900 4905 4910
   Ser Thr Val Pro Tyr Lys Glu Leu Thr Gly Ala Thr Thr Glu Val Ser
   4915 4920 4925
   Arg Thr Asp Val Thr Ser Ser Ser Ser Thr Ser Phe Pro Gly Pro Asp
   4930 4935 4940
   Gln Ser Thr Val Ser Leu Asp Ile Ser Thr Glu Thr Asn Thr Arg Leu
   4945 4950 4955 4960
   Ser Thr Ser Pro Ile Met Thr Glu Ser Ala Glu Ile Thr Ile Thr Thr
   4965 4970 4975
   Gln Thr Gly Pro His Gly Ala Thr Ser Gln Asp Thr Phe Thr Met Asp
   4980 4985 4990
   Pro Ser Asn Thr Thr Pro Gln Ala Gly Ile His Ser Ala Met Thr His
           4995                5000                5005
   Gly Phe Ser Gln Leu Asp Val Thr Thr Leu Met Ser Arg Ile Pro Gln
       5010                5015                5020
   Asp Val Ser Trp Thr Ser Pro Pro Ser Val Asp Lys Thr Ser Ser Pro
   5025                5030                5035                5040
   Ser Ser Phe Leu Ser Ser Pro Ala Met Thr Thr Pro Ser Leu Ile Ser
                   5045                5050                5055
   Ser Thr Leu Pro Glu Asp Lys Leu Ser Ser Pro Met Thr Ser Leu Leu
               5060                5065                5070
   Thr Ser Gly Leu Val Lys Ile Thr Asp Ile Leu Arg Thr Arg Leu Glu
           5075                5080                5085
   Pro Val Thr Ser Ser Leu Pro Asn Phe Ser Ser Thr Ser Asp Lys Ile
       5090                5095                5100
   Leu Ala Thr Ser Lys Asp Ser Lys Asp Thr Lys Glu Ile Phe Pro Ser
   5105                5110                5115                5120
   Ile Asn Thr Glu Glu Thr Asn Val Lys Ala Asn Asn Ser Gly His Glu
                   5125                5130                5135
   Ser His Ser Pro Ala Leu Ala Asp Ser Glu Thr Pro Lys Ala Thr Thr
               5140                5145                5150
   Gln Met Val Ile Thr Thr Thr Val Gly Asp Pro Ala Pro Ser Thr Ser
           5155                5160                5165
   Met Pro Val His Gly Ser Ser Glu Thr Thr Asn Ile Lys Arg Glu Pro
       5170                5175                5180
   Thr Tyr Phe Leu Thr Pro Arg Leu Arg Glu Thr Ser Thr Ser Gln Glu
   5185                5190                5195                5200
   Ser Ser Phe Pro Thr Asp Thr Ser Phe Leu Leu Ser Lys Val Pro Thr
                   5205                5210                5215
   Gly Thr Ile Thr Glu Val Ser Ser Thr Gly Val Asn Ser Ser Ser Lys
               5220                5225                5230
   Ile Ser Thr Pro Asp His Asp Lys Ser Thr Val Pro Pro Asp Thr Phe
           5235                5240                5245
   Thr Gly Glu Ile Pro Arg Val Phe Thr Ser Ser Ile Lys Thr Lys Ser
       5250                5255                5260
   Ala Glu Met Thr Ile Thr Thr Gln Ala Ser Pro Pro Glu Ser Ala Ser
   5265                5270                5275                5280
   His Ser Thr Leu Pro Leu Asp Thr Ser Thr Thr Leu Ser Gln Gly Gly
                   5285                5290                5295
   Thr His Ser Thr Val Thr Gln Gly Phe Pro Tyr Ser Glu Val Thr Thr
               5300                5305                5310
   Leu Met Gly Met Gly Pro Gly Asn Val Ser Trp Met Thr Thr Pro Pro
           5315                5320                5325
   Val Glu Glu Thr Ser Ser Val Ser Ser Leu Met Ser Ser Pro Ala Met
       5330                5335                5340
   Thr Ser Pro Ser Pro Val Ser Ser Thr Ser Pro Gln Ser Ile Pro Ser
   5345                5350                5355                5360
   Ser Pro Leu Pro Val Thr Ala Leu Pro Thr Ser Val Leu Val Thr Thr
                   5365                5370                5375
   Thr Asp Val Leu Gly Thr Thr Ser Pro Glu Ser Val Thr Ser Ser Pro
               5380                5385                5390
   Pro Asn Leu Ser Ser Ile Thr His Glu Arg Pro Ala Thr Tyr Lys Asp
           5395                5400                5405
   Thr Ala His Thr Glu Ala Ala Met His His Ser Thr Asn Thr Ala Val
       5410                5415                5420
   Thr Asn Val Gly Thr Ser Gly Ser Gly His Lys Ser Gln Ser Ser Val
   5425                5430                5435                5440
   Leu Ala Asp Ser Glu Thr Ser Lys Ala Thr Pro Leu Met Ser Thr Thr
                   5445                5450                5455
   Ser Thr Leu Gly Asp Thr Ser Val Ser Thr Ser Thr Pro Asn Ile Ser
               5460                5465                5470
   Gln Thr Asn Gln Ile Gln Thr Glu Pro Thr Ala Ser Leu Ser Pro Arg
           5475                5480                5485
   Leu Arg Glu Ser Ser Thr Ser Glu Lys Thr Ser Ser Thr Thr Glu Thr
       5490                5495                5500
   Asn Thr Ala Phe Ser Tyr Val Pro Thr Gly Ala Ile Thr Gln Ala Ser
   5505                5510                5515                5520
   Arg Thr Glu Ile Ser Ser Ser Arg Thr Ser Ile Ser Asp Leu Asp Arg
                   5525                5530                5535
   Pro Thr Ile Ala Pro Asp Ile Ser Thr Gly Met Ile Thr Arg Leu Phe
               5540                5545                5550
   Thr Ser Pro Ile Met Thr Lys Ser Ala Glu Met Thr Val Thr Thr Gln
           5555                5560                5565
   Thr Thr Thr Pro Gly Ala Thr Ser Gln Gly Ile Leu Pro Trp Asp Thr
       5570                5575                5580
   Ser Thr Thr Leu Phe Gln Gly Gly Thr His Ser Thr Val Ser Gln Gly
   5585                5590                5595                5600
   Phe Pro His Ser Glu Ile Thr Thr Leu Arg Ser Arg Thr Pro Gly Asp
                   5605                5610                5615
   Val Ser Trp Met Thr Thr Pro Pro Val Glu Glu Thr Ser Ser Gly Phe
               5620                5625                5630
   Ser Leu Met Ser Pro Ser Met Thr Ser Pro Ser Pro Val Ser Ser Thr
           5635                5640                5645
   Ser Pro Glu Ser Ile Pro Ser Ser Pro Leu Pro Val Thr Ala Leu Leu
       5650                5655                5660
   Thr Ser Val Leu Val Thr Thr Thr Asn Val Leu Gly Thr Thr Ser Pro
   5665                5670                5675                5680
   Glu Pro Val Thr Ser Ser Pro Pro Asn Leu Ser Ser Pro Thr Gln Glu
                   5685                5690                5695
   Arg Leu Thr Thr Tyr Lys Asp Thr Ala His Thr Glu Ala Met His Ala
               5700                5705                5710
   Ser Met His Thr Asn Thr Ala Val Ala Asn Val Gly Thr Ser Ile Ser
           5715                5720                5725
   Gly His Glu Ser Gln Ser Ser Val Pro Ala Asp Ser His Thr Ser Lys
       5730                5735                5740
   Ala Thr Ser Pro Met Gly Ile Thr Phe Ala Met Gly Asp Thr Ser Val
   5745                5750                5755                5760
   Ser Thr Ser Thr Pro Ala Phe Phe Glu Thr Arg Ile Gln Thr Glu Ser
                   5765                5770                5775
   Thr Ser Ser Leu Ile Pro Gly Leu Arg Asp Thr Arg Thr Ser Glu Glu
               5780                5785                5790
   Ile Asn Thr Val Thr Glu Thr Ser Thr Val Leu Ser Glu Val Pro Thr
           5795                5800                5805
   Thr Thr Thr Thr Glu Val Ser Arg Thr Glu Val Ile Thr Ser Ser Arg
       5810                5815                5820
   Thr Thr Ile Ser Gly Pro Asp His Ser Lys Met Ser Pro Tyr Ile Ser
   5825                5830                5835                5840
   Thr Glu Thr Ile Thr Arg Leu Ser Thr Phe Pro Phe Val Thr Gly Ser
                   5845                5850                5855
   Thr Glu Met Ala Ile Thr Asn Gln Thr Gly Pro Ile Gly Thr Ile Ser
               5860                5865                5870
   Gln Ala Thr Leu Thr Leu Asp Thr Ser Ser Thr Ala Ser Trp Glu Gly
           5875                5880                5885
   Thr His Ser Pro Val Thr Gln Arg Phe Pro His Ser Glu Glu Thr Thr
       5890                5895                5900
   Thr Met Ser Arg Ser Thr Lys Gly Val Ser Trp Gln Ser Pro Pro Ser
   5905                5910                5915                5920
   Val Glu Glu Thr Ser Ser Pro Ser Ser Pro Val Pro Leu Pro Ala Ile
                   5925                5930                5935
   Thr Ser His Ser Ser Leu Tyr Ser Ala Val Ser Gly Ser Ser Pro Thr
               5940                5945                5950
   Ser Ala Leu Pro Val Thr Ser Leu Leu Thr Ser Gly Arg Arg Lys Thr
           5955                5960                5965
   Ile Asp Met Leu Asp Thr His Ser Glu Leu Val Thr Ser Ser Leu Pro
       5970                5975                5980
   Ser Ala Ser Ser Phe Ser Gly Glu Ile Leu Thr Ser Glu Ala Ser Thr
   5985                5990                5995                6000
   Asn Thr Glu Thr Ile His Phe Ser Glu Asn Thr Ala Glu Thr Asn Met
                   6005                6010                6015
   Gly Thr Thr Asn Ser Met His Lys Leu His Ser Ser Val Ser Ile His
               6020                6025                6030
   Ser Gln Pro Ser Gly His Thr Pro Pro Lys Val Thr Gly Ser Met Met
           6035                6040                6045
   Glu Asp Ala Ile Val Ser Thr Ser Thr Pro Gly Ser Pro Glu Thr Lys
       6050                6055                6060
   Asn Val Asp Arg Asp Ser Thr Ser Pro Leu Thr Pro Glu Leu Lys Glu
   6065                6070                6075                6080
   Asp Ser Thr Ala Leu Val Met Asn Ser Thr Thr Glu Ser Asn Thr Val
                   6085                6090                6095
   Phe Ser Ser Val Ser Leu Asp Ala Ala Thr Glu Val Ser Arg Ala Glu
               6100                6105                6110
   Val Thr Tyr Tyr Asp Pro Thr Phe Met Pro Ala Ser Ala Gln Ser Thr
           6115                6120                6125
   Lys Ser Pro Asp Ile Ser Pro Glu Ala Ser Ser Ser His Ser Asn Ser
       6130                6135                6140
   Pro Pro Leu Thr Ile Ser Thr His Lys Thr Ile Ala Thr Gln Thr Gly
   6145                6150                6155                6160
   Pro Ser Gly Val Thr Ser Leu Gly Gln Leu Thr Leu Asp Thr Ser Thr
                   6165                6170                6175
   Ile Ala Thr Ser Ala Gly Thr Pro Ser Ala Arg Thr Gln Asp Phe Val
               6180                6185                6190
   Asp Ser Glu Thr Thr Ser Val Met Asn Asn Asp Leu Asn Asp Val Leu
           6195                6200                6205
   Lys Thr Ser Pro Phe Ser Ala Glu Glu Ala Asn Ser Leu Ser Ser Gln
       6210                6215                6220
   Ala Pro Leu Leu Val Thr Thr Ser Pro Ser Pro Val Thr Ser Thr Leu
   6225                6230                6235                6240
   Gln Glu His Ser Thr Ser Ser Leu Val Ser Val Thr Ser Val Pro Thr
                   6245                6250                6255
   Pro Thr Leu Ala Lys Ile Thr Asp Met Asp Thr Asn Leu Glu Pro Val
               6260                6265                6270
   Thr Arg Ser Pro Gln Asn Leu Arg Asn Thr Leu Ala Thr Ser Glu Ala
           6275                6280                6285
   Thr Thr Asp Thr His Thr Met His Pro Ser Ile Asn Thr Ala Val Ala
       6290                6295                6300
   Asn Val Gly Thr Thr Ser Ser Pro Asn Glu Phe Tyr Phe Thr Val Ser
   6305                6310                6315                6320
   Pro Asp Ser Asp Pro Tyr Lys Ala Thr Ser Ala Val Val Ile Thr Ser
                   6325                6330                6335
   Thr Ser Gly Asp Ser Ile Val Ser Thr Ser Met Pro Arg Ser Ser Ala
               6340                6345                6350
   Met Lys Lys Ile Glu Ser Glu Thr Thr Phe Ser Leu Ile Phe Arg Leu
           6355                6360                6365
   Arg Glu Thr Ser Thr Ser Gln Lys Ile Gly Ser Ser Ser Asp Thr Ser
       6370                6375                6380
   Thr Val Phe Asp Lys Ala Phe Thr Ala Ala Thr Thr Glu Val Ser Arg
   6385                6390                6395                6400
   Thr Glu Leu Thr Ser Ser Ser Arg Thr Ser Ile Gln Gly Thr Glu Lys
                   6405                6410                6415
   Pro Thr Met Ser Pro Asp Thr Ser Thr Arg Ser Val Thr Met Leu Ser
               6420                6425                6430
   Thr Phe Ala Gly Leu Thr Lys Ser Glu Glu Arg Thr Ile Ala Thr Gln
           6435                6440                6445
   Thr Gly Pro His Arg Ala Thr Ser Gln Gly Thr Leu Thr Trp Asp Thr
       6450                6455                6460
   Ser Ile Thr Thr Ser Gln Ala Gly Thr His Ser Ala Met Thr His Gly
   6465                6470                6475                6480
   Phe Ser Gln Leu Asp Leu Ser Thr Leu Thr Ser Arg Val Pro Glu Tyr
                   6485                6490                6495
   Ile Ser Gly Thr Ser Pro Pro Ser Val Glu Lys Thr Ser Ser Ser Ser
               6500                6505                6510
   Ser Leu Leu Ser Leu Pro Ala Ile Thr Ser Pro Ser Pro Val Pro Thr
           6515                6520                6525
   Thr Leu Pro Glu Ser Arg Pro Ser Ser Pro Val His Leu Thr Ser Leu
       6530                6535                6540
   Pro Thr Ser Gly Leu Val Lys Thr Thr Asp Met Leu Ala Ser Val Ala
   6545                6550                6555                6560
   Ser Leu Pro Pro Asn Leu Gly Ser Thr Ser His Lys Ile Pro Thr Thr
                   6565                6570                6575
   Ser Glu Asp Ile Lys Asp Thr Glu Lys Met Tyr Pro Ser Thr Asn Ile
               6580                6585                6590
   Ala Val Thr Asn Val Gly Thr Thr Thr Ser Glu Lys Glu Ser Tyr Ser
           6595                6600                6605
   Ser Val Pro Ala Tyr Ser Glu Pro Pro Lys Val Thr Ser Pro Met Val
       6610                6615                6620
   Thr Ser Phe Asn Ile Arg Asp Thr Ile Val Ser Thr Ser Met Pro Gly
   6625                6630                6635                6640
   Ser Ser Glu Ile Thr Arg Ile Glu Met Glu Ser Thr Phe Ser Leu Ala
                   6645                6650                6655
   His Gly Leu Lys Gly Thr Ser Thr Ser Gln Asp Pro Ile Val Ser Thr
               6660                6665                6670
   Glu Lys Ser Ala Val Leu His Lys Leu Thr Thr Gly Ala Thr Glu Thr
           6675                6680                6685
   Ser Arg Thr Glu Val Ala Ser Ser Arg Arg Thr Ser Ile Pro Gly Pro
       6690                6695                6700
   Asp His Ser Thr Glu Ser Pro Asp Ile Ser Thr Glu Val Ile Pro Ser
   6705                6710                6715                6720
   Leu Pro Ile Ser Leu Gly Ile Thr Glu Ser Ser Asn Met Thr Ile Ile
                   6725                6730                6735
   Thr Arg Thr Gly Pro Pro Leu Gly Ser Thr Ser Gln Gly Thr Phe Thr
               6740                6745                6750
   Leu Asp Thr Pro Thr Thr Ser Ser Arg Ala Gly Thr His Ser Met Ala
           6755                6760                6765
   Thr Gln Glu Phe Pro His Ser Glu Met Thr Thr Val Met Asn Lys Asp
       6770                6775                6780
   Pro Glu Ile Leu Ser Trp Thr Ile Pro Pro Ser Ile Glu Lys Thr Ser
   6785                6790                6795                6800
   Phe Ser Ser Ser Leu Met Pro Ser Pro Ala Met Thr Ser Pro Pro Val
                   6805                6810                6815
   Ser Ser Thr Leu Pro Lys Thr Ile His Thr Thr Pro Ser Pro Met Thr
               6820                6825                6830
   Ser Leu Leu Thr Pro Ser Leu Val Met Thr Thr Asp Thr Leu Gly Thr
           6835                6840                6845
   Ser Pro Glu Pro Thr Thr Ser Ser Pro Pro Asn Leu Ser Ser Thr Ser
       6850                6855                6860
   His Glu Ile Leu Thr Thr Asp Glu Asp Thr Thr Ala Ile Glu Ala Met
   6865                6870                6875                6880
   His Pro Ser Thr Ser Thr Ala Ala Thr Asn Val Glu Thr Thr Ser Ser
                   6885                6890                6895
   Gly His Gly Ser Gln Ser Ser Val Leu Ala Asp Ser Glu Lys Thr Lys
               6900                6905                6910
   Ala Thr Ala Pro Met Asp Thr Thr Ser Thr Met Gly His Thr Thr Val
           6915                6920                6925
   Ser Thr Ser Met Ser Val Ser Ser Glu Thr Thr Lys Ile Lys Arg Glu
       6930                6935                6940
   Ser Thr Tyr Ser Leu Thr Pro Gly Leu Arg Glu Thr Ser Ile Ser Gln
   6945 6950 6955 6960
   Asn Ala Ser Phe Ser Thr Asp Thr Ser Ile Val Leu Ser Glu Val Pro
   6965 6970 6975
   Thr Gly Thr Thr Ala Glu Val Ser Arg Thr Glu Val Thr Ser Ser Gly
   6980 6985 6990
   Arg Thr Ser Ile Pro Gly Pro Ser Gln Ser Thr Val Leu Pro Glu Ile
   6995 7000 7005
   Ser Thr Arg Thr Met Thr Arg Leu Phe Ala Ser Pro Thr Met Thr Glu
   7010 7015 7020
   Ser Ala Glu Met Thr Ile Pro Thr Gln Thr Gly Pro Ser Gly Ser Thr
   7025 7030 7035 7040
   Ser Gln Asp Thr Leu Thr Leu Asp Thr Ser Thr Thr Lys Ser Gln Ala
   7045 7050 7055
   Lys Thr His Ser Thr Leu Thr Gln Arg Phe Pro His Ser Glu Met Thr
   7060 7065 7070
   Thr Leu Met Ser Arg Gly Pro Gly Asp Met Ser Trp Gln Ser Ser Pro
   7075 7080 7085
   Ser Leu Glu Asn Pro Ser Ser Leu Pro Ser Leu Leu Ser Leu Pro Ala
   7090 7095 7100
   Thr Thr Ser Pro Pro Pro Ile Ser Ser Thr Leu Pro Val Thr Ile Ser
   7105 7110 7115 7120
   Ser Ser Pro Leu Pro Val Thr Ser Leu Leu Thr Ser Ser Pro Val Thr
   7125 7130 7135
   Thr Thr Asp Met Leu His Thr Ser Pro Glu Leu Val Thr Ser Ser Pro
   7140 7145 7150
   Pro Lys Leu Ser His Thr Ser Asp Glu Arg Leu Thr Thr Gly Lys Asp
   7155 7160 7165
   Thr Thr Asn Thr Glu Ala Val His Pro Ser Thr Asn Thr Ala Ala Ser
   7170 7175 7180
   Asn Val Glu Ile Pro Ser Ser Gly His Glu Ser Pro Ser Ser Ala Leu
   7185 7190 7195 7200
   Ala Asp Ser Glu Thr Ser Lys Ala Thr Ser Pro Met Phe Ile Thr Ser
   7205 7210 7215
   Thr Gln Glu Asp Thr Thr Val Ala Ile Ser Thr Pro His Phe Leu Glu
   7220 7225 7230
   Thr Ser Arg Ile Gln Lys Glu Ser Ile Ser Ser Leu Ser Pro Lys Leu
   7235 7240 7245
   Arg Glu Thr Gly Ser Ser Val Glu Thr Ser Ser Ala Ile Glu Thr Ser
   7250 7255 7260
   Ala Val Leu Ser Glu Val Ser Ile Gly Ala Thr Thr Glu Ile Ser Arg
   7265 7270 7275 7280
   Thr Glu Val Thr Ser Ser Ser Arg Thr Ser Ile Ser Gly Ser Ala Glu
   7285 7290 7295
   Ser Thr Met Leu Pro Glu Ile Ser Thr Thr Arg Lys Ile Ile Lys Phe
   7300 7305 7310
   Pro Thr Ser Pro Ile Leu Ala Glu Ser Ser Glu Met Thr Ile Lys Thr
   7315 7320 7325
   Gln Thr Ser Pro Pro Gly Ser Thr Ser Glu Ser Thr Phe Thr Leu Asp
   7330 7335 7340
   Thr Ser Thr Thr Pro Ser Leu Val Ile Thr His Ser Thr Met Thr Gln
   7345 7350 7355 7360
   Arg Leu Pro His Ser Glu Ile Thr Thr Leu Val Ser Arg Gly Ala Gly
   7365 7370 7375
   Asp Val Pro Arg Pro Ser Ser Leu Pro Val Glu Glu Thr Ser Pro Pro
   7380 7385 7390
   Ser Ser Gln Leu Ser Leu Ser Ala Met Ile Ser Pro Ser Pro Val Ser
   7395 7400 7405
   Ser Thr Leu Pro Ala Ser Ser His Ser Ser Ser Ala Ser Val Thr Ser
   7410 7415 7420
   Leu Leu Thr Pro Gly Gln Val Lys Thr Thr Glu Val Leu Asp Ala Ser
   7425 7430 7435 7440
   Ala Glu Pro Glu Thr Ser Ser Pro Pro Ser Leu Ser Ser Thr Ser Val
                   7445                7450                7455
   Glu Ile Leu Ala Thr Ser Glu Val Thr Thr Asp Thr Glu Lys Ile His
               7460                7465                7470
   Pro Phe Ser Asn Thr Ala Val Thr Lys Val Gly Thr Ser Ser Ser Gly
           7475                7480                7485
   His Glu Ser Pro Ser Ser Val Leu Pro Asp Ser Glu Thr Thr Lys Ala
       7490                7495                7500
   Thr Ser Ala Met Gly Thr Ile Ser Ile Met Gly Asp Thr Ser Val Ser
   7505                7510                7515                7520
   Thr Leu Thr Pro Ala Leu Ser Asn Thr Arg Lys Ile Gln Ser Glu Pro
                   7525                7530                7535
   Ala Ser Ser Leu Thr Thr Arg Leu Arg Glu Thr Ser Thr Ser Glu Glu
               7540                7545                7550
   Thr Ser Leu Ala Thr Glu Ala Asn Thr Val Leu Ser Lys Val Ser Thr
           7555                7560                7565
   Gly Ala Thr Thr Glu Val Ser Arg Thr Glu Ala Ile Ser Phe Ser Arg
       7570                7575                7580
   Thr Ser Met Ser Gly Pro Glu Gln Ser Thr Met Ser Gln Asp Ile Ser
   7585                7590                7595                7600
   Ile Gly Thr Ile Pro Arg Ile Ser Ala Ser Ser Val Leu Thr Glu Ser
                   7605                7610                7615
   Ala Lys Met Thr Ile Thr Thr Gln Thr Gly Pro Ser Glu Ser Thr Leu
               7620                7625                7630
   Glu Ser Thr Leu Asn Leu Asn Thr Ala Thr Thr Pro Ser Trp Val Glu
           7635                7640                7645
   Thr His Ser Ile Val Ile Gln Gly Phe Pro His Pro Glu Met Thr Thr
       7650                7655                7660
   Ser Met Gly Arg Gly Pro Gly Gly Val Ser Trp Pro Ser Pro Pro Phe
   7665                7670                7675                7680
   Val Lys Glu Thr Ser Pro Pro Ser Ser Pro Leu Ser Leu Pro Ala Val
                   7685                7690                7695
   Thr Ser Pro His Pro Val Ser Thr Thr Phe Leu Ala His Ile Pro Pro
               7700                7705                7710
   Ser Pro Leu Pro Val Thr Ser Leu Leu Thr Ser Gly Pro Ala Thr Thr
           7715                7720                7725
   Thr Asp Ile Leu Gly Thr Ser Thr Glu Pro Gly Thr Ser Ser Ser Ser
       7730                7735                7740
   Ser Leu Ser Thr Thr Ser His Glu Arg Leu Thr Thr Tyr Lys Asp Thr
   7745                7750                7755                7760
   Ala His Thr Glu Ala Val His Pro Ser Thr Asn Thr Gly Gly Thr Asn
                   7765                7770                7775
   Val Ala Thr Thr Ser Ser Gly Tyr Lys Ser Gln Ser Ser Val Leu Ala
               7780                7785                7790
   Asp Ser Ser Pro Met Cys Thr Thr Ser Thr Met Gly Asp Thr Ser Val
           7795                7800                7805
   Leu Thr Ser Thr Pro Ala Phe Leu Glu Thr Arg Arg Ile Gln Thr Glu
       7810                7815                7820
   Leu Ala Ser Ser Leu Thr Pro Gly Leu Arg Glu Ser Ser Gly Ser Glu
   7825                7830                7835                7840
   Gly Thr Ser Ser Gly Thr Lys Met Ser Thr Val Leu Ser Lys Val Pro
                   7845                7850                7855
   Thr Gly Ala Thr Thr Glu Ile Ser Lys Glu Asp Val Thr Ser Ile Pro
               7860                7865                7870
   Gly Pro Ala Gln Ser Thr Ile Ser Pro Asp Ile Ser Thr Arg Thr Val
           7875                7880                7885
   Ser Trp Phe Ser Thr Ser Pro Val Met Thr Glu Ser Ala Glu Ile Thr
       7890                7895                7900
   Met Asn Thr His Thr Ser Pro Leu Gly Ala Thr Thr Gln Gly Thr Ser
   7905                7910                7915                7920
   Thr Leu Asp Thr Ser Ser Thr Thr Ser Leu Thr Met Thr His Ser Thr
                   7925                7930                7935
   Ile Ser Gln Gly Phe Ser His Ser Gln Met Ser Thr Leu Met Arg Arg
               7940                7945                7950
   Gly Pro Glu Asp Val Ser Trp Met Ser Pro Pro Leu Leu Glu Lys Thr
           7955                7960                7965
   Arg Pro Ser Phe Ser Leu Met Ser Ser Pro Ala Thr Thr Ser Pro Ser
       7970                7975                7980
   Pro Val Ser Ser Thr Leu Pro Glu Ser Ile Ser Ser Ser Pro Leu Pro
   7985                7990                7995                8000
   Val Thr Ser Leu Leu Thr Ser Gly Leu Ala Lys Thr Thr Asp Met Leu
                   8005                8010                8015
   His Lys Ser Ser Glu Pro Val Thr Asn Ser Pro Ala Asn Leu Ser Ser
               8020                8025                8030
   Thr Ser Val Glu Ile Leu Ala Thr Ser Glu Val Thr Thr Asp Thr Glu
           8035                8040                8045
   Lys Thr His Pro Ser Ser Asn Arg Thr Val Thr Asp Val Gly Thr Ser
       8050                8055                8060
   Ser Ser Gly His Glu Ser Thr Ser Phe Val Leu Ala Asp Ser Gln Thr
   8065                8070                8075                8080
   Ser Lys Val Thr Ser Pro Met Val Ile Thr Ser Thr Met Glu Asp Thr
                   8085                8090                8095
   Ser Val Ser Thr Ser Thr Pro Gly Phe Phe Glu Thr Ser Arg Ile Gln
               8100                8105                8110
   Thr Glu Pro Thr Ser Ser Leu Thr Leu Gly Leu Arg Lys Thr Ser Ser
           8115                8120                8125
   Ser Glu Gly Thr Ser Leu Ala Thr Glu Met Ser Thr Val Leu Ser Gly
       8130                8135                8140
   Val Pro Thr Gly Ala Thr Ala Glu Val Ser Arg Thr Glu Val Thr Ser
   8145                8150                8155                8160
   Ser Ser Arg Thr Ser Ile Ser Gly Phe Ala Gln Leu Thr Val Ser Pro
                   8165                8170                8175
   Glu Thr Ser Thr Glu Thr Ile Thr Arg Leu Pro Thr Ser Ser Ile Met
               8180                8185                8190
   Thr Glu Ser Ala Glu Met Met Ile Lys Thr Gln Thr Asp Pro Pro Gly
           8195                8200                8205
   Ser Thr Pro Glu Ser Thr His Thr Val Asp Ile Ser Thr Thr Pro Asn
       8210                8215                8220
   Trp Val Glu Thr His Ser Thr Val Thr Gln Arg Phe Ser His Ser Glu
   8225                8230                8235                8240
   Met Thr Thr Leu Val Ser Arg Ser Pro Gly Asp Met Leu Trp Pro Ser
                   8245                8250                8255
   Gln Ser Ser Val Glu Glu Thr Ser Ser Ala Ser Ser Leu Leu Ser Leu
               8260                8265                8270
   Pro Ala Thr Thr Ser Pro Ser Pro Val Ser Ser Thr Leu Val Glu Asp
           8275                8280                8285
   Phe Pro Ser Ala Ser Leu Pro Val Thr Ser Leu Leu Asn Pro Gly Leu
       8290                8295                8300
   Val Ile Thr Thr Asp Arg Met Gly Ile Ser Arg Glu Pro Gly Thr Ser
   8305                8310                8315                8320
   Ser Thr Ser Asn Leu Ser Ser Thr Ser His Glu Arg Leu Thr Thr Leu
                   8325                8330                8335
   Glu Asp Thr Val Asp Thr Glu Asp Met Gln Pro Ser Thr His Thr Ala
               8340                8345                8350
   Val Thr Asn Val Arg Thr Ser Ile Ser Gly His Glu Ser Gln Ser Ser
           8355                8360                8365
   Val Leu Ser Asp Ser Glu Thr Pro Lys Ala Thr Ser Pro Met Gly Thr
       8370                8375                8380
   Thr Tyr Thr Met Gly Glu Thr Ser Val Ser Ile Ser Thr Ser Asp Phe
   8385                8390                8395                8400
   Phe Glu Thr Ser Arg Ile Gln Ile Glu Pro Thr Ser Ser Leu Thr Ser
                   8405                8410                8415
   Gly Leu Arg Glu Thr Ser Ser Ser Glu Arg Ile Ser Ser Ala Thr Glu
               8420                8425                8430
   Gly Ser Thr Val Leu Ser Glu Val Pro Ser Gly Ala Thr Thr Glu Val
           8435                8440                8445
   Ser Arg Thr Glu Val Ile Ser Ser Arg Gly Thr Ser Met Ser Gly Pro
       8450                8455                8460
   Asp Gln Phe Thr Ile Ser Pro Asp Ile Ser Thr Glu Ala Ile Thr Arg
   8465                8470                8475                8480
   Leu Ser Thr Ser Pro Ile Met Thr Glu Ser Ala Glu Ser Ala Ile Thr
                   8485                8490                8495
   Ile Glu Thr Gly Ser Pro Gly Ala Thr Ser Glu Gly Thr Leu Thr Leu
               8500                8505                8510
   Asp Thr Ser Thr Thr Thr Phe Trp Ser Gly Thr His Ser Thr Ala Ser
           8515                8520                8525
   Pro Gly Phe Ser His Ser Glu Met Thr Thr Leu Met Ser Arg Thr Pro
       8530                8535                8540
   Gly Asp Val Pro Trp Pro Ser Leu Pro Ser Val Glu Glu Ala Ser Ser
   8545                8550                8555                8560
   Val Ser Ser Ser Leu Ser Ser Pro Ala Met Thr Ser Thr Ser Phe Phe
                   8565                8570                8575
   Ser Thr Leu Pro Glu Ser Ile Ser Ser Ser Pro His Pro Val Thr Ala
               8580                8585                8590
   Leu Leu Thr Leu Gly Pro Val Lys Thr Thr Asp Met Leu Arg Thr Ser
           8595                8600                8605
   Ser Glu Pro Glu Thr Ser Ser Pro Pro Asn Leu Ser Ser Thr Ser Ala
       8610                8615                8620
   Glu Ile Leu Ala Thr Ser Glu Val Thr Lys Asp Arg Glu Lys Ile His
   8625                8630                8635                8640
   Pro Ser Ser Asn Thr Pro Val Val Asn Val Gly Thr Val Ile Tyr Lys
                   8645                8650                8655
   His Leu Ser Pro Ser Ser Val Leu Ala Asp Leu Val Thr Thr Lys Pro
               8660                8665                8670
   Thr Ser Pro Met Ala Thr Thr Ser Thr Leu Gly Asn Thr Ser Val Ser
           8675                8680                8685
   Thr Ser Thr Pro Ala Phe Pro Glu Thr Met Met Thr Gln Pro Thr Ser
       8690                8695                8700
   Ser Leu Thr Ser Gly Leu Arg Glu Ile Ser Thr Ser Gln Glu Thr Ser
   8705                8710                8715                8720
   Ser Ala Thr Glu Arg Ser Ala Ser Leu Ser Gly Met Pro Thr Gly Ala
                   8725                8730                8735
   Thr Thr Lys Val Ser Arg Thr Glu Ala Leu Ser Leu Gly Arg Thr Ser
               8740                8745                8750
   Thr Pro Gly Pro Ala Gln Ser Thr Ile Ser Pro Glu Ile Ser Thr Glu
           8755                8760                8765
   Thr Ile Thr Arg Ile Ser Thr Pro Leu Thr Thr Thr Gly Ser Ala Glu
       8770                8775                8780
   Met Thr Ile Thr Pro Lys Thr Gly His Ser Gly Ala Ser Ser Gln Gly
   8785                8790                8795                8800
   Thr Phe Thr Leu Asp Thr Ser Ser Arg Ala Ser Trp Pro Gly Thr His
                   8805                8810                8815
   Ser Ala Ala Thr His Arg Ser Pro His Ser Gly Met Thr Thr Pro Met
               8820                8825                8830
   Ser Arg Gly Pro Glu Asp Val Ser Trp Pro Ser Arg Pro Ser Val Glu
           8835                8840                8845
   Lys Thr Ser Pro Pro Ser Ser Leu Val Ser Leu Ser Ala Val Thr Ser
       8850                8855                8860
   Pro Ser Pro Leu Tyr Ser Thr Pro Ser Glu Ser Ser His Ser Ser Pro
   8865                8870                8875                8880
   Leu Arg Val Thr Ser Leu Phe Thr Pro Val Met Met Lys Thr Thr Asp
                   8885                8890                8895
   Met Leu Asp Thr Ser Leu Glu Pro Val Thr Thr Ser Pro Pro Ser Met
               8900                8905                8910
   Asn Ile Thr Ser Asp Glu Ser Leu Ala Thr Ser Lys Ala Thr Met Glu
           8915                8920                8925
   Thr Glu Ala Ile Gln Leu Ser Glu Asn Thr Ala Val Thr Gln Met Gly
       8930                8935                8940
   Thr Ile Ser Ala Arg Gln Glu Phe Tyr Ser Ser Tyr Pro Gly Leu Pro
   8945                8950                8955                8960
   Glu Pro Ser Lys Val Thr Ser Pro Val Val Thr Ser Ser Thr Ile Lys
                   8965                8970                8975
   Asp Ile Val Ser Thr Thr Ile Pro Ala Ser Ser Glu Ile Thr Arg Ile
               8980                8985                8990
   Glu Met Glu Ser Thr Ser Thr Leu Thr Pro Thr Pro Arg Glu Thr Ser
           8995                9000                9005
   Thr Ser Gln Glu Ile His Ser Ala Thr Lys Pro Ser Thr Val Pro Tyr
       9010                9015                9020
   Lys Ala Leu Thr Ser Ala Thr Ile Glu Asp Ser Met Thr Gln Val Met
   9025                9030                9035                9040
   Ser Ser Ser Arg Gly Pro Ser Pro Asp Gln Ser Thr Met Ser Gln Asp
                   9045                9050                9055
   Ile Ser Thr Glu Val Ile Thr Arg Leu Ser Thr Ser Pro Ile Lys Thr
               9060                9065                9070
   Glu Ser Thr Glu Met Thr Ile Thr Thr Gln Thr Gly Ser Pro Gly Ala
           9075                9080                9085
   Thr Ser Arg Gly Thr Leu Thr Leu Asp Thr Ser Thr Thr Phe Met Ser
       9090                9095                9100
   Gly Thr His Ser Thr Ala Ser Gln Gly Phe Ser His Ser Gln Met Thr
   9105                9110                9115                9120
   Ala Leu Met Ser Arg Thr Pro Gly Asp Val Pro Trp Leu Ser His Pro
                   9125                9130                9135
   Ser Val Glu Glu Ala Ser Ser Ala Ser Phe Ser Leu Ser Ser Pro Val
               9140                9145                9150
   Met Thr Ser Ser Ser Pro Val Ser Ser Thr Leu Pro Asp Ser Ile His
           9155                9160                9165
   Ser Ser Ser Leu Pro Val Thr Ser Leu Leu Thr Ser Gly Leu Val Lys
       9170                9175                9180
   Thr Thr Glu Leu Leu Gly Thr Ser Ser Glu Pro Glu Thr Ser Ser Pro
   9185                9190                9195                9200
   Pro Asn Leu Ser Ser Thr Ser Ala Glu Ile Leu Ala Ile Thr Glu Val
                   9205                9210                9215
   Thr Thr Asp Thr Glu Lys Leu Glu Met Thr Asn Val Val Thr Ser Gly
               9220                9225                9230
   Tyr Thr His Glu Ser Pro Ser Ser Val Leu Ala Asp Ser Val Thr Thr
           9235                9240                9245
   Lys Ala Thr Ser Ser Met Gly Ile Thr Tyr Pro Thr Gly Asp Thr Asn
       9250                9255                9260
   Val Leu Thr Ser Thr Pro Ala Phe Ser Asp Thr Ser Arg Ile Gln Thr
   9265                9270                9275                9280
   Lys Ser Lys Leu Ser Leu Thr Pro Gly Leu Met Glu Thr Ser Ile Ser
                   9285                9290                9295
   Glu Glu Thr Ser Ser Ala Thr Glu Lys Ser Thr Val Leu Ser Ser Val
               9300                9305                9310
   Pro Thr Gly Ala Thr Thr Glu Val Ser Arg Thr Glu Ala Ile Ser Ser
           9315                9320                9325
   Ser Arg Thr Ser Ile Pro Gly Pro Ala Gln Ser Thr Met Ser Ser Asp
       9330                9335                9340
   Thr Ser Met Glu Thr Ile Thr Arg Ile Ser Thr Pro Leu Thr Arg Lys
   9345                9350                9355                9360
   Glu Ser Thr Asp Met Ala Ile Thr Pro Lys Thr Gly Pro Ser Gly Ala
                   9365                9370                9375
   Thr Ser Gln Gly Thr Phe Thr Leu Asp Ser Ser Ser Thr Ala Ser Trp
               9380                9385                9390
   Pro Gly Thr His Ser Ala Thr Thr Gln Arg Phe Pro Gln Ser Val Val
           9395                9400                9405
   Thr Thr Pro Met Ser Arg Gly Pro Glu Asp Val Ser Trp Pro Ser Pro
       9410                9415                9420
   Leu Ser Val Glu Lys Asn Ser Pro Pro Ser Ser Leu Val Ser Ser Ser
   9425                9430                9435                9440
   Ser Val Thr Ser Pro Ser Pro Leu Tyr Ser Thr Pro Ser Gly Ser Ser
                   9445                9450                9455
   His Ser Ser Pro Val Pro Val Thr Ser Leu Phe Thr Ser Ile Met Met
               9460                9465                9470
   Lys Ala Thr Asp Met Leu Asp Ala Ser Leu Glu Pro Glu Thr Thr Ser
           9475                9480                9485
   Ala Pro Asn Met Asn Ile Thr Ser Asp Glu Ser Leu Ala Ala Ser Lys
       9490                9495                9500
   Ala Thr Thr Glu Thr Glu Ala Ile His Val Phe Glu Asn Thr Ala Ala
   9505                9510                9515                9520
   Ser His Val Glu Thr Thr Ser Ala Thr Glu Glu Leu Tyr Ser Ser Ser
                   9525                9530                9535
   Pro Gly Phe Ser Glu Pro Thr Lys Val Ile Ser Pro Val Val Thr Ser
               9540                9545                9550
   Ser Ser Ile Arg Asp Asn Met Val Ser Thr Thr Met Pro Gly Ser Ser
           9555                9560                9565
   Gly Ile Thr Arg Ile Glu Ile Glu Ser Met Ser Ser Leu Thr Pro Gly
       9570                9575                9580
   Leu Arg Glu Thr Arg Thr Ser Gln Asp Ile Thr Ser Ser Thr Glu Thr
   9585                9590                9595                9600
   Ser Thr Val Leu Tyr Lys Met Pro Ser Gly Ala Thr Pro Glu Val Ser
                   9605                9610                9615
   Arg Thr Glu Val Met Pro Ser Ser Arg Thr Ser Ile Pro Gly Pro Ala
               9620                9625                9630
   Gln Ser Thr Met Ser Leu Asp Ile Ser Asp Glu Val Val Thr Arg Leu
           9635                9640                9645
   Ser Thr Ser Pro Ile Met Thr Glu Ser Ala Glu Ile Thr Ile Thr Thr
       9650                9655                9660
   Gln Thr Gly Tyr Ser Leu Ala Thr Ser Gln Val Thr Leu Pro Leu Gly
   9665                9670                9675                9680
   Thr Ser Met Thr Phe Leu Ser Gly Thr His Ser Thr Met Ser Gln Gly
                   9685                9690                9695
   Leu Ser His Ser Glu Met Thr Asn Leu Met Ser Arg Gly Pro Glu Ser
               9700                9705                9710
   Leu Ser Trp Thr Ser Pro Arg Phe Val Glu Thr Thr Arg Ser Ser Ser
           9715                9720                9725
   Ser Leu Thr Ser Leu Pro Leu Thr Thr Ser Leu Ser Pro Val Ser Ser
       9730                9735                9740
   Thr Leu Leu Asp Ser Ser Pro Ser Ser Pro Leu Pro Val Thr Ser Leu
   9745                9750                9755                9760
   Ile Leu Pro Gly Leu Val Lys Thr Thr Glu Val Leu Asp Thr Ser Ser
                   9765                9770                9775
   Glu Pro Lys Thr Ser Ser Ser Pro Asn Leu Ser Ser Thr Ser Val Glu
               9780                9785                9790
   Ile Pro Ala Thr Ser Glu Ile Met Thr Asp Thr Glu Lys Ile His Pro
           9795                9800                9805
   Ser Ser Asn Thr Ala Val Ala Lys Val Arg Thr Ser Ser Ser Val His
       9810                9815                9820
   Glu Ser His Ser Ser Val Leu Ala Asp Ser Glu Thr Thr Ile Thr Ile
   9825                9830                9835                9840
   Pro Ser Met Gly Ile Thr Ser Ala Val Asp Asp Thr Thr Val Phe Thr
                   9845                9850                9855
   Ser Asn Pro Ala Phe Ser Glu Thr Arg Arg Ile Pro Thr Glu Pro Thr
               9860                9865                9870
   Phe Ser Leu Thr Pro Gly Phe Arg Glu Thr Ser Thr Ser Glu Glu Thr
           9875                9880                9885
   Thr Ser Ile Thr Glu Thr Ser Ala Val Leu Tyr Gly Val Pro Thr Ser
       9890                9895                9900
   Ala Thr Thr Glu Val Ser Met Thr Glu Ile Met Ser Ser Asn Arg Ile
   9905                9910                9915                9920
   His Ile Pro Asp Ser Asp Gln Ser Thr Met Ser Pro Asp Ile Ile Thr
                   9925                9930                9935
   Glu Val Ile Thr Arg Leu Ser Ser Ser Ser Met Met Ser Glu Ser Thr
               9940                9945                9950
   Gln Met Thr Ile Thr Thr Gln Lys Ser Ser Pro Gly Ala Thr Ala Gln
           9955                9960                9965
   Ser Thr Leu Thr Leu Ala Thr Thr Thr Ala Pro Leu Ala Arg Thr His
       9970                9975                9980
   Ser Thr Val Pro Pro Arg Phe Leu His Ser Glu Met Thr Thr Leu Met
   9985                9990                9995                10000
   Ser Arg Ser Pro Glu Asn Pro Ser Trp Lys Ser Ser Leu Phe Val Glu
                   10005               10010               10015
   Lys Thr Ser Ser Ser Ser Ser Leu Leu Ser Leu Pro Val Thr Thr Ser
               10020               10025               10030
   Pro Ser Val Ser Ser Thr Leu Pro Gln Ser Ile Pro Ser Ser Ser Phe
           10035               10040               10045
   Ser Val Thr Ser Leu Leu Thr Pro Gly Met Val Lys Thr Thr Asp Thr
       10050               10055               10060
   Ser Thr Glu Pro Gly Thr Ser Leu Ser Pro Asn Leu Ser Gly Thr Ser
   10065               10070               10075               10080
   Val Glu Ile Leu Ala Ala Ser Glu Val Thr Thr Asp Thr Glu Lys Ile
                   10085               10090               10095
   His Pro Ser Ser Ser Met Ala Val Thr Asn Val Gly Thr Thr Ser Ser
               10100               10105               10110
   Gly His Glu Leu Tyr Ser Ser Val Ser Ile His Ser Glu Pro Ser Lys
           10115               10120               10125
   Ala Thr Tyr Pro Val Gly Thr Pro Ser Ser Met Ala Glu Thr Ser Ile
       10130               10135               10140
   Ser Thr Ser Met Pro Ala Asn Phe Glu Thr Thr Gly Phe Glu Ala Glu
   10145               10150               10155               10160
   Pro Phe Ser His Leu Thr Ser Gly Phe Arg Lys Thr Asn Met Ser Leu
                   10165               10170               10175
   Asp Thr Ser Ser Val Thr Pro Thr Asn Thr Pro Ser Ser Pro Gly Ser
               10180               10185               10190
   Thr His Leu Leu Gln Ser Ser Lys Thr Asp Phe Thr Ser Ser Ala Lys
           10195               10200               10205
   Thr Ser Ser Pro Asp Trp Pro Pro Ala Ser Gln Tyr Thr Glu Ile Pro
       10210               10215               10220
   Val Asp Ile Ile Thr Pro Phe Asn Ala Ser Pro Ser Ile Thr Glu Ser
   10225               10230               10235               10240
   Thr Gly Ile Thr Ser Phe Pro Glu Ser Arg Phe Thr Met Ser Val Thr
                   10245               10250               10255
   Glu Ser Thr His His Leu Ser Thr Asp Leu Leu Pro Ser Ala Glu Thr
               10260               10265               10270
   Ile Ser Thr Gly Thr Val Met Pro Ser Leu Ser Glu Ala Met Thr Ser
           10275               10280               10285
   Phe Ala Thr Thr Gly Val Pro Arg Ala Ile Ser Gly Ser Gly Ser Pro
       10290               10295               10300
   Phe Ser Arg Thr Glu Ser Gly Pro Gly Asp Ala Thr Leu Ser Thr Ile
   10305               10310               10315               10320
   Ala Glu Ser Leu Pro Ser Ser Thr Pro Val Pro Phe Ser Ser Ser Thr
                   10325               10330               10335
   Phe Thr Thr Thr Asp Ser Ser Thr Ile Pro Ala Leu His Glu Ile Thr
               10340               10345               10350
   Ser Ser Ser Ala Thr Pro Tyr Arg Val Asp Thr Ser Leu Gly Thr Glu
           10355               10360               10365
   Ser Ser Thr Thr Glu Gly Arg Leu Val Met Val Ser Thr Leu Asp Thr
   10370 10375 10380
   Ser Ser Gln Pro Gly Arg Thr Ser Ser Ser Pro Ile Leu Asp Thr Arg
   10385 10390 10395 10400
   Met Thr Glu Ser Val Glu Leu Gly Thr Val Thr Ser Ala Tyr Gln Val
   10405 10410 10415
   Pro Ser Leu Ser Thr Arg Leu Thr Arg Thr Asp Gly Ile Met Glu His
   10420 10425 10430
   Ile Thr Lys Ile Pro Asn Glu Ala Ala His Arg Gly Thr Ile Arg Pro
   10435 10440 10445
   Val Lys Gly Pro Gln Thr Ser Thr Ser Pro Ala Ser Pro Lys Gly Leu
   10450 10455 10460
   His Thr Gly Gly Thr Lys Arg Met Glu Thr Thr Thr Thr Ala Leu Lys
   10465 10470 10475 10480
   Thr Thr Thr Thr Ala Leu Lys Thr Thr Ser Arg Ala Thr Leu Thr Thr
   10485 10490 10495
   Ser Val Tyr Thr Pro Thr Leu Gly Thr Leu Thr Pro Leu Asn Ala Ser
   10500 10505 10510
   Met Gln Met Ala Ser Thr Ile Pro Thr Glu Met Met Ile Thr Thr Pro
   10515 10520 10525
   Tyr Val Phe Pro Asp Val Pro Glu Thr Thr Ser Ser Leu Ala Thr Ser
   10530 10535 10540
   Leu Gly Ala Glu Thr Ser Thr Ala Leu Pro Arg Thr Thr Pro Ser Val
   10545 10550 10555 10560
   Phe Asn Arg Glu Ser Glu Thr Thr Ala Ser Leu Val Ser Arg Ser Gly
   10565 10570 10575
   Ala Glu Arg Ser Pro Val Ile Gln Thr Leu Asp Val Ser Ser Ser Glu
   10580 10585 10590
   Pro Asp Thr Thr Ala Ser Trp Val Ile His Pro Ala Glu Thr Ile Pro
   10595 10600 10605
   Thr Val Ser Lys Thr Thr Pro Asn Phe Phe His Ser Glu Leu Asp Thr
   10610 10615 10620
   Val Ser Ser Thr Ala Thr Ser His Gly Ala Asp Val Ser Ser Ala Ile
   10625 10630 10635 10640
   Pro Thr Asn Ile Ser Pro Ser Glu Leu Asp Ala Leu Thr Pro Leu Val
   10645 10650 10655
   Thr Ile Ser Gly Thr Asp Thr Ser Thr Thr Phe Pro Thr Leu Thr Lys
   10660 10665 10670
   Ser Pro His Glu Thr Glu Thr Arg Thr Thr Trp Leu Thr His Pro Ala
   10675 10680 10685
   Glu Thr Ser Ser Thr Ile Pro Arg Thr Ile Pro Asn Phe Ser His His
   10690 10695 10700
   Glu Ser Asp Ala Thr Pro Ser Ile Ala Thr Ser Pro Gly Ala Glu Thr
   10705 10710 10715 10720
   Ser Ser Ala Ile Pro Ile Met Thr Val Ser Pro Gly Ala Glu Asp Leu
   10725 10730 10735
   Val Thr Ser Gln Val Thr Ser Ser Gly Thr Asp Arg Asn Met Thr Ile
   10740 10745 10750
   Pro Thr Leu Thr Leu Ser Pro Gly Glu Pro Lys Thr Ile Ala Ser Leu
   10755 10760 10765
   Val Thr His Pro Glu Ala Gln Thr Ser Ser Ala Ile Pro Thr Ser Thr
   10770 10775 10780
   Ile Ser Pro Ala Val Ser Arg Leu Val Thr Ser Met Val Thr Ser Leu
   10785 10790 10795 10800
   Ala Ala Lys Thr Ser Thr Thr Asn Arg Ala Leu Thr Asn Ser Pro Gly
   10805 10810 10815
   Glu Pro Ala Thr Thr Val Ser Leu Val Thr His Pro Ala Gln Thr Ser
   10820 10825 10830
   Pro Thr Val Pro Trp Thr Thr Ser Ile Phe Phe His Ser Lys Ser Asp
   10835 10840 10845
   Thr Thr Pro Ser Met Thr Thr Ser His Gly Ala Glu Ser Ser Ser Ala
   10850 10855 10860
   Val Pro Thr Pro Thr Val Ser Thr Glu Val Pro Gly Val Val Thr Pro
   10865 10870 10875 10880
   Leu Val Thr Ser Ser Arg Ala Val Ile Ser Thr Thr Ile Pro Ile Leu
   10885 10890 10895
   Thr Leu Ser Pro Gly Glu Pro Glu Thr Thr Pro Ser Met Ala Thr Ser
   10900 10905 10910
   His Gly Glu Glu Ala Ser Ser Ala Ile Pro Thr Pro Thr Val Ser Pro
   10915 10920 10925
   Gly Val Pro Gly Val Val Thr Ser Leu Val Thr Ser Ser Arg Ala Val
   10930 10935 10940
   Thr Ser Thr Thr Ile Pro Ile Leu Thr Phe Ser Leu Gly Glu Pro Glu
   10945 10950 10955 10960
   Thr Thr Pro Ser Met Ala Thr Ser His Gly Thr Glu Ala Gly Ser Ala
   10965 10970 10975
   Val Pro Thr Val Leu Pro Glu Val Pro Gly Met Val Thr Ser Leu Val
   10980 10985 10990
   Ala Ser Ser Arg Ala Val Thr Ser Thr Thr Leu Pro Thr Leu Thr Leu
   10995 11000 11005
   Ser Pro Gly Glu Pro Glu Thr Thr Pro Ser Met Ala Thr Ser His Gly
   11010 11015 11020
   Ala Glu Ala Ser Ser Thr Val Pro Thr Val Ser Pro Glu Val Pro Gly
   11025 11030 11035 11040
   Val Val Thr Ser Leu Val Thr Ser Ser Ser Gly Val Asn Ser Thr Ser
   11045 11050 11055
   Ile Pro Thr Leu Ile Leu Ser Pro Gly Glu Leu Glu Thr Thr Pro Ser
   11060 11065 11070
   Met Ala Thr Ser His Gly Ala Glu Ala Ser Ser Ala Val Pro Thr Pro
   11075 11080 11085
   Thr Val Ser Pro Gly Val Ser Gly Val Val Thr Pro Leu Val Thr Ser
   11090 11095 11100
   Ser Arg Ala Val Thr Ser Thr Thr Ile Pro Ile Leu Thr Leu Ser Ser
   11105 11110 11115 11120
   Ser Glu Pro Glu Thr Thr Pro Ser Met Ala Thr Ser His Gly Val Glu
   11125 11130 11135
   Ala Ser Ser Ala Val Leu Thr Val Ser Pro Glu Val Pro Gly Met Val
   11140 11145 11150
   Thr Ser Leu Val Thr Ser Ser Arg Ala Val Thr Ser Thr Thr Ile Pro
   11155 11160 11165
   Thr Leu Thr Ile Ser Ser Asp Glu Pro Glu Thr Thr Thr Ser Leu Val
   11170 11175 11180
   Thr His Ser Glu Ala Lys Met Ile Ser Ala Ile Pro Thr Leu Ala Val
   11185 11190 11195 11200
   Ser Pro Thr Val Gln Gly Leu Val Thr Ser Leu Val Thr Ser Ser Gly
   11205 11210 11215
   Ser Glu Thr Ser Ala Phe Ser Asn Leu Thr Val Ala Ser Ser Gln Pro
   11220 11225 11230
   Glu Thr Ile Asp Ser Trp Val Ala His Pro Gly Thr Glu Ala Ser Ser
   11235 11240 11245
   Val Val Pro Thr Leu Thr Val Ser Thr Gly Glu Pro Phe Thr Asn Ile
   11250 11255 11260
   Ser Leu Val Thr His Pro Ala Glu Ser Ser Ser Thr Leu Pro Arg Thr
   11265 11270 11275 11280
   Thr Ser Arg Phe Ser His Ser Glu Leu Asp Thr Met Pro Ser Thr Val
   11285 11290 11295
   Thr Ser Pro Glu Ala Glu Ser Ser Ser Ala Ile Ser Thr Thr Ile Ser
   11300 11305 11310
   Pro Gly Ile Pro Gly Val Leu Thr Ser Leu Val Thr Ser Ser Gly Arg
   11315 11320 11325
   Asp Ile Ser Ala Thr Phe Pro Thr Val Pro Glu Ser Pro His Glu Ser
   11330 11335 11340
   Glu Ala Thr Ala Ser Trp Val Thr His Pro Ala Val Thr Ser Thr Thr
   11345 11350 11355 11360
   Val Pro Arg Thr Thr Pro Asn Tyr Ser His Ser Glu Pro Asp Thr Thr
   11365 11370 11375
   Pro Ser Ile Ala Thr Ser Pro Gly Ala Glu Ala Thr Ser Asp Phe Pro
   11380 11385 11390
   Thr Ile Thr Val Ser Pro Asp Val Pro Asp Met Val Thr Ser Gln Val
   11395 11400 11405
   Thr Ser Ser Gly Thr Asp Thr Ser Ile Thr Ile Pro Thr Leu Thr Leu
   11410 11415 11420
   Ser Ser Gly Glu Pro Glu Thr Thr Thr Ser Phe Ile Thr Tyr Ser Glu
   11425 11430 11435 11440
   Thr His Thr Ser Ser Ala Ile Pro Thr Leu Pro Val Ser Pro Gly Ala
   11445 11450 11455
   Ser Lys Met Leu Thr Ser Leu Val Ile Ser Ser Gly Thr Asp Ser Thr
   11460 11465 11470
   Thr Thr Phe Pro Thr Leu Thr Glu Thr Pro Tyr Glu Pro Glu Thr Thr
   11475 11480 11485
   Ala Ile Gln Leu Ile His Pro Ala Glu Thr Asn Thr Met Val Pro Arg
   11490 11495 11500
   Thr Thr Pro Lys Phe Ser His Ser Lys Ser Asp Thr Thr Leu Pro Val
   11505 11510 11515 11520
   Ala Ile Thr Ser Pro Gly Pro Glu Ala Ser Ser Ala Val Ser Thr Thr
   11525 11530 11535
   Thr Ile Ser Pro Asp Met Ser Asp Leu Val Thr Ser Leu Val Pro Ser
   11540 11545 11550
   Ser Gly Thr Asp Thr Ser Thr Thr Phe Pro Thr Leu Ser Glu Thr Pro
   11555 11560 11565
   Tyr Glu Pro Glu Thr Thr Ala Thr Trp Leu Thr His Pro Ala Glu Thr
   11570 11575 11580
   Ser Thr Thr Val Ser Gly Thr Ile Pro Asn Phe Ser His Arg Gly Ser
   11585 11590 11595 11600
   Asp Thr Ala Pro Ser Met Val Thr Ser Pro Gly Val Asp Thr Arg Ser
   11605 11610 11615
   Gly Val Pro Thr Thr Thr Ile Pro Pro Ser Ile Pro Gly Val Val Thr
   11620 11625 11630
   Ser Gln Val Thr Ser Ser Ala Thr Asp Thr Ser Thr Ala Ile Pro Thr
   11635 11640 11645
   Leu Thr Pro Ser Pro Gly Glu Pro Glu Thr Thr Ala Ser Ser Ala Thr
   11650 11655 11660
   His Pro Gly Thr Gln Thr Gly Phe Thr Val Pro Ile Arg Thr Val Pro
   11665 11670 11675 11680
   Ser Ser Glu Pro Asp Thr Met Ala Ser Trp Val Thr His Pro Pro Gln
   11685 11690 11695
   Thr Ser Thr Pro Val Ser Arg Thr Thr Ser Ser Phe Ser His Ser Ser
   11700 11705 11710
   Pro Asp Ala Thr Pro Val Met Ala Thr Ser Pro Arg Thr Glu Ala Ser
   11715 11720 11725
   Ser Ala Val Leu Thr Thr Ile Ser Pro Gly Ala Pro Glu Met Val Thr
   11730 11735 11740
   Ser Gln Ile Thr Ser Ser Gly Ala Ala Thr Ser Thr Thr Val Pro Thr
   11745 11750 11755 11760
   Leu Thr His Ser Pro Gly Met Pro Glu Thr Thr Ala Leu Leu Ser Thr
   11765 11770 11775
   His Pro Arg Thr Glu Thr Ser Lys Thr Phe Pro Ala Ser Thr Val Phe
   11780 11785 11790
   Pro Gln Val Ser Glu Thr Thr Ala Ser Leu Thr Ile Arg Pro Gly Ala
   11795 11800 11805
   Glu Thr Ser Thr Ala Leu Pro Thr Gln Thr Thr Ser Ser Leu Phe Thr
   11810 11815 11820
   Leu Leu Val Thr Gly Thr Ser Arg Val Asp Leu Ser Pro Thr Ala Ser
   11825               11830               11835               11840
   Pro Gly Val Ser Ala Lys Thr Ala Pro Leu Ser Thr His Pro Gly Thr
                   11845               11850               11855
   Glu Thr Ser Thr Met Ile Pro Thr Ser Thr Leu Ser Leu Gly Leu Leu
               11860               11865               11870
   Glu Thr Thr Gly Leu Leu Ala Thr Ser Ser Ser Ala Glu Thr Ser Thr
           11875               11880               11885
   Ser Thr Leu Thr Leu Thr Val Ser Pro Ala Val Ser Gly Leu Ser Ser
       11890               11895               11900
   Ala Ser Ile Thr Thr Asp Lys Pro Gln Thr Val Thr Ser Trp Asn Thr
   11905               11910               11915               11920
   Glu Thr Ser Pro Ser Val Thr Ser Val Gly Pro Pro Glu Phe Ser Arg
                   11925               11930               11935
   Thr Val Thr Gly Thr Thr Met Thr Leu Ile Pro Ser Glu Met Pro Thr
               11940               11945               11950
   Pro Pro Lys Thr Ser His Gly Glu Gly Val Ser Pro Thr Thr Ile Leu
           11955               11960               11965
   Arg Thr Thr Met Val Glu Ala Thr Asn Leu Ala Thr Thr Gly Ser Ser
       11970               11975               11980
   Pro Thr Val Ala Lys Thr Thr Thr Thr Phe Asn Thr Leu Ala Gly Ser
   11985               11990               11995               12000
   Leu Phe Thr Pro Leu Thr Thr Pro Gly Met Ser Thr Leu Ala Ser Glu
                   12005               12010               12015
   Ser Val Thr Ser Arg Thr Ser Tyr Asn His Arg Ser Trp Ile Ser Thr
               12020               12025               12030
   Thr Ser Ser Tyr Asn Arg Arg Tyr Trp Thr Pro Ala Thr Ser Thr Pro
           12035               12040               12045
   Val Thr Ser Thr Phe Ser Pro Gly Ile Ser Thr Ser Ser Ile Pro Ser
       12050               12055               12060
   Ser Thr Ala Ala Thr Val Pro Phe Met Val Pro Phe Thr Leu Asn Phe
   12065               12070               12075               12080
   Thr Ile Thr Asn Leu Gln Tyr Glu Glu Asp Met Arg His Pro Gly Ser
                   12085               12090               12095
   Arg Lys Phe Asn Ala Thr Glu Arg Glu Leu Gln Gly Leu Leu Lys Pro
               12100               12105               12110
   Leu Phe Arg Asn Ser Ser Leu Glu Tyr Leu Tyr Ser Gly Cys Arg Leu
           12115               12120               12125
   Ala Ser Leu Arg Pro Glu Lys Asp Ser Ser Ala Thr Ala Val Asp Ala
       12130               12135               12140
   Ile Cys Thr His Arg Pro Asp Pro Glu Asp Leu Gly Leu Asp Arg Glu
   12145               12150               12155               12160
   Arg Leu Tyr Trp Glu Leu Ser Asn Leu Thr Asn Gly Ile Gln Glu Leu
                   12165               12170               12175
   Gly Pro Tyr Thr Leu Asp Arg Asn Ser Leu Tyr Val Asn Gly Phe Thr
               12180               12185               12190
   His Arg Ser Ser Met Pro Thr Thr Ser Thr Pro Gly Thr Ser Thr Val
           12195               12200               12205
   Asp Val Gly Thr Ser Gly Thr Pro Ser Ser Ser Pro Ser Pro Thr Thr
       12210               12215               12220
   Ala Gly Pro Leu Leu Met Pro Phe Thr Leu Asn Phe Thr Ile Thr Asn
   12225               12230               12235               12240
   Leu Gln Tyr Glu Glu Asp Met Arg Arg Thr Gly Ser Arg Lys Phe Asn
                   12245               12250               12255
   Thr Met Glu Ser Val Leu Gln Gly Leu Leu Lys Pro Leu Phe Lys Asn
               12260               12265               12270
   Thr Ser Val Gly Pro Leu Tyr Ser Gly Cys Arg Leu Thr Leu Leu Arg
           12275               12280               12285
   Pro Glu Lys Asp Gly Ala Ala Thr Gly Val Asp Ala Ile Cys Thr His
       12290               12295               12300
   Arg Leu Asp Pro Lys Ser Pro Gly Leu Asn Arg Glu Gln Leu Tyr Trp
   12305               12310               12315               12320
   Glu Leu Ser Lys Leu Thr Asn Asp Ile Glu Glu Leu Gly Pro Tyr Thr
                   12325               12330               12335
   Leu Asp Arg Asn Ser Leu Tyr Val Asn Gly Phe Thr His Gln Ser Ser
               12340               12345               12350
   Val Ser Thr Thr Ser Thr Pro Gly Thr Ser Thr Val Asp Leu Arg Thr
           12355               12360               12365
   Ser Gly Thr Pro Ser Ser Leu Ser Ser Pro Thr Ile Met Ala Ala Gly
       12370               12375               12380
   Pro Leu Leu Val Pro Phe Thr Leu Asn Phe Thr Ile Thr Asn Leu Gln
   12385               12390               12395               12400
   Tyr Gly Glu Asp Met Gly His Pro Gly Ser Arg Lys Phe Asn Thr Thr
                   12405               12410               12415
   Glu Arg Val Leu Gln Gly Leu Leu Gly Pro Ile Phe Lys Asn Thr Ser
               12420               12425               12430
   Val Gly Pro Leu Tyr Ser Gly Cys Arg Leu Thr Ser Leu Arg Ser Glu
           12435               12440               12445
   Lys Asp Gly Ala Ala Thr Gly Val Asp Ala Ile Cys Ile His His Leu
       12450               12455               12460
   Asp Pro Lys Ser Pro Gly Leu Asn Arg Glu Arg Leu Tyr Trp Glu Leu
   12465               12470               12475               12480
   Ser Gln Leu Thr Asn Gly Ile Lys Glu Leu Gly Pro Tyr Thr Leu Asp
                   12485               12490               12495
   Arg Asn Ser Leu Tyr Val Asn Gly Phe Thr His Arg Thr Ser Val Pro
               12500               12505               12510
   Thr Ser Ser Thr Pro Gly Thr Ser Thr Val Asp Leu Gly Thr Ser Gly
           12515               12520               12525
   Thr Pro Phe Ser Leu Pro Ser Pro Ala Thr Ala Gly Pro Leu Leu Val
       12530               12535               12540
   Leu Phe Thr Leu Asn Phe Thr Ile Thr Asn Leu Lys Tyr Glu Glu Asp
   12545               12550               12555               12560
   Met His Arg Pro Gly Ser Arg Lys Phe Asn Thr Thr Glu Arg Val Leu
                   12565               12570               12575
   Gln Thr Leu Leu Gly Pro Met Phe Lys Asn Thr Ser Val Gly Leu Leu
               12580               12585               12590
   Tyr Ser Gly Cys Arg Leu Thr Leu Leu Arg Ser Glu Lys Asp Gly Ala
           12595               12600               12605
   Ala Thr Gly Val Asp Ala Ile Cys Thr His Arg Leu Asp Pro Lys Ser
       12610               12615               12620
   Pro Gly Val Asp Arg Glu Gln Leu Tyr Trp Glu Leu Ser Gln Leu Thr
   12625               12630               12635               12640
   Asn Gly Ile Lys Glu Leu Gly Pro Tyr Thr Leu Asp Arg Asn Ser Leu
                   12645               12650               12655
   Tyr Val Asn Gly Phe Thr His Trp Ile Pro Val Pro Thr Ser Ser Thr
               12660               12665               12670
   Pro Gly Thr Ser Thr Val Asp Leu Gly Ser Gly Thr Pro Ser Ser Leu
           12675               12680               12685
   Pro Ser Pro Thr Thr Ala Gly Pro Leu Leu Val Pro Phe Thr Leu Asn
       12690               12695               12700
   Phe Thr Ile Thr Asn Leu Lys Tyr Glu Glu Asp Met His Cys Pro Gly
   12705               12710               12715               12720
   Ser Arg Lys Phe Asn Thr Thr Glu Arg Val Leu Gln Ser Leu Leu Gly
                   12725               12730               12735
   Pro Met Phe Lys Asn Thr Ser Val Gly Pro Leu Tyr Ser Gly Cys Arg
               12740               12745               12750
   Leu Thr Leu Leu Arg Ser Glu Lys Asp Gly Ala Ala Thr Gly Val Asp
           12755               12760               12765
   Ala Ile Cys Thr His Arg Leu Asp Pro Lys Ser Pro Gly Val Asp Arg
       12770               12775               12780
   Glu Gln Leu Tyr Trp Glu Leu Ser Gln Leu Thr Asn Gly Ile Lys Glu
   12785               12790               12795               12800
   Leu Gly Pro Tyr Thr Leu Asp Arg Asn Ser Leu Tyr Val Asn Gly Phe
   12805 12810 12815
   Thr His Gln Thr Ser Ala Pro Asn Thr Ser Thr Pro Gly Thr Ser Thr
   12820 12825 12830
   Val Asp Leu Gly Thr Ser Gly Thr Pro Ser Ser Leu Pro Ser Pro Thr
   12835 12840 12845
   Ser Ala Gly Pro Leu Leu Val Pro Phe Thr Leu Asn Phe Thr Ile Thr
   12850 12855 12860
   Asn Leu Gln Tyr Glu Glu Asp Met His His Pro Gly Ser Arg Lys Phe
   12865 12870 12875 12880
   Asn Thr Thr Glu Arg Val Leu Gln Gly Leu Leu Gly Pro Met Phe Lys
   12885 12890 12895
   Asn Thr Ser Val Gly Leu Leu Tyr Ser Gly Cys Arg Leu Thr Leu Leu
   12900 12905 12910
   Arg Pro Glu Lys Asn Gly Ala Ala Thr Gly Met Asp Ala Ile Cys Ser
   12915 12920 12925
   His Arg Leu Asp Pro Lys Ser Pro Gly Leu Asn Arg Glu Gln Leu Tyr
   12930 12935 12940
   Trp Glu Leu Ser Gln Leu Thr His Gly Ile Lys Glu Leu Gly Pro Tyr
   12945 12950 12955 12960
   Thr Leu Asp Arg Asn Ser Leu Tyr Val Asn Gly Phe Thr His Arg Ser
   12965 12970 12975
   Ser Val Ala Pro Thr Ser Thr Pro Gly Thr Ser Thr Val Asp Leu Gly
   12980 12985 12990
   Thr Ser Gly Thr Pro Ser Ser Leu Pro Ser Pro Thr Thr Ala Val Pro
   12995 13000 13005
   Leu Leu Val Pro Phe Thr Leu Asn Phe Thr Ile Thr Asn Leu Gln Tyr
   13010 13015 13020
   Gly Glu Asp Met Arg His Pro Gly Ser Arg Lys Phe Asn Thr Thr Glu
   13025 13030 13035 13040
   Arg Val Leu Gln Gly Leu Leu Gly Pro Leu Phe Lys Asn Ser Ser Val
   13045 13050 13055
   Gly Pro Leu Tyr Ser Gly Cys Arg Leu Ile Ser Leu Arg Ser Glu Lys
   13060 13065 13070
   Asp Gly Ala Ala Thr Gly Val Asp Ala Ile Cys Thr His His Leu Asn
   13075 13080 13085
   Pro Gln Ser Pro Gly Leu Asp Arg Glu Gln Leu Tyr Trp Gln Leu Ser
   13090 13095 13100
   Gln Met Thr Asn Gly Ile Lys Glu Leu Gly Pro Tyr Thr Leu Asp Arg
   13105 13110 13115 13120
   Asn Ser Leu Tyr Val Asn Gly Phe Thr His Arg Ser Ser Gly Leu Thr
   13125 13130 13135
   Thr Ser Thr Pro Trp Thr Ser Thr Val Asp Leu Gly Thr Ser Gly Thr
   13140 13145 13150
   Pro Ser Pro Val Pro Ser Pro Thr Thr Thr Gly Pro Leu Leu Val Pro
   13155 13160 13165
   Phe Thr Leu Asn Phe Thr Ile Thr Asn Leu Gln Tyr Glu Glu Asn Met
   13170 13175 13180
   Gly His Pro Gly Ser Arg Lys Phe Asn Ile Thr Glu Ser Val Leu Gln
   13185 13190 13195 13200
   Gly Leu Leu Lys Pro Leu Phe Lys Ser Thr Ser Val Gly Pro Leu Tyr
   13205 13210 13215
   Ser Gly Cys Arg Leu Thr Leu Leu Arg Pro Glu Lys Asp Gly Val Ala
   13220 13225 13230
   Thr Arg Val Asp Ala Ile Cys Thr His Arg Pro Asp Pro Lys Ile Pro
   13235 13240 13245
   Gly Leu Asp Arg Gln Gln Leu Tyr Trp Glu Leu Ser Gln Leu Thr His
   13250 13255 13260
   Ser Ile Thr Glu Leu Gly Pro Tyr Thr Leu Asp Arg Asp Ser Leu Tyr
   13265 13270 13275 13280
   Val Asn Gly Phe Thr Gln Arg Ser Ser Val Pro Thr Thr Ser Thr Pro
   13285 13290 13295
   Gly Thr Phe Thr Val Gln Pro Glu Thr Ser Glu Thr Pro Ser Ser Leu
               13300               13305               13310
   Pro Gly Pro Thr Ala Thr Gly Pro Val Leu Leu Pro Phe Thr Leu Asn
           13315               13320               13325
   Phe Thr Ile Thr Asn Leu Gln Tyr Glu Glu Asp Met Arg Arg Pro Gly
       13330               13335               13340
   Ser Arg Lys Phe Asn Thr Thr Glu Arg Val Leu Gln Gly Leu Leu Met
   13345               13350               13355               13360
   Pro Leu Phe Lys Asn Thr Ser Val Ser Ser Leu Tyr Ser Gly Cys Arg
                   13365               13370               13375
   Leu Thr Leu Leu Arg Pro Glu Lys Asp Gly Ala Ala Thr Arg Val Asp
               13380               13385               13390
   Ala Val Cys Thr His Arg Pro Asp Pro Lys Ser Pro Gly Leu Asp Arg
           13395               13400               13405
   Glu Arg Leu Tyr Trp Lys Leu Ser Gln Leu Thr His Gly Ile Thr Glu
       13410               13415               13420
   Leu Gly Pro Tyr Thr Leu Asp Arg His Ser Leu Tyr Val Asn Gly Phe
   13425               13430               13435               13440
   Thr His Gln Ser Ser Met Thr Thr Thr Arg Thr Pro Asp Thr Ser Thr
                   13445               13450               13455
   Met His Leu Ala Thr Ser Arg Thr Pro Ala Ser Leu Ser Gly Pro Met
               13460               13465               13470
   Thr Ala Ser Pro Leu Leu Val Leu Phe Thr Ile Asn Phe Thr Ile Thr
           13475               13480               13485
   Asn Leu Arg Tyr Glu Glu Asn Met His His Pro Gly Ser Arg Lys Phe
       13490               13495               13500
   Asn Thr Thr Glu Arg Val Leu Gln Gly Leu Leu Arg Pro Val Phe Lys
   13505               13510               13515               13520
   Asn Thr Ser Val Gly Pro Leu Tyr Ser Gly Cys Arg Leu Thr Leu Leu
                   13525               13530               13535
   Arg Pro Lys Lys Asp Gly Ala Ala Thr Lys Val Asp Ala Ile Cys Thr
               13540               13545               13550
   Tyr Arg Pro Asp Pro Lys Ser Pro Gly Leu Asp Arg Glu Gln Leu Tyr
           13555               13560               13565
   Trp Glu Leu Ser Gln Leu Thr His Ser Ile Thr Glu Leu Gly Pro Tyr
       13570               13575               13580
   Thr Leu Asp Arg Asp Ser Leu Tyr Val Asn Gly Phe Thr Gln Arg Ser
   13585               13590               13595               13600
   Ser Val Pro Thr Thr Ser Ile Pro Gly Thr Pro Thr Val Asp Leu Gly
                   13605               13610               13615
   Thr Ser Gly Thr Pro Val Ser Lys Pro Gly Pro Ser Ala Ala Ser Pro
               13620               13625               13630
   Leu Leu Val Leu Phe Thr Leu Asn Phe Thr Ile Thr Asn Leu Arg Tyr
           13635               13640               13645
   Glu Glu Asn Met Gln His Pro Gly Ser Arg Lys Phe Asn Thr Thr Glu
       13650               13655               13660
   Arg Val Leu Gln Gly Leu Leu Arg Ser Leu Phe Lys Ser Thr Ser Val
   13665               13670               13675               13680
   Gly Pro Leu Tyr Ser Gly Cys Arg Leu Thr Leu Leu Arg Pro Glu Lys
                   13685               13690               13695
   Asp Gly Thr Ala Thr Gly Val Asp Ala Ile Cys Thr His His Pro Asp
               13700               13705               13710
   Pro Lys Ser Pro Arg Leu Asp Arg Glu Gln Leu Tyr Trp Glu Leu Ser
           13715               13720               13725
   Gln Leu Thr His Asn Ile Thr Glu Leu Gly Pro Tyr Ala Leu Asp Asn
       13730               13735               13740
   Asp Ser Leu Phe Val Asn Gly Phe Thr His Arg Ser Ser Val Ser Thr
   13745               13750               13755               13760
   Thr Ser Thr Pro Gly Thr Pro Thr Val Tyr Leu Gly Ala Ser Lys Thr
                   13765               13770               13775
   Pro Ala Ser Ile Phe Gly Pro Ser Ala Ala Ser His Leu Leu Ile Leu
                   13780                   13785                   13790
   Phe Thr Leu Asn Phe Thr Ile Thr Asn Leu Arg Tyr Glu Glu Asn Met
               13795                   13800                   13805
   Trp Pro Gly Ser Arg Lys Phe Asn Thr Thr Glu Arg Val Leu Gln Gly
           13810                   13815                   13820
   Leu Leu Arg Pro Leu Phe Lys Asn Thr Ser Val Gly Pro Leu Tyr Ser
   13825                   13830                   13835                   13840
   Gly Cys Arg Leu Thr Leu Leu Arg Pro Glu Lys Asp Gly Glu Ala Thr
                       13845                   13850                   13855
   Gly Val Asp Ala Ile Cys Thr His Arg Pro Asp Pro Thr Gly Pro Gly
                   13860                   13865                   13870
   Leu Asp Arg Glu Gln Leu Tyr Leu Glu Leu Ser Gln Leu Thr His Ser
               13875                   13880                   13885
   Ile Thr Glu Leu Gly Pro Tyr Thr Leu Asp Arg Asp Ser Leu Tyr Val
           13890                   13895                   13900
   Asn Gly Phe Thr His Arg Ser Ser Val Pro Thr Thr Ser Thr Gly Val
   13905                   13910                   13915                   13920
   Val Ser Glu Glu Pro Phe Thr Leu Asn Phe Thr Ile Asn Asn Leu Arg
                       13925                   13930                   13935
   Tyr Met Ala Asp Met Gly Gln Pro Gly Ser Leu Lys Phe Asn Ile Thr
                   13940                   13945                   13950
   Asp Asn Val Met Gln His Leu Leu Ser Pro Leu Phe Gln Arg Ser Ser
               13955                   13960                   13965
   Leu Gly Ala Arg Tyr Thr Gly Cys Arg Val Ile Ala Leu Arg Ser Val
           13970                   13975                   13980
   Lys Asn Gly Ala Glu Thr Arg Val Asp Leu Leu Cys Thr Tyr Leu Gln
   13985                   13990                   13995                   14000
   Pro Leu Ser Gly Pro Gly Leu Pro Ile Lys Gln Val Phe His Glu Leu
                       14005                   14010                   14015
   Ser Gln Gln Thr His Gly Ile Thr Arg Leu Gly Pro Tyr Ser Leu Asp
                   14020                   14025                   14030
   Lys Asp Ser Leu Tyr Leu Asn Gly Tyr Asn Glu Pro Gly Pro Asp Glu
               14035                   14040                   14045
   Pro Pro Thr Thr Pro Lys Pro Ala Thr Thr Phe Leu Pro Pro Leu Ser
           14050                   14055                   14060
   Glu Ala Thr Thr Ala Met Gly Tyr His Leu Lys Thr Leu Thr Leu Asn
   14065                   14070                   14075                   14080
   Phe Thr Ile Ser Asn Leu Gln Tyr Ser Pro Asp Met Gly Lys Gly Ser
                       14085                   14090                   14095
   Ala Thr Phe Asn Ser Thr Glu Gly Val Leu Gln His Leu Leu Arg Pro
                   14100                   14105                   14110
   Leu Phe Gln Lys Ser Ser Met Gly Pro Phe Tyr Leu Gly Cys Gln Leu
               14115                   14120                   14125
   Ile Ser Leu Arg Pro Glu Lys Asp Gly Ala Ala Thr Gly Val Asp Thr
           14130                   14135                   14140
   Thr Cys Thr Tyr His Pro Asp Pro Val Gly Pro Gly Leu Asp Ile Gln
   14145                   14150                   14155                   14160
   Gln Leu Tyr Trp Glu Leu Ser Gln Leu Thr His Gly Val Thr Gln Leu
                       14165                   14170                   14175
   Gly Phe Tyr Val Leu Asp Arg Asp Ser Leu Phe Ile Asn Gly Tyr Ala
                   14180                   14185                   14190
   Pro Gln Asn Leu Ser Ile Arg Gly Glu Tyr Gln Ile Asn Phe His Ile
               14195                   14200                   14205
   Val Asn Trp Asn Leu Ser Asn Pro Asp Pro Thr Ser Ser Glu Tyr Ile
           14210                   14215                   14220
   Thr Leu Leu Arg Asp Ile Gln Asp Lys Val Thr Thr Leu Tyr Lys Gly
   14225                   14230                   14235                   14240
   Ser Gln Leu His Asp Thr Phe Arg Phe Cys Leu Val Thr Asn Leu Thr
                       14245                   14250                   14255
   Met Asp Ser Val Leu Val Thr Val Lys Ala Leu Phe Ser Ser Asn Leu
                   14260                   14265                   14270
   Asp Pro Ser Leu Val Glu Gln Val Phe Leu Asp Lys Thr Leu Asn Ala
           14275                   14280                   14285
   Ser Phe His Trp Leu Gly Ser Thr Tyr Gln Leu Val Asp Ile His Val
       14290                   14295                   14300
   Thr Glu Met Glu Ser Ser Val Tyr Gln Pro Thr Ser Ser Ser Ser Thr
   14305                   14310                   14315                   14320
   Gln His Phe Tyr Leu Asn Phe Thr Ile Thr Asn Leu Pro Tyr Ser Gln
                   14325                   14330                   14335
   Asp Lys Ala Gln Pro Gly Thr Thr Asn Tyr Gln Arg Asn Lys Arg Asn
               14340                   14345                   14350
   Ile Glu Asp Ala Leu Asn Gln Leu Phe Arg Asn Ser Ser Ile Lys Ser
           14355                   14360                   14365
   Tyr Phe Ser Asp Cys Gln Val Ser Thr Phe Arg Ser Val Pro Asn Arg
       14370                   14375                   14380
   His His Thr Gly Val Asp Ser Leu Cys Asn Phe Ser Pro Leu Ala Arg
   14385                   14390                   14395                   14400
   Arg Val Asp Arg Val Ala Ile Tyr Glu Glu Phe Leu Arg Met Thr Arg
                   14405                   14410                   14415
   Asn Gly Thr Gln Leu Gln Asn Phe Thr Leu Asp Arg Ser Ser Val Leu
               14420                   14425                   14430
   Val Asp Gly Tyr Ser Pro Asn Arg Asn Glu Pro Leu Thr Gly Asn Ser
           14435                   14440                   14445
   Asp Leu Pro Phe Trp Ala Val Ile Leu Ile Gly Leu Ala Gly Leu Leu
       14450                   14455                   14460
   Gly Val Ile Thr Cys Leu Ile Cys Gly Val Leu Val Thr Thr Arg Arg
   14465                   14470                   14475                   14480
   Arg Lys Lys Glu Gly Glu Tyr Asn Val Gln Gln Gln Cys Pro Gly Tyr
                   14485                   14490                   14495
   Tyr Gln Ser His Leu Asp Leu Glu Asp Leu Gln
               14500                   14505
<210> 58
   <211> 43816
   <212> DNA
   <213> Homo sapiens
<400> 58
   aagcgttgca caattccccc aacctccata catacggcag ctcttctaga cacaggtttt 60
   cccaggtcaa atgcggggac cccagccata tctcccaccc tgagaaattt tggagtttca 120
   gggagctcag aagctctgca gaggccaccc tctctgaggg gattcttctt agacctccat 180
   ccagaggcaa atgttgacct gtccatgctg aaaccctcag gccttcctgg gtcatcttct 240
   cccacccgct ccttgatgac agggagcagg agcactaaag ccacaccaga aatggattca 300
   ggactgacag gagccacctt gtcacctaag acatctacag gtgcaatcgt ggtgacagaa 360
   catactctgc cctttacttc cccagataag accttggcca gtcctacatc ttcggttgtg 420
   ggaagaacca cccagtcttt gggggtgatg tcctctgctc tccctgagtc aacctctaga 480
   ggaatgacac actccgagca aagaaccagc ccatcgctga gtccccaggt caatggaact 540
   ccctctagga actaccctgc tacaagcatg gtttcaggat tgagttcccc aaggaccagg 600
   accagttcca cagaaggaaa ttttaccaaa gaagcatcta catacacact cactgtagag 660
   accacaagtg gcccagtcac tgagaagtac acagtcccca ctgagacctc aacaactgaa 720
   ggtgacagca cagagacccc ctgggacaca agatatattc ctgtaaaaat cacatctcca 780
   atgaaaacat ttgcagattc aactgcatcc aaggaaaatg ccccagtgtc tatgactcca 840
   gctgagacca cagttactga ctcacatact ccaggaagga caaacccatc atttgggaca 900
   ctttattctt ccttccttga cctatcacct aaagggaccc caaattccag aggtgaaaca 960
   agcctggaac tgattctatc aaccactgga tatcccttct cctctcctga acctggctct 1020
   gcaggacaca gcagaataag taccagtgcg cctttgtcat catctgcttc agttctcgat 1080
   aataaaatat cagagaccag catattctca ggccagagtc tcacctcccc tctgtctcct 1140
   ggggtgcccg aggccagagc cagcacaatg cccaactcag ctatcccttt ttccatgaca 1200
   ctaagcaatg cagaaacaag tgccgaaagg gtcagaagca caatttcctc tctggggact 1260
   ccatcaatat ccacaaagca gacagcagag actatcctta ccttccatgc cttcgctgag 1320
   accatggata tacccagcac ccacatagcc aagactttgg cttcagaatg gttgggaagt 1380
   ccaggtaccc ttggtggcac cagcacttca gcgctgacaa ccacatctcc atctaccact 1440
   ttagtctcag aggagaccaa cacccatcac tccacgagtg gaaaggaaac agaaggaact 1500
   ttgaatacat ctatgactcc acttgagacc tctgctcctg gagaagagtc cgaaatgact 1560
   gccaccttgg tccccactct aggttttaca actcttgaca gcaagatcag aagtccatct 1620
   caggtctctt catcccaccc aacaagagag ctcagaacca caggcagcac ctctgggagg 1680
   cagagttcca gcacagctgc ccacgggagc tctgacatcc tgagggcaac cacttccagc 1740
   acctcaaaag catcatcatg gaccagtgaa agcacagctc agcaatttag tgaaccccag 1800
   cacacacagt gggtggagac aagtcctagc atgaaaacag agagaccccc agcatcaacc 1860
   agtgtggcag cccctatcac cacttctgtt ccctcagtgg tctctggctt caccaccctg 1920
   aagaccagct ccacaaaagg gatttggctt gaagaaacat ctgcagacac actcatcgga 1980
   gaatccacag ctggcccaac cacccatcag tttgctgttc ccactgggat ttcaatgaca 2040
   ggaggcagca gcaccagggg aagccagggc acaacccacc tactcaccag agccacagca 2100
   tcatctgaga catccgcaga tttgactctg gccacgaacg gtgtcccagt ctccgtgtct 2160
   ccagcagtga gcaagacggc tgctggctca agtcctccag gagggacaaa gccatcatat 2220
   acaatggttt cttctgtcat ccctgagaca tcatctctac agtcctcagc tttcagggaa 2280
   ggaaccagcc tgggactgac tccattaaac actagacatc ccttctcttc ccctgaacca 2340
   gactctgcag gacacaccaa gataagcacc agcattcctc tgttgtcatc tgcttcagtt 2400
   cttgaggata aagtgtcagc gaccagcaca ttctcacacc acaaagccac ctcatctatt 2460
   accacaggga ctcctgaaat ctcaacaaag acaaagccca gctcagccgt tctttcctcc 2520
   atgaccctaa gcaatgcagc aacaagtcct gaaagagtca gaaatgcaac ttcccctctg 2580
   actcatccat ctccatcagg ggaagagaca gcagggagtg tcctcactct cagcacctct 2640
   gctgagacta cagactcacc taacatccac ccaactggga cactgacttc agaatcgtca 2700
   gagagtccta gcactctcag cctcccaagt gtctctggag tcaaaaccac attttcttca 2760
   tctactcctt ccactcatct atttactagt ggagaagaaa cagaggaaac ttcgaatcca 2820
   tctgtgtctc aacctgagac ttctgtttcc agagtaagga ccaccttggc cagcacctct 2880
   gtccctaccc cagtattccc caccatggac acctggccta cacgttcagc tcagttctct 2940
   tcatcccacc tagtgagtga gctcagagct acgagcagta cctcagttac aaactcaact 3000
   ggttcagctc ttcctaaaat atctcacctc actgggacgg caacaatgtc acagaccaat 3060
   agagacacgt ttaatgactc tgctgcaccc caaagcacaa cttggccaga gactagtccc 3120
   agattcaaga cagggttacc ttcagcaaca accactgttt caacctctgc cacttctctc 3180
   tctgctactg taatggtctc taaattcact tctccagcaa ctagttccat ggaagcaact 3240
   tctatcaggg aaccatcaac aaccatcctc acaacagaga ccacgaatgg cccaggctct 3300
   atggctgtgg cttctaccaa catcccaatt ggaaagggct acattactga aggaagattg 3360
   gacacaagcc atctgcccat tggaaccaca gcttcctctg agacatctat ggattttacc 3420
   atggccaaag aaagtgtctc aatgtcagta tctccatctc agtccatgga tgctgctggc 3480
   tcaagcactc caggaaggac aagccaattc gttgacacat tttctgatga tgtctatcat 3540
   ttaacatcca gagaaattac aatacctaga gatggaacaa gctcagctct gactccacaa 3600
   atgactgcaa ctcaccctcc atctcctgat cctggctctg ctagaagcac ctggcttggc 3660
   atcttgtcct catctccttc ttctcctact cccaaagtca caatgagctc cacattttca 3720
   actcagagag tcaccacaag catgataatg gacacagttg aaactagtcg gtggaacatg 3780
   cccaacttac cttccacgac ttccttgaca ccaagtaata ttccaacaag tggtgccata 3840
   ggaaaaagca ccctggttcc cttggacact ccatctccag ccacatcatt ggaggcatca 3900
   gaagggggac ttccaaccct cagcacctac cctgaatcaa caaacacacc cagcatccac 3960
   ctcggagcac acgctagttc agaaagtcca agcaccatca aacttaccat ggcttcagta 4020
   gtaaaacctg gctcttacac acctctcacc ttcccctcaa tagagaccca cattcatgta 4080
   tcaacagcca gaatggctta ctcttctggg tcttcacctg agatgacagc tcctggagag 4140
   actaacactg gtagtacctg ggaccccacc acctacatca ccactacgga tcctaaggat 4200
   acaagttcag ctcaggtctc tacaccccac tcagtgagga cactcagaac cacagaaaac 4260
   catccaaaga cagagtccgc caccccagct gcttactctg gaagtcctaa aatctcaagt 4320
   tcacccaatc tcaccagtcc ggccacaaaa gcatggacca tcacagacac aactgaacac 4380
   tccactcaat tacattacac aaaattggca gaaaaatcat ctggatttga gacacagtca 4440
   gctccaggac ctgtctctgt agtaatccct acctccccta ccattggaag cagcacattg 4500
   gaactaactt ctgatgtccc aggggaaccc ctggtccttg ctcccagtga gcagaccaca 4560
   atcactctcc ccatggcaac atggctgagt accagtttga cagaggaaat ggcttcaaca 4620
   gaccttgata tttcaagtcc aagttcaccc atgagtacat ttgctatttt tccacctatg 4680
   tccacacctt ctcatgaact ttcaaagtca gaggcagata ccagtgccat tagaaataca 4740
   gattcaacaa cgttggatca gcacctagga atcaggagtt tgggcagaac tggggactta 4800
   acaactgttc ctatcacccc actgacaacc acgtggacca gtgtgattga acactcaaca 4860
   caagcacagg acaccctttc tgcaacgatg agtcctactc acgtgacaca gtcactcaaa 4920
   gatcaaacat ctataccagc ctcagcatcc ccttcccatc ttactgaagt ctaccctgag 4980
   ctcgggacac aagggagaag ctcctctgag gcaaccactt tttggaaacc atctacagac 5040
   acactgtcca gagagattga gactggccca acaaacattc aatccactcc acccatggac 5100
   aacacaacaa cagggagcag tagtagtgga gtcaccctgg gcatagccca ccttcccata 5160
   ggaacatcct ccccagctga gacatccaca aacatggcac tggaaagaag aagttctaca 5220
   gccactgtct ctatggctgg gacaatggga ctccttgtta ctagtgctcc aggaagaagc 5280
   atcagccagt cattaggaag agtttcctct gtcctttctg agtcaactac tgaaggagtc 5340
   acagattcta gtaagggaag cagcccaagg ctgaacacac agggaaatac agctctctcc 5400
   tcctctcttg aacccagcta tgctgaagga agccagatga gcacaagcat ccctctaacc 5460
   tcatctccta caactcctga tgtggaattc atagggggca gcacattttg gaccaaggag 5520
   gtcaccacag ttatgacctc agacatctcc aagtcttcag caaggacaga gtccagctca 5580
   gctaccctta tgtccacagc tttgggaagc actgaaaata caggaaaaga aaaactcaga 5640
   actgcctcta tggatcttcc atctccaact ccatcaatgg aggtgacacc atggatttct 5700
   ctcactctca gtaatgcccc caataccaca gattcacttg acctcagcca tggggtgcac 5760
   accagctctg cagggacttt ggccactgac aggtcattga atactggtgt cactagagcc 5820
   tccagattgg aaaacggctc tgatacctct tctaagtccc tgtctatggg aaacagcact 5880
   cacacttcca tgacttacac agagaagagt gaagtgtctt cttcaatcca tccccgacct 5940
   gagacctcag ctcctggagc agagaccact ttgacttcca ctcctggaaa cagggccata 6000
   agcttaacat tgcctttttc atccattcca gtggaagaag tcatttctac aggcataacc 6060
   tcaggaccag acatcaactc agcacccatg acacattctc ccatcacccc accaacaatt 6120
   gtatggacca gtacaggcac aattgaacag tccactcaac cactacatgc agtttcttca 6180
   gaaaaagttt ctgtgcagac acagtcaact ccatatgtca actctgtggc agtgtctgct 6240
   tcccctaccc atgagaattc agtctcttct ggaagcagca catcctctcc atattcctca 6300
   gcctcacttg aatccttgga ttccacaatc agtaggagga atgcaatcac ttcctggcta 6360
   tgggacctca ctacatctct ccccactaca acttggccaa gtactagttt atctgaggca 6420
   ctgtcctcag gccattctgg ggtttcaaac ccaagttcaa ctacgactga atttccactc 6480
   ttttcagctg catccacatc tgctgctaag caaagaaatc cagaaacaga gacccatggt 6540
   ccccagaata cagccgcgag tactttgaac actgatgcat cctcggtcac aggtctttct 6600
   gagactcctg tgggggcaag tatcagctct gaagtccctc ttccaatggc cataacttct 6660
   agatcagatg tttctggcct tacatctgag agtactgcta acccgagttt aggcacagcc 6720
   tcttcagcag ggaccaaatt aactaggaca atatccctgc ccacttcaga gtctttggtt 6780
   tcctttagaa tgaacaagga tccatggaca gtgtcaatcc ctttggggtc ccatccaact 6840
   actaatacag aaacaagcat cccagtaaac agcgcaggtc cacctggctt gtccacagta 6900
   gcatcagatg taattgacac accttcagat ggggctgaga gtattcccac tgtctccttt 6960
   tccccctccc ctgatactga agtgacaact atctcacatt tcccagaaaa gacaactcat 7020
   tcatttagaa ccatttcatc tctcactcat gagttgactt caagagtgac acctattcct 7080
   ggggattgga tgagttcagc tatgtctaca aagcccacag gagccagtcc ctccattaca 7140
   ctgggagaga gaaggacaat cacctctgct gctccaacca cttcccccat agttctcact 7200
   gctagtttca cagagaccag cacagtttca ctggataatg aaactacagt aaaaacctca 7260
   gatatccttg acgcacggaa aacaaatgag ctcccctcag atagcagttc ttcttctgat 7320
   ctgatcaaca cctccatagc ttcttcaact atggatgtca ctaaaacagc ctccatcagt 7380
   cccactagca tctcaggaat gacagcaagt tcctccccat ctctcttctc ttcagataga 7440
   ccccaggttc ccacatctac aacagagaca aatacagcca cctctccatc tgtttccagt 7500
   aacacctatt ctcttgatgg gggctccaat gtgggtggca ctccatccac tttaccaccc 7560
   tttacaatca cccaccctgt cgagacaagc tcggccctat tagcctggtc tagaccagta 7620
   agaactttca gcaccatggt cagcactgac actgcctccg gagaaaatcc tacctctagc 7680
   aattctgtgg tgacttctgt tccagcacca ggtacatgga ccagtgtagg cagtactact 7740
   gacttacctg ccatgggctt tctcaagaca agtcctgcag gagaggcaca ctcacttcta 7800
   gcatcaacta ttgaaccagc cactgccttc actccccatc tctcagcagc agtggtcact 7860
   ggatccagtg ctacatcaga agccagtctt ctcactacga gtgaaagcaa agccattcat 7920
   tcttcaccac agaccccaac tacacccacc tctggagcaa actgggaaac ttcagctact 7980
   cctgagagcc ttttggtagt cactgagact tcagacacaa cacttacctc aaagattttg 8040
   gtcacagata ccatcttgtt ttcaactgtg tccacgccac cttctaaatt tccaagtacg 8100
   gggactctgt ctggagcttc cttccctact ttactcccgg acactccagc catccctctc 8160
   actgccactg agccaacaag ttcattagct acatcctttg attccacccc actggtgact 8220
   atagcttcgg atagtcttgg cacagtccca gagactaccc tgaccatgtc agagacctca 8280
   aatggtgatg cactggttct taagacagta agtaacccag ataggagcat ccctggaatc 8340
   actatccaag gagtaacaga aagtccactc catccttctt ccacttcccc ctctaagatt 8400
   gttgctccac ggaatacaac ctatgaaggt tcgatcacag tggcactttc tactttgcct 8460
   gcgggaacta ctggttccct tgtattcagt cagagttctg aaaactcaga gacaacggct 8520
   ttggtagact catcagctgg gcttgagagg gcatctgtga tgccactaac cacaggaagc 8580
   cagggtatgg ctagctctgg aggaatcaga agtgggtcca ctcactcaac tggaaccaaa 8640
   acattttctt ctctccctct gaccatgaac ccaggtgagg ttacagccat gtctgaaatc 8700
   accacgaaca gactgacagc tactcaatca acagcaccca aagggatacc tgtgaagccc 8760
   accagtgctg agtcaggcct cctaacacct gtctctgcct cctcaagccc atcaaaggcc 8820
   tttgcctcac tgactacagc tcccccaact tgggggatcc cacagtctac cttgacattt 8880
   gagttttctg aggtcccaag tttggatact aagtccgctt ctttaccaac tcctggacag 8940
   tccctgaaca ccattccaga ctcagatgca agcacagcat cttcctcact gtccaagtct 9000
   ccagaaaaaa acccaagggc aaggatgatg acttccacaa aggccataag tgcaagctca 9060
   tttcaatcaa caggttttac tgaaacccct gagggatctg cctccccttc tatggcaggg 9120
   catgaaccca gagtccccac ttcaggaaca ggggacccta gatatgcctc agagagcatg 9180
   tcttatccag acccaagcaa ggcatcatca gctatgacat cgacctctct tgcatcaaaa 9240
   ctcacaactc tcttcagcac aggtcaagca gcaaggtctg gttctagttc ctctcccata 9300
   agcctatcca ctgagaaaga aacaagcttc ctttccccca ctgcatccac ctccagaaag 9360
   acttcactat ttcttgggcc ttccatggca aggcagccca acatattggt gcatcttcag 9420
   acttcagctc tgacactttc tccaacatcc actctaaata tgtcccagga ggagcctcct 9480
   gagttaacct caagccagac cattgcagaa gaagagggaa caacagctga aacacagacg 9540
   ttaaccttca caccatctga gaccccaaca tccttgttac ctgtctcttc tcccacagaa 9600
   cccacagcca gaagaaagag ttctccagaa acatgggcaa gctctatttc agttcctgcc 9660
   aagacctcct tggttgaaac aactgatgga acgctagtga ccaccataaa gatgtcaagc 9720
   caggcagcac aaggaaattc cacgtggcct gccccagcag aggagacggg gagcagtcca 9780
   gcaggcacat ccccaggaag cccagaaatg tctaccactc tcaaaatcat gagctccaag 9840
   gaacccagca tcagcccaga gatcaggtcc actgtgagaa attctccttg gaagactcca 9900
   gaaacaactg ttcccatgga gaccacagtg gaaccagtca cccttcagtc cacagcccta 9960
   ggaagtggca gcaccagcat ctctcacctg cccacaggaa ccacatcacc aaccaagtca 10020
   ccaacagaaa atatgttggc tacagaaagg gtctccctct ccccatcccc acctgaggct 10080
   tggaccaacc tttattctgg aactccagga gggaccaggc agtcactggc cacaatgtcc 10140
   tctgtctccc tagagtcacc aactgctaga agcatcacag ggactggtca gcaaagcagt 10200
   ccagaactgg tttcaaagac aactggaatg gaattctcta tgtggcatgg ctctactgga 10260
   gggaccacag gggacacaca tgtctctctg agcacatctt ccaatatcct tgaagaccct 10320
   gtaaccagcc caaactctgt gagctcattg acagataaat ccaaacataa aaccgagaca 10380
   tgggtaagca ccacagccat tccctccact gtcctgaata ataagataat ggcagctgaa 10440
   caacagacaa gtcgatctgt ggatgaggct tattcatcaa ctagttcttg gtcagatcag 10500
   acatctggga gtgacatcac ccttggtgca tctcctgatg tcacaaacac attatacatc 10560
   acctccacag cacaaaccac ctcactagtg tctctgccct ctggagacca aggcattaca 10620
   agcctcacca atccctcagg aggaaaaaca agctctgcgt catctgtcac atctccttca 10680
   atagggcttg agactctgag ggccaatgta agtgcagtga aaagtgacat tgcccctact 10740
   gctgggcatc tatctcagac ttcatctcct gcggaagtga gcatcctgga cgtaaccaca 10800
   gctcctactc caggtatctc caccaccatc accaccatgg gaaccaactc aatctcaact 10860
   accacaccca acccagaagt gggtatgagt accatggaca gcaccccggc cacagagagg 10920
   cgcacaactt ctacagaaca cccttccacc tggtcttcca cagctgcatc agattcctgg 10980
   actgtcacag acatgacttc aaacttgaaa gttgcaagat ctcctggaac aatttccaca 11040
   atgcatacaa cttcattctt agcctcaagc actgaattag actccatgtc tactccccat 11100
   ggccgtataa ctgtcattgg aaccagcctg gtcactccat cctctgatgc ttcagctgta 11160
   aagacagaga ccagtacaag tgaaagaaca ttgagtcctt cagacacaac tgcatctact 11220
   cccatctcaa ctttttctcg tgtccagagg atgagcatct cagttcctga cattttaagt 11280
   acaagttgga ctcccagtag tacagaagca gaagatgtgc ctgtttcaat ggtttctaca 11340
   gatcatgcta gtacaaagac tgacccaaat acgcccctgt ccacttttct gtttgattct 11400
   ctgtccactc ttgactggga cactgggaga tctctgtcat cagccacagc cactacctca 11460
   gctcctcagg gggccacaac tccccaggaa ctcactttgg aaaccatgat cagcccagct 11520
   acctcacagt tgcccttctc tatagggcac attacaagtg cagtcacacc agctgcaatg 11580
   gcaaggagct ctggagttac tttttcaaga ccagatccca caagcaaaaa ggcagagcag 11640
   acttccactc agcttcccac caccacttct gcacatccag ggcaggtgcc cagatcagca 11700
   gcaacaactc tggatgtgat cccacacaca gcaaaaactc cagatgcaac ttttcagaga 11760
   caagggcaga cagctcttac aacagaggca agagctacat ctgactcctg gaatgagaaa 11820
   gaaaaatcaa ccccaagtgc accttggatc actgagatga tgaattctgt ctcagaagat 11880
   accatcaagg aggttaccag ctcctccagt gtattaagga ccctgaatac gctggacata 11940
   aacttggaat ctgggacgac ttcatcccca agttggaaaa gcagcccata tgagagaatt 12000
   gccccttctg agtccaccac agacaaagag gcaattcacc cttctacaaa cacagtagag 12060
   accacaggct gggtcacaag ttccgaacat gcttctcatt ccactatccc agcccactca 12120
   gcgtcatcca aactcacatc tccagtggtt acaacctcca ccagggaaca agcaatagtt 12180
   tctatgtcaa caaccacatg gccagagtct acaagggcta gaacagagcc taattccttc 12240
   ttgactattg aactgaggga cgtcagccct tacatggaca ccagctcaac cacacaaaca 12300
   agtattatct cttccccagg ttccactgcg atcaccaagg ggcctagaac agaaattacc 12360
   tcctctaaga gaatatccag ctcattcctt gcccagtcta tgaggtcgtc agacagcccc 12420
   tcagaagcca tcaccaggct gtctaacttt cctgccatga cagaatctgg aggaatgatc 12480
   cttgctatgc aaacaagtcc acctggcgct acatcactaa gtgcacctac tttggataca 12540
   tcagccacag cctcctggac agggactcca ctggctacga ctcagagatt tacatactca 12600
   gagaagacca ctctctttag caaaggtcct gaggatacat cacagccaag ccctccctct 12660
   gtggaagaaa ccagctcttc ctcttccctg gtacctatcc atgctacaac ctcgccttcc 12720
   aatattttgt tgacatcaca agggcacagt ccctcctcta ctccacctgt gacctcagtt 12780
   ttcttgtctg agacctctgg cctggggaag accacagaca tgtcgaggat aagcttggaa 12840
   cctggcacaa gtttacctcc caatttgagc agtacagcag gtgaggcgtt atccacttat 12900
   gaagcctcca gagatacaaa ggcaattcat cattctgcag acacagcagt gacgaatatg 12960
   gaggcaacca gttctgaata ttctcctatc ccaggccata caaagccatc caaagccaca 13020
   tctccattgg ttacctccca catcatgggg gacatcactt cttccacatc agtatttggc 13080
   tcctccgaga ccacagagat tgagacagtg tcctctgtga accagggact tcaggagaga 13140
   agcacatccc aggtggccag ctctgctaca gagacaagca ctgtcattac ccatgtgtct 13200
   agtggtgatg ctactactca tgtcaccaag acacaagcca ctttctctag cggaacatcc 13260
   atctcaagcc ctcatcagtt tataacttct accaacacat ttacagatgt gagcaccaac 13320
   ccctccacct ctctgataat gacagaatct tcaggagtga ccatcaccac ccaaacaggt 13380
   cctactggag ctgcaacaca gggtccatat ctcttggaca catcaaccat gccttacttg 13440
   acagagactc cattagctgt gactccagat tttatgcaat cagagaagac cactctcata 13500
   agcaaaggtc ccaaggatgt gtcctggaca agccctccct ctgtggcaga aaccagctat 13560
   ccctcttccc tgacaccttt cttggtcaca accatacctc ctgccacttc cacgttacaa 13620
   gggcaacata catcctctcc tgtttctgcg acttcagttc ttacctctgg actggtgaag 13680
   accacagata tgttgaacac aagcatggaa cctgtgacca attcacctca aaatttgaac 13740
   aatccatcaa atgagatact ggccactttg gcagccacca cagatataga gactattcat 13800
   ccttccataa acaaagcagt gaccaatatg gggactgcca gttcagcaca tgtactgcat 13860
   tccactctcc cagtcagctc agaaccatct acagccacat ctccaatggt tcctgcctcc 13920
   agcatggggg acgctcttgc ttctatatca atacctggtt ctgagaccac agacattgag 13980
   ggagagccaa catcctccct gactgctgga cgaaaagaga acagcaccct ccaggagatg 14040
   aactcaacta cagagtcaaa catcatcctc tccaatgtgt ctgtgggggc tattactgaa 14100
   gccacaaaaa tggaagtccc ctcttttgat gcaacattca taccaactcc tgctcagtca 14160
   acaaagttcc cagatatttt ctcagtagcc agcagtagac tttcaaactc tcctcccatg 14220
   acaatatcta cccacatgac caccacccag acagggtctt ctggagctac atcaaagatt 14280
   ccacttgcct tagacacatc aaccttggaa acctcagcag ggactccatc agtggtgact 14340
   gaggggtttg cccactcaaa aataaccact gcaatgaaca atgatgtcaa ggacgtgtca 14400
   cagacaaacc ctccctttca ggatgaagcc agctctccct cttctcaagc acctgtcctt 14460
   gtcacaacct taccttcttc tgttgctttc acaccgcaat ggcacagtac ctcctctcct 14520
   gtttctatgt cctcagttct tacttcttca ctggtaaaga ccgcaggcaa ggtggataca 14580
   agcttagaaa cagtgaccag ttcacctcaa agtatgagca acactttgga tgacatatcg 14640
   gtcacttcag cagccaccac agatatagag acaacgcatc cttccataaa cacagtagtt 14700
   accaatgtgg ggaccaccgg ttcagcattt gaatcacatt ctactgtctc agcttaccca 14760
   gagccatcta aagtcacatc tccaaatgtt accacctcca ccatggaaga caccacaatt 14820
   tccagatcaa tacctaaatc ctctaagact acaagaactg agactgagac aacttcctcc 14880
   ctgactccta aactgaggga gaccagcatc tcccaggaga tcacctcgtc cacagagaca 14940
   agcactgttc cttacaaaga gctcactggt gccactaccg aggtatccag gacagatgtc 15000
   acttcctcta gcagtacatc cttccctggc cctgatcagt ccacagtgtc actagacatc 15060
   tccacagaaa ccaacaccag gctgtctacc tccccaataa tgacagaatc tgcagaaata 15120
   accatcacca cccaaacagg tcctcatggg gctacatcac aggatacttt taccatggac 15180
   ccatcaaata caacccccca ggcagggatc cactcagcta tgactcatgg attttcacaa 15240
   ttggatgtga ccactcttat gagcagaatt ccacaggatg tatcatggac aagtcctccc 15300
   tctgtggata aaaccagctc cccctcttcc tttctgtcct cacctgcaat gaccacacct 15360
   tccctgattt cttctacctt accagaggat aagctctcct ctcctatgac ttcacttctc 15420
   acctctggcc tagtgaagat tacagacata ttacgtacac gcttggaacc tgtgaccagc 15480
   tcacttccaa atttcagcag cacctcagat aagatactgg ccacttctaa agacagtaaa 15540
   gacacaaagg aaatttttcc ttctataaac acagaagaga ccaatgtgaa agccaacaac 15600
   tctggacatg aatcccattc ccctgcactg gctgactcag agacacccaa agccacaact 15660
   caaatggtta tcaccaccac tgtgggagat ccagctcctt ccacatcaat gccagtgcat 15720
   ggttcctctg agactacaaa cattaagaga gagccaacat atttcttgac tcctagactg 15780
   agagagacca gtacctctca ggagtccagc tttcccacgg acacaagttt tctactttcc 15840
   aaagtcccca ctggtactat tactgaggtc tccagtacag gggtcaactc ttctagcaaa 15900
   atttccaccc cagaccatga taagtccaca gtgccacctg acaccttcac aggagagatc 15960
   cccagggtct tcacctcctc tattaagaca aaatctgcag aaatgacgat caccacccaa 16020
   gcaagtcctc ctgagtctgc atcgcacagt acccttccct tggacacatc aaccacactt 16080
   tcccagggag ggactcattc aactgtgact cagggattcc catactcaga ggtgaccact 16140
   ctcatgggca tgggtcctgg gaatgtgtca tggatgacaa ctccccctgt ggaagaaacc 16200
   agctctgtgt cttccctgat gtcttcacct gccatgacat ccccttctcc tgtttcctcc 16260
   acatcaccac agagcatccc ctcctctcct cttcctgtga ctgcacttcc tacttctgtt 16320
   ctggtgacaa ccacagatgt gttgggcaca acaagcccag agtctgtaac cagttcacct 16380
   ccaaatttga gcagcatcac tcatgagaga ccggccactt acaaagacac tgcacacaca 16440
   gaagccgcca tgcatcattc cacaaacacc gcagtgacca atgtagggac ttccgggtct 16500
   ggacataaat cacaatcctc tgtcctagct gactcagaga catcgaaagc cacacctctg 16560
   atgagtacca cctccaccct gggggacaca agtgtttcca catcaactcc taatatctct 16620
   cagactaacc aaattcaaac agagccaaca gcatccctga gccctagact gagggagagc 16680
   agcacgtctg agaagaccag ctcaacaaca gagacaaata ctgccttttc ttatgtgccc 16740
   acaggtgcta ttactcaggc ctccagaaca gaaatctcct ctagcagaac atccatctca 16800
   gaccttgatc ggcccacaat agcacccgac atctccacag gaatgatcac caggctcttc 16860
   acctccccca tcatgacaaa atctgcagaa atgaccgtca ccactcaaac aactactcct 16920
   ggggctacat cacagggtat ccttccctgg gacacatcaa ccacactttt ccagggaggg 16980
   actcattcaa ccgtgtctca gggattccca cactcagaga taaccactct tcggagcaga 17040
   acccctggag atgtgtcatg gatgacaact ccccctgtgg aagaaaccag ctctgggttt 17100
   tccctgatgt caccttccat gacatcccct tctcctgttt cctccacatc accagagagc 17160
   atcccctcct ctcctctccc tgtgactgca cttcttactt ctgttctggt gacaaccaca 17220
   aatgtattgg gcacaacaag cccagagccc gtaacgagtt cacctccaaa tttaagcagc 17280
   cccacacagg agagactgac cacttacaaa gacactgcgc acacagaagc catgcatgct 17340
   tccatgcata caaacactgc agtggccaac gtggggacct ccatttctgg acatgaatca 17400
   caatcttctg tcccagctga ttcacacaca tccaaagcca catctccaat gggtatcacc 17460
   ttcgccatgg gggatacaag tgtttctaca tcaactcctg ccttctttga gactagaatt 17520
   cagactgaat caacatcctc tttgattcct ggattaaggg acaccaggac gtctgaggag 17580
   atcaacactg tgacagagac cagcactgtc ctttcagaag tgcccactac tactactact 17640
   gaggtctcca ggacagaagt tatcacttcc agcagaacaa ccatctcagg gcctgatcat 17700
   tccaaaatgt caccctacat ctccacagaa accatcacca ggctctccac ttttcctttt 17760
   gtaacaggat ccacagaaat ggccatcacc aaccaaacag gtcctatagg gactatctca 17820
   caggctaccc ttaccctgga cacatcaagc acagcttcct gggaagggac tcactcacct 17880
   gtgactcaga gatttccaca ctcagaggag accactacta tgagcagaag tactaagggc 17940
   gtgtcatggc aaagccctcc ctctgtggaa gaaaccagtt ctccttcttc cccagtgcct 18000
   ttacctgcaa taacctcaca ttcatctctt tattccgcag tatcaggaag tagccccact 18060
   tctgctctcc ctgtgacttc ccttctcacc tctggcagga ggaagaccat agacatgttg 18120
   gacacacact cagaacttgt gaccagctcc ttaccaagtg caagtagctt ctcaggtgag 18180
   atactcactt ctgaagcctc cacaaataca gagacaattc acttttcaga gaacacagca 18240
   gaaaccaata tggggaccac caattctatg cataaactac attcctctgt ctcaatccac 18300
   tcccagccat ccggacacac acctccaaag gttactggat ctatgatgga ggacgctatt 18360
   gtttccacat caacacctgg ttctcctgag actaaaaatg ttgacagaga ctcaacatcc 18420
   cctctgactc ctgaactgaa agaggacagc accgccctgg tgatgaactc aactacagag 18480
   tcaaacactg ttttctccag tgtgtccctg gatgctgcta ctgaggtctc cagggcagaa 18540
   gtcacctact atgatcctac attcatgcca gcttctgctc agtcaacaaa gtccccagac 18600
   atttcacctg aagccagcag cagtcattct aactctcctc ccttgacaat atctacacac 18660
   aagaccatcg ccacacaaac aggtccttct ggggtgacat ctcttggcca actgaccctg 18720
   gacacatcaa ccatagccac ctcagcagga actccatcag ccagaactca ggattttgta 18780
   gattcagaaa caaccagtgt catgaacaat gatctcaatg atgtgttgaa gacaagccct 18840
   ttctctgcag aagaagccaa ctctctctct tctcaggcac ctctccttgt gacaacctca 18900
   ccttctcctg taacttccac attgcaagag cacagtacct cctctcttgt ttctgtgacc 18960
   tcagtaccca cccctacact ggcgaagatc acagacatgg acacaaactt agaacctgtg 19020
   actcgttcac ctcaaaattt aaggaacacc ttggccactt cagaagccac cacagataca 19080
   cacacaatgc atccttctat aaacacagca gtggccaatg tggggaccac cagttcacca 19140
   aatgaattct attttactgt ctcacctgac tcagacccat ataaagccac atccgcagta 19200
   gttatcactt ccacctcggg ggactcaata gtttccacat caatgcctag atcctctgcg 19260
   atgaaaaaga ttgagtctga gacaactttc tccctgatat ttagactgag ggagactagc 19320
   acctcccaga aaattggctc atcctcagac acaagcacgg tctttgacaa agcattcact 19380
   gctgctacta ctgaggtctc cagaacagaa ctcacctcct ctagcagaac atccatccaa 19440
   ggcactgaaa agcccacaat gtcaccggac acctccacaa gatctgtcac catgctttct 19500
   acttttgctg gcctgacaaa atccgaagaa aggaccattg ccacccaaac aggtcctcat 19560
   agggcgacat cacagggtac ccttacctgg gacacatcaa tcacaacctc acaggcaggg 19620
   acccactcag ctatgactca tggattttca caattagatt tgtccactct tacgagtaga 19680
   gttcctgagt acatatcagg gacaagccca ccctctgtgg aaaaaaccag ctcttcctct 19740
   tcccttctgt ctttaccagc aataacctca ccgtcccctg tacctactac attaccagaa 19800
   agtaggccgt cttctcctgt tcatctgact tcactcccca cctctggcct agtgaagacc 19860
   acagatatgc tggcatctgt ggccagttta cctccaaact tgggcagcac ctcacataag 19920
   ataccgacta cttcagaaga cattaaagat acagagaaaa tgtatccttc cacaaacata 19980
   gcagtaacca atgtggggac caccacttct gaaaaggaat cttattcgtc tgtcccagcc 20040
   tactcagaac cacccaaagt cacctctcca atggttacct ctttcaacat aagggacacc 20100
   attgtttcca catccatgcc tggctcctct gagattacaa ggattgagat ggagtcaaca 20160
   ttctccctgg ctcatgggct gaagggaacc agcacctccc aggaccccat cgtatccaca 20220
   gagaaaagtg ctgtccttca caagttgacc actggtgcta ctgagacctc taggacagaa 20280
   gttgcctctt ctagaagaac atccattcca ggccctgatc attccacaga gtcaccagac 20340
   atctccactg aagtgatccc cagcctgcct atctcccttg gcattacaga atcttcaaat 20400
   atgaccatca tcactcgaac aggtcctcct cttggctcta catcacaggg cacatttacc 20460
   ttggacacac caactacatc ctccagggca ggaacacact cgatggcgac tcaggaattt 20520
   ccacactcag aaatgaccac tgtcatgaac aaggaccctg agattctatc atggacaatc 20580
   cctccttcta tagagaaaac cagcttctcc tcttccctga tgccttcacc agccatgact 20640
   tcacctcctg tttcctcaac attaccaaag accattcaca ccactccttc tcctatgacc 20700
   tcactgctca cccctagcct agtgatgacc acagacacat tgggcacaag cccagaacct 20760
   acaaccagtt cacctccaaa tttgagcagt acctcacatg agatactgac aacagatgaa 20820
   gacaccacag ctatagaagc catgcatcct tccacaagca cagcagcgac taatgtggaa 20880
   accaccagtt ctggacatgg gtcacaatcc tctgtcctag ctgactcaga aaaaaccaag 20940
   gccacagctc caatggatac cacctccacc atggggcata caactgtttc cacatcaatg 21000
   tctgtttcct ctgagactac aaaaattaag agagagtcaa catattcctt gactcctgga 21060
   ctgagagaga ccagcatttc ccaaaatgcc agcttttcca ctgacacaag tattgttctt 21120
   tcagaagtcc ccactggtac tactgctgag gtctccagga cagaagtcac ctcctctggt 21180
   agaacatcca tccctggccc ttctcagtcc acagttttgc cagaaatatc cacaagaaca 21240
   atgacaaggc tctttgcctc gcccaccatg acagaatcag cagaaatgac catccccact 21300
   caaacaggtc cttctgggtc tacctcacag gataccctta ccttggacac atccaccaca 21360
   aagtcccagg caaagactca ttcaactttg actcagagat ttccacactc agagatgacc 21420
   actctcatga gcagaggtcc tggagatatg tcatggcaaa gctctccctc tctggaaaat 21480
   cccagctctc tcccttccct gctgtcttta cctgccacaa cctcacctcc tcccatttcc 21540
   tccacattac cagtgactat ctcctcctct cctcttcctg tgacttcact tctcacctct 21600
   agcccggtaa cgaccacaga catgttacac acaagcccag aacttgtaac cagttcacct 21660
   ccaaagctga gccacacttc agatgagaga ctgaccactg gcaaggacac cacaaataca 21720
   gaagctgtgc atccttccac aaacacagca gcgtccaatg tggagattcc cagctctgga 21780
   catgaatccc cttcctctgc cttagctgac tcagagacat ccaaagccac atcaccaatg 21840
   tttattacct ccacccagga ggatacaact gttgccatat caacccctca cttcttggag 21900
   actagcagaa ttcagaaaga gtcaatttcc tccctgagcc ctaaattgag ggagacaggc 21960
   agttctgtgg agacaagctc agccatagag acaagtgctg tcctttctga agtgtccatt 22020
   ggtgctacta ctgagatctc caggacagaa gtcacctcct ctagcagaac atccatctct 22080
   ggttctgctg agtccacaat gttgccagaa atatccacca caagaaaaat cattaagttc 22140
   cctacttccc ccatcctggc agaatcatca gaaatgacca tcaagaccca aacaagtcct 22200
   cctgggtcta catcagagag tacctttaca ttagacacat caaccactcc ctccttggta 22260
   ataacccatt cgactatgac tcagagattg ccacactcag agataaccac tcttgtgagt 22320
   agaggtgctg gggatgtgcc acggcccagc tctctccctg tggaagaaac aagccctcca 22380
   tcttcccagc tgtctttatc tgccatgatc tcaccttctc ctgtttcttc cacattacca 22440
   gcaagtagcc actcctcttc tgcttctgtg acttcacttc tcacaccagg ccaagtgaag 22500
   actactgagg tgttggacgc aagtgcagaa cctgaaacca gttcacctcc aagtttgagc 22560
   agcacctcag ttgaaatact ggccacctct gaagtcacca cagatacgga gaaaattcat 22620
   cctttctcaa acacggcagt aaccaaagtt ggaacttcca gttctggaca tgaatcccct 22680
   tcctctgtcc tacctgactc agagacaacc aaagccacat cggcaatggg taccatctcc 22740
   attatggggg atacaagtgt ttctacatta actcctgcct tatctaacac taggaaaatt 22800
   cagtcagagc cagcttcctc actgaccacc agattgaggg agaccagcac ctctgaagag 22860
   accagcttag ccacagaagc aaacactgtt ctttctaaag tgtccactgg tgctactact 22920
   gaggtctcca ggacagaagc catctccttt agcagaacat ccatgtcagg ccctgagcag 22980
   tccacaatgt cacaagacat ctccatagga accatcccca ggatttctgc ctcctctgtc 23040
   ctgacagaat ctgcaaaaat gaccatcaca acccaaacag gtccttcgga gtctacacta 23100
   gaaagtaccc ttaatttgaa cacagcaacc acaccctctt gggtggaaac ccactctata 23160
   gtaattcagg gatttccaca cccagagatg accacttcca tgggcagagg tcctggaggt 23220
   gtgtcatggc ctagccctcc ctttgtgaaa gaaaccagcc ctccatcctc cccgctgtct 23280
   ttacctgccg tgacctcacc tcatcctgtt tccaccacat tcctagcaca tatccccccc 23340
   tctccccttc ctgtgacttc acttctcacc tctggcccgg cgacaaccac agatatcttg 23400
   ggtacaagca cagaacctgg aaccagttca tcttcaagtt tgagcaccac ctcccatgag 23460
   agactgacca cttacaaaga cactgcacat acagaagccg tgcatccttc cacaaacaca 23520
   ggagggacca atgtggcaac caccagctct ggatataaat cacagtcctc tgtcctagct 23580
   gactcatctc caatgtgtac cacctccacc atgggggata caagtgttct cacatcaact 23640
   cctgccttcc ttgagactag gaggattcag acagagctag cttcctccct gacccctgga 23700
   ttgagggagt ccagcggctc tgaagggacc agctcaggca ccaagatgag cactgtcctc 23760
   tctaaagtgc ccactggtgc tactactgag atctccaagg aagacgtcac ctccatccca 23820
   ggtcccgctc aatccacaat atcaccagac atctccacaa gaaccgtcag ctggttctct 23880
   acatcccctg tcatgacaga atcagcagaa ataaccatga acacccatac aagtccttta 23940
   ggggccacaa cacaaggcac cagtactttg gacacgtcaa gcacaacctc tttgacaatg 24000
   acacactcaa ctatatctca aggattttca cactcacaga tgagcactct tatgaggagg 24060
   ggtcctgagg atgtatcatg gatgagccct ccccttctgg aaaaaactag accttccttt 24120
   tctctgatgt cttcaccagc cacaacttca ccttctcctg tttcctccac attaccagag 24180
   agcatctctt cctctcctct tcctgtgact tcactcctca cgtctggctt ggcaaaaact 24240
   acagatatgt tgcacaaaag ctcagaacct gtaaccaact cacctgcaaa tttgagcagc 24300
   acctcagttg aaatactggc cacctctgaa gtcaccacag atacagagaa aactcatcct 24360
   tcttcaaaca gaacagtgac cgatgtgggg acctccagtt ctggacatga atccacttcc 24420
   tttgtcctag ctgactcaca gacatccaaa gtcacatctc caatggttat tacctccacc 24480
   atggaggata cgagtgtctc cacatcaact cctggctttt ttgagactag cagaattcag 24540
   acagaaccaa catcctccct gacccttgga ctgagaaaga ccagcagctc tgaggggacc 24600
   agcttagcca cagagatgag cactgtcctt tctggagtgc ccactggtgc cactgctgaa 24660
   gtctccagga cagaagtcac ctcctctagc agaacatcca tctcaggctt tgctcagctc 24720
   acagtgtcac cagagacttc cacagaaacc atcaccagac tccctacctc cagcataatg 24780
   acagaatcag cagaaatgat gatcaagaca caaacagatc ctcctgggtc tacaccagag 24840
   agtactcata ctgtggacat atcaacaaca cccaactggg tagaaaccca ctcgactgtg 24900
   actcagagat tttcacactc agagatgacc actcttgtga gcagaagccc tggtgatatg 24960
   ttatggccta gtcaatcctc tgtggaagaa accagctctg cctcttccct gctgtctctg 25020
   cctgccacga cctcaccttc tcctgtttcc tctacattag tagaggattt cccttccgct 25080
   tctcttcctg tgacttctct tctcaaccct ggcctggtga taaccacaga caggatgggc 25140
   ataagcagag aacctggaac cagttccact tcaaatttga gcagcacctc ccatgagaga 25200
   ctgaccactt tggaagacac tgtagataca gaagacatgc agccttccac acacacagca 25260
   gtgaccaacg tgaggacctc catttctgga catgaatcac aatcttctgt cctatctgac 25320
   tcagagacac ccaaagccac atctccaatg ggtaccacct acaccatggg ggaaacgagt 25380
   gtttccatat ccacttctga cttctttgag accagcagaa ttcagataga accaacatcc 25440
   tccctgactt ctggattgag ggagaccagc agctctgaga ggatcagctc agccacagag 25500
   ggaagcactg tcctttctga agtgcccagt ggtgctacca ctgaggtctc caggacagaa 25560
   gtgatatcct ctaggggaac atccatgtca gggcctgatc agttcaccat atcaccagac 25620
   atctctactg aagcgatcac caggctttct acttccccca ttatgacaga atcagcagaa 25680
   agtgccatca ctattgagac aggttctcct ggggctacat cagagggtac cctcaccttg 25740
   gacacctcaa caacaacctt ttggtcaggg acccactcaa ctgcatctcc aggattttca 25800
   cactcagaga tgaccactct tatgagtaga actcctggag atgtgccatg gccgagcctt 25860
   ccctctgtgg aagaagccag ctctgtctct tcctcactgt cttcacctgc catgacctca 25920
   acttcttttt tctccacatt accagagagc atctcctcct ctcctcatcc tgtgactgca 25980
   cttctcaccc ttggcccagt gaagaccaca gacatgttgc gcacaagctc agaacctgaa 26040
   accagttcac ctccaaattt gagcagcacc tcagctgaaa tattagccac gtctgaagtc 26100
   accaaagata gagagaaaat tcatccctcc tcaaacacac ctgtagtcaa tgtagggact 26160
   gtgatttata aacatctatc cccttcctct gttttggctg acttagtgac aacaaaaccc 26220
   acatctccaa tggctaccac ctccactctg gggaatacaa gtgtttccac atcaactcct 26280
   gccttcccag aaactatgat gacacagcca acttcctccc tgacttctgg attaagggag 26340
   atcagtacct ctcaagagac cagctcagca acagagagaa gtgcttctct ttctggaatg 26400
   cccactggtg ctactactaa ggtctccaga acagaagccc tctccttagg cagaacatcc 26460
   accccaggtc ctgctcaatc cacaatatca ccagaaatct ccacggaaac catcactaga 26520
   atttctactc ccctcaccac gacaggatca gcagaaatga ccatcacccc caaaacaggt 26580
   cattctgggg catcctcaca aggtaccttt accttggaca catcaagcag agcctcctgg 26640
   ccaggaactc actcagctgc aactcacaga tctccacact cagggatgac cactcctatg 26700
   agcagaggtc ctgaggatgt gtcatggcca agccgcccat cagtggaaaa aactagccct 26760
   ccatcttccc tggtgtcttt atctgcagta acctcacctt cgccacttta ttccacacca 26820
   tctgagagta gccactcatc tcctctccgg gtgacttctc ttttcacccc tgtcatgatg 26880
   aagaccacag acatgttgga cacaagcttg gaacctgtga ccacttcacc tcccagtatg 26940
   aatatcacct cagatgagag tctggccact tctaaagcca ccatggagac agaggcaatt 27000
   cagctttcag aaaacacagc tgtgactcag atgggcacca tcagcgctag acaagaattc 27060
   tattcctctt atccaggcct cccagagcca tccaaagtga catctccagt ggtcacctct 27120
   tccaccataa aagacattgt ttctacaacc atacctgctt cctctgagat aacaagaatt 27180
   gagatggagt caacatccac cctgaccccc acaccaaggg agaccagcac ctcccaggag 27240
   atccactcag ccacaaagcc aagcactgtt ccttacaagg cactcactag tgccacgatt 27300
   gaggactcca tgacacaagt catgtcctct agcagaggac ctagccctga tcagtccaca 27360
   atgtcacaag acatatccac tgaagtgatc accaggctct ctacctcccc catcaagaca 27420
   gaatctacag aaatgaccat taccacccaa acaggttctc ctggggctac atcaaggggt 27480
   acccttacct tggacacttc aacaactttt atgtcaggga cccactcaac tgcatctcaa 27540
   ggattttcac actcacagat gaccgctctt atgagtagaa ctcctggaga tgtgccatgg 27600
   ctaagccatc cctctgtgga agaagccagc tctgcctctt tctcactgtc ttcacctgtc 27660
   atgacctcat cttctcccgt ttcttccaca ttaccagaca gcatccactc ttcttcgctt 27720
   cctgtgacat cacttctcac ctcagggctg gtgaagacca cagagctgtt gggcacaagc 27780
   tcagaacctg aaaccagttc acccccaaat ttgagcagca cctcagctga aatactggcc 27840
   atcactgaag tcactacaga tacagagaaa ctggagatga ccaatgtggt aacctcaggt 27900
   tatacacatg aatctccttc ctctgtccta gctgactcag tgacaacaaa ggccacatct 27960
   tcaatgggta tcacctaccc cacaggagat acaaatgttc tcacatcaac ccctgccttc 28020
   tctgacacca gtaggattca aacaaagtca aagctctcac tgactcctgg gttgatggag 28080
   accagcatct ctgaagagac cagctctgcc acagaaaaaa gcactgtcct ttctagtgtg 28140
   cccactggtg ctactactga ggtctccagg acagaagcca tctcttctag cagaacatcc 28200
   atcccaggcc ctgctcaatc cacaatgtca tcagacacct ccatggaaac catcactaga 28260
   atttctaccc ccctcacaag gaaagaatca acagacatgg ccatcacccc caaaacaggt 28320
   ccttctgggg ctacctcgca gggtaccttt accttggact catcaagcac agcctcctgg 28380
   ccaggaactc actcagctac aactcagaga tttccacagt cagtggtgac aactcctatg 28440
   agcagaggtc ctgaggatgt gtcatggcca agcccgctgt ctgtggaaaa aaacagccct 28500
   ccatcttccc tggtatcttc atcttcagta acctcacctt cgccacttta ttccacacca 28560
   tctgggagta gccactcctc tcctgtccct gtcacttctc ttttcacctc tatcatgatg 28620
   aaggccacag acatgttgga tgcaagtttg gaacctgaga ccacttcagc tcccaatatg 28680
   aatatcacct cagatgagag tctggccgct tctaaagcca ccacggagac agaggcaatt 28740
   cacgtttttg aaaatacagc agcgtcccat gtggaaacca ccagtgctac agaggaactc 28800
   tattcctctt ccccaggctt ctcagagcca acaaaagtga tatctccagt ggtcacctct 28860
   tcctctataa gagacaacat ggtttccaca acaatgcctg gctcctctgg cattacaagg 28920
   attgagatag agtcaatgtc atctctgacc cctggactga gggagaccag aacctcccag 28980
   gacatcacct catccacaga gacaagcact gtcctttaca agatgccctc tggtgccact 29040
   cctgaggtct ccaggacaga agttatgccc tctagcagaa catccattcc tggccctgct 29100
   cagtccacaa tgtcactaga catctccgat gaagttgtca ccaggctgtc tacctctccc 29160
   atcatgacag aatctgcaga aataaccatc accacccaaa caggttattc tctggctaca 29220
   tcccaggtta cccttccctt gggcacctca atgacctttt tgtcagggac ccactcaact 29280
   atgtctcaag gactttcaca ctcagagatg accaatctta tgagcagggg tcctgaaagt 29340
   ctgtcatgga cgagccctcg ctttgtggaa acaactagat cttcctcttc tctgacatca 29400
   ttacctctca cgacctcact ttctcctgtg tcctccacat tactagacag tagcccctcc 29460
   tctcctcttc ctgtgacttc acttatcctc ccaggcctgg tgaagactac agaagtgttg 29520
   gatacaagct cagagcctaa aaccagttca tctccaaatt tgagcagcac ctcagttgaa 29580
   ataccggcca cctctgaaat catgacagat acagagaaaa ttcatccttc ctcaaacaca 29640
   gcggtggcca aagtgaggac ctccagttct gttcatgaat ctcattcctc tgtcctagct 29700
   gactcagaaa caaccataac cataccttca atgggtatca cctccgctgt ggacgatacc 29760
   actgttttca catcaaatcc tgccttctct gagactagga ggattccgac agagccaaca 29820
   ttctcattga ctcctggatt cagggagact agcacctctg aagagaccac ctcaatcaca 29880
   gaaacaagtg cagtccttta tggagtgccc actagtgcta ctactgaagt ctccatgaca 29940
   gaaatcatgt cctctaatag aatacacatc cctgactctg atcagtccac gatgtctcca 30000
   gacatcatca ctgaagtgat caccaggctc tcttcctcat ccatgatgtc agaatcaaca 30060
   caaatgacca tcaccaccca aaaaagttct cctggggcta cagcacagag tactcttacc 30120
   ttggccacaa caacagcccc cttggcaagg acccactcaa ctgttcctcc tagattttta 30180
   cactcagaga tgacaactct tatgagtagg agtcctgaaa atccatcatg gaagagctct 30240
   ctctttgtgg aaaaaactag ctcttcatct tctctgttgt ccttacctgt cacgacctca 30300
   ccttctgttt cttccacatt accgcagagt atcccttcct cctctttttc tgtgacttca 30360
   ctcctcaccc caggcatggt gaagactaca gacacaagca cagaacctgg aaccagttta 30420
   tctccaaatc tgagtggcac ctcagttgaa atactggctg cctctgaagt caccacagat 30480
   acagagaaaa ttcatccttc ttcaagcatg gcagtgacca atgtgggaac caccagttct 30540
   ggacatgaac tatattcctc tgtttcaatc cactcggagc catccaaggc tacataccca 30600
   gtgggtactc cctcttccat ggctgaaacc tctatttcca catcaatgcc tgctaatttt 30660
   gagaccacag gatttgaggc tgagccattt tctcatttga cttctggatt taggaagaca 30720
   aacatgtccc tggacaccag ctcagtcaca ccaacaaata caccttcttc tcctgggtcc 30780
   actcaccttt tacagagttc caagactgat ttcacctctt ctgcaaaaac atcatcccca 30840
   gactggcctc cagcctcaca gtatactgaa attccagtgg acataatcac cccctttaat 30900
   gcttctccat ctattacgga gtccactggg ataacctcct tcccagaatc caggtttact 30960
   atgtctgtaa cagaaagtac tcatcatctg agtacagatt tgctgccttc agctgagact 31020
   atttccactg gcacagtgat gccttctcta tcagaggcca tgacttcatt tgccaccact 31080
   ggagttccac gagccatctc aggttcaggt agtccattct ctaggacaga gtcaggccct 31140
   ggggatgcta ctctgtccac cattgcagag agcctgcctt catccactcc tgtgccattc 31200
   tcctcttcaa ccttcactac cactgattct tcaaccatcc cagccctcca tgagataact 31260
   tcctcttcag ctaccccata tagagtggac accagtcttg ggacagagag cagcactact 31320
   gaaggacgct tggttatggt cagtactttg gacacttcaa gccaaccagg caggacatct 31380
   tcatcaccca ttttggatac cagaatgaca gagagcgttg agctgggaac agtgacaagt 31440
   gcttatcaag ttccttcact ctcaacacgg ttgacaagaa ctgatggcat tatggaacac 31500
   atcacaaaaa tacccaatga agcagcacac agaggtacca taagaccagt caaaggccct 31560
   cagacatcca cttcgcctgc cagtcctaaa ggactacaca caggagggac aaaaagaatg 31620
   gagaccacca ccacagctct gaagaccacc accacagctc tgaagaccac ttccagagcc 31680
   accttgacca ccagtgtcta tactcccact ttgggaacac tgactcccct caatgcatca 31740
   atgcaaatgg ccagcacaat ccccacagaa atgatgatca caaccccata tgttttccct 31800
   gatgttccag aaacgacatc ctcattggct accagcctgg gagcagaaac cagcacagct 31860
   cttcccagga caaccccatc tgttttcaat agagaatcag agaccacagc ctcactggtc 31920
   tctcgttctg gggcagagag aagtccggtt attcaaactc tagatgtttc ttctagtgag 31980
   ccagatacaa cagcttcatg ggttatccat cctgcagaga ccatcccaac tgtttccaag 32040
   acaaccccca attttttcca cagtgaatta gacactgtat cttccacagc caccagtcat 32100
   ggggcagacg tcagctcagc cattccaaca aatatctcac ctagtgaact agatgcactg 32160
   accccactgg tcactatttc ggggacagat actagtacaa cattcccaac actgactaag 32220
   tccccacatg aaacagagac aagaaccaca tggctcactc atcctgcaga gaccagctca 32280
   actattccca gaacaatccc caatttttct catcatgaat cagatgccac accttcaata 32340
   gccaccagtc ctggggcaga aaccagttca gctattccaa ttatgactgt ctcacctggt 32400
   gcagaagatc tggtgacctc acaggtcact agttctggga cagacagaaa tatgactatt 32460
   ccaactttga ctctttctcc tggtgaacca aagacgatag cctcattagt cacccatcct 32520
   gaagcacaga caagttcggc cattccaact tcaactatct cgcctgctgt atcacggttg 32580
   gtgacctcaa tggtcaccag tttggcggca aagacaagta caactaatcg agctctgaca 32640
   aactcccctg gtgaaccagc tacaacagtt tcattggtca cgcatcctgc acagaccagc 32700
   ccaacagttc cctggacaac ttccattttt ttccatagta aatcagacac cacaccttca 32760
   atgaccacca gtcatggggc agaatccagt tcagctgttc caactccaac tgtttcaact 32820
   gaggtaccag gagtagtgac ccctttggtc accagttcta gggcagtgat cagtacaact 32880
   attccaattc tgactctttc tcctggtgaa ccagagacca caccttcaat ggccaccagt 32940
   catggggaag aagccagttc tgctattcca actccaactg tttcacctgg ggtaccagga 33000
   gtggtgacct ctctggtcac tagttctagg gcagtgacta gtacaactat tccaattctg 33060
   actttttctc ttggtgaacc agagaccaca ccttcaatgg ccaccagtca tgggacagaa 33120
   gctggctcag ctgttccaac tgttttacct gaggtaccag gaatggtgac ctctctggtt 33180
   gctagttcta gggcagtaac cagtacaact cttccaactc tgactctttc tcctggtgaa 33240
   ccagagacca caccttcaat ggccaccagt catggggcag aagccagctc aactgttcca 33300
   actgtttcac ctgaggtacc aggagtggtg acctctctgg tcactagttc tagtggagta 33360
   aacagtacaa gtattccaac tctgattctt tctcctggtg aactagaaac cacaccttca 33420
   atggccacca gtcatggggc agaagccagc tcagctgttc caactccaac tgtttcacct 33480
   ggggtatcag gagtggtgac ccctctggtc actagttcca gggcagtgac cagtacaact 33540
   attccaattc taactctttc ttctagtgag ccagagacca caccttcaat ggccaccagt 33600
   catggggtag aagccagctc agctgttcta actgtttcac ctgaggtacc aggaatggtg 33660
   acctctctgg tcactagttc tagagcagta accagtacaa ctattccaac tctgactatt 33720
   tcttctgatg aaccagagac cacaacttca ttggtcaccc attctgaggc aaagatgatt 33780
   tcagccattc caactttagc tgtctcccct actgtacaag ggctggtgac ttcactggtc 33840
   actagttctg ggtcagagac cagtgcgttt tcaaatctaa ctgttgcctc aagtcaacca 33900
   gagaccatag actcatgggt cgctcatcct gggacagaag caagttctgt tgttccaact 33960
   ttgactgtct ccactggtga gccgtttaca aatatctcat tggtcaccca tcctgcagag 34020
   agtagctcaa ctcttcccag gacaacctca aggttttccc acagtgaatt agacactatg 34080
   ccttctacag tcaccagtcc tgaggcagaa tccagctcag ccatttcaac aactatttca 34140
   cctggtatac caggtgtgct gacatcactg gtcactagct ctgggagaga catcagtgca 34200
   acttttccaa cagtgcctga gtccccacat gaatcagagg caacagcctc atgggttact 34260
   catcctgcag tcaccagcac aacagttccc aggacaaccc ctaattattc tcatagtgaa 34320
   ccagacacca caccatcaat agccaccagt cctggggcag aagccacttc agattttcca 34380
   acaataactg tctcacctga tgtaccagat atggtaacct cacaggtcac tagttctggg 34440
   acagacacca gtataactat tccaactctg actctttctt ctggtgagcc agagaccaca 34500
   acctcattta tcacctattc tgagacacac acaagttcag ccattccaac tctccctgtc 34560
   tcccctggtg catcaaagat gctgacctca ctggtcatca gttctgggac agacagcact 34620
   acaactttcc caacactgac ggagacccca tatgaaccag agacaacagc catacagctc 34680
   attcatcctg cagagaccaa cacaatggtt cccaggacaa ctcccaagtt ttcccatagt 34740
   aagtcagaca ccacactccc agtagccatc accagtcctg ggccagaagc cagttcagct 34800
   gtttcaacga caactatctc acctgatatg tcagatctgg tgacctcact ggtccctagt 34860
   tctgggacag acaccagtac aaccttccca acattgagtg agaccccata tgaaccagag 34920
   actacagcca cgtggctcac tcatcctgca gaaaccagca caacggtttc tgggacaatt 34980
   cccaactttt cccatagggg atcagacact gcaccctcaa tggtcaccag tcctggagta 35040
   gacacgaggt caggtgttcc aactacaacc atcccaccca gtataccagg ggtagtgacc 35100
   tcacaggtca ctagttctgc aacagacact agtacagcta ttccaacttt gactccttct 35160
   cctggtgaac cagagaccac agcctcatca gctacccatc ctgggacaca gactggcttc 35220
   actgttccaa ttcggactgt tccctctagt gagccagata caatggcttc ctgggtcact 35280
   catcctccac agaccagcac acctgtttcc agaacaacct ccagtttttc ccatagtagt 35340
   ccagatgcca cacctgtaat ggccaccagt cctaggacag aagccagttc agctgtactg 35400
   acaacaatct cacctggtgc accagagatg gtgacttcac agatcactag ttctggggca 35460
   gcaaccagta caactgttcc aactttgact cattctcctg gtatgccaga gaccacagcc 35520
   ttattgagca cccatcccag aacagagaca agtaaaacat ttcctgcttc aactgtgttt 35580
   cctcaagtat cagagaccac agcctcactc accattagac ctggtgcaga gactagcaca 35640
   gctctcccaa ctcagacaac atcctctctc ttcaccctac ttgtaactgg aaccagcaga 35700
   gttgatctaa gtccaactgc ttcacctggt gtttctgcaa aaacagcccc actttccacc 35760
   catccaggga cagaaaccag cacaatgatt ccaacttcaa ctctttccct tggtttacta 35820
   gagactacag gcttactggc caccagctct tcagcagaga ccagcacgag tactctaact 35880
   ctgactgttt cccctgctgt ctctgggctt tccagtgcct ctataacaac tgataagccc 35940
   caaactgtga cctcctggaa cacagaaacc tcaccatctg taacttcagt tggaccccca 36000
   gaattttcca ggactgtcac aggcaccact atgaccttga taccatcaga gatgccaaca 36060
   ccacctaaaa ccagtcatgg agaaggagtg agtccaacca ctatcttgag aactacaatg 36120
   gttgaagcca ctaatttagc taccacaggt tccagtccca ctgtggccaa gacaacaacc 36180
   accttcaata cactggctgg aagcctcttt actcctctga ccacacctgg gatgtccacc 36240
   ttggcctctg agagtgtgac ctcaagaaca agttataacc atcggtcctg gatctccacc 36300
   accagcagtt ataaccgtcg gtactggacc cctgccacca gcactccagt gacttctaca 36360
   ttctccccag ggatttccac atcctccatc cccagctcca cagcagccac agtcccattc 36420
   atggtgccat tcaccctcaa cttcaccatc accaacctgc agtacgagga ggacatgcgg 36480
   caccctggtt ccaggaagtt caacgccaca gagagagaac tgcagggtct gctcaaaccc 36540
   ttgttcagga atagcagtct ggaatacctc tattcaggct gcagactagc ctcactcagg 36600
   ccagagaagg atagctcagc cacggcagtg gatgccatct gcacacatcg ccctgaccct 36660
   gaagacctcg gactggacag agagcgactg tactgggagc tgagcaatct gacaaatggc 36720
   atccaggagc tgggccccta caccctggac cggaacagtc tctatgtcaa tggtttcacc 36780
   catcgaagct ctatgcccac caccagcact cctgggacct ccacagtgga tgtgggaacc 36840
   tcagggactc catcctccag ccccagcccc acgactgctg gccctctcct gatgccgttc 36900
   accctcaact tcaccatcac caacctgcag tacgaggagg acatgcgtcg cactggctcc 36960
   aggaagttca acaccatgga gagtgtcctg cagggtctgc tcaagccctt gttcaagaac 37020
   accagtgttg gccctctgta ctctggctgc agattgacct tgctcaggcc cgagaaagat 37080
   ggggcagcca ctggagtgga tgccatctgc acccaccgcc ttgaccccaa aagccctgga 37140
   ctcaacaggg agcagctgta ctgggagcta agcaaactga ccaatgacat tgaagagctg 37200
   ggcccctaca ccctggacag gaacagtctc tatgtcaatg gtttcaccca tcagagctct 37260
   gtgtccacca ccagcactcc tgggacctcc acagtggatc tcagaacctc agggactcca 37320
   tcctccctct ccagccccac aattatggct gctggccctc tcctggtacc attcaccctc 37380
   aacttcacca tcaccaacct gcagtatggg gaggacatgg gtcaccctgg ctccaggaag 37440
   ttcaacacca cagagagggt cctgcagggt ctgcttggtc ccatattcaa gaacaccagt 37500
   gttggccctc tgtactctgg ctgcagactg acctctctca ggtctgagaa ggatggagca 37560
   gccactggag tggatgccat ctgcatccat catcttgacc ccaaaagccc tggactcaac 37620
   agagagcggc tgtactggga gctgagccaa ctgaccaatg gcatcaaaga gctgggcccc 37680
   tacaccctgg acaggaacag tctctatgtc aatggtttca cccatcggac ctctgtgccc 37740
   accagcagca ctcctgggac ctccacagtg gaccttggaa cctcagggac tccattctcc 37800
   ctcccaagcc ccgcaactgc tggccctctc ctggtgctgt tcaccctcaa cttcaccatc 37860
   accaacctga agtatgagga ggacatgcat cgccctggct ccaggaagtt caacaccact 37920
   gagagggtcc tgcagactct gcttggtcct atgttcaaga acaccagtgt tggccttctg 37980
   tactctggct gcagactgac cttgctcagg tccgagaagg atggagcagc cactggagtg 38040
   gatgccatct gcacccaccg tcttgacccc aaaagccctg gagtggacag ggagcagcta 38100
   tactgggagc tgagccagct gaccaatggc atcaaagagc tgggccccta caccctggac 38160
   aggaacagtc tctatgtcaa tggtttcacc cattggatcc ctgtgcccac cagcagcact 38220
   cctgggacct ccacagtgga ccttgggtca gggactccat cctccctccc cagccccaca 38280
   actgctggcc ctctcctggt gccgttcacc ctcaacttca ccatcaccaa cctgaagtac 38340
   gaggaggaca tgcattgccc tggctccagg aagttcaaca ccacagagag agtcctgcag 38400
   agtctgcttg gtcccatgtt caagaacacc agtgttggcc ctctgtactc tggctgcaga 38460
   ctgaccttgc tcaggtccga gaaggatgga gcagccactg gagtggatgc catctgcacc 38520
   caccgtcttg accccaaaag ccctggagtg gacagggagc agctatactg ggagctgagc 38580
   cagctgacca atggcatcaa agagctgggt ccctacaccc tggacagaaa cagtctctat 38640
   gtcaatggtt tcacccatca gacctctgcg cccaacacca gcactcctgg gacctccaca 38700
   gtggaccttg ggacctcagg gactccatcc tccctcccca gccctacatc tgctggccct 38760
   ctcctggtgc cattcaccct caacttcacc atcaccaacc tgcagtacga ggaggacatg 38820
   catcacccag gctccaggaa gttcaacacc acggagcggg tcctgcaggg tctgcttggt 38880
   cccatgttca agaacaccag tgtcggcctt ctgtactctg gctgcagact gaccttgctc 38940
   aggcctgaga agaatggggc agccactgga atggatgcca tctgcagcca ccgtcttgac 39000
   cccaaaagcc ctggactcaa cagagagcag ctgtactggg agctgagcca gctgacccat 39060
   ggcatcaaag agctgggccc ctacaccctg gacaggaaca gtctctatgt caatggtttc 39120
   acccatcgga gctctgtggc ccccaccagc actcctggga cctccacagt ggaccttggg 39180
   acctcaggga ctccatcctc cctccccagc cccacaacag ctgttcctct cctggtgccg 39240
   ttcaccctca actttaccat caccaatctg cagtatgggg aggacatgcg tcaccctggc 39300
   tccaggaagt tcaacaccac agagagggtc ctgcagggtc tgcttggtcc cttgttcaag 39360
   aactccagtg tcggccctct gtactctggc tgcagactga tctctctcag gtctgagaag 39420
   gatggggcag ccactggagt ggatgccatc tgcacccacc accttaaccc tcaaagccct 39480
   ggactggaca gggagcagct gtactggcag ctgagccaga tgaccaatgg catcaaagag 39540
   ctgggcccct acaccctgga ccggaacagt ctctacgtca atggtttcac ccatcggagc 39600
   tctgggctca ccaccagcac tccttggact tccacagttg accttggaac ctcagggact 39660
   ccatcccccg tccccagccc cacaaccacc ggccctctcc tggtgccatt cacactcaac 39720
   ttcaccatca ctaacctaca gtatgaggag aacatgggtc accctggctc caggaagttc 39780
   aacatcacgg agagtgttct gcagggtctg ctcaagccct tgttcaagag caccagtgtt 39840
   ggccctctgt attctggctg cagactgacc ttgctcaggc ctgagaagga tggagtagcc 39900
   accagagtgg acgccatctg cacccaccgc cctgacccca aaatccctgg gctagacaga 39960
   cagcagctat actgggagct gagccagctg acccacagca tcactgagct gggaccctac 40020
   accctggata gggacagtct ctatgtcaat ggtttcaccc agcggagctc tgtgcccacc 40080
   accagcactc ctgggacttt cacagtacag ccggaaacct ctgagactcc atcatccctc 40140
   cctggcccca cagccactgg ccctgtcctg ctgccattca ccctcaattt taccatcact 40200
   aacctgcagt atgaggagga catgcgtcgc cctggctcca ggaagttcaa caccacggag 40260
   agggtccttc agggtctgct tatgcccttg ttcaagaaca ccagtgtcag ctctctgtac 40320
   tctggttgca gactgacctt gctcaggcct gagaaggatg gggcagccac cagagtggat 40380
   gctgtctgca cccatcgtcc tgaccccaaa agccctggac tggacagaga gcggctgtac 40440
   tggaagctga gccagctgac ccacggcatc actgagctgg gcccctacac cctggacagg 40500
   cacagtctct atgtcaatgg tttcacccat cagagctcta tgacgaccac cagaactcct 40560
   gatacctcca caatgcacct ggcaacctcg agaactccag cctccctgtc tggacccatg 40620
   accgccagcc ctctcctggt gctattcaca attaacttca ccatcactaa cctgcggtat 40680
   gaggagaaca tgcatcaccc tggctctaga aagtttaaca ccacggagag agtccttcag 40740
   ggtctgctca ggcctgtgtt caagaacacc agtgttggcc ctctgtactc tggctgcaga 40800
   ctgaccttgc tcaggcccaa gaaggatggg gcagccacca aagtggatgc catctgcacc 40860
   taccgccctg atcccaaaag ccctggactg gacagagagc agctatactg ggagctgagc 40920
   cagctgaccc acagcatcac tgagctgggc ccctacaccc tggacaggga cagtctctat 40980
   gtcaatggtt tcacacagcg gagctctgtg cccaccacta gcattcctgg gacccccaca 41040
   gtggacctgg gaacatctgg gactccagtt tctaaacctg gtccctcggc tgccagccct 41100
   ctcctggtgc tattcactct caacttcacc atcaccaacc tgcggtatga ggagaacatg 41160
   cagcaccctg gctccaggaa gttcaacacc acggagaggg tccttcaggg cctgctcagg 41220
   tccctgttca agagcaccag tgttggccct ctgtactctg gctgcagact gactttgctc 41280
   aggcctgaaa aggatgggac agccactgga gtggatgcca tctgcaccca ccaccctgac 41340
   cccaaaagcc ctaggctgga cagagagcag ctgtattggg agctgagcca gctgacccac 41400
   aatatcactg agctgggccc ctatgccctg gacaacgaca gcctctttgt caatggtttc 41460
   actcatcgga gctctgtgtc caccaccagc actcctggga cccccacagt gtatctggga 41520
   gcatctaaga ctccagcctc gatatttggc ccttcagctg ccagccatct cctgatacta 41580
   ttcaccctca acttcaccat cactaacctg cggtatgagg agaacatgtg gcctggctcc 41640
   aggaagttca acactacaga gagggtcctt cagggcctgc taaggccctt gttcaagaac 41700
   accagtgttg gccctctgta ctctggctgc aggctgacct tgctcaggcc agagaaagat 41760
   ggggaagcca ccggagtgga tgccatctgc acccaccgcc ctgaccccac aggccctggg 41820
   ctggacagag agcagctgta tttggagctg agccagctga cccacagcat cactgagctg 41880
   ggcccctaca cactggacag ggacagtctc tatgtcaatg gtttcaccca tcggagctct 41940
   gtacccacca ccagcaccgg ggtggtcagc gaggagccat tcacactgaa cttcaccatc 42000
   aacaacctgc gctacatggc ggacatgggc caacccggct ccctcaagtt caacatcaca 42060
   gacaacgtca tgcagcacct gctcagtcct ttgttccaga ggagcagcct gggtgcacgg 42120
   tacacaggct gcagggtcat cgcactaagg tctgtgaaga acggtgctga gacacgggtg 42180
   gacctcctct gcacctacct gcagcccctc agcggcccag gtctgcctat caagcaggtg 42240
   ttccatgagc tgagccagca gacccatggc atcacccggc tgggccccta ctctctggac 42300
   aaagacagcc tctaccttaa cggttacaat gaacctggtc cagatgagcc tcctacaact 42360
   cccaagccag ccaccacatt cctgcctcct ctgtcagaag ccacaacagc catggggtac 42420
   cacctgaaga ccctcacact caacttcacc atctccaatc tccagtattc accagatatg 42480
   ggcaagggct cagctacatt caactccacc gagggggtcc ttcagcacct gctcagaccc 42540
   ttgttccaga agagcagcat gggccccttc tacttgggtt gccaactgat ctccctcagg 42600
   cctgagaagg atggggcagc cactggtgtg gacaccacct gcacctacca ccctgaccct 42660
   gtgggccccg ggctggacat acagcagctt tactgggagc tgagtcagct gacccatggt 42720
   gtcacccaac tgggcttcta tgtcctggac agggatagcc tcttcatcaa tggctatgca 42780
   ccccagaatt tatcaatccg gggcgagtac cagataaatt tccacattgt caactggaac 42840
   ctcagtaatc cagaccccac atcctcagag tacatcaccc tgctgaggga catccaggac 42900
   aaggtcacca cactctacaa aggcagtcaa ctacatgaca cattccgctt ctgcctggtc 42960
   accaacttga cgatggactc cgtgttggtc actgtcaagg cattgttctc ctccaatttg 43020
   gaccccagcc tggtggagca agtctttcta gataagaccc tgaatgcctc attccattgg 43080
   ctgggctcca cctaccagtt ggtggacatc catgtgacag aaatggagtc atcagtttat 43140
   caaccaacaa gcagctccag cacccagcac ttctacctga atttcaccat caccaaccta 43200
   ccatattccc aggacaaagc ccagccaggc accaccaatt accagaggaa caaaaggaat 43260
   attgaggatg cgctcaacca actcttccga aacagcagca tcaagagtta tttttctgac 43320
   tgtcaagttt caacattcag gtctgtcccc aacaggcacc acaccggggt ggactccctg 43380
   tgtaacttct cgccactggc tcggagagta gacagagttg ccatctatga ggaatttctg 43440
   cggatgaccc ggaatggtac ccagctgcag aacttcaccc tggacaggag cagtgtcctt 43500
   gtggatgggt attctcccaa cagaaatgag cccttaactg ggaattctga ccttcccttc 43560
   tgggctgtca tcctcatcgg cttggcagga ctcctgggag tcatcacatg cctgatctgc 43620
   ggtgtcctgg tgaccacccg ccggcggaag aaggaaggag aatacaacgt ccagcaacag 43680
   tgcccaggct actaccagtc acacctagac ctggaggatc tgcaatgact ggaacttgcc 43740
   ggtgcctggg gtgcctttcc cccagccagg gtccaaagaa gcttggctgg ggcagaaata 43800
   aaccatattg gtcgga 43816
<210> 59
   <211> 267
   <212> PRT
   <213> Homo sapiens
<400> 59
   Met Arg Leu Thr Val Leu Cys Ala Val Cys Leu Leu Pro Gly Ser Leu
    1               5                  10                  15
   Ala Leu Pro Leu Pro Gln Glu Ala Gly Gly Met Ser Glu Leu Gln Trp
               20                  25                  30
   Glu Gln Ala Gln Asp Tyr Leu Lys Arg Phe Tyr Leu Tyr Asp Ser Glu
           35                  40                  45
   Thr Lys Asn Ala Asn Ser Leu Glu Ala Lys Leu Lys Glu Met Gln Lys
       50                  55                  60
   Phe Phe Gly Leu Pro Ile Thr Gly Met Leu Asn Ser Arg Val Ile Glu
   65                  70                  75                  80
   Ile Met Gln Lys Pro Arg Cys Gly Val Pro Asp Val Ala Glu Tyr Ser
                   85                  90                  95
   Leu Phe Pro Asn Ser Pro Lys Trp Thr Ser Lys Val Val Thr Tyr Arg
               100                 105                 110
   Ile Val Ser Tyr Thr Arg Asp Leu Pro His Ile Thr Val Asp Arg Leu
           115                 120                 125
   Val Ser Lys Ala Leu Asn Met Trp Gly Lys Glu Ile Pro Leu His Phe
       130                 135                 140
   Arg Lys Val Val Trp Gly Thr Ala Asp Ile Met Ile Gly Phe Ala Arg
   145                 150                 155                 160
   Gly Ala His Gly Asp Ser Tyr Pro Phe Asp Gly Pro Gly Asn Thr Leu
                   165                 170                 175
   Ala His Ala Phe Ala Pro Gly Thr Gly Leu Gly Gly Asp Ala His Phe
               180                 185                 190
   Asp Glu Asp Glu Arg Trp Thr Asp Gly Ser Ser Leu Gly Ile Asn Phe
           195                 200                 205
   Leu Tyr Ala Ala Thr His Glu Leu Gly His Ser Leu Gly Met Gly His
       210                 215                 220
   Ser Ser Asp Pro Asn Ala Val Met Tyr Pro Thr Tyr Gly Asn Gly Asp
   225                 230                 235                 240
   Pro Gln Asn Phe Lys Leu Ser Gln Asp Asp Ile Lys Gly Ile Gln Lys
                   245                 250                 255
   Leu Tyr Gly Lys Arg Ser Asn Ser Arg Lys Lys
               260                 265
<210> 60
   <211> 1147
   <212> DNA
   <213> Homo sapiens
<400> 60
   accaaatcaa ccataggtcc aagaacaatt gtctctggac ggcagctatg cgactcaccg 60
   tgctgtgtgc tgtgtgcctg ctgcctggca gcctggccct gccgctgcct caggaggcgg 120
   gaggcatgag tgagctacag tgggaacagg ctcaggacta tctcaagaga ttttatctct 180
   atgactcaga aacaaaaaat gccaacagtt tagaagccaa actcaaggag atgcaaaaat 240
   tctttggcct acctataact ggaatgttaa actcccgcgt catagaaata atgcagaagc 300
   ccagatgtgg agtgccagat gttgcagaat actcactatt tccaaatagc ccaaaatgga 360
   cttccaaagt ggtcacctac aggatcgtat catatactcg agacttaccg catattacag 420
   tggatcgatt agtgtcaaag gctttaaaca tgtggggcaa agagatcccc ctgcatttca 480
   ggaaagttgt atggggaact gctgacatca tgattggctt tgcgcgagga gctcatgggg 540
   actcctaccc atttgatggg ccaggaaaca cgctggctca tgcctttgcg cctgggacag 600
   gtctcggagg agatgctcac ttcgatgagg atgaacgctg gacggatggt agcagtctag 660
   ggattaactt cctgtatgct gcaactcatg aacttggcca ttctttgggt atgggacatt 720
   cctctgatcc taatgcagtg atgtatccaa cctatggaaa tggagatccc caaaatttta 780
   aactttccca ggatgatatt aaaggcattc agaaactata tggaaagaga agtaattcaa 840
   gaaagaaata gaaacttcag gcagaacatc cattcattca ttcattggat tgtatatcat 900
   tgttgcacaa tcagaattga taagcactgt tcctccactc catttagcaa ttatgtcacc 960
   cttttttatt gcagttggtt tttgaatgtc tttcactcct tttaaggata aactccttta 1020
   tggtgtgact gtgtcttatt catctatact tgcagtgggt agatgtcaat aaatgttaca 1080
   tacacaaata aataaaatgt ttattccatg gtaaatttaa aaaaaaaaaa aaaaaaaaaa 1140
   aaaaaaa                                                           1147
<210> 61
   <211> 227
   <212> PRT
   <213> Homo sapiens
<400> 61
   Met Asn Ile Lys Gly Ser Pro Trp Lys Gly Ser Leu Leu Leu Leu Leu
    1               5                  10                  15
   Val Ser Asn Leu Leu Leu Cys Gln Ser Val Ala Pro Leu Pro Ile Cys
               20                  25                  30
   Pro Gly Gly Ala Ala Arg Cys Gln Val Thr Leu Arg Asp Leu Phe Asp
           35                  40                  45
   Arg Ala Val Val Leu Ser His Tyr Ile His Asn Leu Ser Ser Glu Met
       50                  55                  60
   Phe Ser Glu Phe Asp Lys Arg Tyr Thr His Gly Arg Gly Phe Ile Thr
   65                  70                  75                  80
   Lys Ala Ile Asn Ser Cys His Thr Ser Ser Leu Ala Thr Pro Glu Asp
                   85                  90                  95
   Lys Glu Gln Ala Gln Gln Met Asn Gln Lys Asp Phe Leu Ser Leu Ile
               100                 105                 110
   Val Ser Ile Leu Arg Ser Trp Asn Glu Pro Leu Tyr His Leu Val Thr
           115                 120                 125
   Glu Val Arg Gly Met Gln Glu Ala Pro Glu Ala Ile Leu Ser Lys Ala
       130                 135                 140
   Val Glu Ile Glu Glu Gln Thr Lys Arg Leu Leu Glu Gly Met Glu Leu
   145                 150                 155                 160
   Ile Val Ser Gln Val His Pro Glu Thr Lys Glu Asn Glu Ile Tyr Pro
                   165                 170                 175
   Val Trp Ser Gly Leu Pro Ser Leu Gln Met Ala Asp Glu Glu Ser Arg
               180                 185                 190
   Leu Ser Ala Tyr Tyr Asn Leu Leu His Cys Leu Arg Arg Asp Ser His
           195                 200                 205
   Lys Ile Asp Asn Tyr Leu Lys Leu Leu Lys Cys Arg Ile Ile His Asn
       210                 215                 220
   Asn Asn Cys
   225
<210> 62
   <211> 1388
   <212> DNA
   <213> Homo sapiens
<400> 62
   gggatcctta ttctatatct cttggtattt agtgtaaaaa ttttaaaatc tttacctagc 60
   aatcttgagg aagaaacttg ataactgata atacatgaga ttgttaccta agtgaaatat 120
   aatcctatat attcaacaaa ctttagagaa ataagataaa ttttaaagta aatgacttct 180
   gtagttttat agatcctcca aaccaatcta gtctcagatc tcaccttcat catttctctc 240
   atttcctttt ggcctaatta atcaaaatcc ttcctagaat gttcatttct ggccagtatg 300
   tcttcctgaa tatgaataag aaataaaata ccatttgatg tttgaaatta tgggggtaat 360
   ctcaatgacg gaaatagatg accaggaaaa gggaaacgaa tgcctgattc attatattca 420
   tgaagatatc aaaggtttat aaagccaata tctgggaaag agaaaaccgt gagacttcca 480
   gatcttctct ggtgaagtgt gtttcctgca acgatcacga acatgaacat caaaggatcg 540
   ccatggaaag ggtccctcct gctgctgctg gtgtcaaacc tgctcctgtg ccagagcgtg 600
   gcccccttgc ccatctgtcc cggcggggct gcccgatgcc aggtgaccct tcgagacctg 660
   tttgaccgcg ccgtcgtcct gtcccactac atccataacc tctcctcaga aatgttcagc 720
   gaattcgata aacggtatac ccatggccgg gggttcatta ccaaggccat caacagctgc 780
   cacacttctt cccttgccac ccccgaagac aaggagcaag cccaacagat gaatcaaaaa 840
   gactttctga gcctgatagt cagcatattg cgatcctgga atgagcctct gtatcatctg 900
   gtcacggaag tacgtggtat gcaagaagcc ccggaggcta tcctatccaa agctgtagag 960
   attgaggagc aaaccaaacg gcttctagag ggcatggagc tgatagtcag ccaggttcat 1020
   cctgaaacca aagaaaatga gatctaccct gtctggtcgg gacttccatc cctgcagatg 1080
   gctgatgaag agtctcgcct ttctgcttat tataacctgc tccactgcct acgcagggat 1140
   tcacataaaa tcgacaatta tctcaagctc ctgaagtgcc gaatcatcca caacaacaac 1200
   tgctaagccc acatccattt catctatttc tgagaaggtc cttaatgatc cgttccattg 1260
   caagcttctt ttagttgtat ctcttttgaa tccatgcttg ggtgtaacag gtctcctctt 1320
   aaaaaataaa aactgactcc ttagagacat caaaatccaa aaaaaaaaaa aaaaaaaaaa 1380
   aaaaaaaa                                                          1388
<210> 63
   <211> 132
   <212> PRT
   <213> Homo sapiens
<400> 63
   Met Lys Ser Ser Gly Leu Phe Pro Phe Leu Val Leu Leu Ala Leu Gly
    1               5                  10                  15
   Thr Leu Ala Pro Trp Ala Val Glu Gly Ser Gly Lys Ser Phe Lys Ala
               20                  25                  30
   Gly Val Cys Pro Pro Lys Lys Ser Ala Gln Cys Leu Arg Tyr Lys Lys
           35                  40                  45
   Pro Glu Cys Gln Ser Asp Trp Gln Cys Pro Gly Lys Lys Arg Cys Cys
       50                  55                  60
   Pro Asp Thr Cys Gly Ile Lys Cys Leu Asp Pro Val Asp Thr Pro Asn
   65                  70                  75                  80
   Pro Thr Arg Arg Lys Pro Gly Lys Cys Pro Val Thr Tyr Gly Gln Cys
                   85                  90                  95
   Leu Met Leu Asn Pro Pro Asn Phe Cys Glu Met Asp Gly Gln Cys Lys
               100                 105                 110
   Arg Asp Leu Lys Cys Cys Met Gly Met Cys Gly Lys Ser Cys Val Ser
           115                 120                 125
   Pro Val Lys Ala
       130
<210> 64
   <211> 598
   <212> DNA
   <213> Homo sapiens
<400> 64
   cagagtcact cctgccttca ccatgaagtc cagcggcctc ttccccttcc tggtgctgct 60
   tgccctggga actctggcac cttgggctgt ggaaggctct ggaaagtcct tcaaagctgg 120
   agtctgtcct cctaagaaat ctgcccagtg ccttagatac aagaaacctg agtgccagag 180
   tgactggcag tgtccaggga agaagagatg ttgtcctgac acttgtggca tcaaatgcct 240
   ggatcctgtt gacaccccaa acccaacaag gaggaagcct gggaagtgcc cagtgactta 300
   tggccaatgt ttgatgctta acccccccaa tttctgtgag atggatggcc agtgcaagcg 360
   tgacttgaag tgttgcatgg gcatgtgtgg gaaatcctgc gtttcccctg tgaaagcttg 420
   attcctgcca tatggaggag gctctggagt cctgctctgt gtggtccagg tcctttccac 480
   cctgagactt ggctccacca ctgatatcct cctttgggga aaggcttggc acacagcagg 540
   ctttcaagaa gtgccagttg atcaatgaat aaataaacga gcctatttct ctttgcac   598

## Description

### FIELD OF THE INVENTION

The present invention relates to methods and compositions for identifying women having an increased likelihood of having ovarian cancer.

### BACKGROUND OF THE INVENTION

Ovarian cancer represents a heterogeneous group of diseases that affect women on a global basis. There are several forms of ovarian cancer which include epithelial cancer, germ-line cancer of the ovaries and ovarian stromal cancer. Epithelial ovarian cancer represents the most common form of the disease. Approximately 5-10% of epithelial ovarian cancer represents a hereditary form of the disease and three common patterns are recognized: ovarian cancer alone; ovarian and breast cancer linked to BRAC1 and BRCA2 genetic linkage on chromosomes 17q21 and 13q12 respectively; and ovarian and colon cancer. The most important risk factor for ovarian cancer is a first degree relative with the disease (e.g., a mother, sister or daughter with ovarian cancer). See, for example, Patridge et al. (1999) CA-A Cancer Journal for Clinicians 49:297-320In 2005, there were an estimated 22,000 new cases of ovarian cancer diagnoses and 16,000 deaths from ovarian cancer. Ovarian cancer is a disease that primarily affects post-menopausal women with the median age for diagnosis at 63 years of age. However, the disease can affect women at all age groups.

The classification of ovarian cancer stage is based upon the extent of localization versus spread of the disease beyond the ovaries. Stage 1 ovarian cancer is confined to one or both of the ovaries. Stage 2 disease involves a tumor in one or both ovaries with pelvic extension. In Stage 3 ovarian cancer, a tumor is present in or both ovaries with microscopically confirmed peritoneal metastasis outside the pelvis and/or regional lymph node metastasis. Stage 4 ovarian cancer is characterized by distant metastasis beyond the peritoneal cavity. Ovarian cancer is generally diagnosed in an advance stage of the disease due to the lack of specific clinical symptoms that would indicate the presence of small tumors. For women under the age of 50, less than 40% of ovarian cancers are detected when tumors are localized to one or both ovaries and when disease prognosis is best. For women over the age of 50, that number drops to less than 15%. Approximately 68% of women of all age groups afflicted with ovarian cancer are not diagnosed until distant metastasis is present.

Ovarian cancer spreads via local shedding from the ovarian epithelium into the peritoneal cavity followed by implantation on the peritoneum and local invasion of the bowel and bladder. The presence of lymph node involvement in ovarian cancer is evident in all stages of diagnosed ovarian cancer. The percentage of positive lymph nodes increases significantly with progression of the disease (i.e., Stage 1, 24%; Stage 2, 50%, Stage 3, 74%; Stage 4, 73%). *Id*.

The survival of patients with ovarian cancer is a function of the stage at which the disease is diagnosed, with the 5-year survival decreasing with advanced disease. More than 90% of women diagnosed with ovarian cancer in Stage 1 survive for at least 5 years following diagnosis. The 5-year survival rate drops to less than 30% when the disease is not diagnosed until Stage 4 (i.e., distant metastasis). *Id*.

Epithelial ovarian cancer is the most common form of the disease. There are four recognized major histological classes of epithelial ovarian cancer and include serous, endometrioid, clear cell, and mucinous subtypes. The pathogenesis of ovarian cancer is poorly understood but is believed to arise from ovarian surface epithelium. See Bell (2005) Mod. Pathol.18 (Suppl 2):S19-32. Life factors that provide the greatest reduction in risk of ovarian cancer include multiparity, use of oral contraceptives, and breast feeding, all of which prevent ovulation. Because ovulation results in epithelial damage, followed by repair and possible inflammatory responses, repetition of this process throughout a woman's reproductive life without interruption appears to lead to cell damage and to increase the risk of ovarian cancer. See, for example, Ness et al. (1999) J. Natl. Cancer Inst. 91:1459-1467. However, there is no recognized, stepwise progression of ovarian cancer through defined precursor lesions, such as those recognized for both cervical carcinoma and colon cancer. Hence, considerable research has been directed at understanding the molecular basis for ovarian cancer and to understand the basic differences between the various histological subtypes of ovarian cancer. These studies have utilized gene expression analysis to provide this understanding and have identified a series of potential biomarkers for evaluation in diagnostic applications. See for example Ono et al. (2000) Cancer Res. 60:5007-11; Welsh et al. (2001) Proc. Natl. Acad. Sci. USA 98:1176-1181; Donninger et al. (2004) Oncogene 23:8065-8077; and Lee et al. (2004) Int. J. Oncol. 24(4):847-851.

Ovarian cancer is often detected with the presentation of overt clinical symptoms, most notably the presentation of abdominal pain, an adnexal mass, abdominal bloating, and urinary urgency. As such, the detection of ovarian cancer is often detected at an advanced stage, where the prognosis and clinical outcome is poor. Detection of ovarian cancer at an early stage (i.e., Stage 1) results in approximately 90% cure rate using standard surgery and chemotherapy; hence there is a clinical need to detect ovarian cancer at an early stage where treatment will be most effective. Unfortunately, current screening methods to detect early stage ovarian cancer are insufficient. The current practice for ovarian cancer screening employs the use of CA125 and transvaginal ultrasound (sonogaphy). Rising serum levels of CA125 are associated with ovarian cancer and subsequent utilization of transvaginal ultrasound helps detect the presence of ovarian cancer. Confirmation of ovarian disease is based upon invasive procedures such as laprotomy. However, the use of CA125 is ineffective for general population screening due to issues of limited sensitivity, limited specificity, and a poor positive predictive value of <3%. Bast (2003) J Clin Oncol. 21(10 Suppl):200-205. As a result, there is no consensus on the recommendations for generally screening for ovarian cancer in the asymptomatic patient population. For high risk patients, the generally accepted procedures for the detection of ovarian cancer include the use of pelvic examinations, the use of CA125 serum testing, and transvaginal ultrasound (sonography). Patridge et al. (1999) CA-A Cancer Journal for Clinicians 49:297-320.

CA125 is a well characterized tumor marker normally expressed on the surface of epithelial cells and is often detected in the serum of normal patients at 35U/mL. Elevated serum levels of CA125 (>35U/mL) are often detected in approximately 85% of ovarian cancer patients; the remaining 15% of ovarian cancer patients have normal serum levels of CA125. Furthermore, CA125 is elevated in only 50% of stage 1 ovarian cancer patients, thereby limiting its clinical utility in the early detection of ovarian cancer. However, elevated serum levels of CA 125 are used for the monitoring of disease recurrence following therapeutic intervention and this represents the currently approved use for CA125 by the FDA. In addition, elevated serum levels of CA125 are predictive of future detectable ovarian cancer. Bast et al. discloses a two-step method of detecting ovarian cancer by screening for CA125 as a first marker. Further markers are screened if the first marker is over-expressed. (Bast et al., 2002. In Cancer treatment and research, Nijhoff, Dordrecht, NL. Vol 107, pages 61-97).

The low prevalence rates of ovarian cancer in the general population create significant challenges for the development of a screening test that would promote early detection of the disease. Screening methods for diseases with low prevalence rates such as ovarian cancer often result in a high ratio of false positives to true positives that limits the clinical utility of such screening programs. Given the significant risks associated with surgical exploration for possible ovarian cancer, a clinically useful screening test should refer to surgery no more than 10 women for every woman who actually has ovarian cancer (i.e., a positive predictive value (PPV) of at least 10%). Skates et al. (2004) J. Clin. Oncol. 22:4059-4066. PPV is highly dependent upon the prevalence rates for a particular disease or condition and will shift dramatically as a result of differences in disease prevalence. Therefore, with low-prevalence diseases, such as ovarian cancer, screening diagnostic tests with a relatively low PPV still have significant clinical utility. Potential ovarian cancer screening programs must be adjusted for the low prevalence of ovarian cancer and assessed for biomarker performance and clinical need. See, for example, Skates *et al.*

(2004) J. Clin. Oncol. 22:4059-4066; Bast et al. (2005) Int. J. Gynecol. Cancer 15:274-281; and Rosen et al. (2005) Gyn. Oncol. 99:267-277. Despite efforts to identify a biomarker or panel of biomarkers for the detection, particularly early detection, of ovarian cancer, no adequate screening or diagnostic test that satisfies clinical needs currently exists. Currently available methods, such as detection of CA125, exhibit unacceptably high false-positive rates.

The current recommendations from the National Cancer Institute state that "there is insufficient evidence to establish that screening for ovarian cancer with serum markers such as CA125, transvaginal ultrasound or pelvic examinations would result in a decrease in mortality from ovarian cancer" (*NCI Summary of Evidence (Level 4,* 5); dated February 2005). In light of the serious risk of false-positives with currently available screening techniques, the NCI has not supported institution of general screening procedures for ovarian cancer. As such, no standardized screening test exists for ovarian cancer, despite the fact that early diagnosis significantly improves 5-year survival rates.

Therefore, a significant need exists in the art for reliable methods and compositions that are capable of specifically identifying women that have ovarian cancer. In particular, screening methods for identifying patients with an increased likelihood of having ovarian cancer are needed. Women identified as having an increased likelihood of having ovarian cancer could be selected for more aggressive diagnostic methods to definitively determine if they presently have the disease. Moreover, such screening methods could be performed in the general female patient population on a routine basis to facilitate the detection of ovarian cancer in the early stages of the disease when prognosis and disease outcome are best.

### BRIEF SUMMARY OF THE INVENTION

Screening methods for identifying patients with an increased likelihood of having ovarian cancer are provided. The methods of the invention generally comprise detecting in a patient body sample expression of a plurality of biomarkers that are selectively overexpressed in ovarian cancer. Overexpression of the biomarkers is indicative of an increased likelihood that the patient has ovarian cancer. The methods of the invention comprise a "two-step" analysis, wherein a first assay step is performed to detect the expression of a first biomarker. If the first biomarker is overexpressed, a second assay step is performed to detect the expression of a panel of biomarkers. Overexpression of the first and panel of biomarkers is indicative of an increased likelihood that the patient has ovarian cancer. The first assay step may be designed to enrich the patient population under review by eliminating a large percentage of women that are "true negatives." The second assay step is typically intended to rule out those patients in the enriched population that do not presently have ovarian cancer by eliminating additional true negative patients from the enriched population. These assay steps may be performed as a single test encompassing both assay steps or as two distinct tests, wherein each test comprises one of the assay steps.

A patient that is identified by the screening methods of the invention as having an increased likelihood of having ovarian cancer may be subjected to further diagnostic tests to definitively determine if the patient has ovarian cancer. Patients that are classified as having an increased likelihood of having ovarian cancer in accordance with the methods disclosed herein, but that are determined not to currently have the disease, are generally monitored on a regular basis for the development of ovarian cancer. Moreover, the present methods may be used to screen the general female patient population for ovarian cancer on a routine basis. Thus, the screening methods of the invention may permit the diagnosis of ovarian cancer at earlier stages of the disease when prognosis is significantly better.

Biomarker expression can be assessed at the protein or nucleic acid level. In some embodiments, biomarker expression is detected at the protein level using biomarker-specific antibodies. Expression of the biomarkers of the invention can also be detected by nucleic acid-based techniques, including, for example, hybridization and RT-PCR. Biomarker expression can be assessed in a variety of body samples, including but not limited to blood (e.g., whole blood, blood serum, blood having platelets removed, etc.), lymph, ascitic fluids, urine, gynecological fluids (e.g., ovarian, fallopian, and uterine secretion, menses, etc.), biopsies, and fluids obtained during laparoscopy.

The invention therefore provides a screening method for identifying patients with an increased likelihood of having ovarian cancer, the method comprising:
a) performing a first assay step comprising detecting the expression of HE4 in a body sample and determining if HE4 is overexpressed; and
b) performing a second assay step if HE4 is determined to be overexpressed in step (a), the second assay step comprising detecting the expression of a panel of biomarkers in the body sample, wherein the panel comprises the biomarkers CA125, glycodelin, PAI-1, and PLAU-R, and determining if the panel of biomarkers is overexpressed;
wherein overexpression of both HE4 and the panel of biomarkers of (b) is indicative of an increased likelihood of the patient having ovarian cancer.

### BRIEF DESCRIPTION OF THE DRAWINGS

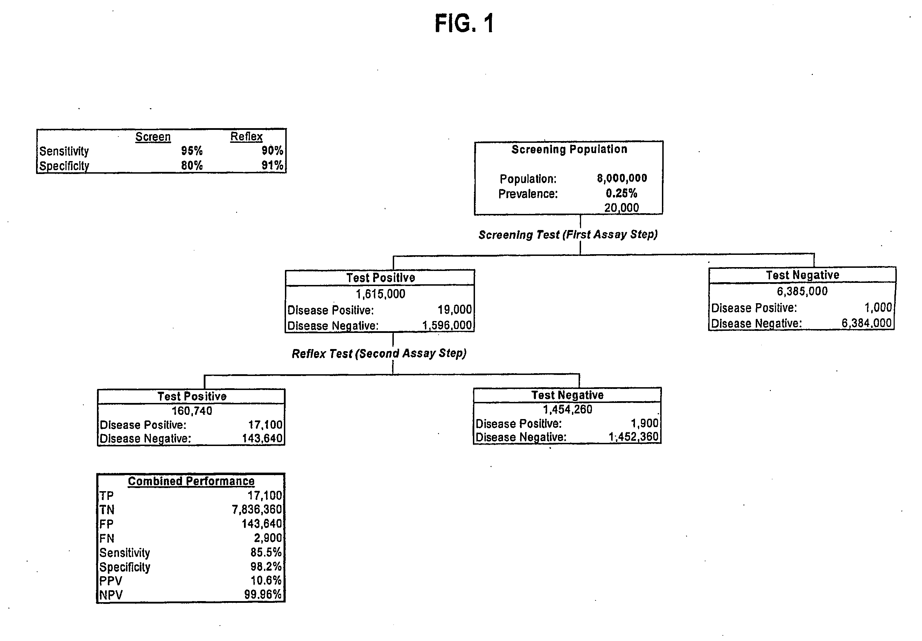

Figure 1 provides a schematic representation of an exemplary "two-step" screening test for identifying patients with an increased likelihood of having ovarian cancer. The 8 million female patients screened in this example represent the high-risk, asymptomatic U.S. patient population, as defined herein below. In the first assay step, a significant number of true negatives are eliminated from further testing, thereby leaving an enriched population for further analysis in the second assay step. The second assay step further rules out additional patients that are true negatives in order to identify those women with the highest risk of having ovarian cancer. Additional details regarding the two-step screening method are provided in the text.
Figure 2 provides the Receiver Operating Characteristic (ROC) plots for HE4 obtained with samples from patients over the age of 55 (A) and with all patient samples. Additional experimental details are provided in Example 2.
Figure 3 provides the ROC plots for inhibin A (INH) obtained with samples from patients over the age of 55 (A) and with all patient samples. Additional experimental details are provided in Example 2.
Figure 4 provides the ROC plot for prolactin obtained with all patient samples. Additional experimental details are provided in Example 2.
Figure 5 provides the ROC plot for PLAU-R obtained with all patient samples. Additional experimental details are provided in Example 2.
Figure 6 provides the ROC plots for glycodelin (GLY) obtained with samples from patients over the age of 55 (A) and with all patient samples. Additional experimental details are provided in Example 2.
Figure 7 provides the ROC plot for SLPI obtained with all patient samples. Additional experimental details are provided in Example 2.
Figure 8 provides the ROC plot for CTHRC1 obtained with all patient samples. Additional experimental details are provided in Example 2.
Figure 9 provides the ROC plot for PAI-1 obtained with all patient samples. Additional experimental details are provided in Example 2.
Figure 10 provides the ROC plot for KLK-10 obtained with all patient samples. Additional experimental details are provided in Example 2.
Figure 11 provides the ROC plots for CA125 obtained with samples from patients over the age of 55 (A) and with all patient samples. Additional experimental details are provided in Example 2.
Figure 12 provides the ROC plot for KLK-6 obtained with all patient samples. Additional experimental details are provided in Example 2.
Figure 13 provides the ROC plot for Muc-1 (MU) obtained with all patient samples. Additional experimental details are provided in Example 2.
Figure 14 provides the ROC plot for MMP-7 (MM) obtained with all patient samples. Additional experimental details are provided in Example 2.

### DETAILED DESCRIPTION OF THE INVENTION

The present invention provides screening methods for identifying patients with ovarian cancer. The screening methods generally comprise detecting the expression of a plurality of biomarkers in a body sample, particularly a blood sample, more particularly a serum sample, from the patient. Overexpression of the biomarkers used in the practice of the invention is indicative of an increased likelihood of the presence of ovarian cancer. A two-step analysis is used to identify patients having an increased likelihood of having ovarian cancer. The first assay step is performed to detect the expression of a first biomarker in a patient body sample. If the first biomarker is determined to be overexpressed in the sample, a second assay step is performed to detect the expression of a second panel of biomarkers. Overexpression of the first biomarker and second panel of biomarkers is indicative of an increased likelihood that the patient has ovarian cancer. In certain embodiments, antibodies are used to detect biomarker protein expression. In other aspects of the invention, when the expression of a panel of biomarkers is detected, overexpression of only a subset of the biomarkers analyzed may be sufficient to be indicative of an increased likelihood of the patient having ovarian cancer.

Although the invention is not limited to a particular mechanism, the first assay step is generally designed to enrich the true-positive patient population (i.e., on a percentage basis) by eliminating a large number of true negatives from further testing. The first assay step may employ a higher sensitivity and negative predictive value (NPV) in order to achieve the desired enrichment of the true-positive patient population. The second assay step is typically intended to rule out those patients in the enriched population that do not presently have ovarian cancer, that is, to eliminate additional true negatives from the enriched population. The second assay step may employ a higher specificity and positive predictive value (PPV), while maintaining a reasonable sensitivity, to further eliminate true negatives from the enriched population of true-positive patients. A schematic representation of the two-step screening test and the results that can be obtained with this method is provided in Figure 1.

One of skill in the art will recognize that these assay steps may be performed as a single screening test encompassing both assay steps or as two distinct tests, wherein each test encompasses one of the assay steps.

The level of expression of a particular biomarker that is sufficient to constitute "overexpression" will vary depending on the specific biomarker used. In particular embodiments of the invention, a "threshold level" of expression is established for a particular biomarker, wherein expression levels above this value are deemed overexpression. A variety of statistical and mathematical methods for establishing the threshold level of expression are known in the art. A threshold expression level for a particular biomarker may be selected, for example, based on data from Receiver Operating Characteristic (ROC) plots, as described in Examples 2 and 3, or on compilations of data from normal patient samples (i.e., a normal patient population). For example, the threshold expression level may be established at the mean expression level plus two times the standard deviation, based on analysis of samples from normal patients not afflicted with ovarian cancer. One of skill in the art will appreciate that these threshold expression levels can be varied, for example, by moving along the ROC plot for a particular biomarker, to obtain different values for sensitivity, specificity, positive predictive value (PPV), and negative predictive value (NPV), thereby affecting overall assay performance.

A patient that is identified as having an increased likelihood of having ovarian cancer in accordance with the disclosed methods may be subjected to further diagnostic testing to definitively determine if the patient has ovarian cancer. "Further diagnostic testing" includes but is not limited to pelvic examination, transvaginal ultrasound, CT scan, MRI, laparotomy, laparoscopy, and biopsy. Such diagnostic methods are well known in the art. Moreover, patients classified as having an increased likelihood of having ovarian cancer that are determined by further diagnostic testing not to currently have ovarian cancer may be closely monitored on a regular basis for the development of ovarian cancer. Monitoring of such patients may include but is not limited to periodic pelvic examination, transvaginal ultrasound, CT scan, and MRI. A physician of ordinary skill in the art will appreciate appropriate techniques for monitoring patients for the development of ovarian cancer. By identifying and monitoring patients having an increased likelihood of having ovarian cancer, the screening methods of the invention may permit the detection of ovarian cancer at an earlier stage of the disease, particularly Stage 1 or Stage 2, when prognosis and disease outcome are greatly improved.

In particular embodiments, antibodies are used to detect biomarker expression at the protein level. In other aspects of the invention, biomarker expression is detected at the nucleic acid level.

By "ovarian cancer" is intended those conditions classified by post-exploratory laparotomy as premalignant pathology, malignant pathology, and cancer (FIGO Stages 1-4). Staging and classification of ovarian cancer are described in detail above. "Early-stage ovarian cancer" refers to those disease states classified as Stage 1 or Stage 2 carcinoma. Early detection of ovarian cancer significantly increases 5-year survival rates. The term "screening method" refers to strategies to identify patients that have an increased likelihood of having ovarian cancer so that such patients can be selected for more aggressive diagnostic methods to definitively determine if the patients have ovarian cancer. The "screening methods" or "diagnostic screening methods" of the invention are generally not intended to definitively diagnose a patient as having or not having ovarian cancer. Rather, such methods are intended to identify women having an increased likelihood of having ovarian cancer so that these women may be definitively diagnosed using other methods (e.g., pelvic examination, transvaginal ultrasound, CT scan, MRI, laparotomy, laparoscopy, and biopsy of tissue samples). Regimens may also be instituted for monitoring patients identified as having an increased likelihood of having ovarian cancer by the present methods but that are determined not to currently have the disease.

The screening methods of the invention may be performed on a case-by-case basis or as a periodic routine screening test for the general female population. In some embodiments, the screening methods for identifying patients with an increased likelihood of having ovarian cancer may be viewed as comparable to Pap smears for the identification of patients having an increased likelihood of having cervical cancer. As used herein, "identifying patients with an increased likelihood of having ovarian cancer" is intended methods for detecting those females that are more likely to have ovarian cancer. An "increased likelihood of having ovarian cancer" is intended to mean that patients who are determined in accordance with the present methods to exhibit overexpression of particular biomarkers are more likely to have ovarian cancer than those patients who do not.

"Diagnosing ovarian cancer" is intended to include, for example, diagnosing or detecting the presence of ovarian cancer, monitoring the progression of the disease, and identifying or detecting cells or samples that are indicative of ovarian cancer. The terms diagnosing, detecting, and identifying ovarian cancer are used interchangeably herein. Definitive diagnosis of ovarian cancer will generally comprise performing a biopsy on a tissue sample from the patient.

The methods of the present invention permit superior assessment of the likelihood of having ovarian cancer when compared with proposed screening methods currently known in the art (e.g., measurement of CA125 levels). As used herein, "specificity" refers to the level at which a method of the invention can accurately identify samples that have been confirmed as nonmalignant by exploratory laparotomy (i.e., true negatives). That is, specificity is the proportion of disease negatives that are test-negative. In a clinical study, specificity is calculated by dividing the number of true negatives by the sum of true negatives and false positives. By "sensitivity" is intended the level at which a method of the invention can accurately identify samples that have been laparotomy-confirmed as positive for ovarian cancer (i.e., true positives). Thus, sensitivity is the proportion of disease positives that are test-positive. Sensitivity is calculated in a clinical study by dividing the number of true positives by the sum of true positives and false negatives. The sensitivity of the disclosed methods for the detection of ovarian cancer is at least about 70%, particularly at least about 80%, more particularly at least about 90, 91, 92, 93, 94, 95, 96, 97, 98, 99% or more. Furthermore, the specificity of the present methods is at least about 70%, particularly at least about 80%, most particularly at least about 90, 91, 92, 93, 94, 95, 96, 97, 98, 99% or more.

The term "positive predictive value" or "PPV" refers to the percentage of patients with a positive test result in the methods of the invention who actually have ovarian cancer. PPV is calculated in a clinical study by dividing the number of true positives by the sum of true positives and false positives. PPV is highly dependent upon the prevalence rates for a particular disease or condition and will shift dramatically as a result of differences in disease prevalence. Therefore, with low-prevalence diseases, such as ovarian cancer, screening tests with a relatively low PPV still have significant clinical utility. In contrast, a disease with high prevalence rates would require a higher PPV to be clinically useful. See, for example, Skates et al. (2004) J. Clin. Oncol. 22:4059-4066; Bast et al. (2005) Int. J. Gynecol. Cancer 15:274-281; Rosen et al. (2005) Gyn. Oncol. 99:267-277; and Pepe (2004) The Statistical Evaluation of Medical Tests for Classification and Prediction (Oxford University Press). The PPV for the present methods of identifying patients with an increased likelihood of having ovarian cancer is generally at least about 7, 8, 9, 10, 15, 20, 25, 30% or more. In some embodiments, the PPV of a method of the invention is at least about 10%. A PPV of at least about 10% for a diagnostic screening method is considered in the art to be of clinical utility. See Skates *et al., supra.* The "negative predictive value" or "NPV" of a test is the percentage of patients with a negative test result who actually do not have ovarian cancer. NPV is calculated in a clinical study by dividing the number of true negatives by the sum of true negatives and false negatives. The NPV for the present methods of identifying patients with an increased likelihood of having ovarian cancer is generally at least about 80%, particularly at least about 90%, more particularly at least about 91, 92, 93, 94, 95, 96, 97, 98, 99, 99.1, 99.2, 99.3, 99.4, 99.5, 99.6, 99.7, 99.8, 99.9% or more. In some embodiments, the NPV of a method of the invention for is at least about 99%. One of skill in the art will appreciate that the PPV and NPV values for the methods of the invention are based on a prevalence-adjusted population and are reflective of the prevalence rates of ovarian cancer within the U.S. female population.

A "biomarker" is any gene or protein whose level of expression in a tissue or cell is altered compared to that of a normal or healthy cell or tissue. Biomarkers of the invention are selective for ovarian cancer. By "selectively overexpressed in ovarian cancer" is intended that the biomarker of interest is overexpressed in ovarian cancer but is not overexpressed in conditions classified as nonmalignant, benign, and other conditions that are not considered to be clinical disease. Thus, detection of the biomarkers of the invention permits the differentiation of samples indicative of an increased likelihood of having ovarian cancer or the presence of ovarian cancer from normal samples (i.e., samples from patients that are ovarian-cancer free) and samples that are indicative of nonmalignant and benign proliferation. Biomarkers of the invention may be referred to herein interchangeably as "ovarian cancer biomarkers," "markers," or "ovarian cancer markers."

The biomarkers of the invention include genes and proteins. Such biomarkers include DNA comprising the entire or partial sequence of the nucleic acid sequence encoding the biomarker, or the complement of such a sequence. The biomarker nucleic acids also include RNA comprising the entire or partial sequence of any of the nucleic acid sequences of interest. A biomarker protein is a protein encoded by or corresponding to a DNA biomarker of the invention. A biomarker protein comprises the entire or partial amino acid sequence of any of the biomarker proteins or polypeptides. Fragments and variants of biomarker genes and proteins are also encompassed by the present invention. By "fragment" is intended a portion of the polynucleotide or a portion of the amino acid sequence and hence protein encoded thereby. Polynucleotides that are fragments of a biomarker nucleotide sequence generally comprise at least 10, 15, 20, 50, 75, 100, 150, 200, 250, 300, 350, 400, 450, 500, 550, 600, 650, 700, 800, 900, 1,000, 1,100, 1,200, 1,300, or 1,400 contiguous nucleotides, or up to the number of nucleotides present in a full-length biomarker polynucleotide disclosed herein. A fragment of a biomarker polynucleotide will generally encode at least 15, 25, 30, 50, 100, 150, 200, or 250 contiguous amino acids, or up to the total number of amino acids present in a full-length biomarker protein of the invention. "Variant" is intended to mean substantially similar sequences. Generally, variants of a particular biomarker of the invention will have at least about 40%, 45%, 50%, 55%, 60%, 65%, 70%, 75%, 80%, 85%, 90%, 91%, 92%, 93%, 94%, 95%, 96%, 97%, 98%, 99% or more sequence identity to that biomarker as determined by sequence alignment programs.

The biomarkers of the invention include HE4, CA125, glycodelin, PAI-1, and PLAU-R.

HE4 is a protein that was first observed in human epididymis tissue, and the name "HE4" is an abbreviation of "Human Epididymis Protein 4". Subsequent studies have shown that HE4 protein is also present in the female reproductive tract and other epithelial tissues. The HE4 gene resides on human chromosome 20q12-13.1, and the 20q 12 chromosome region has been found to be frequently amplified in ovarian carcinomas. Studies have shown that HE4 is expressed by ovarian carcinoma cells. The protein is N-glycosylated and is secreted extracellularly. See, for example, Drapin et al. (2005) Cancer Research 65(6): 2162-9; Hellström et al. (2003) Cancer Research 63: 3695-3700; and Bingle et al. (2002) Oncogene 21: 2768-2773.

CA-125 is a high molecular weight, cell surface glycoprotein detected in the serum of a large proportion of patients with ovarian epithelial cancer (OEC). However, while the percentage is high (75-90%) in advanced stages of this disease, it is only elevated in 50% of the patients with Stage 1 disease. Use of CA-125 as a marker for OEC is problematic because the molecule is also expressed in a number of normal and pathological conditions including menstruation, pregnancy, endometriosis, inflammatory diseases and other types of cancer. Improved sensitivity and specificity for OEC has been reported among post menopausal women. See, for example, Bast et al. (1998) Int'l J. Biological Markers 13:170-187; and Moss et al. (2005) J. Clin. Pathol. 58:308-312.

Glycodelin is a member of the lipocalin superfamily with several distinctive actions in cell recognition and differentiation principally in the reproductive axis. Previously, this glycoprotein has been named progesterone-associated endometrial protein (PAEP) and placental protein 14 (PP-14). The name change was in part initiated because glycodelin is not synthesized by the endometrium or placenta. Glycodelin has been purified from amniotic fluid as 28kDa molecule in SDS gels and reported to be synthesized by the normal ovary and by malignant ovarian tumors. Its presence has been reported in serum. See generally Seppala et al. (2002) Endocrine Reviews 23:401-430; Pala et al. (1997) J. Chromatography B 704:25-34; Meerit Kamarainen et al. (1996) Amer. J. Pathology 148:1435-1443.

PAI-1 (i.e., plasminogen activator inhibitor type 1) is a serine or cysteine proteinase inhibitor. The PAI-1 mRNA is 2876 bp in length, and the encoded protein is 402 amino acids long. The calculated molecular weight is 42,769 Da, whereas the affinity-purified protein is reported to be approximately 50,000 Da, as determined by SDS gel electrophoresis. PAI-1 plays a role in inhibiting extracellular matrix degradation by PLAU and is a putative unregulated c-Myc target gene. PAI-1 also plays a key role in controlling coagulation and tissue remodeling. PAI-1 limits the production of plasmin and serves to keep fibrinolysis in check. Some physiological functions involving the inhibition of plasmin by PAI-1 include ovulation, cell migration, and epithelial cell differentiation. High PAI-1 levels in cancer indicates poor prognosis for survival of many human cancers, including breast and lung cancers. Pappot et al. (Nov. 29, 2005) Lung Cancer [Epub ahead of print]; Chazaud et. al. (2002) American Journal of Pathology 160:237-246.

Plasminogen activator urokinase-receptor (PLAU-R or UPAR) is a cell surface glycoprotein with a molecular weight of approximately 60 kDa that is attached by its carboxy-terminal end to the cell membrane by GPI linkage. PLAU-R serves as a specific receptor for the serine protease urokinase plasminogen activator (uPA) that is involved in basement membrane/extracellular matrix remodeling in both normal and pathological processes. Soluble PLAU-R, released from the cell surface, has been reported to be at elevated concentrations in serum in several types of human cancer including colorectal, breast and ovarian cancer. See for example Sier et al. (1998) Cancer Res. 58:1843-1849; and Begum et al. (2004) Anticancer Research 24:1981-1986.

By "selectively overexpressed in early-stage ovarian cancer" is intended that the biomarker is overexpressed in stage 1 or stage 2 ovarian cancer states but is not overexpressed in normal samples or in conditions classified as nonmalignant, benign, and other conditions that are not considered to be clinical disease. One of skill in the art will appreciate that early-stage ovarian cancer biomarkers include those genes and proteins specific for ovarian cancer that are initially overexpressed in stage 1 or stage 2 and whose overexpression persists throughout the advanced stages of the disease, as well as biomarkers that are only overexpressed in stage 1 or stage 2 ovarian cancer. Detection of expression of biomarkers that are selectively overexpressed in early-stage ovarian cancer may permit the earlier detection and diagnosis of ovarian cancer and, accordingly, improve patient prognosis.

The two-step screening method of the invention is used to identify patients having an increased likelihood of having ovarian cancer, a plurality of biomarkers refers to the detection of one biomarker during the first assay step and additional biomarkers during the second assay step. A panel of biomarkers comprising CA125, glycodelin, PLAU-R and PAI-1 is provided.

Any female patient or patient population may be assessed using the screening and diagnostic methods of the invention. For example, the methods disclosed herein may be performed on the general female patient population, as well as on the narrower population of post-menopausal women. The term "post-menopausal" is understood by those of skill in the art. In particular embodiments, post-menopausal generally refers to, for example, women over the age of 55. In particular embodiments, the screening methods are performed routinely (e.g., annually, every two years, etc.) on the general female population. Regular screening of patients may begin, for example, at the onset of menses, at age 30, or at the beginning of menopause. Screening of the high-risk patient population, as defined herein below, will typically be performed on a routine basis independent of patient age. Patients who are both asymptomatic and symptomatic (i.e., displaying characteristic symptoms of ovarian cancer, such as pelvic or abdominal pain or swelling) can be assessed for an increased likelihood of having ovarian using the screening and diagnostic methods of the invention. Women that are at a low-risk of developing ovarian and those that are considered high-risk based on clinical and family history risk factors may also be assessed using the present methods. Patients considered "high-risk" based on such clinical and family history risk factors include but are not limited to patients living with breast cancer, colon cancer, or breast/ovarian syndrome, women with a first-degree relative with ovarian cancer (e.g., mother, daughter, or sister), patients positive for at least one breast cancer gene (BRCA 1 or 2), and women suffering from HNPCC (i.e., Hereditary non-polyposis colorectal cancer).

In one aspect of the invention, the target population for the screening and diagnostic methods is the group of asymptomatic patients classified as high-risk on the basis of, for example, the above-referenced clinical and familial risk factors. This high-risk, asymptomatic population represents more than 8 million women in the U.S. It is recognized that the methods, compositions, and kits of the invention will be of particular utility to patients having an enhanced risk of developing ovarian cancers and to their physicians. Patients recognized in the art as having an increased risk of developing ovarian cancers include, for example, patients having a familial history of ovarian cancer and patients of advancing age (i.e., typically women over 55 years of age).

A number of clinical conditions or characteristics not directly related to ovarian cancer may exist in the patient populations tested in accordance with the methods of the invention, thereby interfering with the results of the screening and diagnostic methods disclosed herein. Such clinical conditions and characteristics are referred to herein as "interfering substances and pathologies" and include but are not limited to pregnancy (first trimester), breast cancer, chronic hepatitis, colon cancer, oral contraceptive therapy, coronary artery disease, deep vein thrombosis, diabetes, endometriosis, hormone replacement therapy, menstruation, multiple myeloma, ovarian cysts/polycystic disease, polymyalgia, polymyositis, rectal cancer, rheumatoid arthritis, systemic lupus rythematosus (SLE), and warfarin treatment. Additional exemplary interfering pathologies include uterine conditions (e.g., myomas, adenomyosis, and endometrial cancer), ovarian conditions (e.g., benign growths such as functional cysts, theca-lutein cysts, pregnancy luteoma, sclerocystic ovaries, serous cystadenoma, mucinous cystadenoma, cystic teratoma, fibroma, thecoma, and Brenner tumor; neoplasms; and malignant conditions such as cystadenocarcinoma and adenocarcinoma), fallopian tube conditions (e.g., tubo-ovarian abscess, hydrosaplinx, parovarian cyst, ectopic pregnancy, and cancer of the fallopian tubes), bowel conditions (e.g., distention with gas and/or feces, diverticulitis, ileitis, appendicitis, and colon cancer), and other miscellaneous conditions (e.g., distended bladder, pelvic kidney, urachal cyst, abdominal wall hematoma, abdominal wall abscess, and retroperitoneal neoplasms, such as lymphoma, sarcoma, and teratoma. In particular embodiments, the biomarkers, threshold expression levels, and mathematical models used in the screening and diagnostic methods described herein will be selected so as to minimize the effects of interfering substances and pathologies on the performance (i.e., the specificity, sensitivity, PPV, and NPV) of the claimed methods.

By "body sample" is intended any sampling of cells, tissues, or bodily fluids from a patient in which expression of a biomarker can be detected. Examples of such body samples include but are not limited to blood (e.g., whole blood, blood serum, blood having platelets removed, etc.), lymph, ascitic fluids, urine, gynecological fluids (e.g., ovarian, fallopian, and uterine secretion, menses, etc.), biopsies, and fluids obtained during laparoscopy. Body samples may be obtained from a patient by a variety of techniques including, for example, by venipuncture, by scraping or swabbing an area, or by using a needle to aspirate bodily fluids or tissues. Methods for collecting various body samples are well known in the art. In particular embodiments, the body sample comprises blood or serum. The present inventors have recognized that the methods for the collection and storage of blood samples, more particularly serum samples, affect the performance of the methods disclosed herein. Several studies indicate that body sample, particularly serum sample, collection and storage methods are critical to achieve acceptable assay performance. See, for example, Diamandis (2004) J. Natl. Cancer Institute 95:353-356 and Thavasu et al. (1992) J. Immunol. Meth. 153:115-124. An exemplary method for serum collection and storage is provided in Example 1.

Any methods available in the art for the detection biomarker expression can be used to practice the invention. The expression of a biomarker of the invention can be detected on a nucleic acid level or a protein level. In order to determine overexpression, the body sample to be examined may be compared with a corresponding body sample that originates from a healthy person. That is, the "normal" level of expression is the level of expression of the biomarker in a body sample from a patient that is not afflicted with ovarian cancer. Such a sample can be present in standardized form. In some embodiments, determination of biomarker overexpression requires no comparison between the body sample and a corresponding body sample that originates from a normal person.

In general, it is preferable to use biomarkers for which the difference between the level of expression of the biomarker in a body sample from a patient afflicted with ovarian cancer and the level of expression of the same biomarker in a "normal" body sample (i.e., from an ovarian cancer free patient) is as great as possible. Although this difference can be as small as the limit of detection of the method for assessing expression of the biomarker, it is preferred that the difference be at least greater than the standard error of the assessment method, and optimally a difference of at least 2, 3, 4, 5, 6, 7, 8, 9, 10, 15, 20, 25-fold or greater than the level of expression of the same biomarker in a normal body sample. The "normal" level of expression of a biomarker of the invention may be determined by assessing expression of the biomarker in a body sample obtained from a non-ovarian cancer afflicted patient. Alternatively, and particularly as further information becomes available as a result of the routine performance of the methods described herein, average values for biomarker expression levels may be used.

As described above, in some aspects of the invention, a threshold level of expression of a particular biomarker, above which the biomarker is considered to be overexpressed in a patient sample, may be established. Methods for selecting the threshold value include but are not limited to analysis of the ROC plot for the biomarker or analysis of the biomarker expression level for a normal patient population. Exemplary threshold or "cutoff" values include (1) the mean expression level plus two times the standard deviation, as determined from a population of normal patient samples and (2) expression levels selected from the ROC curve that represent the highest value of sensitivity plus specificity. The threshold level of expression for a particular biomarker may be determined relative to the expression level in a normal patient population. Persons of skill in the art will appreciate that other methods for selecting appropriate threshold expression values can be used to practice the invention.

Methods for detecting biomarkers of the invention comprise any methods that determine the quantity or the presence of the biomarkers either at the nucleic acid or protein level. Such methods are well known in the art and include but are not limited to western blots, northern blots, southern blots, ELISA, immunoprecipitation, immunofluorescence, flow cytometry, immunocytochemistry, multiplex bead-based immunoassays, nucleic acid hybridization techniques, nucleic acid reverse transcription methods, and nucleic acid amplification methods. In particular embodiments, overexpression of a biomarker is detected on a protein level using, for example, antibodies that are directed against specific biomarker proteins. These antibodies can be used in various methods such as Western blot, ELISA, multiplex bead-based immunoassay, immunoprecipitation, or immunocytochemistry techniques. The multiplex bead-based assays used to practice the present invention include but are not limited to the Luminex technology described in U.S. Patent Nos. 6,599,331, 6,592,822, and 6,268,222. In particular embodiments, the Luminex LabMAP® system is utilized, as described in International Publication No. WO 2005/016126.

In some embodiments of the invention, antibodies specific for biomarker proteins are utilized to detect the expression of a plurality of biomarker proteins in a body sample in order to identify patients with an increased likelihood of having ovarian cancer. The method comprises obtaining a body sample from a patient, particularly a serum sample, contacting the body sample with antibodies directed to a plurality of biomarkers that are selectively overexpressed in ovarian cancer, and detecting antibody binding to determine if the biomarkers are overexpressed in the patient sample. In the screening methods of the invention, overexpression of the biomarkers is indicative of an increased likelihood of having ovarian cancer. Patients classified by the present screening methods as having an increased likelihood of having ovarian cancer are subjected to further diagnostic testing to detect the presence of ovarian cancer. Female patients identified by the present screening methods for ovarian cancer that are determined to not be currently suffering from ovarian cancer are regularly monitored in order to potentially detect ovarian cancer at an earlier stage.

As described above the screening methods for identifying patients with an increased likelihood of having ovarian cancer comprises a two-step analysis. That is, the method comprises performing a first assay step comprising detecting the expression of a first biomarker in a body sample and determining if the first biomarker is overexpressed. If a positive result is obtained in the first assay step (i.e., the first biomarker is overexpressed in the body sample), the method further comprises performing a second assay step comprising detecting the expression of a second panel of biomarkers and determining if the panel of biomarkers is overexpressed. Overexpression of both the first biomarker and second panel of biomarkers (i.e., a positive result in both assay steps) is indicative of an increased likelihood of having ovarian cancer. In particular embodiments, detection of expression of the biomarkers in the first and second assay steps is performed at the protein level and comprises contacting the body sample, more particularly a serum sample, with antibodies specific for the particular biomarkers of interest and detecting antibody binding. In certain methods, biomarker protein expression is detected using an ELISA or multiplex bead-based immunoassay format.

In the "two-step" screening methods of the invention, the biomarker used in the first step may be employed to maximize sensitivity and NPV. That is, the first screening step may be designed to maximize the number of true negatives classified as negative by the methods of the invention, thereby eliminating a significant percentage of true negatives from further analysis. Therefore, the patient population classified as positive by the first assay step will be enriched in true positive patients. The second screening step in the two-step methods may be designed to maximize PPV and specificity, while maintaining a reasonable sensitivity, in order to identify women with the highest likelihood of having ovarian cancer. Specificity, sensitivity, PPV, and NPV values may be determined for each individual assay step in the method, as described above. When a two-step screening method is used, combined sensitivity, specificity, PPV, and NPV values may determined for the method as a whole. In particular embodiments, the two-step method for identifying patients having an increased likelihood of having ovarian cancer will have a combined sensitivity of at least 90%, a combined specificity of at least 98%, a combined PPV of at least 10%, and a combined NPV of at least 99.9%.

Algorithms or mathematical models may be applied to develop "test rules" for determining when a patient sample is "positive" (i.e., indicative of an increased likelihood of having ovarian cancer).

In certain aspects of the invention, biomarker expression is detected at the protein level using antibodies. The terms "antibody" and "antibodies" broadly encompass naturally occurring forms of antibodies and recombinant antibodies such as single-chain antibodies, chimeric and humanized antibodies and multi-specific antibodies as well as fragments and derivatives of all of the foregoing, which fragments and derivatives have at least an antigenic binding site. Antibody derivatives may comprise a protein or chemical moiety conjugated to the antibody.

"Antibodies" and "immunoglobulins" (Igs) are glycoproteins having the same structural characteristics. While antibodies exhibit binding specificity to an antigen, immunoglobulins include both antibodies and other antibody-like molecules that lack antigen specificity. Polypeptides of the latter kind are, for example, produced at low levels by the lymph system and at increased levels by myelomas.

The term "antibody" is used in the broadest sense and covers fully assembled antibodies, antibody fragments that can bind antigen ( e.g., Fab', F'(ab)₂, Fv, single chain antibodies, diabodies), and recombinant peptides comprising the foregoing.

The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Antibody fragments" comprise a portion of an intact antibody, preferably the antigen-binding or variable region of the intact antibody. Examples of antibody fragments include Fab, Fab', F(ab')2, and Fv fragments; diabodies; linear antibodies (Zapata et al. (1995) Protein Eng. 8(10):1057-1062); single-chain antibody molecules; and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize 35 readily. Pepsin treatment yields an F(ab')2 fragment that has two antigen-combining sites and is still capable of cross-linking antigen.

"Fv" is the minimum antibody fragment that contains a complete antigen recognition and binding site. In a two-chain Fv species, this region consists of a dimer of one heavy- and one light-chain variable domain in tight, non-covalent association. In a single-chain Fv species, one heavy- and one light-chain variable domain can be covalently linked by flexible peptide linker such that the light and heavy chains can associate in a "dimeric" structure analogous to that in a two-chain Fv species. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (C_{H}1) of the heavy chain. Fab fragments differ from Fab' fragments by the addition of a few residues at the carboxy terminus of the heavy-chain C_{H}1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments that have hinge cysteines between them.

Polyclonal antibodies can be prepared by immunizing a suitable subject (e.g., rabbit, goat, mouse, or other mammal) with a biomarker protein immunogen. The antibody titer in the immunized subject can be monitored over time by standard techniques, such as with an enzyme linked immunosorbent assay (ELISA) using immobilized biomarker protein. At an appropriate time after immunization, e.g., when the antibody titers are highest, antibody-producing cells can be obtained from the subject and used to prepare monoclonal antibodies by standard techniques, such as the hybridoma technique originally described by Kohler and Milstein (1975) Nature 256:495-497, the human B cell hybridoma technique (Kozbor et al. (1983) Immuno/. Today 4:72), the EBV-hybridoma technique (Cole et al. (1985) in Monoclonal Antibodies and Cancer Therapy, ed. Reisfeld and Sell (Alan R. Liss, Inc., New York, NY), pp. 77-96) or trioma techniques. The technology for producing hybridomas is well known (see generally Coligan et al., eds. (1994) Current Protocols in Immunology (John Wiley & Sons, Inc., New York, NY); Galfre et al. (1977) Nature 266:550-52; Kenneth (1980) in Monoclonal Antibodies: A New Dimension In Biological Analyses (Plenum Publishing Corp., NY); and Lerner (1981) Yale J. Biol. Med., 54:387-402).

Alternative to preparing monoclonal antibody-secreting hybridomas, a monoclonal antibody can be identified and isolated by screening a recombinant combinatorial immunoglobulin library (e.g., an antibody phage display library) with a biomarker protein to thereby isolate immunoglobulin library members that bind the biomarker protein. Kits for generating and screening phage display libraries are commercially available (e.g., the Pharmacia *Recombinant Phage Antibody System,* Catalog No. 27-9400-01; and the Stratagene *SurfZAP ϑ Phage Display Kit,* Catalog No. 240612). Additionally, examples of methods and reagents particularly amenable for use in generating and screening antibody display library can be found in, for example, U.S. Patent No. 5,223,409; PCT Publication Nos. WO 92/18619; WO 91/17271; WO 92/20791; WO 92/15679; 93/01288; WO 92/01047; 92/09690; and 90/02809; Fuchs et al. (1991) Bio/Technology 9:1370-1372; Hay et al. (1992) Hum. Antibod. Hybridomas 3:81-85; Huse et al. (1989) Science 246:1275-1281; Griffiths et al. (1993) EMBO J. 12:725-734.

One of skill in the art will recognize that optimization of reagents and conditions, for example, antibody titer and parameters for detection of antigen-antibody binding, is needed to maximize the signal to noise ratio for a particular antibody. Antibody concentrations that maximize specific binding to the biomarkers of the invention and minimize non-specific binding (or "background") will be determined. Appropriate antibody titers are determined by initially testing various antibody dilutions on patient serum samples. The design of assays to optimize antibody titer and detection conditions is standard and well within the routine capabilities of those of ordinary skill in the art. Some antibodies require additional optimization to reduce background and/or to increase specificity and sensitivity.

Furthermore, one of skill in the art will recognize that the concentration of a particular antibody used to practice the methods of the invention will vary depending on such factors as time for binding, level of specificity of the antibody for the biomarker protein, and method of body sample preparation. Moreover, when multiple antibodies are used in a single sample, the required concentration may be affected by the order in which the antibodies are applied to the sample, i.e., simultaneously as a cocktail or sequentially as individual antibody reagents. Furthermore, the detection chemistry used to visualize antibody binding to a biomarker of interest must also be optimized to produce the desired signal to noise ratio.

Detection of antibody binding can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include ¹²⁵I, ¹³¹I, ³⁵S, or ³H.

The antibodies used to practice the invention are selected to have high specificity for the biomarker proteins of interest. Methods for making antibodies and for selecting appropriate antibodies are known in the art. See, for example, Celis, ed. (in press) Cell Biology & Laboratory Handbook, 3rd edition (Academic Press, New York). In some embodiments, commercial antibodies directed to specific biomarker proteins may be used to practice the invention. The antibodies of the invention may be selected on the basis of desirable staining of cytological, rather than histological, samples. That is, in particular embodiments the antibodies are selected with the end sample type (i.e., serum samples) in mind and for binding specificity.

In other embodiments, the expression of a biomarker of interest is detected at the nucleic acid level. Nucleic acid-based techniques for assessing expression are well known in the art and include, for example, determining the level of biomarker mRNA in a body sample. Many expression detection methods use isolated RNA. Any RNA isolation technique that does not select against the isolation of mRNA can be utilized for the purification of RNA from serum samples (see, e.g., Ausubel et al., ed., (1987-1999) Current Protocols in Molecular Biology (John Wiley & Sons, New York). Additionally, large numbers of blood, serum, or tissue samples can readily be processed using techniques well known to those of skill in the art, such as, for example, the single-step RNA isolation process of Chomczynski (1989, U.S. Pat. No. 4,843,155).

The term "probe" refers to any molecule that is capable of selectively binding to a specifically intended target biomolecule, for example, a nucleotide transcript or a protein encoded by or corresponding to a biomarker. Probes can be synthesized by one of skill in the art, or derived from appropriate biological preparations. Probes may be specifically designed to be labeled. Examples of molecules that can be utilized as probes include, but are not limited to, RNA, DNA, proteins, antibodies, and organic molecules.

Isolated mRNA can be used in hybridization or amplification assays that include, but are not limited to, Southern or Northern analyses, polymerase chain reaction analyses and probe arrays. One method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to an mRNA or genomic DNA encoding a biomarker of the present invention. Hybridization of an mRNA with the probe indicates that the biomarker in question is being expressed.

In one embodiment, the RNA is immobilized on a solid surface and contacted with a probe, for example by running the isolated mRNA on an agarose gel and transferring the mRNA from the gel to a membrane, such as nitrocellulose. In an alternative embodiment, the probe(s) are immobilized on a solid surface and the mRNA is contacted with the probe(s), for example, in an Affymetrix gene chip array. A skilled artisan can readily adapt known mRNA detection methods for use in detecting the level of mRNA encoded by the biomarkers of the present invention.

An alternative method for determining the level of biomarker mRNA in a sample involves the process of nucleic acid amplification, e.g., by RT-PCR (the experimental embodiment set forth in Mullis, 1987, U.S. Pat. No. 4,683,202), ligase chain reaction (Barany (1991) Proc. Natl. Acad. Sci. USA 88:189-193), self sustained sequence replication (Guatelli et al. (1990) Proc. Natl. Acad. Sci. USA 87:1874-1878), transcriptional amplification system (Kwoh et al. (1989) Proc. Natl. Acad. Sci. USA 86:1173-1177), Q-Beta Replicase (Lizardi et al. (1988) BiolTechnology 6:1197), rolling circle replication *(*Lizardi et al., U.S. Pat. No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. In particular aspects of the invention, biomarker expression is assessed by quantitative fluorogenic RT-PCR (i.e., the TaqMan® System). Such methods typically utilize pairs of oligonucleotide primers that are specific for the biomarker of interest. Methods for designing oligonucleotide primers specific for a known sequence are well known in the art.

Biomarker expression levels of RNA may be monitored using a membrane blot (such as used in hybridization analysis such as Northern, Southern, dot, and the like), or microwells, sample tubes, gels, beads or fibers (or any solid support comprising bound nucleic acids). See U.S. Patent Nos. 5,770,722, 5,874,219, 5,744,305, 5,677,195 and 5,445,934. The detection of biomarker expression may also comprise using nucleic acid probes in solution.

In one embodiment of the invention, microarrays are used to detect biomarker expression. Microarrays are particularly well suited for this purpose because of the reproducibility between different experiments. DNA microarrays provide one method for the simultaneous measurement of the expression levels of large numbers of genes. Each array consists of a reproducible pattern of capture probes attached to a solid support Labeled RNA or DNA is hybridized to complementary probes on the array and then detected by laser scanning. Hybridization intensities for each probe on the array are determined and converted to a quantitative value representing relative gene expression levels. See, U.S. Pat Nos. 6,040,138, 5,800,992 and 6,020,135, 6,033,860, and 6,344,316. High-density oligonucleotide arrays are particularly useful for determining the gene expression profile for a large number of RNA's in a sample.

Techniques for the synthesis of these arrays using mechanical synthesis methods are described in, e.g., U.S. Patent No. 5,384,261. Although a planar array surface is preferred, the array may be fabricated on a surface of virtually any shape or even a multiplicity of surfaces. Arrays may be peptides or nucleic acids on beads, gels, polymeric surfaces, fibers such as fiber optics, glass or any other appropriate substrate, see U.S. Pat. Nos. 5,770,358, 5,789,162, 5,708,153, 6,040,193 and 5,800,992. Arrays may be packaged in such a manner as to allow for diagnostics or other manipulation of an all-inclusive device. *See,* for example, U.S. Pat. Nos. 5,856,174 and 5,922,591.

In one approach, total mRNA isolated from the sample is converted to labeled cRNA and then hybridized to an oligonucleotide array. Each sample is hybridized to a separate array. Relative transcript levels may be calculated by reference to appropriate controls present on the array and in the sample.

One of skill in the art will further appreciate that any or all steps in the screening and diagnostic methods of the invention could be implemented by personnel or, alternatively, performed in an automated fashion. That is, the methods can be performed in an automated, semi-automated, or manual fashion. Furthermore, the methods disclosed herein can also be combined with other methods known or later developed to permit a more accurate identification of patients having an increased likelihood of having ovarian cancer or a more reliable diagnosis of ovarian cancer.

The article "a" and "an" are used herein to refer to one or more than one (i.e., to at least one) of the grammatical object of the article. By way of example, "an element" means one or more element.

Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps.

The following examples are offered by way of illustration and not by way of limitation:

In the following examples, only a two-step screening method in which expression of HE4 is detected as a first biomarker, and if HE4 is over expressed, expression of a panel of biomarkers comprising CA125, glycodelin, PAI-1, and PLAU-R, is detected, forms part of the invention. All other biomarkers and all other sequential screening decisions serve merely as comparative examples and do not form part of the invention.

### EXPERIMENTAL

### Example 1: Exemplary Method for Collection of Blood, Processing of Blood Into Serum, Serum Storage Conditions, and Serum Shopping Conditions

### Blood Collection

A sufficient quantity of blood was drawn by venipuncture into a Becton Dickinson Vacutainer red-topped collection tubes containing no additive (catalog number 366430). The lot number of the blood collection tube was recorded. Whole blood was not refrigerated or frozen. Processing of the whole blood to serum began immediately after collection of the blood.

### Serum Preparation Procedure

Blood collection tubes were placed upright in a rack and stored at room temperature. The blood was allowed to clot for a minimum of 60 minutes and a maximum of 90 minutes from time of collection. Blood was thoroughly clotted before centrifugation. The time required for complete clotting for each sample was recorded.

After blood was thoroughly clotted, the samples were centrifuged at 1300xg for 10 minutes using either a swing-head or fixed-angle centrifuge rotor. Tubes were carefully removed from the centrifuge to avoid disturbing the red blood cells on the bottom.

The stopper was removed from each tube without disturbing the cell pellet. The serum from each donor was combined into a labeled tube using a disposable transfer pipette without disturbing the cell layer or allowing any cells into the pipette. If cells were disturbed during transfer, the samples were re-centrifuged as described above.

Serum samples from each donor were mixed by gently inverting the tube five times. One mL aliquots were placed in labeled tubes and frozen at -20°C to -80°C. Serum was not stored at room temperature for more than two hours or at 2-8°C for more than eight hours prior to freezing.

### Example 2: Ovarian Biomarker Selection Study - Individual Biomarker Analysis

The purpose of the biomarker selection study was to evaluate the initial clinical utility of a set of fourteen candidate ovarian cancer biomarkers. The premise of the study was to be able to identify and quantitate the amount of each biomarker in a patient's serum. Biomarker protein expression in serum samples was detected using co*mmercial or novel biomarker-specific antibodies in an ELISA format. Fourteen target biomarkers were analyzed, as described below in Table 1:

**Table 1. Candidate markers**

| | |
|---|---|
| HE4 | CA125 |
| SLPI | Glycodelin |
| KLK6 | MMP7 |
| KLK10 | PLAU-R |
| CTHRC1 | Inhibin |
| PAI-1 | Prolactin |
| MUC1 | Alpha-1 anti-trypsin |

### Target population and demographics of the study

In evaluations of this type, it is essential that the samples tested are representative of the target clinical population and that normal controls are demographically matched. A total of 900 patient serum samples were analyzed in this study. 200 of the samples were from ovarian cancer patients in various stages of the disease (i.e., the 200 samples were evenly split among Stage 1, 2, 3 ovarian cancer samples). A total of 500 normal sera were used in the study. 104 of the normal serum samples were from post-menopausal women (set as women over the age of 55 for the present study) to establish the baseline levels of each biomarker and to calculate the appropriate threshold cut-off values. The remaining 396 normal serum samples were evenly split between pre-menopausal women under the age of 55 and post-menopausal women over the age of 55. This distribution permitted the investigation of any age or hormone-related patient distributions. The remaining 200 samples in the study were collected from donors with various conditions/diseases, as described herein above (i.e., "interfering conditions" or "interfering pathologies"). The inclusion of these samples allowed for the analysis of any interfering pathologies that may affect individual biomarker performance. Summaries of the patient populations analyzed and study demographics are provided in Tables 2-4.

**Table 2: Demographics of sample cases in the study**

| Characteristics | Ovarian Cancer | Study Normals | Interfering Pathologies | Threshold Normals |
|---|---|---|---|---|
| Total number of samples Age at diagnosis (years) Mean (std) Range Age group distribution <=55 >55 | 200 | 396 | 200 | 104 |
| | 55.5(11.5) 19-81 | 55.3 (9.7) 40-84 | 57.0 (18.3) 20-97 | 63.0(6.9) 41-80 |
| | 100 (50%) | 201 (50.8%) | 92 (46%) | 2 (1.9%) |
| | 100 (50%) | 195 (49.2%) | 108 (54%) | 102 (98.1%) |
| Race, n African-American Caucasian Other Missing | 10 (5%) | 3 (1%) | 12 (6%) | 0 |
| | 183 (92%) | 389 (99%) | 185 (92.5%) | 98 (100%) |
| | 6 (3%) | 1(<1%) | 3 (1,5%) | 0 |
| | 1 | 3 | 0 | 6 |

**Table 3: Clinical Characteristics of Ovarian Cancer Patients in Study**

| | Frequency | Percent |
|---|---|---|
| Ovarian Cancer Stage, n | 200 | |
| 1 | 67 | 33.5% |
| 2 | 66 | 33% |
| 3 | 67 | 33.5% |
| Histological Classification, n | 197 | |
| | | |
| Clear Cell | 10 | 5% |
| Endometrioid | 39 | 19% |
| Serous | 23 | 12% |
| Mucinous | 36 | 18% |
| Adenocarcinoma | 49 | 25% |
| Papillary | 25 | 13% |
| Unclassified | 13 | 7% |
| Stromal Sarcoma | 2 | 1% |

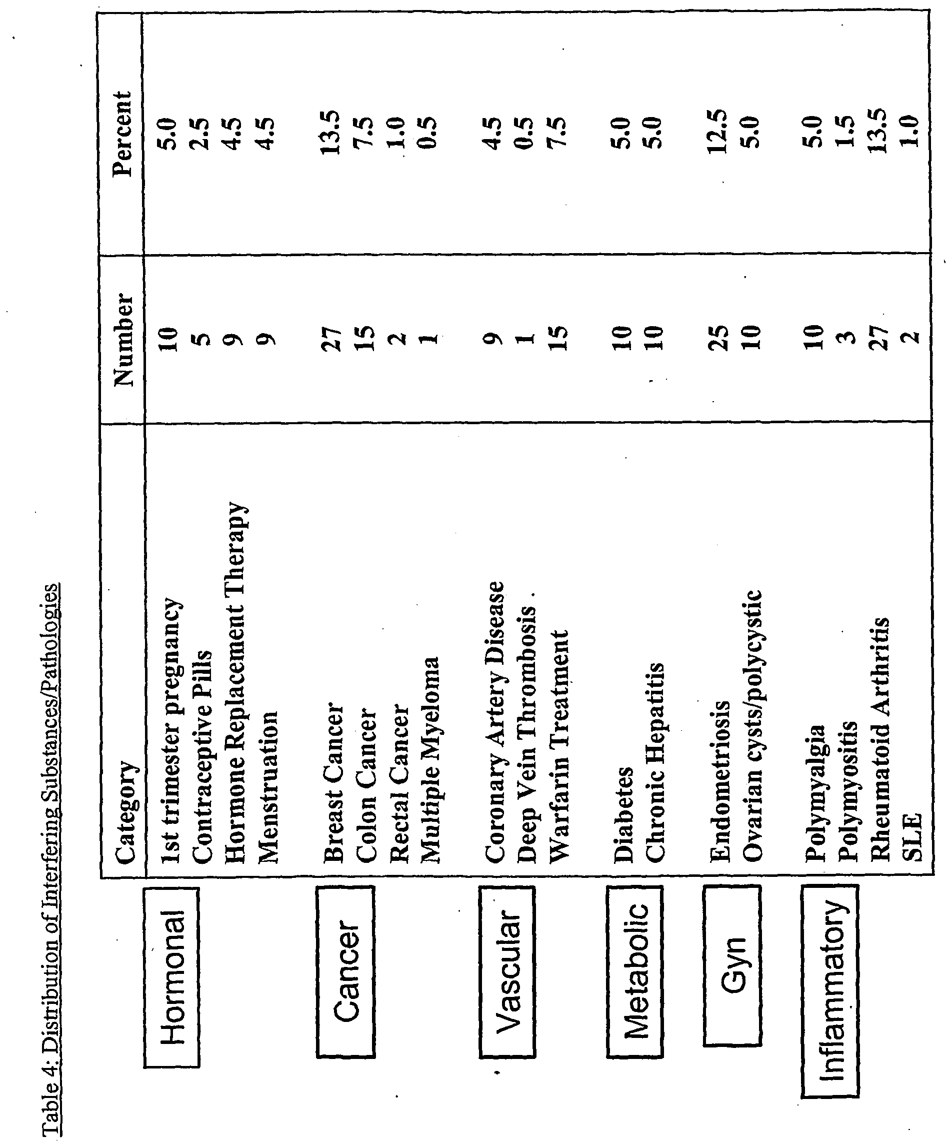

### General Description of Automation and Validation Analysis for the Study

All 900 sera samples were processed in duplicate for each of the 14 markers. An automated assay system with barcode tracking was utilized in generating the marker selection data. The assays run in this marker selection study were all completed using a Tecan Evo automated robotic liquid handler. The use of this platform allowed all 900 samples to be processed for each marker in a single experimental run (one run per day). The precision of the automated platform was verified before any study samples were processed.

All of the ELISA assays were processed for this study using a buffered solution as the diluent for the standard curve. This was done to standardize, as much as possible, the protocols for each of the assays.

The normal sera used in this study were obtained from community blood collection centers compliant with local IRB customs. While these sera can be considered 'normal' it must be noted that these donors were not screened by a medical professional to confirm their disease free or normal status

### Detection of Expression of HE4 in Serum Samples (A Representative Example)

### Materials and Methods

### A. Coating of assay plates:

ELISA 96-well plates were coated with 100 µl/well of the primary antibody, anti-HE4 monoclonal 90.1 #6, at 2 µg/ml in PBS, then the plates were incubated at 4°C overnight. The next day, the plates were washed once with PBS, then 250 µl/well of PBS-3% BSA was added to all wells, and the plates were incubated for 2 hours at 30°C. The plates were then emptied and dried in a vacuum oven for 2 hours at room temperature. Then they were heat-sealed inside a mylar foil bag along with a desiccant pack and stored at 4°C until used in an assay.

### B. Assay Methods

The foil bags containing the assay plates were warmed to room temperature immediately prior to use in the assay. The serum samples were diluted 1:4 into PBS-1% bovine Serum-0.05% Tween 20-1 mg/ml mouse IgG. The HE4 antigen protein was diluted to 100 ng/ml, then serially diluted two-fold into PBS-1% Bovine Serum-0.05% Tween 20-1 mg/ml Mouse IgG, then all the individual standard curve samples were further diluted 1:4 into the buffer so that they would be diluted the same as the serum samples.

The diluted sera samples and standard curve samples were added to the anti-HE4 coated assay plates in 100 µl/well volumes. Then the plates were incubated for 2 hours at 30°C. The plates were next washed 5x with 250µl/well of PBS-0.05% Tween 20.

The secondary antibody, anti-HE4 monoclonal 71.1#1.13-HRP, was diluted 1:16,000 into PBS-1% bovine IgG-0.05% Tween 20-1 mg/ml mouse IgG. The secondary antibody solution was then added to the aspirated plates in 100 µl/well volumes. The plates were incubated for 1 hour at 30°C.

The plates were washed 5x with 250 µl/well of PBS-0.05% Tween 20. The developing solution, TMB(3,3',5,5'- Tetramethylbenzidine) was warmed to room temperature prior to use, and then the TMB was added to the aspirated plates in 100 µl/well volumes.

The plates were incubated for 10 minutes at room temperature and then the Stop solution, 2N H2SO4, was added to the plates (containing the TMB) in 100 µl/well volumes.

The plates were incubated for 10 minutes at room temperature, and then they were read on the Molecular Devices SpectraMax plate reader at 450 nm, with a reference wavelength of 650 nm, using SoftMax Pro software.

The data was saved as SoftMax Pro files, and also exported as Text files for use with MS Excel.

| | |
|---|---|
| Controls: | Serum used as the High Control: Uniglobe #72372 |
| | Serum used as the Low Control: Uniglobe #72404 |
| | Buffer Control used was PBS-1% Bovine Serum-0.05% Tween 20- 1 mg/ml Mouse IgG |

The CV's for the controls across all 25 plates are summarized in Table 5.

**Table 5: The CV's for the Controls Across All 25 Plates**

| Standards point-per-point CV% across all 25 plates | | | |
|---|---|---|---|
| ng/ml | Mean OD | SD | CV% |
| 100 | 2.614 | 0.145 | 5.6 |
| 50 | 1.681 | 0.101 | 6.0 |
| 25 | 0.931 | 0.075 | 8.1 |
| 12.5 | 0.510 | 0.043 | 8.4 |
| 6.25 | 0.263 | 0.027 | 10.4 |
| 3.13 | 0.139 | 0.014 | 10.0 |
| 1.56 | 0.076 | 0.008 | 10.5 |
| 0.78 | 0.044 | 0.012 | 27.6 |

### Results (Standard Curves)

A standard curve for HE4 was prepared by diluting the HE4 protein in PBS/1% bovine serum/0.05% Tween 20-1 mg/mL of mouse IgG. Preparation of such standard curves is well known in the art. Standard curves and the levels or concentrations of the biomarkers in all serum samples were produced for the remaining biomarkers, essentially as described above for HE4, with variations that could be appreciated and implemented by one of skill in the art. Standard curve ranges and curve fit equations for the biomarkers analyzed (i.e., HE4, glycodelin, SLPI, PLAU-R, MUC-1, PAI-1, MMP-7, inhibin, CA125, CTHRC1, KLK-6; KLK-10, alpha-1 anti-trypsin, and prolactin) are presented below:

### HE4

- Standard Curve experimental range = 0.78 ng/ml to 100 ng/ml, in serial two-fold dilutions
- Curve fit = third order polynomial, y = -1E-07x³ - 0.001x² + 0.0402x + 0.0157 ; R² = 0.9999 .
- The standard curve samples and the serum samples were then both diluted 1:4 to run in the assay; since both the serum samples and the standard curve samples were diluted in the same manner there was no need to use a dilution factor during data analysis to determine the concentrations of the unknown serum samples.

### Glycodelin

- Standard Curve experimental range = 3 ng/ml to 100 ng/ml, in serial two-fold dilutions
- Curve fit = quadratic, y = -0.0003x² + 0.0478x + 0.0952 ; R² = 0.9851
- The serum samples were run in the assay undiluted, so no dilution factor was used in the data analysis.

### SLPI

- Standard Curve experimental range = 2.38 ng/ml to 305 ng/ml, in serial two-fold dilutions
- Curve fit = third order polynomial, y = 7E-08x³- 7E-05x² + 0.0237x - 0.039 ; R² = 0.9997
- The serum samples were diluted 1:6.1 in an early step of the automated sample preparation technique. Then those diluted serum samples were further diluted 1:6.6 in another automated step in order to achieve a total dilution of 1:40. Those 1:40 diluted samples were then run in the assay.
- The standard curve samples were prepared at the concentrations stated above (experimental range), then those standard curve samples were all further diluted 1:6.1, and those 1:6.1 diluted samples were then run in the assay.
- Since there was a discrepancy between the dilution of the serum samples (1:40) as compared to the dilution of the standard curve samples (1:6.1), all the serum sample concentration values obtained from the standard curve were multiplied by a dilution factor of "6.6" to correct for the discrepancy (1:40 = 1:6.1 x 1:6.6).

### uPar (PLAU-R)

- Standard Curve experimental range = 62.5 pg/ml to 4000 pg/ml, in serial two-fold dilutions
- Curve fit = linear, y = 0.0006x + 0.0522 ; R² = 0.9977
- The serum samples were diluted 1:6 in an early step of the automated sample preparation technique. Then those diluted serum samples were further diluted 1:3 in another automated step in order to achieve a total dilution of 1:18. Those 1:18 diluted samples were then run in the assay.
- The standard curve samples were prepared at the concentrations stated above (experimental range), then those standard curve samples were all further diluted 1:3, and those 1:3 diluted samples were then run in the assay.
- Since there was a discrepancy between the dilution of the serum samples (1:18) as compared to the dilution of the standard curve samples (1:3), all the serum sample concentration values obtained from the standard curve were multiplied by a dilution factor of "6" to correct for the discrepancy (1:18 =1:3 x1:6).

### MUC-1

- Standard Curve experimental range = 0.3 U/ml to 600 U/ml, in serial threefold dilutions
- Curve fit = third order polynomial, y = 3E-07x³ - 0.0001 x² + 0.0236x + 0.0371; R² = 1.0
- The standard curve samples and the serum samples were then both diluted 1:2 to run in the assay; since both the serum samples and the standard curve samples were diluted in the same manner there was no need to use a dilution factor during data analysis to determine the concentrations of the unknown serum samples.

### PAI-1

- Standard Curve experimental range = 0.78 ng/ml to 100 ng/ml, in serial two-fold dilutions
- Curve fit = linear, y = 0.0139x + 0.0151; R²= 0.9996
- The standard curve samples and the serum samples were then both diluted 1:4 to run in the assay; since both the serum samples and the standard curve
- samples were diluted in the same manner there was no need to use a dilution factor during data analysis to determine the concentrations of the unknown serum samples.

### MMP-7

- Standard Curve experimental range = 0.16 ng/ml to 20 ng/ml, in serial two-fold dilutions
- Curve fit = third order polynomial, y = 0.0001x³ - 0.0046x² + 0.1578x +
- 0.0936 ; R²= 0.9999
- The standard curve samples and the serum samples were then both diluted 1:2 to run in the assay; since both the serum samples and the standard curve samples were diluted in the same manner there was no need to use a dilution factor during data analysis to determine the concentrations of the unknown serum samples.

### Inhibin

- Standard Curve experimental range =10 pg/ml to 1000 pg/ml, in serial two-fold dilutions
- Curve fit = linear, y = 0.0007x + 0.0136 ; R² = 0.9997
- The standard curve samples and the serum samples were then both diluted 1:3 to run in the assay; since both the serum samples and the standard curve samples were diluted in the same manner there was no need to use a dilution factor during data analysis to determine the concentrations of the unknown serum samples.

### CA125

- Standard Curve experimental range = 15 U/ml to 400 U/ml, in serial two-fold dilutions
- Curve fit = linear, y = 0.0021x + 0.0892 ; R² = 0.9986
- The serum samples were run in the assay undiluted, so no dilution factor was used in the data analysis.

### CTHRC1

- Standard Curve experimental range = 0.78 ng/ml to 100 ng/ml, in serial two-fold dilutions
- Curve fit = linear, y = 0.0261 x + 0.042 ; R² = 0.9991
- The standard curve samples and the serum samples were then both diluted 1:2 to run in the assay; since both the serum samples and the standard curve samples were diluted in the same manner there was no need to use a dilution factor during data analysis to determine the concentrations of the unknown serum samples.

### KLK-6

- Standard Curve experimental range = 0.78 ng/ml to 100 ng/ml, in serial two-fold dilutions
- Curve fit = third order polynomial, y = -4E-07x³ + 0.0001x² + 0.0133x + 0.0662 ;
   R²= 1.0
- The standard curve samples and the serum samples were then both diluted 1:2 to run in the assay; since both the serum samples and the standard curve samples were diluted in the same manner there was no need to use a dilution factor during data analysis to determine the concentrations of the unknown serum samples.

### KLK-10

- Standard Curve experimental range = 1 ng/ml to 80 ng/ml, in serial two-fold dilutions
- Curve fit = linear, y = 0.0758x - 0.0312 ; R² = 0.9976
- The standard curve samples and the serum samples were then both diluted 1:4 to run in the assay; since both the serum samples and the standard curve samples were diluted in the same manner there was no need to use a dilution factor during data analysis to determine the concentrations of the unknown serum samples.

### Alpha 1 anti-tryosin

- Standard Curve experimental range = 3.3 ng/ml to 90 ng/ml, in serial two-fold dilutions
- Curve fit = quadratic, y = -0.0002x² + 0.0412x + 0.0592 ; R² = 0.9997
- The serum samples were diluted 1:250,000, and then those diluted samples were run in the assay. The standard curve samples were serially diluted as stated above (experimental range), and then those samples were run in the assay.
- Since there was a discrepancy between the dilution of the serum samples (1:250,0.00) as compared to the dilution of the standard curve samples (undiluted beyond the serial dilutions), all the serum sample concentration values obtained from the standard curve were multiplied by a dilution factor of "250,000" to correct for the discrepancy.

### Prolactin

- Standard Curve experimental range = 2 ng/ml to 240 ng/ml, in serial two-fold dilutions
- Curve fit = linear, y = 0.0049x + 0.0363 ; R² = 0.9917
- The standard curve samples and the serum samples were then both diluted 1:5 to run in the assay; since both the serum samples and the standard curve samples were diluted in the same manner there was no need to use a dilution factor during data analysis to determine the concentrations of the unknown serum samples.

### Results - Individual Marker Performance

Among the 500 normal cases used in the present study, 104 normal cases were used as control group to select the threshold cutoff point. Table 6 provides the mean, standard deviation, range, mean+2*std, mean+3*std based on these 104 threshold normal cases for each marker. Table 7 and Table 8 provide the mean standard deviation, range, mean+2*std in all normal study cases (396 cases) and with normal study cases from patients over the age of 55 (195 or less). The mean and standard deviations were very similar between the normal threshold control cases and normal study cases for patients over the age of 55 for each biomarker except for markers HE4 and Inhibin. See Tables 6 and 8.

**Table 6: Mean and STD of Each Biomarker in 104 Normal Control Cases**

| Variable | N | Mean | stud Dev | Min | Max | Mean+2*std | Mean+3*std | Best Cutoff |
|---|---|---|---|---|---|---|---|---|
| HE4 | 104 | 1.7 | 0.91 | 0.5 | 6.6 | 3.5 | 4.4 | 2.2 |
| CA125 | 104 | 6.9 | 6.42 | 0.6 | 42.6 | 19.7 | 26.1 | 20 |
| GLY | 104 | 1.8 | 2.75 | 0.0 | 18.6 | 7.4 | 10.1 | 5 |
| MMP | 104 | 3.4 | 1.05 | 1.9 | 7.0 | 5.5 | 6.5 | 4 |
| Mucl | 104 | 12.5 | 10.71 | 0.1 | 51.8 | 33.9 | 44.6 | 12 |
| PAI | 104 | 74.8 | 24.92 | 16.4 | 143.2 | 124.7 | | 95 |
| CTRHC1 | 104 | 3.2 | 1.16 | 1.2 | 6.2 | 5.5 | | 4 |
| INH | 104 | 0.6 | 2.80 | 0.0 | 26.9 | 6.2 | 9.0 | 2 |
| Plau-R | 104 | 1945.4 | 534.03 | 1056.00 | 4248.0 | 3013.4 | 3547.5 | 2399.9 |
| PROLAC | 104 | 10.2 | 10.94 | 3.1 | 102.2 | 32.0 | 43.0 | 11 |
| KLK10 | 104 | 0.8 | 0.51 | 0.3 | 2.3 | 1.8 | | 0.9 |
| KLK6 | 104 | 2.2 | 2.39 | 0.3 | 16.3 | 7.0 | 9.4 | 5 |
| SLPI | 104 | 65.7 | 14.38 | 40.2 | 134.5 | 94.5 | 108.9 | 65 |

**Table 7: Mean and STD of Each Biomarker in 396 Normal Study Cases**

| **Variable** | **N** | **Mean** | **Std Dev** | **Min** | **Max** | Mean+2*std |
|---|---|---|---|---|---|---|
| HE4 | 396 | 1.6 | 2.1 | 0.2 | 36.4 | 5.8* |
| CA125 | 396 | 10.4 | 14.2 | 1.7 | 212.4 | 38.7* |
| GLY | 396 | 5.0 | 10.2 | 0.0 | 98.4 | 25.3* |
| MMP | 396 | 3.0 | 1.1 | 0.8 | 10.4 | 5.1 |
| Muc1 | 396 | 8.3 | 8.2 | 0.0 | 52.1 | 24.7 |
| PAI | 396 | 73.2 | 28.0 | 18.8 | 145.7 | 129.2 |
| CTRHC1 | 396 | 2.9 | 1.2 | 0.4 | 8.7 | 5.2 |
| INH | 360 | 11.3 | 28.1 | 0.0 | 265.4 | 67.5* |
| PLAUR | 396 | 2086.0 | 782.0 | 822.0 | 6384.00 | 3650 |
| PROLAC | 396 | 11.6 | 9.5 | 2.0 | 138.0 | 30.7 |
| KLK10 | 396 | 0.9 | 0.4 | 0.2 | 2.5 | 1.7 |
| KLK6 | 396 | 1.7 | 1.7 | 0.1 | 18.0 | 5.1 |
| SLPI | 396 | 59.6 | 18.0 | 31.3 | 305.0 | 95.6 |
| * The values in normal cases were very different from the values in normal control cases, which means these markers might be sensitive due to age effect. | | | | | | |

**Table 8: Mean and STD of Each Biomarker in Normal Study Cases (Patients Over the Age of 55)**

| Variable | N | Mean | Std Dev | Min | Max | Mean+2*std |
|---|---|---|---|---|---|---|
| HE4 | 195 | 2.0 | 2.8 | 0.2 | 36.4 | 7.6* |
| CA125 | 194 | 10.6 | 5.6 | 2.3 | 35.7 | 21.9 |
| GLY | 195 | 1.7 | 2.3 | 0.0 | 24.8 | 6.3 |
| MMP | 195 | 3.2 | 1.2 | 0.8 | 10.4 | 5.6 |
| Muc1 | 195 | 8.9 | 8.4 | 0.0 | 36.6 | 25.7 |
| PAI | 195 | 75.6 | 27.9 | 19.1 | 145.7 | 131.3 |
| CTRHC1 | 195 | 3.2 | 1.1 | 1.2 | 7.1 | 5.4 |
| INH | 177 | 5.2 | 26.6 | 0.0 | 265.4 | 58.5** |
| PLAUR | 195 | 2209.5 | 752.1 | 1122.0 | 5100.0 | 3713.7 |
| PROLAC | 195 | 10.8 | 11.1 | 2.0 | 138.0 | 32.9 |
| KLK10 | 195 | 0.9 | 0.4 | 0.2 | 2.4 | 1.7 |
| KLK6 | 195 | 1.8 | 2.0 | 0.1 | 18.0 | 5.7 |
| SLPI | 195 | 60.3 | 14.2 | 31.3 | 126.9 | 88.6 |
| * The standard deviation was larger in the normal study cases than in the normal control cases for patients over the age of 55 | | | | | | |
| ** The mean and standard deviation were larger in the normal study cases than in the normal control cases for patients over the age of 55 | | | | | | |

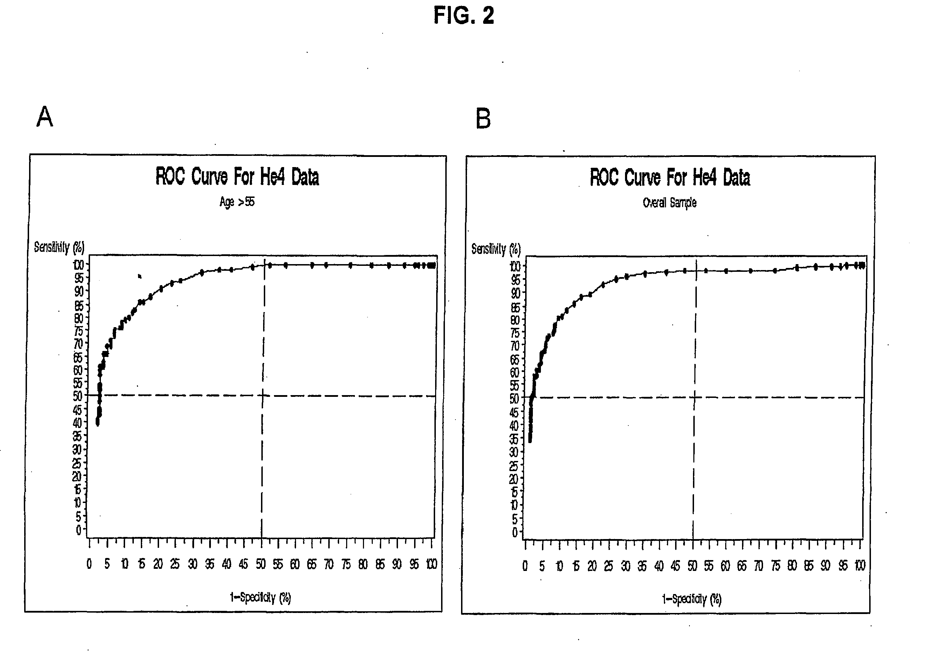

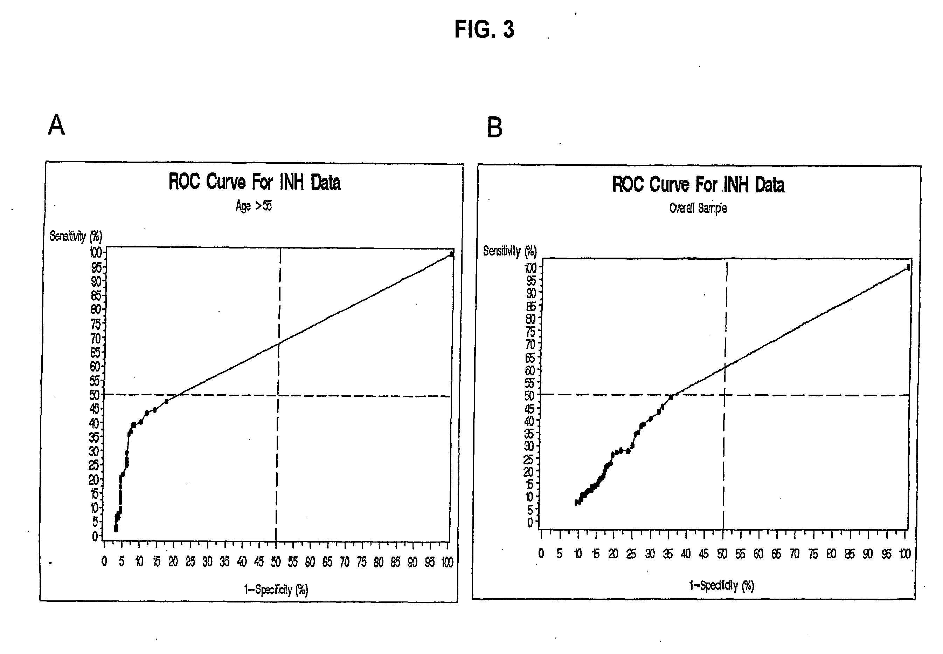

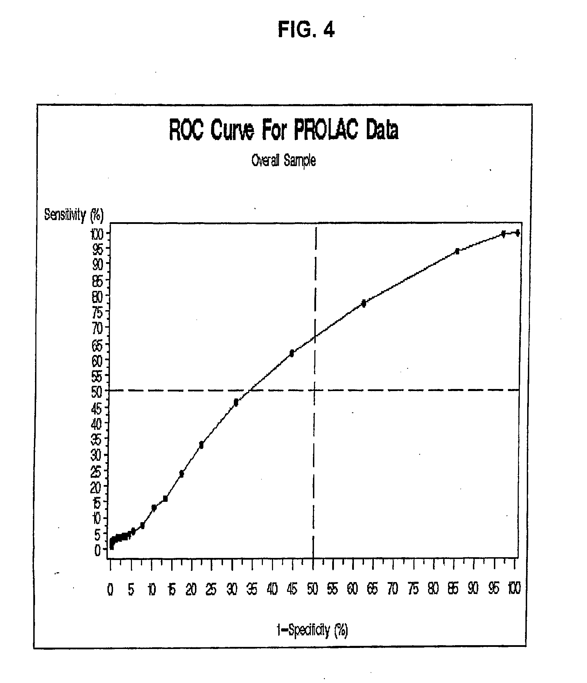

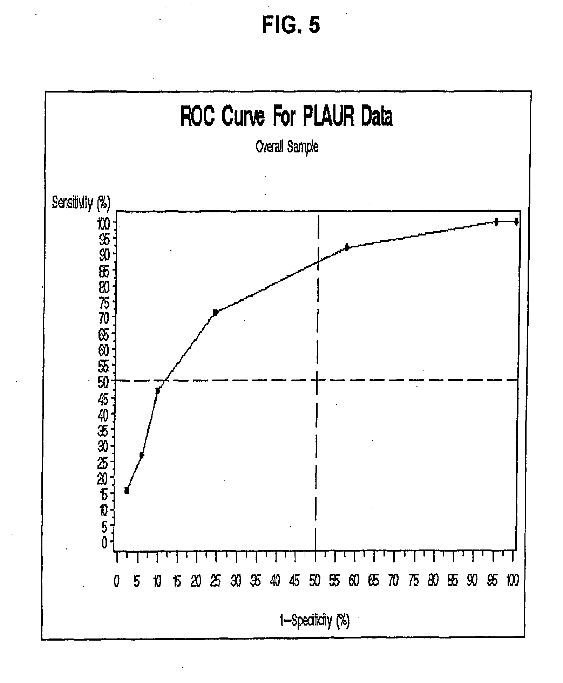

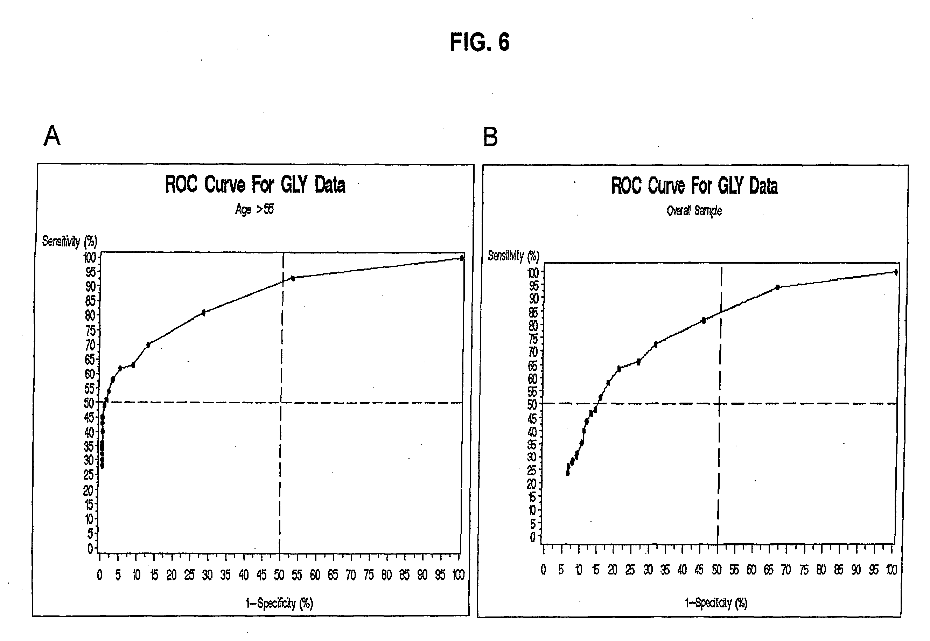

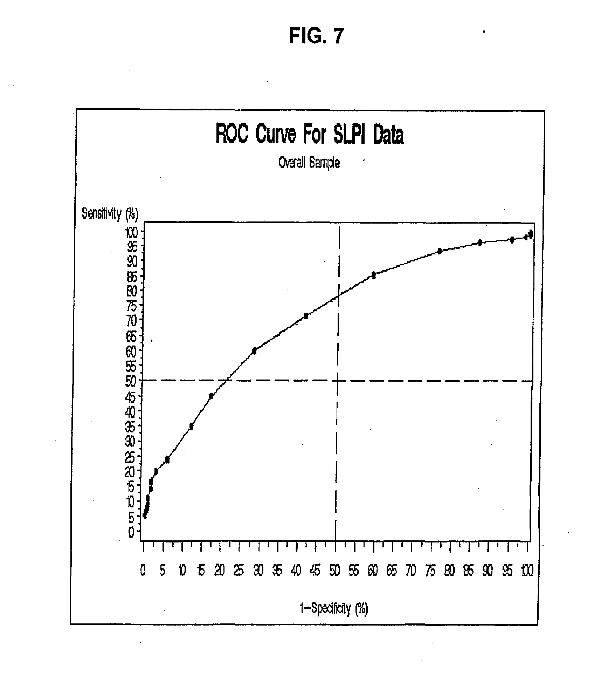

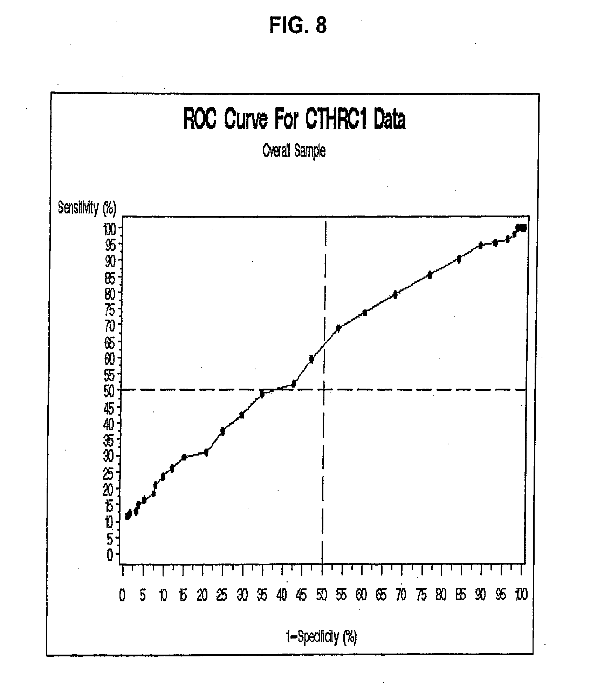

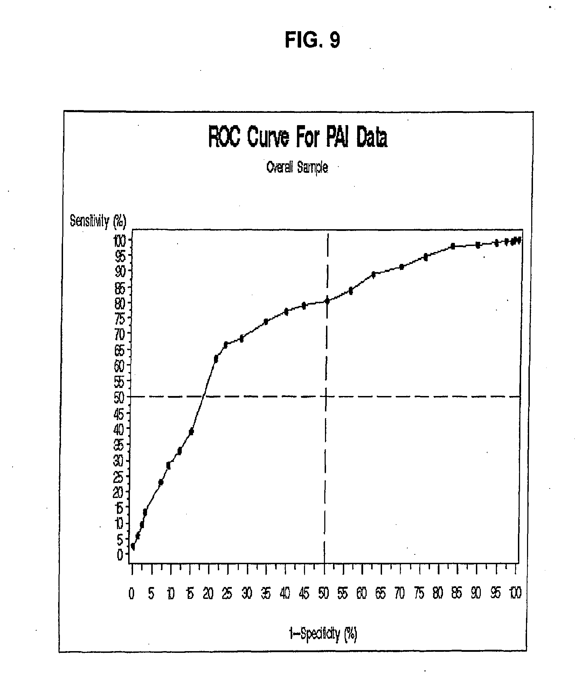

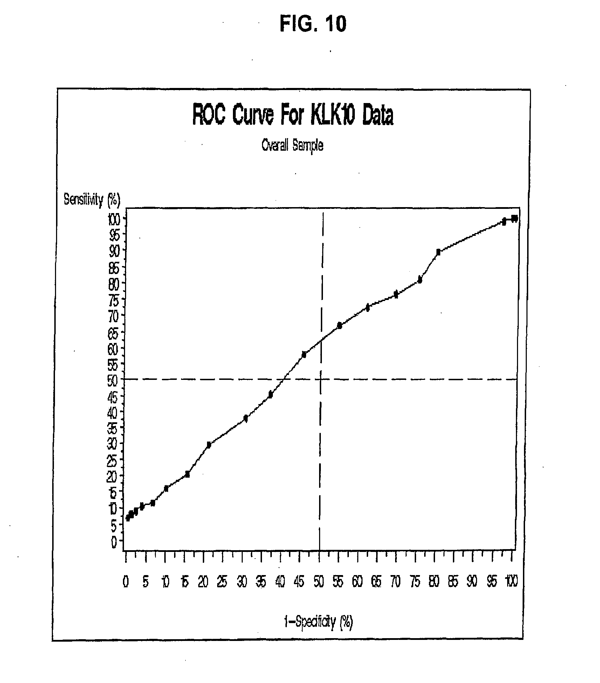

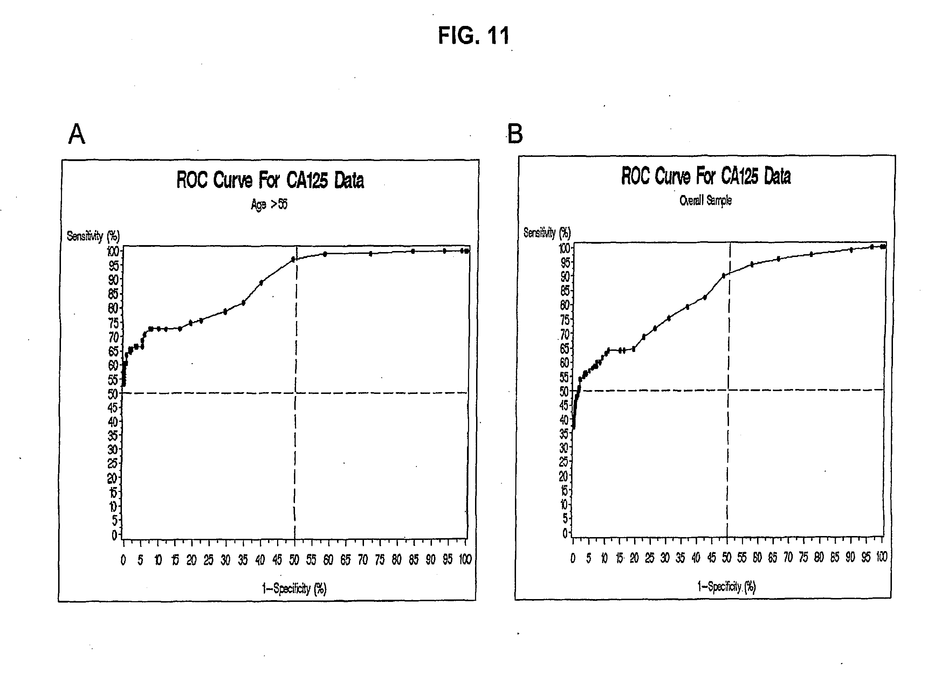

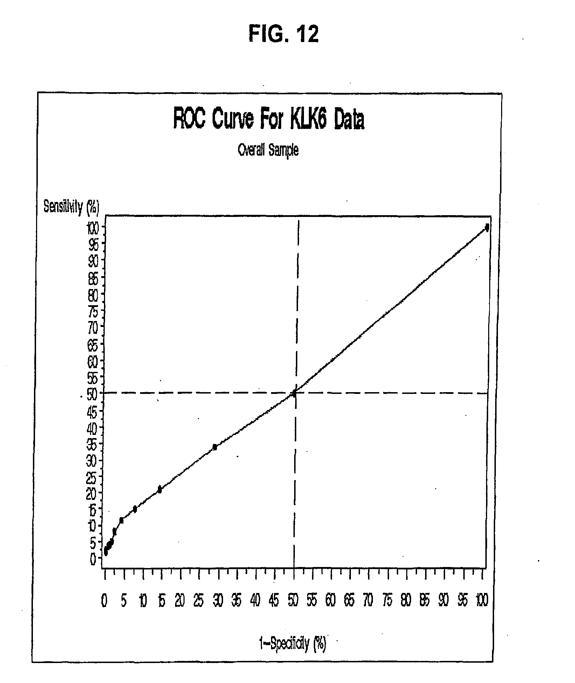

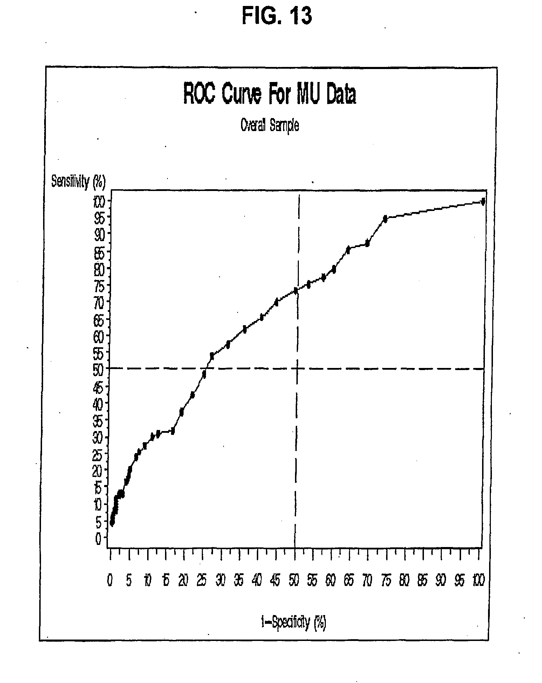

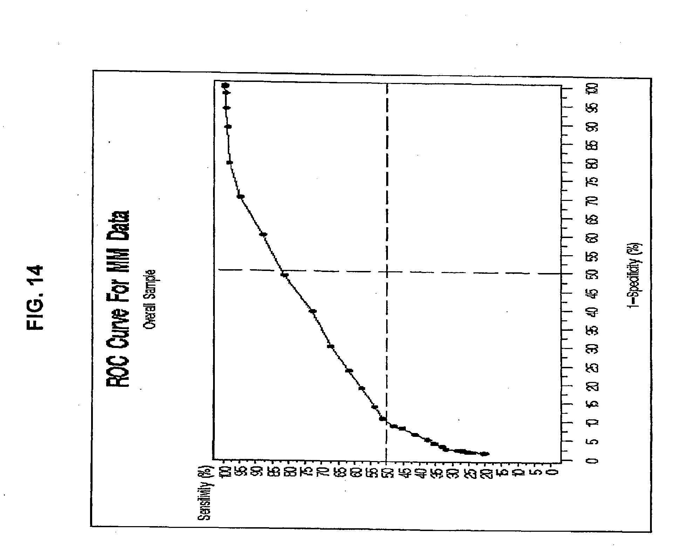

For each individual marker, the ROC curve was obtained. The ROC curve only consists of the 396 normal cases and the 200 ovarian cancer cases. The 104 normal cases as control and 200 interfering substance/pathology cases were not included in this analysis. The ROC curves for HE4, inhibin A, prolactin, PLAU-R, glycodelin, SLPI, CTHRC1, PAI-1, KLK-10, CA125, KLK-6, MUC-1, and MMP-7 are presented in Figures 2-14, respectively.

Also, the percentage of positive and negative samples were determined for each marker by age group, by cancer stage, and by interfering substance/pathology category. These determinations were based on two kinds of cutoff points: (1) mean+2*std, and (2) the best cutoff from the ROC curve. The best cutoff points were selected from the ROC curve with the highest value of sensitivity plus specificity. The best cutoff point was listed in Table 6. Summaries of the positive results for each biomarker by age group, cancer stage, and interfering substance/pathology category are presented in tables 9 to 47.

### Biomarker: HE4

**Table 9: Summary by Age Group for HE4**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 57 | 63.33 | 98 | 90.74 | 41 | 45.56 | 81 | 75.00 |
| | **Negative** | 33 | 36.67 | 10 | 9.26 | 49 | 54.44 | 27 | 25.00 |
| | **Total** | 90 | 100.00 | 108 | 100.0 0 | 90 | 100.00 | 108 | 100.00 |
| **NORMAL** | **Positive** | 15 | 7.46 | 39 | 20.00 | 3 | 1.49 | 13 | 6.67 |
| | **Negative** | 186 | 92.54 | 156 | 80.00 | 198 | 98.51 | 182 | 93.33 |
| | **Total** | 201 | 100.00 | 195 | 100.0 0 | 201 | 100.00 | 195 | 100.00 |
| **OvCA** | **Positive** | 81 | 81.00 | 91 | 91.00 | 58 | 58.00 | 75 | 75.00 |
| | **Negative** | 19 | 19.00 | 9 | 9.00 | 42 | 42.00 | 25 | 25.00 |
| | **Total** | 100 | 100.00 | 100 | 100.0 0 | 100 | 100.00 | 100 | - 100.00 |

**Table 10: Summary by Interfering Substance/Pathology Category for HE4**

| | | Best Cutoff from ROC plot | | Mean+2*std | |
|---|---|---|---|---|---|
| Group | Result | N | % | N | % |
| Tumors | Positive | 38 | 84.44 | 27 | 60.00 |
| | Negative | 7 | 15.56 | 18 | 40.00 |
| | Total | 45 | 100.00 | 45 | 100.00 |
| Metabolic Disorder | Positive | 20 | 100.00 | 20 | 100.00 |
| | Negative | 0 | 0.00 | 0 | 0.00 |
| | Total | 20 | 100.00 | 20 | 100.00 |
| Hormonal Fluctuation | Positive | 23 | 69.70 | 16 | 48.48 |
| | Negative | 10 | 30.30 | 17 | 51.52 |
| | Total | 33 | 100.00 | 33 | 100.00 |
| Gynecologic Disorder | Positive | 17 | 48.57 | 12 | 34.29 |
| | Negative | 18 | 51.43 | 23 | 65.71 |
| | Total | 35 | 100.00 | 35 | 100.00 |
| Vascular Disorder | Positive | 22 | 88.00 | 19 | 76.00 |
| | Negative | 3 | 12.00 | 6 | 24.00 |
| | Total | 25 | 100.00 | 25 | 100.00 |
| Chronic Immuno. Disorder | Positive | 37 | 88.10 | 29 | 69.05 |
| | Negative | 5 | 11.90 | 13 | 30.95 |
| | Total | 42 | 100.00 | 42 | 100.00 |

**Table 11: Summary by Cancer Stage for HE4**

| | | Best Cutoff from ROC plot | | Mean+2*std | |
|---|---|---|---|---|---|
| Stage | Result | N | % | N | % |
| **1** | **Positive** | 57 | 85.07 | 42 | 62.69 |
| | **Negative** | 10 | 14.93 | 25 | 37.31 |
| | **Total** | 67 | 100.00 | 67 | 100.00 |
| **2** | **Positive** | 53 | 80.30 | 41 | 62.12 |
| | **Negative** | 13 | 19.70 | 25 | 37.88 |
| | **Total** | 66 | 100.00 | 66 | 100.00 |
| **3** | **Positive** | 62 | 92.54 | 50 | 74.63 |
| | **Negative** | 5 | 7.46 | 17 | 25.37 |

### Biomarker Muc-1

**Table 12: Summary by Age Group for Muc-1**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 38 | 42.22 | 53 | 49.07 | 4 | 4.44 | 9 | 8.33 |
| | **Negative** | 52 | 57.78 | 55 | 50.93 | 86 | 95.56 | 99 | 91.67 |
| | **Total** | 90 | 100.00 | 108 | 100.00 | 90 | 100.00 | 108 | 100.00 |
| **NORMAL** | **Positive** | 49 | 24.38 | 58 | 29.74 | 2 | 1.00 | 4 | 2.05 |
| | **Negative** | 152 | 75.62 | 137 | 70.26 | 199 | 99.00 | 191 | 97.95 |
| | **Total** | 201 | 100.00 | 195 | 100.00 | 201 | 100.00 | 195 | 100.00 |
| **OvCA** | **Positive** | 56 | 56.00 | 52 | 52.00 | 4 | 4.00 | 12 | 12.00 |
| | **Negative** | 44 | 44.00 | 48 | 48.00 | 96 | 96.00 | 88 | 88.00 |
| | **Total** | 100 | 100.00 | 100 | 100.00 | 100 | 100.00 | 100 | 100.00 |

**Table 13: Summary by Interfering Substance/Pathology Category for Muc-1**

| | | Best Cutoff from ROC plot | | Mean+2*std | |
|---|---|---|---|---|---|
| Group | Result | N | % | N | % |
| Tumors | Positive | 20 | 44.44 | 3 | 6.67 |
| | Negative | 25 | 55.56 | 42 | 93.33 |
| | Total | 45 | 100.00 | 45 | 100.00 |
| Metabolic Disorder | Positive | 8 | 40.00 | 1 | 5.00 |
| | Negative | 12 | 60.00 | 19 | 95.00 |
| | Total | 20 | 100.00 | 20 | 100.00 |
| Hormonal Fluctuation | Positive | 14 | 42.42 | 1 | 3.03 |
| | Negative | 19 | 57.58 | 32 | 96.97 |
| | Total | 33 | 100.00 | 33 | 100.00 |
| Gynecologic Disorder | Positive | 17 | 48.57 | 1 | 2.86 |
| | Negative | 18 | 51.43 | 34 | 97.14 |
| | Total | 35 | 100.00 | 35 | 100.00 |
| Vascular Disorder | Positive | 11 | 44.00 | 2 | 8.00 |
| | Negative | 14 | 56.00 | 23 | 92.00 |
| | Total | 25 | 100.00 | 25 | 100.00 |
| Chronic Immuno. Disorder | Positive | 22 | 52.38 | 5 | 11.90 |
| | Negative | 20 | 47.62 | 37 | 88.10 |
| | Total | 42 | 100.00 | 42 | 100.00 |

**Table 14: Summary by Cancer Stage for Muc-1**

| | | Best Cutoff from ROC plot | | Mean+2*std | |
|---|---|---|---|---|---|
| Stage | Result | N | % | N | % |
| **1** | **Positive** | 44 | 65.67 | 6 | 8.96 |
| | **Negative** | 23 | 34.33 | 61 | 91.04 |
| | **Total** | 67 | 100.00 | 67 | 100.00 |
| **2** | **Positive** | 27 | 40.91 | 3 | 4.55 |
| | **Negative** | 39 | 59.09 | 63 | 95.45 |
| | **Total** | 66 | 100.00 | 66 | 100.00 |
| **3** | **Positive** | 37 | 55.22 | 7 | 10.45 |
| | **Negative** | 30 | 44.78 | 60 | 89.55 |
| | **Total** | 67 | 100.00 | 67 | 100.00 |

### Biomarker: KLK-6

**Table 15: Summary by Age Group for KLK-6**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 5 | 5.56 | 8 | 7.41 | 4 | 4.44 | 4 | 3.70 |
| | **Negative** | 85 | 94.44 | 100 | 92.59 | 86 | 95.56 | 104 | 96.30 |
| | **Total** | 90 | 100.00 | 108 | 100.00 | 90 | 100.00 | 108 | 100.00 |
| **NORMAL** | **Positive** | 6 | 2.99 | 11 | 5.64 | 2 | 1.00 | 5 | 2.56 |
| | **Negative** | 195. | 97.01 | 184 | 94.36 | 199 | 99.00 | 190 | 97.44 |
| | **Total** | 201 | 100.00 | 195 | 100.00 | 201 | 100.00 | 195 | 100.00 |
| **OvCA** | **Positive** | 10 | 10.00 | 13 | 13.00 | 4 | 4.00 | 6 | 6.00 |
| | **Negative** | 90 | 90.00 | 87 | 87.00 | 96 | 96.00 | 94 | 94.00 |
| | Total | 100 | 100.00 | 100 | 100.00 | 100 | 100.00 | 100 | 100.00 |

**Table 16: Summary by Interfering Substance/Pathology Category for KLK.6**

| | | Best Cutoff from ROC plot | | Mean+2*std | |
|---|---|---|---|---|---|
| Group | Result | N | % | N | % |
| Tumors | Positive | 5 | 11.11 | 4 | 8.89 |
| | Negative | 40 | 88.89 | 41 | 91.11 |
| | Total | 45 | 100.00 | 45 | 100.00 |
| Metabolic Disorder | Positive | 0 | 0.00 | 0 | 0.00 |
| | Negative | 20 | 100.00 | 20 | 100.00 |
| | Total | 20 | 100.00 | 20 | 100.00 |
| Hormonal Fluctuation | Positive | 0 | 0.00 | 0 | 0.00 |
| | Negative | 33 | 100.00 | 33 | 100.00 |
| | Total | 33 | 100.00 | 33 | 100.00 |
| Gynecologic Disorder | Positive | 3 | 8.57 | 2 | 5.71 |
| | Negative | 32 | 91.43 | 33 | 94.29 |
| | Total | 35 | 100.00 | 35 | 100.00 |
| Vascular Disorder | Positive | 3 | 12.00 | 1 | 4.00 |
| | Negative | 22 | 88.00 | 24 | 96.00 |
| | Total | 25 | 100.00 | 25 | 100.00 |
| Chronic Immuno. Disorder | Positive | 2 | 4.76 | 1 | 2.38 |
| | Negative | 40 | 95.24 | 41 | 97.62 |
| | Total | 42 | 100.00 | 42 | 100.00 |

**Table 17: Summary by Cancer Stage Group for KLK-6**

| | | Best Cutoff from ROC plot | | Mean+2*std | |
|---|---|---|---|---|---|
| Stage | Result | N | % | N | % |
| **1** | **Positive** | 10 | 14.93 | 4 | 5.97 |
| | **Negative** | 57 | 85.07 | 63 | 94.03 |
| | **Total** | 67 | 100.00 | 67 | 100.00 |
| **2** | **Positive** | 7 | 10.61 | 3 | 4.55 |
| | **Negative** | 59 | 89.39 | 63 | 95.45 |
| | **Total** | 66 | 100.00 | 66 | 100.00 |
| **3** | **Positive** | 6 | 8.96 | 3 | 4.48 |
| | **Negative** | 61 | 91.04 | 64 | 95.52 |
| | **Total** | 67 | 100.00 | 67 | 100.00 |

### Biomarker: KLK-10

**Table 18: Summary by Age Group for KLK-10**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 56 | 62.22 | 51 | 47.22 | 7 | 7.78 | 6 | 5.56 |
| | **Negative** | 34 | 37.78 | 57 | 52.78 | 83 | 92.22 | 102 | 94.44 |
| | **Total** | 90 | 100.00 | 108 | 100.00 | 90 | 100.00 | 108 | 100.00 |
| **NORMAL** | **Positive** | 92 | 45.77 | 89 | 45.64 | 4 | 1.99 | 2 | 1.03 |
| | **Negative** | 109 | 54.23 | 106 | 54.36 | 197 | 98.01 | 193 | 98.97 |
| | **Total** | 201 | 100.00 | 195 | 100.00 | 201 | 100.00 | 195 | 100.00 |
| **OvCA** | **Positive** | 63 | 63.00 | 53 | 53.00 | 9 | 9.00 | 7 | 7.00 |
| | **Negative** | 37 | 37.00 | 47 | 47.00 | 91 | 91.00 | 93 | 93.00 |
| | **Total** | 100 | 100.00 | 100 | 100.00 | 100 | 100.00 | 100 | 100.00 |

**Table 19: Summary by Interfering Substance/Pathology Group for KLK-10**

| | | Best Cutoff from ROC plot | | Mean+2*std | |
|---|---|---|---|---|---|
| Group | Result | N | % | N | % |
| Tumors | Positive | 28 | 62.22 | 3 | 6.67 |
| | Negative | 17 | 37.78 | 42 | 93.33 |
| | Total | 45 | 100.00 | 45 | 100.00 |
| Metabolic Disorder | Positive | 10 | 50.00 | 0 | 0.00 |
| | Negative | 10 | 50.00 | 20 | 100.00 |
| | Total | 20 | 100.00 | 20 | 100.00 |
| Hormonal Fluctuation | Positive | 14 | 42.42 | 0 | 0.00 |
| | Negative | 19 | 57.58 | 33 | 100.00 |
| | Total | 33 | 100.00 | 33 | 100.00 |
| Gynecologic Disorder | Positive | 23 | 65.71 | 3 | 8.57 |
| | Negative | 12 | 34.29 | 32 | 91.43 |
| | Total | 35 | 100.00 | 35 | 100.00 |
| Vascular Disorder | Positive | 11 | 44.00 | 1 | 4.00 |
| | Negative | 14 | 56.00 | 24 | 96.00 |
| | Total | 25 | 100.00 | 25 | 100.00 |
| Chronic Immuno. Disorder | Positive | 23 | 54.76 | 6 | 14.29 |
| | Negative | 19 | 45.24 | 36 | 85.71 |
| | Total | 42 | 100.00 | 42 | 100.00 |

**Table 20: Summary by Cancer Stage Group for KLK-10**

| | | Best Cutoff from ROC plot | | Mean+2*std | |
|---|---|---|---|---|---|
| Stage | Result | N | % | N | % |
| **1** | **Positive** | 37 | 55.22 | 8 | 11.94 |
| | **Negative** | 30 | 44.78 | 59 | 88.06 |
| | **Total** | 67 | 100.00 | 67 | 100.00 |
| **2** | **Positive** | 37 | 56.06 | 3 | 4.55 |
| | **Negative** | 29 | 43.94 | 63 | 95.45 |
| | **Total** | 66 | 100.00 | 66 | 100.00 |
| **3** | **Positive** | 42 | 62.69 | 5 | 7.46 |
| | **Negative** | 25 | 37.31 | 62 | 92.54 |
| | **Total** | 67 | 100.00 | 67 | 100.00 |

### Biomarker: PAI-1

**Table 21: Summary by Age Group for PAI-1**

| | | Best Cutoff from ROC plot | | | | Mean+2*std | | | |
|---|---|---|---|---|---|---|---|---|---|
| | | Age <=5 | | Age>55 | | Age <=55 | | Age>55 | |
| **Group** | **Result** | N | % | N | % | N | % | N | % |
| **IP** | **Positive** | 52 | 57.78 | 67 | 62.04 | 12 | 13.33 | 10 | 9.26 |
| | **Negative** | 38 | 42.22 | 41 | 37.96 | 78 | 86.67 | 98 | 90.74 |
| | **Total** | 90 | 100.00 | 108 | 100.00 | 90 | 100.00 | 108 | 100.00 |
| **NORMAL** | **Positive** | 43 | 21.39 | 52 | 26.67 | 5 | 2.49 | 10 | 5.13 |
| | **Negative** | 158 | 78.61 | 143 | 73.33 | 196 | 97.51 | 185 | 94.87 |
| | **Total** | 201 | 100.00 | 195 | 100.00 | 201 | 100.00 | 195 | 100.00 . |
| **OvCA** | **Positive** | 71 | 71.00 | 62 | 62.00 | 19 | 19.00 | 11 | 11.00 |
| | **Negative** | 29 | 29.00 | 38 | 38.00 | 81 | 81.00 | 89 | 89.00 |
| | **Total** | 100 | 100.00 | 100 | 100.00 | 100 | 100.00 | 100 | 100.00 |

**Table 22: Summary by Interfering Substance/Pathology Group for PAI-1**

| | | Best Cutoff from ROC plot | | Mean+2*std | |
|---|---|---|---|---|---|
| Group | Result | N | % | N | % |
| Tumors | Positive | 17 | 37.78 | 4 | 8.89 |
| | Negative | 28 | 62.22 | 41 | 91.11 |
| | Total | 45 | 100.00 | 45 | 100.00 |
| Metabolic Disorder | Positive | 16 | 80.00 | 4 | 20.00 |
| | Negative | 4 | 20.00 | 16 | 80.00 |
| | Total | 20 | 100.00 | 20 | 100.00 |
| Hormonal Fluctuation | Positive | 24 | 72.73 | 5 | 15.15 |
| | Negative | 9 | 27.27 | 28 | 84.85 |
| | Total | 33 | 100.00 | 33 | 100.00 |
| Gynecologic Disorder | Positive | 17 | 48.57 | 2 | 5.71 |
| | Negative | 18 | 51.43 | 33 | 94.29 |
| | Total | 35 | 100.00 | 35 | 100.00 |
| Vascular Disorder | Positive | 17 | 68.00 | 1 | 4.00 |
| | Negative | 8 | 32.00 | 24 | 96.00 |
| | Total | 25 | 100.00 | 25 | 1000.00 |
| Chronic Immuno. Disorder | Positive | 30 | 71.43 | 6 | 14.29 |
| | Negative | 12 | 28.57 | 36 | 85.71 |
| | Total | 42 | 100.00 | 42 | 100.00 |

**Table 23: Summary by Cancer Stage Group for PAI-1**

| | | Best Cutoff from ROC plot | | Mean+2*std | |
|---|---|---|---|---|---|
| Stage | Result | N | % | N | % |
| 1 | **Positive** | 54 | 80.60 | 15 | 22.39 |
| | **Negative** | 13 | 19.40 | 52 | 77.61 |
| | | | | | |
| | **Total** | 67 | 100.00 | 67 | 100.00 |
| **2** | **Positive** | 33 | 50.00 | 4 | 6.06 |
| | **Negative** | 33 | 50.00 | 62 | 93.94 |
| | | | | | |
| | **Total** | 66 | 100.00 | 66 | 100.00 |
| **3** | **Positive** | 46 | 68.66 | 11 | 16.42 |
| | **Negative** | 21 | 31.34 | 56 | 83.58 |
| | | | | | |
| | **Total** | 67 | 100.00 | 67 | 100.0 |

### Biomarker: CTHRC1

**Table 24: Summary by Age Group for CTHRC1**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 12 | 13.33 | 35 | 32.41 | 6 | 6.67 | 11 | 10.19 |
| | **Negative** | 78 | 86.67 | 73 | 67.59 | 84 | 93.33 | 97 | 89.81 |
| | **Total** | 90 | 100.00 | 108 | 100.00 | 90 | 100.00 | 108 | 100.00 |
| **NORMAL** | **Positive** | 20 | 9.95 | 40 | 20.51 | 4 | 1.99 | 4 | 2.05 |
| | **Negative** | 181 | 90.05 | 155 | 79.49 | 197 | 98.01 | 191 | 97.95 |
| | **Total** | 201 | 100.00 | 195 | 100.00 | 201 | 100.00 | 195 | 100.00 |
| **OvCA** | **Positive** | 24 | 24.00 | 35 | 35.00 | 10 | 10.00 | 15 | 15.00 |
| | **Negative** | 76 | 76.00 | 65 | 65.00 | 90 | 90.00 | 85 | 85.00 |
| | **Total** | 100 | 100.00 | 100 | 100.00 | 100 | 100.00 | 100 | 100.00 |

**Table 25: Summary by Interfering Substance/Pathology_Group for CTHRC1**

| | | Best Cutoff from ROC plot | | Mean+2*std | |
|---|---|---|---|---|---|
| Group | Result | N | % | N | % |
| Tumors | Positive | 13 | 28.89 | 7 | 15.56 |
| | Negative | 32 | 71.11 | 38 | 84.44 |
| | Total | 45 | 100.00 | 45 | 100.00 |
| Metabolic Disorder | Positive | 2 | 10.00 | 0 | 0.00 |
| | Negative | 18 | 90.00 | 20 | 100.00 |
| | Total | 20 | 100.00 | 20 | 100.00 |
| Hormonal Fluctuation | Positive | 1 | 3.03 | 1 | 3.03 |
| | Negative | 32 | 96.97 | 32 | 96.97 |
| | Total | 33 | 100.00 | 33 | 100.00 |
| Gynecologic Disorder | Positive | 2 | 5.71 | 0 | 0.00 |
| | Negative | 33 | 94.29 | 35 | 100.00 |
| | Total | 35 | 100.00 | 35 | 100.00 |
| Vascular Disorder | Positive | 11 | 44.00 | 5 | 20.00 |
| | Negative | 14 | 56.00 | 20 | 80.00 |
| | Total | 25 | 100.00 | 25 | 100.00 |
| Chronic Immuno. Disorder | Positive | 18 | 42.86 | 4 | 9.52 |
| | Negative | 24 | 57.14 | 38 | 90.48 |
| | Total | 42 | 100.00 | 42 | 100.00 |

**Table 26: Summary by Cancer Stage Group for CTHRC1**

| | | Best Cutoff from ROC plot | | Mean+2*std | |
|---|---|---|---|---|---|
| Stage | Result | N | % | N | % |
| **1** | **Positive** | 16 | 23.88 | 7 | 10.45 |
| | **Negative** | 51 | 76.12 | 60 | 89.55 |
| | **Total** | 67 | 100.00 | 67 | 100.00 |
| **2** | **Positive** | 19 | 28.79 | 8 | 12.12 |
| | **Negative** | 47 | 71.21 | 58 | 87.88 |
| | **Total** | 66 | 100.00 | 66 | 100.00 |
| **3** | **Positive** | 24 | 35.82 | 10 | 14.93 |
| | **Negative** | 43 | 64.18 | 57 | 85.07 |
| | **Total** | 67 | 100.00 | 67 | 100.00 |

### Biomarker: SLPI

**Table 27: Summary by Age Group for SLPI**

| | | **Best** Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 27 | 30.00 | 61 | 56.48 | 4 | 4.44 | 23 | 21.30 |
| | **Negative** | 63 | 70.00 | 47 | 43.52 | 86 | 95.56 | 85 | 78.70 |
| | **Total** | 90 | 100.00 | 108 | 100.00 | 90 | 100.00 | 108 | 100.00 |
| **NORMAL** | **Positive** | 49 | 24.38 | 63 | 32.31 | 4 | 1.99 | 3 | 1.54 |
| | **Negative** | 152 | 75.62 | 132 | 67.69 | 197 | 98.01 | 192 | 98.46 |
| | **Total** | 201 | 100.00 | 195 | 100.00 | 201 | 100.00 | 195 | 100.00 |
| **OvCA** | **Positive** | 53 | 53.00 | 67 | 67.00 | 14 | 14.00 | 16 | 16.00 |
| | **Negative** | 47 | 47.00 | 33 | 33.00 | 86 | 86.00 | 84 | 84.00 |
| | **Total** | 100 | 100.00 | 100 | 100.00 | 100 | 100.00 | 100 | 100.00 |

**Table 28: Summary by Interfering Substance/Pathology Group for SLPI**

| | | Best Cutoff from ROC plot | | Mean+2*std | |
|---|---|---|---|---|---|
| Group | Result | N | % | N | % |
| Tumors | Positive | 20 | 44.44 | 7 | 15.56 |
| | Negative | 25 | 55.56 | 38 | 84.44 |
| | Total | 45 | 100.00 | 45 | 100.00 |
| Metabolic Disorder | Positive | 10 | 50.00 | 1 | 5.00 |
| | Negative | 10 | 50.00 | 19 | 95.00 - |
| | Total | 20 | 100.00 | 20 | 100.00 |
| Hormonal Fluctuation | Positive | 6 | 18.18 | 0 | 0.00 |
| | Negative | 27 | 81.82 | 33 | 100.00 |
| | Total | 33 | 100.00 | 33 | 100.00 |
| Gynecologic Disorder | Positive | 12 | 34.29 | 4 | 11.43 |
| | Negative | 23 | 65.71 | 31 | 88.57 |
| | Total | 35 | 100.00 | 35 | 100.00 |
| Vascular Disorder | Positive | 16 | 64.00 | 9 | 36.00 |
| | Negative | 9 | 36.00 | 16 | 64.00 |
| | Total | 25 | 100.00 | 25 | 100.00 |
| Chronic Immuno. Disorder | Positive | 26 | 61.90 | 7. | 16.67 |
| | Negative | 16 | 38.10 | 35 | 83.33 |
| | Total | 42 | 100.00 | 42 | 100.00 |

**Table 29: Summary by Cancer Stage Group for SLPI**

| | | Best Cutoff from ROC plot | | Mean+2*std | |
|---|---|---|---|---|---|
| Stage | Result | N | % | N | % |
| **1** | **Positive** | 34 | 50.75 | 9 | 13.43 |
| | **Negative** | 33 | 49.25 | 58 | 86.57 |
| | **Total** | 67 | 100.00 | 67 | 100.00 |
| **2** | **Positive** | 38 | 57.58 | 4 | 6.06 |
| | **Negative** | 28 | 42.42 | 62 | 93.94 |
| | **Total** | 66 | 100.00 | 66 | 100.00 |
| **3** | **Positive** | 48 | 71.64 | 17 | 25.37 |
| | **Negative** | 19 | 28.36 | 50 | 74.63 |
| | **Total** | 67 | 100.00 | 67 | 100.00 |

### Biomarker: Inhibin A

**Table 30: Summary by Age Group for Inhibin**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 53 | 58.89 | 11 | 10.19 | 48 | 53.33 | 7 | 6.48 |
| | **Negative** | 37 | 41.11 | 97 | 89.81 | 42 | 46.67 | 101 | 93.52 |
| | **Total** | 90 | 100.00 | 108 | 100.00 | 90 | 100.00 | 108 | 100.00 |
| **NORMAL** | **Positive** | 94 | 46.77 | 25 | 12.82 | 83 | 41.29 | 14 | 7.18 |
| | **Negative** | 107 | 53.23 | 170 | 87.18 | 118 | 58.71 | 181 | 92.82 |
| | **Total** | 201 | 100.00 | 195 | 100.00 | 201 | 100.00 | 195 | 100.00 |
| **OvCA** | **Positive** | 42 | 42.00 | 41 | 41.00 | 32 | 32.00 | 36 | 36.00 |
| | **Negative** | 58 | 58.00 | 59 | 59.00 | 68 | 68.00 | 64 | 64.00 |
| | **Total** | 100 | 100.00 | 100 | 100.00 | 100 | 100.00 | 100 | 100.00 |

**Table 31: Summary by Interfering Substance/Pathology Group for Inhibin**

| | | Best Cutoff from ROC plot | | Mean+2*std | |
|---|---|---|---|---|---|
| Group | Result | N | % | N | % |
| Tumors | Positive | 4 | 8.89 | 2 | 4.44 |
| | Negative | 41 | 91.11 | 43 | 95.56 |
| | Total | 45 | 100.00 | 45 | 100.00 |
| Metabolic Disorder | Positive | 8 | 40.00 | 8 | 40.00 |
| | Negative | 12 | 60.00 | 12 | 60.00 |
| | Total | 20 | 100.00 | 20 | 100.00 |
| Hormonal Fluctuation | Positive | 16 | 48.48 | 16 | 48.48 |
| | Negative | 17 | 51.52 | 17 | 51.52 |
| | Total | 33 | 100.00 | 33 | 100.00 |
| Gynecologic Disorder | Positive | 27 | 77.14 | 25 | 71.43 |
| | Negative | 8 | 22.86 | 10 | 28.57 |
| | Total | 35 | 100.00 | 35 | 100.00 |
| Vascular Disorder | Positive | 3 | 12.00 | 2 | 8.00 |
| | Negative | 22 | 88.00 | 23 | 92.00 |
| | Total | 25 | 100.00 | 25 | 100.00 |
| Chronic Immuno. Disorder | Positive | 8 | 19.05 | 4 | 9.52 |
| | Negative | 34 | 80.95 | 38 | 90.48 |
| | Total | 42 | 100.00 | 42 | 100.00 |

**Table 32: Summary by Cancer Stage Group for Inhibin**

| | | Best Cutoff from ROC plot | | Mean+2*std | |
|---|---|---|---|---|---|
| Stage | Result | N | % | N | % |
| **1** | **Positive** | 25 | 37.31 | 20 | 29.85 |
| | **Negative** | 42 | 62.69 | 47 | 70.15 |
| | **Total** | 67 | 100.00 | 67 | 100.00 |
| **2** | **Positive** | 35 | 53.03 | 32 | 48.48 |
| | **Negative** | 31 | 46.97 | 34 | 51.52 |
| | **Total** | 66 | 100.00 | 66 | 100.00 |
| **3** | **Positive** | 23 | 34.33 | 16 | 23.88 |
| | **Negative** | 44 | 65.67 | 51 | 76.12 |
| | **Total** | 67 | 100.00 | 67 | 100.00 |

### Biomarker: Glycodelin

**Table 33: Summary by Age Group for Glycodelin**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 53 | 58.89 | 18 | 16.67 | 36 | 40.00 | 10 | 9.26 |
| | **Negative** | 37 | 41.11 | 90 | 83.33 | 54 | 60.00 | 98 | 90.74 |
| | **Total** | 90 | 100.00 | 108 | 100.00 | 90 | 100.00 | 108 | 100.00 |
| **NORMAL** | **Positive** | 73 | 36.32 | 10 | 5.13 | 56 | 27.86 | 3 | 1.54 |
| | **Negative** | 128 | 63.68 | 185 | 94.87 | 145 | 72.14 | 192 | 98.46 |
| | **Total** | 201 | 100.00 | 195 | 100.00 | 201 | 100.00 | 195 | 100.00 |
| **OvCA** | **Positive** | 65 | 65.00 | 62 | 62.00 | 49 | 49.00 | 52 | 52.00 |
| | **Negative** | 35 | 35.00 | 38 | 38.00 | 51 | 51.00 | 48 | 48.00 |
| | **Total** | 100 | 100.00 | 100 | 100.00 | 100 | 100.00 | 100 | 100.00 |

**Table 34: Summary by Interfering Substance/Pathology Group for-Glycodelin**

| | | Best Cutoff from ROC plot | | Mean+2*std | |
|---|---|---|---|---|---|
| Group | Result | N | % | N | % |
| Tumors | Positive | 13 | 28.89 | 6 | 13.33 |
| | Negative | 32 | 71.11 | 39 | 86.67 |
| | Total | 45 | 100.00 | 45 | 100.00 |
| Metabolic Disorder | Positive | 9 | 45.00 | 5 | 25.00 |
| | Negative | 11 | 55.00 | 15 | 75.00 |
| | Total | 20 | 100.00 | 20 | 100.00 |
| Hormonal Fluctuation | Positive | 18 | 54.55 | 15 | 45.45 |
| | Negative | 15 | 45.45 | 18 | 54.55 |
| | Total | 33 | 100.00 | 33 | 100.00 |
| Gynecologic Disorder | Positive | 21 | 60.00 | 14 | 40.00 |
| | Negative | 14 | 40.00 | 21 | 60.00 |
| | Total | 35 | 100.00 | 35 | 100.00 |
| Vascular Disorder | Positive | 1 | 4.00 | 1 | 4.00 |
| | Negative | 24 | 96.00 | 24 | 96.00 |
| | Total | 25 | 100.00 | 25 | 100.00 |
| Chronic Immuno. Disorder | Positive | 9 | 21.43 | 5 | 11.90 |
| | Negative | 33 | 78.57 | 37 | 88.10 |
| | Total | 42 | 100.00 | 42 | 100.00 |

**Table 35: Summary by Cancer Stage Group for Glycodelin**

| | | Best Cutoff from ROC plot | | Mean+2*std | |
|---|---|---|---|---|---|
| Stage | Result | N | % | N | % |
| **1** | **Positive** | 43 | 64.18 | 34 | 50.75 |
| | **Negative** | 24 | 35.82 | 33 | 49.25 |
| | **Total** | 67 | 100.00 | 67 | 100.00 |
| **2** | **Positive** | 41 | 62.12 | 37 | 56.06 |
| | **Negative** | 25 | 37.88 | 29 | 43.94 |
| | **Total** | 66 | 100.00 | 66 | 100.00 |
| **3** | **Positive** | 43 | 64.18 | 30 | 44.78 |
| | **Negative** | 24 | 35.82 | 37 | 55.22 |
| | **Total** | 67 | 100.00 | 67 | 100.00 |

### Biomarker: MMP-7

**Table 36: Summary by Age Group for MMP-7**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 19 | 21.11 | 78 | 72.22 | 9 | 10.00 | 44 | 40.74 |
| | **Negative** | 71 | 78.89 | 30 | 27.78 | 81 | 90.00 | 64 | 59.26 |
| | **Total** | 90 | 100.00 | 108 | 100.00 | 90 | 100.00 | 108 | 100.00 |
| **NORMAL** | **Positive** | 12 | 5.97 | 30 | 15.38 | 2 | 1.00 | 7 | 3.59 |
| | **Negative** | 189 | 94.03 | 165 | 84.62 | 199 | 99.00 | 188 | 96.41 |
| | **Total** | 201 | 100.00 | 195 | 100.00 | 201 | 100.00 | 195 | 100.00 |
| **OvCA** | **Positive** | 50 | 50.00 | 53 | 53.00 | 28 | 28.00 | 33 | 33.00 |
| | **Negative** | 50 | 50.00 | 47 | 47.00 | 72 | 72.00 | 67 | 67.00 |
| | **Total** | 100 | 100.00 | 100 | 100.00 | 100 | 100.00 | 100 | 100.00 |

**Table 37: Summary by Interfering Substance/Pathology Group for MMP-7**

| | | Best Cutoff from ROC plot | | Mean+2*std | |
|---|---|---|---|---|---|
| Group | Result | N | % | N | % |
| Tumors | Positive | 27 | 60.00 | 15 | 33.33 |
| | Negative | 18 | 40.00 | 30 | 66.67 |
| | Total | 45 | 100.00 | 45 | 100.00 |
| Metabolic Disorder | Positive | 8 | 40.00 | 5 | 25.00 |
| | Negative | 12 | 60.00 | 15 | 75.00 |
| | Total | 20 | 100.00 | 20 | 100.00 |
| Hormonal Fluctuation | Positive | 3 | 9.09 | 0 | 0.00 |
| | Negative | 30 | 90.91 | 33 | 100.00 |
| | Total | 33 | 100.00 | 33 | 100.00 |
| Gynecologic Disorder | Positive | 8 | 22.86 | 3 | 8.57 |
| | Negative | 27 | 77.14 | 32 | 91.43 |
| | Total | 35 | 100.00 | 35 | 100.00 |
| Vascular Disorder | Positive | 22 | 88.00 | 10 | 40.00 |
| | Negative | 3 | 12.00 | 15 | 60.00 |
| | Total | 25 | 100.00 | 25 | 100.00 |
| Chronic Immuno. Disorder | Positive | 29 | 69.05 | 20 | 47.62 |
| | Negative | 13 | 30.95 | 22 | 52.38 |
| | Total | 42 | 100.00 | 42 | 100.00 |

**Table 38: Summary by Cancer Stage Group for MMP-7**

| | | Best Cutoff from ROC plot | | Mean+2*std | |
|---|---|---|---|---|---|
| Stage | Result | N | % | N | % |
| **1** | **Positive** | 33 | 49.25 | 20 | 29.85 |
| | **Negative** | 34 | 50.75 | 47 | 70.15 |
| | **Total** | 67 | 100.00 | 67 | 100.00 |
| **2** | **Positive** | 28 | 42.42 | 11 | 16.67 |
| | **Negative** | 38 | 57.58 | 55 | 83.33 |
| | **Total** | 66 | 100.00 | 66 | 100.00 |
| **3** | **Positive** | 42 | 62.69 | 30 | 44.78 |
| | **Negative** | 25 | 37.31 | 37 | 55.22 |
| | **Total** | 67 | 100.00 | 67 | 100.00 |

### Biomarker: PLAU-R

**Table 39: Summary by Age Group for PLAU-R**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 50 | 55.56 | 71 | 65.74 | 24 | 26.67 | 56 | 51.85 |
| | **Negative** | 40 | 44.44 | 37 | 34.26 | 66 | 73.33 | 52 | 48.15 |
| | **Total** | 90 | 100.00 | 108 | 100.00 | 90 | 100.00 | 108 | 100.00 |
| **NORMAL** | **Positive** | 35 | 17.41 | 61 | 31.28 | 16 | 7.96 | 23 | 11.79 |
| | **Negative** | 166 | 82.59 | 134 | 68.72 | 185 | 92.04 | 172 | 88.21 |
| | **Total** | 201 | 100.00 | 195 | 100.00 | 201 | 100.00 | 195 | 100.00 |
| **OvCA** | **Positive** | 71 | 71.00 | 72 | 72.00 | 44 | 44.00 | 50 | 50.00 |
| | **Negative** | 29 | 29.00 | 28 | 28.00 | 56 | 56.00 | 50 | 50.00 |
| | **Total** | 100 | 100.00 | 100 | 100.00 | 100 | 100.00 | 100 | 100.00 |

**Table 40: Summary by Interfering Substance/Pathology Group for PLAU-R**

| | | Best Cutoff from ROC plot | | Mean+2*std | |
|---|---|---|---|---|---|
| Group | Result | N | % | N | % |
| Tumors | Positive | 30 | 66.67 | 20 | 44.44 |
| | Negative | 15 | 33.33 | 25 | 55.56 |
| | Total | 45 | 100.00 | 45 | 100.00 |
| Metabolic Disorder | Positive | 13 | 65.00 | 11 | 55.00 |
| | Negative | 7 | 35.00 | 9 | 45.00 |
| | Total | 20 | 100.00 | 20 | 100.00 |
| Hormonal Fluctuation | Positive | 13 | 39.39 | 1 | 3.03 |
| | Negative | 20 | 60.61 | 32 | 96.97 |
| | Total | 33 | 100.00 | 33 | 100.00 |
| Gynecologic Disorder | Positive | 15 | 42.86 | 9 | 25.71 |
| | Negative | 20 | 57.14 | 26 | 74.29 |
| | Total | 35 | 100.00 | 35 | 100.00 |
| Vascular Disorder | Positive | 18 | 72.00 | 11 | 44.00 |
| | Negative | 7 | 28.00 | 14 | 56.00 |
| | Total | 25 | 100.00 | 25 | 100.00 |
| Chronic Immuno. Disorder | Positive | 32 | 76.19 | 28 | 66.67 |
| | Negative | 10 | 23.81 | 14 | 33.33 |
| | Total | 42 | 100.00 | 42 | 100.00 |

**Table 41: Summary by Cancer Stage Group for PLAU-R**

| | | Best Cutoff from ROC plot | | Mean+2*std | |
|---|---|---|---|---|---|
| Stage | Result | N | % | N | % |
| **1** | **Positive** | 53 | 79.10 | 30 | 44.78 |
| | **Negative** | 14 | 20.90 | 37 | 55.22 |
| | **Total** | 67 | 100.00 | 67 | 100.00 |
| **2** | **Positive** | 40 | 60.61 | 29 | 43.94 |
| | **Negative** | 26 | 39.39 | 37 | 56.06 |
| | **Total** | 66 | 100.00 | 66 | 100.00 |
| **3** | **Positive** | 50 | 74.63 | 35 | 52.24 |
| | **Negative** | 17 | 25.37 | 32 | 47.76 |
| | **Total** | 67 | 100.00 | 67 | 100.00 |

### Biomarker: Prolactin

**Table 42: Summary by Age Group for Prolactin**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 50 | 55.56 | 49 | 45.37 | 9 | 10.00 | 6 | 5.56 |
| | **Negative** | 40 | 44.44 | 59 | 54.63 | 81 | 90.00 | 102 | 94.44 |
| | **Total** | 90 | 100.00 | 108 | 100.00 | 90 | 100.00 | 108 | 100.00 |
| **NORMAL** | **Positive** | 92 | 45.77 | 54 | 27.69 | 6 | 2.99 | 3 | 1.54 |
| | **Negative** | 109 | 54.23 | 141 | 72.31 | 195 | 97.01 | 192 | 98.46 |
| | **Total** | 201 | 100.00 | 195 | 100.00 | 201 | 100.00 | 195 | 100.00 |
| **OvCA** | **Positive** | 64 | 64.00 | 48 | 48.00 | 5 | 5.00 | 2 | 2.00 |
| | **Negative** | 36 | 36.00 | 52 | 52.00 | 95 | 95.00 | 98 | 98.00 |
| | **Total** | 100 | 100.00 | 100 | 100.00 | 100 | 100.00 | 100 | 100.00 |

**Table 43: Summary by Interfering Substance/Pathology Group for Prolactin**

| | | Best Cutoff from ROC plot | | Mean+2*std | |
|---|---|---|---|---|---|
| Group | Result | N | % | N | % |
| Tumors | Positive | 17 | 37.78 | 3 | 6.67 |
| | Negative | 28 | 62.22 | 42 | 93.33 |
| | Total | 45 | 100.00 | 45 | 100.00 |
| Metabolic Disorder | Positive | 4 | 20.00 | 2 | 10.00 |
| | Negative | 16 | 80.00 | 18 | 90.00 |
| | Total | 20 | 100.00 | 20 | 100.00 |
| Hormonal Fluctuation | Positive | 23 | 69.70 | 4 | 12.12 |
| | Negative | 10 | 30.30 | 29 | 87.88 |
| | Total | 33 | 100.00 | 33 | 100.00 |
| Gynecologic Disorder | Positive | 18 | 51.43 | 3 | 8.57 |
| | Negative | 17 | 48.57 | 32 | 91.43 |
| | Total | 35 | 100.00 | 35 | 100.00 |
| Vascular Disorder | Positive | 13 | 52.00 | 2 | 8.00 |
| | Negative | 12 | 48.00 | 23 | 92.00 |
| | Total | 25 | 100.00 | 25 | 100.00 |
| Chronic Immuno. Disorder | Positive | 26 | 61.90 | 2 | 4.76 |
| | Negative | 16 | 38.10 | 40 | 95.24 |
| | Total | 42 | 100.00. | 42 | 100.00 |

**Table 44: Summary by Cancer Stage Group for Prolactin**

| | | **Best** Cutoff from ROC plot | | Mean+2*std | |
|---|---|---|---|---|---|
| Stage | Result | N | % | N | % |
| **1** | **Positive** | 42 | 62.69 | 2 | 2.99 |
| | **Negative** | 25 | 37.31 | 65 | 97.01 |
| | **Total** | 67 | 100.00 | 67 | 100.0 0 |
| **2** | **Positive** | 30 | 45.45 | 2 | 3.03 |
| | **Negative** | 36 | 54.55 | 64 | 96.97 |
| | **Total** | 66 | 100.00 | 66 | 100.0 0 |
| **3** | **Positive** | 40 | 59.70 | 3 | 4.48 |
| | **Negative** | 27 | 40.30 | 64 | 95.52 |
| | **Total** | 67 | 100.00 | 67 | 100.0 0 |

### Biomarker: CA125 (Lab Corp Data)

**Table 45: Summary by Age Group for CA125 (LabCorp Data)**

| | | Cutoff at 35 | | | |
|---|---|---|---|---|---|
| | | Age <=55 | | Age>55 | |
| **Group** | **Result** | N | % | N | % |
| **IP** | **Positive** | 13 | 14.94 | 6 | 5.66 |
| | **Negative** | 74 | 85.06 | 100 | 94.34 |
| | **Total** | 87 | 100.00 | 106 | 100.00 |
| **NORMAL** | **Positive** | 5 | 2.51 | 1 | 0.52 |
| | **Negative** | 194 | 97.49 | 193 | 99.48 |
| | **Total** | 199 | 100.00 | 194 | 100.00 |
| **OvCA** | **Positive** | 39 | 39.39 | 60 | 60.61 |
| | **Negative** | 60 | 60.61 | 39 | 39.39 |
| | **Total** | 99 | 100.00 | 99 | 100.00 |

**Table 46: Summary by Interfering Substance/Pathology Group for CA125 (LabCorp data)**

| | | Cutoff at 35 | |
|---|---|---|---|
| Group | Result | N | % |
| Tumors | Positive | 5 | 11.11 |
| | Negative | 40 | 88.89 |
| | Total | 45 | 100.00 |
| Metabolic Disorder | Positive | 1 | 5.56 |
| | Negative | 17 | 94.44 |
| | Total | 18 | 100.00 |
| Hormonal Fluctuation | Positive | 7 | 21.21 |
| | Negative | 26 | 78.79 |
| | Total | 33 | 100.00 |
| Gynecologic Disorder | Positive | 4 | 12.50 |
| | Negative | 28 | 87.50 |
| | Total | 32 | 100.00 |
| Vascular Disorder | Positive | 0 | 0.00 |
| | Negative | 25 | 100.00 |
| | Total | 25 | 100.00 |
| Chronic Immuno. Disorder | Positive | 2 | 4.76 |
| | Negative | 40 | 95.24 |
| | Total | 42 | 100.00 |

**Table 47: Summary by Cancer Stage Group for CA 125 (LabCorp data)**

| | | **Cutoff** at 35 | |
|---|---|---|---|
| Stage | Result | N | % |
| **1** | **Positive** | 24 | 35.82 |
| | **Negative** | 43 | 64.18 |
| | **Total** | 67 | 100.00 |
| **2** | **Positive** | 37 | 56.06 |
| | **Negative** | 29 | 43.94 |
| | **Total** | 66 | 100.00 |
| **3** | **Positive** | 38 | 58.46 |
| | **Negative** | 27 | 41.54 |
| | **Total** | 65 | 100.00 |

Summaries of the sensitivity of the thirteen biomarkers analyzed are presented in Tables 48-50, based on tumor stage, normal 2-standard deviation limits, or "best fit," respectively.

**Table 48: Individual marker sensitivity summary by tumor stage (Based on best cutoff)**

| Marker | Stage 1 % pos | Stage 2 % pos | Stage 3 % pos |
|---|---|---|---|
| HE4 | 85.1 | 80.3 | 92.5 |
| Glycodelin | 64.2 | 62.1 | 64.2 |
| MMP7 | 49.3 | 42.4 | 62.7 |
| SLPI | 50.8 | 57.6 | 71.6 |
| PAI-1 | 80.6 | 50.0 | 68.7 |
| MUG-1 | 65.7 | 40.9 | 55.2 |
| CA125 | 35.8 | 56.1 | 58.5 |
| Plau-R | 79.1 | 60.6 | 74.6 |
| Inhibin A | 37.3 | 53.0 | 34.3 |
| CTHRC1 | 23.9 | 28.8 | 35.8 |
| KLK6 | 14.9 | 10.6 | 9.0 |
| KLK10 | 55.2 | 56.1 | 62.7 |
| Prolactin | 62.7 | 45.5 | 59.7 |

**Table 49: Individual Marker Sensitivity Summary (based on Normal 2SD limit)**

| Marker | Sensitivity | Specificity |
|---|---|---|
| HE4 | 66.5 | 96 |
| Glycodelin | 50.5 | 85.3 |
| MMP7 | 30.5 | 97.7 |
| SLPI | 15.0 | 98.3 |
| Plau-R | 47.0 | 90.1 |
| MUC1 | 8.0 | 98.5 |
| Inhibin A | 34.0 | 75.8 |
| PAI-1 | 15.0 | 96.2 |
| CA125 (>=35u/ml) | 50.0 | 98.5 |
| CTHRC1 | 12.5 | 98.0 |
| KLK6 | 5.0 | 98.2 |
| KLK10 | 8.0 | 98.5 |
| Prolactin | 3.5 | 97.7 |

**Table 50: Individual Marker Sensitivity Summary (based on best cutoff)**

| Marker | Sensitivity | Specificity |
|---|---|---|
| HE4 | 86.0 | 86.3 |
| Glycodelin | 63.5 | 79.0 |
| MMP7 | 51.5 | 89.4 |
| SLPI | 60.0 | 71.7 |
| Plau-R | 71.5 | 77.2 |
| MUC1 | 54.0 | 73.0 |
| Inhibin A | 40.7 | 70.3 |
| PAI-1 | 66.5 | 76.0 |
| CA125 (>=35u/ml) | 50.5 | 98.2 |
| CTHRC1 | 29.5 | 84.8 |
| KLK6 | 11.5 | 95.7 |
| KLK10 | 58.0 | 54.3 |
| Prolactin | 56.0 | 63.1 |

### Example 3 : Ovarian Biomarker Selection Study - Multiple Biomarker Analysis

The logistic regression method was used for selection of optimal biomarkers for this study. Using logistic regression, stepwise selection starts off by finding the biomarker that produces the largest R-square value with the dependent variable. Then, given that the 'best' biomarker is in the model, logistic regression finds that next best biomarker in terms of adding to the R-square. This process of finding a set of biomarkers that adds to the R-square is stopped when biomarkers can no longer add to the R-square, in accordance with certain statistical criteria. These sets of biomarkers are not unique because some of the biomarkers have the same predictive performance.

Based on one selected model, a ROC plot can be produced, which provides the relationship between the sensitivity and specificity rates. Therefore, an appropriate specificity and sensitivity can be chosen in order to obtain an expected PPV with accepted sensitivity.

Tables 51 and 52 provide several examples involving selection of a set of biomarkers with a higher specificity and an appropriate sensitivity with a PPV of approximately 10% and a combined sensitivity rate around 70%-80%, under the assumption that the population is 8,000,000 and the disease prevalence is 0.25%. Different target values for sensitivity, specificity, NPV and PPV can be obtained by selecting different assay cutoffs, different biomarker combinations, and different rules for combining the biomarkers. Biomarker performance was analyzed using the biomarker values as continuous variables (Table 51) and as categorical variables (Table 52).

By way of definition, when two assay steps are performed, the first assay step may be referred to as the "screen test," and the second step may be referred to as the "reflex test." "Best cutoff point" refers to the value obtained from the ROC plot for a particular biomarker that produces the best sensitivity and specificity. "Mean+2*std" refers to the average expression level plus twice the standard deviation, based on analysis of normal samples from patients not afflicted with ovarian cancer.

### Biomarker Performance as Continuous Variables

**Table 51: Estimated Sensitivity, Specificity, PPV, and NPV Adjusted by Disease Prevalence for Different Models (Biomarker values as continuous variables)**

| Example | Marker Selected and rule | Screen/ Reflex Test Sensitivity | Screen/ Reflex Test Specificity | Combined Performance* | | | |
|---|---|---|---|---|---|---|---|
| | | | | Sensitivity | Specificity | PPV | NPV |
| Age >55 | | | | | | | |
| 1 | Screen Test: If HE4 <=1.8 then test is negative | 90.2% | 62.2% | | | | |
| | Reflex Test: If HE4 > 1.8 then the following marker selected: CA125, GLY, PAI, Plau-R (actual value used**) | 87% | 96% | 78.474% | 98.4880% | 11.5105% | 99.9453% |
| 2† | Marker selected: HE4, CA125, GLY, MMP-7, PAI, Plau-R (actual value used) | 81.7°/ | 98% | 81.7% | 98% | 9.2873% | 99.9532% |
| *Assume the population=8,000,000; prevalence=0.25%, estimated cancer cases=20,000 in the population. | | | | | | | |
| **Actual value refers to the actual biomarker expression level obtained in the test | | | | | | | |
| †Although all listed biomarkers were assayed in a single step, additional algorithms were applied to obtain the values for sensitivity, speciificity, PPV, and NPV indicated in the table. | | | | | | | |

### Biomarker Performance as Categorical Variable

**Table 52: Estimated Sensitivity, Specificity, PPV, and NPV Adjusted by Disease Prevalence for Different Models (Biomarker Values as Categorical Variables)**

| Example | Marker Selected and rule | Screen/Reflex Test Sensitivity | Screen/ Reflex Test Specificity | Combined Performance* | | | |
|---|---|---|---|---|---|---|---|
| | | | | Sensitivity | Specificity | PPV | NPV |
| Overall Sample | | | | | | | |
| 1 | **Screen Test:** If HE4 <=1.8 then test is negative | 91.9% | 73% | | | | |
| | **Reflex Test:** If HE4 >1.8 then the following marker selected: CA125, MUC1, GLY, PAI-1, Plau-R (Best cutoff points used) | 80% | 92.5% | 73.52% | 98.704% | 12.4479% | 99.9328% |
| Age >55 | | | | | | | |
| 1 | **Screen Test:** If HE4 <=1.8 then test is negative | 90.2% | 62.2% | | | | |
| | **Reflex Test:** If HE4 >1.8 then the following marker selected (best cutoff points used) : CA125, MUC-1, MMP-7, | 78% | 95.9% | 70.356% | 98.4502% | 10.2154% | 99.9246% |

| Example | Marker Selected and rule | Screen/Reflex Test Sensitivity | Screen/Reflex Test Specificity | Combined Performance* | | | |
|---|---|---|---|---|---|---|---|
| | | | | Sensitivity | Specificity | PPV | NPV |
| | Plau-R, GLY | | | | | | |
| 2 | **Screen Test:** If HE4 <=1.8 then test is negative | 90.2% | 62.2% | | | | |
| | **Reflex Test:** If HE4 >1.8 then the following marker selected (Mean+2*std cutoff points used): CA125, MUC-1, MMP-7, Plau-R, Inhibin | 82% | 95.8% | 73.964% | 98.4124% | 10.4555% | 99.9337% |
| 3† | Marker selected: PLAU-R, GLY, HE4, CA125, MMP-7, PAI-1 (Best cutoff points used) | 80.8% | 98.5% | 80.8% | 98.5% | 11.8946% | 99.9512% |
| 4† | Marker selected: PLAU-R, GLY, HE4, CA125, MMP-7 (Mean+2*std cutoff points used) | 75% | 99% | 75% | 99% | 15.8228% | 99.9368% |
| *: CA125 cutoff>=35 in either best cutoff or mean+2std cases | | | | | | | |
| **Assume the population=8,000,000; prevalence=0.25%, the estimate cancer cases=20,000 in the population. | | | | | | | |
| † Although all listed biomarkers were assayed in a single step, additional algorithms were applied to obtain the values for sensitivity, specificity, PPV, and NPV indicated in the table. | | | | | | | |

### Sensitivity, Specificity, PPV, and NPV Results Obtained with Application of "Test Rules"

In the current study, the biomarkers HE4, CA125, PLAU-R, glycodelin, Muc-1, PAI-1, MMP-7, and inhibin were the most frequently selected markers based on marker performance either as continuous or categorical variables. In contrast to the statistical methods described above, Table 53 provides the estimated sensitivity, specificity, PPV, and NPV (adjusted by disease prevalence) based on two test rules: (1) if any two of the above biomarkers are positive (i.e., overexpressed), then the test is declared positive or (2) if any 3 of the above biomarkers are positive (i.e., overexpressed), the test is declared positive. Contrary to the results presented above in Tables 51 and 52, though, the application of either test rule did not produce a high specificity rate, which in turn lead to an unacceptably low PPV (<1.5%) when adjusted for the low prevalence rate of ovarian cancer. The application of "test rules," such as those described herein, is representative of the current state of the art in the identification of patients having an increased risk of ovarian cancer.

**Table 53: Estimated Sensitivity, Specificity, PPV, and NPV Adjusted by Disease Prevalence Based on Test Rules**

| Example | Marker Selected and rule | Performance* | | | |
|---|---|---|---|---|---|
| | | Sensitivity | Specificity | PPV | NPV |
| Overall Sample | | | | | |
| 1 | Any of two following markers are positive: HE4, CA125, Plau-R, GLY, MUC-1, PAI-1, MMP-7, Inhibin (best cutoff points used) | 97.475% | 51.654% | 0.5028% | 99.9879% |
| 2 | Any of 3 following markers are positive: HE4, CA125, Plau-R, GLY, MUC-1, PAI-1, MMP-7, Inhibin (best cutoff points used) | 91.414% | 78.372% | 1.0482% | 99.9726% |
| Age >55 Year Older | | | | | |
| 1 | Any of two following markers are positive: HE4, CA125, Plau-R, GLY, MUC-1, PAI-1, MMP-7, Inhibin (best cutoff points used) | 96.970% | 56.186% | 0.5516% | 99.9865% |
| 2 | Any of 3 following markers are positive: HE4, CA125, Plau-R, GLY, MUC-1, PAI-1, MMP-7, Inhibin (best cutoff points used) | 94.949% | 78.866% | 1.1135% | 99.9840% |
| * CA125 cutoff>=35 in either best cutoff or mean+2std cases | | | | | |
| **Assume the population=8,000,000; prevalence=0.25%, estimated cancer cases=20,000 in the population. | | | | | |

### Example 4: Ovarian Biomarker Selection Study - Multiple Biomarker Analysis Using One-Step Screening Method'

Expression of the biomarkers HE4, CA125, and glycodelin and combinations thereof for all patients and patient age over 55 was assessed using a one-step screening method, as described herein above. In particular, Table 54 provides the results for a one-step algorithm for patients over age 55. The test algorithm was based on a linear logistic regression model, and the actual value of each marker was used in the model. Linear logistic regression models (and other algorithms) are routine and well known in the art. See, for example, Fleiss (2003) Statistical Methods for Rates and Proportions (3^{rd} Ed.), which is herein incorporated by reference in its entirety. The algorithm offered 76.8% sensitivity at 96.4% specificity. A test outcome defined as positive was based on the following linear logistic regression model: ln(P/(1-P))= - 4.2391 + 0.0888*CA125 + 0.1790*HE4 + 0.2349*GLY, where P is the conditional probability of ovarian cancer given CA125, HE4, and glycodelin being overexpressed in the patient body sample. A similar result was obtained with CA125 determined using manufacture's recommended cutoff value of ≥ 35u/mL.

**Table 54: Sensitivity and specificity of single-step screening assay to distinguish ovarian cancer (all stages) from controls in patients over age 55**

| Marker | Variable | Algorithm | Sensitivity | Specificity |
|---|---|---|---|---|
| HE4, CA125, GLYCODELIN | Actual value | 1 Step: based on linear logistic model | 76.8% | 96.4% |
| HE4, CA125, GLYCODELIN | Actual value, except CA125 | 1 Step: based on linear logistic model | 71.4% | 95.4% |

### Example 5: Bootstrap Method of Analysis

### Validation of Study Methods

A bootstrap method of analysis was also conducted to assure a robust estimate of sensitivity and specificity for the above studies given that an independent and unique set of cancer sera was not available to validate the model. The bootstrap method consisted of artificially creating 1000 random datasets with replacement selection from the study sample set. A set of the most frequently selected markers in the study based on optimal performance was selected: HE4, CA125, PLAU-R, Glycodelin, MUC1 and PAI-1. A "test" was defined as follows: Test is positive if either (1) HE4 is positive and any one of CA125, Glycodelin, MMP-7, PLAU-R is positive or (2) HE4 is negative but CA125, Glycodelin, MMP-7, PLAU-R are all positive; otherwise, the "test" is negative. From the 1000 random samples, the mean/standard deviation and 95% confidence interval of the sensitivity/specificity of this defined test were obtained.

**Table 55: Biomarker Sequence Information**

| | **Amino Acid Sequence** | | **Nucleotide Sequence** | |
|---|---|---|---|---|
| **Biomarker Name** | **Accession No.** | **Sequence Identifier** | **Accession No.** | **Sequence Identifier** |
| HE4 (Isoform 1) | NP_006094 | SEQ ID NO:1 | NM_006103 | SEQ ID NO:2 |
| HE4 (Isoform 3) | NP_542771 | SEQ ID NO:3 | NM_080733 | SEQ ID NO:4 |
| HE4 (Isoform 4) | NP_542772 | SEQ ID NO:5 | NM_080734 | SEQ ID NO:6 |
| HE4 (Isoform 2) | NP_542774 | SEQ ID NO:7 | NM_080736 | SEQ ID NO:8 |
| HE4 (Isoform 5) | NP_542773 | SEQ ID NO:9 | NM_080735 | SEQ ID NO:10 |
| KLK6 (Variant A) | NP_002765 | SEQ ID NO:1 | NM_002774 | SEQ ID NO:12 |
| KLK6 (Variant B) | NP_001012982 | SEQ ID NO:13 | NM_001012964 | SEQ ID NO:14 |
| KLK6 (Variant C) | NP_001012983 | SEQ ID NO:15 | NM_001012965 | SEQ ID NO:16 |
| KLK (Variant D) | NP_001012984 | SEQ ID NO:17 | NM_001012966 | SEQ ID NO:18 |
| KLK10 (Variant 1) | NP_002767 | SEQ ID NO:19 | NM_002776 | SEQ ID NO:20 |
| KLK10 (Variant 2) | NP_665895 | SEQ ID NO:21 | NM_145888 | SEQ ID NO:22 |
| Glycodelin (Variant 1) | NP_001018059 | SEQ ID NO:23 | NM_001018409 | SEQ ID NO:24 |
| Glycodelin (Variant 2) | NP_002562 | SEQ ID NO:25 | NM_002571 | SEQ ID NO:26 |
| PAI-1 | NP_000593 | SEQ ID NO:27 | NM_000602 | SEQ ID NO:28 |
| Muc-1 (Variant 1) | NP_002447 | SEQ ID NO:29 | NM_002456 | SEQ ID NO:30 |
| Muc-1 (Variant 2) | NP_001018016 | SEQ ID NO:31 | NM_001018016 | SEQ ID NO:32 |
| Muc-1 (Variant 3) | NP_001018017 | SEQ ID NO:33 | NM_001018017 | SFQ ID NO:34 |
| Muc-1 (Variant 4) | NP_001018021 | SEQ ID NO:35 | NM_001018021 | SEQ ID NO:36 |
| Alpha-1 anti-trypsin | NP_000286 | SEQ ID NO:37 | NM_000295 | SEQ ID NO:38 |
| Alpha-1 anti-trypsin | NP_001002235 | SEQ ID NO:39 | NM_001002235 | SEQ ID NO:40 |
| Alpha-1 anti-trypsin | NP_001002236 | SEQ ID NO:41 | NM_001002236 | SEQ ID NO:42 |
| PLAUR (Variant 1) | NP_002650 | SEQ ID NO:43 | NM_002659 | SEQ ID NO:44 |
| PLAUR (Variant 2) | NP_001005376 | SEQ ID NO:45 | NM_001005376 | SEQ ID NO:46 |
| PLAUR (Variant 3) | NP_001005377 | SEQ ID NO:47 | NM_001005377 | SEQ ID NO:48 |
| CTHRC1 | NP_612464 | SEQ ID NO:49 | NM_138455 | SEQ ID NO:50 |
| Inhibin (INHA) | NP_002182 | SEQ ID NO:51 | NM_002191 | SEQ ID NO:52 |
| Inhibin (INHBB) | NP_002184 | SEQ ID NO:53 | NM_002193 | SEQ ID NO:54 |
| Inhibin (INHBA) | NP_002183 | SEQ ID NO:55 | NM_002192 | SEQ ID NO:56 |
| CA125 (Muc-16) | NP_078966 | SEQ ID NO:57 | NM_024690 | SEQ ID NO:58 |
| MMP-7 | NP_002414 | SEQ ID NO:59 | NM_002423 | SEQ ID NO:60 |
| Prolactin | NP_000939 | SEQ ID NO:61 | NM_000948 | SEQ ID NO:62 |
| SLPI | NP_003055 | SEQ ID NO:63 | NM_003064 | SEQ ID NO:64 |

## Claims

1. A screening method for identifying patients with ovarian cancer or an increased likelihood of having ovarian cancer, the method comprising:
(a) performing a first assay step comprising detecting the expression of HE4 in a body sample and determining if HE4 is overexpressed; and
(b) performing a second assay step if HE4 is determined to be overexpressed in step (a), the second assay step comprising detecting the expression of a panel of biomarkers in the body sample, wherein the panel comprises the biomarkers CA125, glycodelin, PAI-1, and PLAU-R, and determining if the panel of biomarkers is overexpressed;
wherein overexpression of both HE4 and the panel of biomarkers of (b) is indicative of the patient having ovarian cancer or an increased likelihood of the patient having ovarian cancer.

2. The method of claim 1, wherein patients identified as having an increased likelihood of having ovarian cancer are subjected to additional diagnostic testing to determine if the patient has ovarian cancer, wherein the additional diagnostic testing is selected from the group consisting of pelvic examination, transvaginal ultrasound, CT scan, MRI, laparotomy, laparoscopy, and tissue sample biopsy.

3. The method of claim 1, wherein patients identified as having an increased likelihood of having ovarian cancer that are determined not to currently have ovarian cancer are further monitored on a regular basis for the development of ovarian cancer.

4. The method of claim 1, wherein the method is used to detect early-stage ovarian cancer.

5. The method of claim 1, wherein a threshold level of expression for each biomarker is determined by analyzing a Receiver Operating Characteristic (ROC) plot for each biomarker, wherein an expression level above a threshold level indicates that a particular biomarker is overexpressed.

6. The method of claim 5, wherein the threshold level of expression for each biomarker is determined relative to the expression level in a normal patient population.

7. The method of claim 1, wherein the patients screened in accordance with the method of claim 1 are asymptomatic and have a family history of ovarian cancer or clinical risk factors indicating a high-risk for developing ovarian cancer.

8. The method of claim 1, wherein the screening method is performed in a population of post-menopausal female patients.

9. The method of claim 1, wherein the body sample is a blood or serum sample.

10. The method of claim 1, wherein expression of HE4 and the panel of biomarkers of claim 1(b) is detected at the protein level using antibodies.

11. The method of claim 10, wherein expression of HE4 and the panel of biomarker proteins is detected using an ELISA format or a multiplex bead-based immunoassay.

12. The method of claim 1, wherein expression of HE4 and the panel of biomarkers is detected at the nucleic acid level using RT-PCR.

13. The method of claim 1, wherein the screening method is performed in an automated, semi-automated, or manual fashion.

## Patentansprüche

1. Screening-Verfahren zum Identifizieren von Patientinnen mit Eierstockkrebs oder mit erhöhter Wahrscheinlichkeit, Eierstockkrebs zu haben, wobei das Verfahren umfasst:
(a) Durchführen eines ersten Assay-Schritts, umfassend Detektieren der Expression von HE4 in einer Körperprobe und Bestimmen, ob HE4 überexprimiert wird, und
(b) Durchführen eines zweiten Assay-Schritts, wenn in Schritt (a) bestimmt wird, dass HE4 überexprimiert wird, wobei der zweite Assay-Schritt Detektieren der Expression einer Gruppe von Biomarkern in der Körperprobe, wobei die Gruppe die Biomarker CA125, Glycodelin, PAI-1 und PLAU-R umfasst, und Bestimmen, ob die Gruppe von Biomarkern überexprimiert wird, umfasst,
wobei eine Überexpression von beiden, HE4 und der Gruppe von Biomarkern von (b), indikativ dafür ist, dass die Patientin Eierstockkrebs hat oder eine erhöhte Wahrscheinlichkeit, dass sie Eierstockkrebs hat, hat.

2. Verfahren gemäß Anspruch 1, wobei Patientinnen, die als eine erhöhte Wahrscheinlichkeit aufweisend, dass sie Eierstockkrebs haben, identifiziert werden, einer weiteren diagnostischen Untersuchung unterzogen werden, um zu bestimmen, ob die Patientin Eierstockkrebs hat, wobei die zusätzliche diagnostische Untersuchung ausgewählt wird aus der Gruppe, bestehend aus Beckenuntersuchung, transvaginalem Ultraschall, CT-Scan, MRI, Laparotomie, Laparoskopie und Gewebeprobenbiopsie.

3. Verfahren gemäß Anspruch 1, wobei Patientinnen, die als erhöhte Wahrscheinlichkeit, Eierstockkrebs zu haben, aufweisend identifiziert wurden, bei denen nicht bestimmt wurde, dass sie Eierstockkrebs haben, auf regelmäßiger Basis bezüglich der Entwicklung von Eierstockkrebs weiter überwacht werden.

4. Verfahren gemäß Anspruch 1, wobei das Verfahren eingesetzt wird, um Eierstockkrebs im frühen Stadium zu detektieren.

5. Verfahren gemäß Anspruch 1, wobei ein Expressionsschwellenlevel für jeden Biomarker bestimmt wird, indem ein Receiver-Operating-Characteristic (ROC)-Plot für jeden Biomarker analysiert wird, wobei ein Expressionslevel über einem Schwellenlevel anzeigt, dass ein bestimmter Biomarker überexprimiert wird.

6. Verfahren gemäß Anspruch 5, wobei der Expressionsschwellenlevel für jeden Biomarker relativ zu dem Expressionslevel bei einer Bevölkerungsgruppe normaler Patientinnen bestimmt wird.

7. Verfahren gemäß Anspruch 1, wobei die Patientinnen, die gemäß dem Verfahren gemäß Anspruch 1 durchgemustert werden, asymptomatisch sind und eine Familiengeschichte mit Eierstockkrebs oder klinische Risikofaktoren, die ein hohes Risiko zur Entwicklung von Eierstockkrebs anzeigen, haben.

8. Verfahren gemäß Anspruch 1, wobei das Screening-Verfahren bei einer Bevölkerungsgruppe von postmenopausalen Patientinnen durchgeführt wird.

9. Verfahren gemäß Anspruch 1, wobei die Körperprobe eine Blut- oder Serumprobe ist.

10. Verfahren gemäß Anspruch 1, wobei eine Expression von HE4 und der Gruppe von Biomarkern von Anspruch 1(b) auf dem Proteinlevel unter Verwendung von Antikörpern detektiert wird.

11. Verfahren gemäß Anspruch 10, wobei eine Expression von HE4 und der Gruppe von Biomarkerproteinen unter Verwendung eines ELISA-Formats oder eines Multiplex-Bead-basierten Immunoassays detektiert wird.

12. Verfahren gemäß Anspruch 1, wobei eine Expression von HE4 und der Gruppe von Biomarkern auf dem Nukleinsäurelevel unter Verwendung von RT-PCR detektiert wird.

13. Verfahren gemäß Anspruch 1, wobei das Screening-Verfahren in automatisierter, halbautomatisierter oder manueller Art durchgeführt wird.

## Revendications

1. Procédé d'identification, par criblage, de patientes atteintes d'un cancer des ovaires ou présentant une forte probabilité d'avoir un cancer des ovaires, lequel procédé comporte :
a) le fait de réaliser une première étape de test, comprenant le fait de détecter l'expression de la protéine HE4 dans un échantillon corporel et le fait de déterminer s'il y a surexpression de HE4 ;
b) et le fait de réaliser, si l'on constate dans l'étape (a) une surexpression de HE4, une deuxième étape de test comprenant le fait de détecter l'expression, dans l'échantillon corporel, d'un jeu de biomarqueurs comprenant les biomarqueurs CA125, glycodéline, PAI-1 et PLAU-R, et le fait de déterminer s'il y a surexpression des biomarqueurs de ce jeu ;
étant entendu qu'une surexpression conjointe de la protéine HE-4 et des biomarqueurs du jeu indiqué en (b) est un indice de ce que la patiente est atteinte d'un cancer des ovaires ou présente une forte probabilité d'avoir un cancer des ovaires.

2. Procédé conforme à la revendication 1, dans lequel on fait subir aux patientes identifiées comme présentant une forte probabilité d'avoir un cancer des ovaires, pour déterminer si la patiente est bien atteinte d'un cancer des ovaires, un test diagnostique supplémentaire choisi dans l'ensemble formé par un examen pelvien, un examen transvaginal aux ultrasons, une tomodensitométrie, un examen par IRM, une laparotomie, une laparoscopie et une biopsie tissulaire.

3. Procédé conforme à la revendication 1, dans lequel les patientes qui ont été identifiées comme présentant une forte probabilité d'avoir un cancer des ovaires, mais dont on a établi qu'elles ne sont pas présentement atteintes d'un cancer des ovaires, sont ensuite soumises à des examens réguliers de surveillance du développement d'un cancer des ovaires.

4. Procédé conforme à la revendication 1, lequel procédé est utilisé pour détecter un cancer des ovaires à un stade précoce.

5. Procédé conforme à la revendication 1, dans lequel on détermine la valeur seuil de l'expression de chaque biomarqueur en analysant la courbe ROC (Receiver Operating Characteristic) établie pour chaque biomarqueur, étant entendu qu'un niveau d'expression supérieur à une valeur seuil est l'indice de ce qu'un biomarqueur particulier est surexprimé.

6. Procédé conforme à la revendication 5, dans lequel on détermine la valeur seuil de l'expression de chaque biomarqueur par rapport au niveau d'expression observé chez une population de patientes normales.

7. Procédé conforme à la revendication 1, dans lequel les patientes examinées selon le procédé de la revendication 1 sont asymptomatiques, mais ont des antécédents familiaux de cancer des ovaires ou présentent des facteurs de risque cliniques indiquant un risque élevé de développement d'un cancer des ovaires.

8. Procédé conforme à la revendication 1, lequel procédé par criblage est mis en oeuvre au sein d'une population de patientes ménopausées.

9. Procédé conforme à la revendication 1, dans lequel l'échantillon corporel est un échantillon de sang ou de sérum.

10. Procédé conforme à la revendication 1, dans lequel l'expression de la protéine HE-4 et du jeu de biomarqueurs indiqué dans la revendication 1, item (b), est détectée à l'échelon des protéines, au moyen d'anticorps.

11. Procédé conforme à la revendication 10, dans lequel l'expression de la protéine HE-4 et des protéines du jeu de biomarqueurs est détectée au moyen d'un test en format ELISA ou d'un immunotest multiplex sur perles.

12. Procédé conforme à la revendication 1, dans lequel l'expression de HE-4 et du jeu de biomarqueurs est détectée à l'échelon des acides nucléiques, à l'aide de la technique RT-PCR.

13. Procédé conforme à la revendication 1, lequel procédé par criblage est mis en oeuvre en mode automatique, semi-automatique ou manuel.
